# EUROPEAN PATENT APPLICATION

(11) **EP 3 912 989 A1**
(43) Date of publication of application: **24.11.2021**
(21) Application number: 19877803.7
(22) Date of filing: 01.11.2019
(51) Int. Cl.: C07K 14/11, C07K 19/00, C12N 7/00, C12N 15/00, A61K 39/145, A61P 35/00, A61P 25/00, A61P 37/00

(54) **CELL-PENETRATING PEPTIDE BASED ON INFLUENZA VIRUS M2 PROTEIN**

(30) Priority: 02.11.2018 WO PCT/CN2018/113755
(71) Applicant: Institute of Basic Medical Sciences Chinese Academy of Medical Sciences, Beijing 100005 (CN)
(72) Inventor: JIANG, Chengyu, Beijing 100005 (CN); ZHAO, Yan, Beijing 100005 (CN); WU, Shuangxiu, Beijing 100005 (CN); SHANG, Daozhen, Beijing 100005 (CN)
(74) Representative: Danner, Stefan
(86) International application number: PCT/CN2019/115107
(87) International publication number: WO 2020/088667

(57) **Abstract**

Provided are a cell-penetrating peptide based on influenza virus M2 protein and a method for preparing and transforming same. Further provided are a conjugate containing the cell-penetrating peptide, a fusion protein and a composition, wherein the cell-penetrating peptide can be used for penetrating cells, introducing a molecule into cells and treating diseases.

## Description

The present application claims the priority of the patent application filed on November 2, 2018, with the invention title of "Cell-Penetrating Peptide Based on Influenza Virus M2 Protein" under PCT application number of PCT/CN2018/113755, the content as a whole is incorporated herein by reference.

### FIELD OF THE INVENTION

The present invention belongs to the field of protein engineering and relates to a cell-penetrating peptide. Specifically, the present invention provides cell-penetrating peptides based on influenza virus M2 protein, preparation and modification methods thereof, and related uses. The present invention also provides conjugates, fusion proteins and compositions comprising these cell-penetrating peptides, as well as methods for penetrating cells by using the cell-penetrating peptides, for introducing molecules into cells, and methods for treating diseases.

### BACKGROUND OF THE INVENTION

Cell-penetrating peptides (CPPs) are a class of peptides that can "penetrate" cells. Cell-penetrating peptides have different sources, sequence lengths, and amino acid compositions, but they all have the function of translocating the plasma membrane and transporting biologically active molecules such as proteins, peptides, DNA, RNA and small molecule drugs into cells. Since it was first reported in 1988 that the trans-activator of transcription (TAT) of HIV-1 virus can be efficiently internalized by cells, some other cell-penetrating peptides have been discovered in the field, including Penetratin, DPV1047, MPG, Pep-1, and the like.

Many therapeutic molecules, such as antibiotics, anti-inflammatory drugs, anti-tumor drugs and neuroprotective agents, cannot reach their specific targets due to the existence of cell and tissue barriers, especially when the targets exist inside the cells, therefore restricting their therapeutic effects. Since cell-penetrating peptides can penetrate cells, they can be used as a carrier to transport various types of therapeutic molecules into cells to reach their targets. At present, the value of some cell-penetrating peptides in disease treatment has been evaluated by a large number of preclinical studies, and promising results have been obtained in many disease models, including cancer and cardiovascular diseases.

Although some cell-penetrating peptides have been identified and evaluated, however, the existing cell-penetrating peptides have various issues, such as low efficiency in penetrating cells and transporting molecules into cells, and limited cell types that can be penetrated, and the like. Therefore, there is a need in the art for new cell-penetrating peptides which have high penetrating efficiency, so that bioactive molecules can be efficiently delivered into various types of cells at low concentrations.

Influenza virus M2 (Matrix-2) protein is a proton-selective ion channel protein that exists in the viral envelope of influenza A virus. This channel exists as a homotetramer (composed of 4 identical M2 units). In influenza A virus, the proton conduction of the M2 protein is essential for virus replication, for it can form a highly selective, pH-adjusted proton conduction channel.

In influenza A virus, the M2 protein unit consists of three domains consisting of 97 amino acid residues, which are: (i) extracellular N-terminal domain (amino acid residues 1-24); (ii) transmembrane segment (amino acid residues 25-43); and (iii) intracellular C-terminal domain (amino acid residues 44-97), respectively. Among them, the transmembrane segment forms the pore of the ion channel. The first 17 residues (amino acid residues 45-62) of the cytoplasmic tail of the M2 protein form a highly conserved amphipathic helix, which plays a role in virus budding and assembly. In addition, amino acid residues 70-77 of the cytoplasmic tail are important for the binding to M1 protein and production of infectious virus particles.

### SUMMARY OF THE INVENTION

### Cell-penetrating peptides

The inventors have unexpectedly discovered that polypeptides derived from influenza virus M2 protein and fragments thereof have the function of cell-penetrating peptides, and their penetrating efficiencies are much higher than those of existing cell-penetrating peptides in the art, and can enter many types of cells at relatively low concentrations. Further research has found that the cell-penetrating peptide function of M2 protein is mainly focused in the sequence of amino acids 44-67. The inventors have obtained cell-penetrating peptides with even higher penetration efficiency by modifying the polypeptide fragments of the influenza virus M2 protein, thereby completing the present invention.

Therefore, in one aspect, the present invention relates to a polypeptide, which has an amino acid sequence selected from the group consisting of:
a. amino acid sequences comprising amino acid sequence 44-67 of the influenza virus M2 protein or the fragment thereof;
b. amino acid sequences comprising amino acid sequence 44-67 of the influenza virus M2 protein or the fragment thereof with modification of one or more amino acid residue(s); and
c. amino acid sequences comprising an amino acid sequence that has at least 60%, at least 70%, at least 80%, at least 85%, at least 90%, at least 95%, or 100% sequence identity with amino acid sequence 44-67 of the influenza virus M2 protein or the fragment thereof,
wherein the length of the fragment is at least 8 amino acids, and the polypeptide has the function of cell-penetrating peptides.

The term "cell-penetrating peptide" ("CPP") is generally used to refer to a short peptide, which can transport different types of cargo molecules across the cell membrane, thereby promoting a variety of molecular cargoes (from nanoscale particles to small chemical molecules, macromolecules and large DNA fragments) to be taken up by cells. A "cargo" molecule binds to the cell-penetrating peptide through chemical linkage by covalent bonds or through non-covalent interactions. A cell-penetrating peptide usually has an amino acid composition that includes relatively high abundance of positively charged amino acids, such as lysine or arginine, or it has a sequence with an alternating pattern of polar/charged amino acids and non-polar, hydrophobic amino acids. These two structures are called polycationic or amphiphilic, respectively. Cell-penetrating peptides have different sizes, amino acid sequences, and charges, but all CPPs have features in common, i.e. the ability to translocate the cell membrane and to promote the delivery of various molecular cargoes to the cytoplasm or organelles of the cell. At present, the theory of CPP translocation refers to three different main entry mechanisms: direct penetration in the membrane, entry regulated by endocytosis, and translocation through the formation of temporary structures. CPP transduction is a filed of ongoing research. It has been found that cell-penetrating peptides have a large number of applications in medicine, as drug delivery agents in the treatment of different diseases (including cancer) and as viral inhibitors, as well as contrast agents for cell labeling and imaging.

A "cargo molecule" herein refers to a molecule that is linked to a cell-penetrating peptide through covalent or non-covalent binding, and the presence of the cell-penetrating peptide allows its cell internalization to be promoted or achieved. In the present invention, a "cargo molecule" includes peptide, protein, polysaccharide, lipid, compositions thereof including lipoprotein and glycoprotein, nucleic acid (such as DNA, siRNA, shRNA, antisense oligonucleotide, decoy DNA, plasmid), small molecule drug (such as cyclosporine A, paclitaxel, doxorubicin, methotrexate, 5-aminolevulinic acid), imaging agent (such as fluorophore, quantum dot (QD), tracer, metal chelating agent such as small molecular weight chelating agent of gadolinium (Gd³⁺) and superparamagnetic ionic oxide (SPIO)). It should be understood that when the cargo molecule is a peptide, polypeptide or protein, it can comprise one or more peptides, polypeptides or proteins that are linked together. In addition, when the cargo molecule is a nucleic acid, the nucleic acid can comprise one or more nucleic acids, each of which encodes one or more peptides or polypeptides. The cargo molecule can also be a combination of protein, lipid and/or polysaccharide, including lipoprotein and glycoprotein. The nucleic acid can be a natural or artificial, single-stranded or double-stranded DNA molecule or RNA molecule. The nucleic acid molecule can be one or more nucleic acids of the same type (e.g., having the same nucleotide sequence) or different types of nucleic acids. The nucleic acid molecule includes, but is not limited to, one or more of the following: DNA, complementary DNA (cDNA), decoy DNA, RNA, small interfering RNA (siRNA), microRNA (miRNA), small hairpin RNA (shRNA), small temporal RNA (stRNA), small nucleolar RNA (snoRNA), small nuclear RNA (snRNA), pentose nucleic acid (PNA), antisense oligomer, plasmid, and other modified nucleic acids.

In the present invention, the terms "protein" and "polypeptide" are used interchangeably herein and generally refer to a polymer of amino acid residues connected by peptide bonds, without limitation on the minimum length of the product. Therefore, the above terms include peptide, oligopeptide, polypeptide, dimer (heterologous and homologous), multimer (heterologous and homologous), and the like. "Protein" and "polypeptide" encompass the full-length protein and fragments thereof. The terms also include post-expression modifications of the polypeptide, such as glycosylation, acetylation, phosphorylation, and the like.

In some embodiments, the polypeptide includes the amino acids 44-67 sequence of the influenza virus M2 protein. The polypeptide can include other amino acid sequences at the N-terminus and/or C-terminus of amino acids 44-67 of the M2 protein. For example, the polypeptide can include the natural sequences at both ends of amino acids 44-67 of M2 protein. For example, the polypeptide can include a N-terminal extension sequence of amino acids 44-67 of the M2 protein, such as the amino acids 43-67, 42-67, 41-67, 40-67, 39-67, 38-67, 37-67, 36-67, 35-67, 34-67 sequences of the M2 protein, and the like. Alternatively, the polypeptide can include a C-terminal extension sequence of amino acids 44-67 of the M2 protein. For example, the polypeptide can include the amino acids 43-68, 42-69, 41-70, 40-71, 39-72, 38-73, 37-74, 36-75, 35-76, 34-77 sequences of the M2 protein, and the like. The polypeptide can include both N-terminal and C-terminal extension natural sequences of amino acids 44-67 of the M2 protein.

In addition, the polypeptide can also include non-natural sequences, such as heterologous sequences, at the N-terminus and/or C-terminus of amino acids 44-67 of the M2 protein. The heterologous sequence can be selected from various tags, fluorescent protein, functional moiety, and amino acid sequences derived from other proteins or polypeptides, as long as the heterologous sequence does not affect the cell-penetrating peptide function of the polypeptide.

In other embodiments, the polypeptide comprises a fragment of the above sequences, and the fragment has the function of cell-penetrating peptides. The present invention has identified multiple functional fragments of the amino acids 44-67 sequence of the influenza virus M2 protein. In some embodiments, the length of the fragment can be at least 8 amino acids, at least 9 amino acids, at least 10 amino acids, at least 11 amino acids, at least 12 amino acids, at least 13 amino acids, at least 14 amino acids, at least 15 amino acids, at least 16 amino acids, at least 17 amino acids, at least 18 amino acids, at least 19 amino acids, at least 20 amino acids, at least 21 amino acids, at least 22 amino acids or at least 23 amino acids. There is no specific limitation on the selection of the amino acid fragment, as long as the fragment has the function of cell-penetrating peptides.

In some embodiments, the fragment includes the amino acids 44-48 or 45-48 of the M2 protein. A polypeptide fragment with amino acids 44-48 or amino acids 45-48 of the M2 protein has a strong function of cell-penetrating peptides. However, the polypeptide fragment may not include amino acids 44-48 or amino acids 45-48 of the M2 protein.

In some embodiments, the fragment includes or consists of amino acids 44-65, 44-61, 45-62 or 44-56 of the M2 protein. The fragment can also include a sequence with one or more amino acid residue(s) extended and/or truncated at the N-terminus and/or C-terminus of the above sequences.

In some embodiments, the polypeptide comprises an amino acid sequence of amino acids 44-67 sequence of the influenza virus M2 protein or the fragment thereof, with modifications of one or more amino acid residue(s). The modification of the amino acid residues includes substitution, insertion, deletion and/or addition of amino acid residues. In some embodiments, the amino acid modification is amino acid substitution.

In some embodiments, the number of amino acid residues can be determined based on the length of the polypeptide and/or its amino acid composition. For example, when the length of the polypeptide is about 25 amino acids, the number of amino acid substitutions, insertions and deletions can be 1-12, such as 1, 2, 3, 4, 5, 6, 7, 8, 9, 10, 11 or 12. When the length of the polypeptide is about 20 amino acids, the number of amino acid substitutions, insertions and deletions can be 1-10, such as 1, 2, 3, 4, 5, 6, 7, 8, 9 or 10. When the length of the polypeptide is about 15 amino acids, the number of amino acid substitutions, insertions and deletions can be 1-7, such as 1, 2, 3, 4, 5, 6 or 7.

There is not particular limitation on the manner of the amino acid substitution, insertion and deletion. In some embodiments, the amino acid substitution is conservative amino acid substitution. A "conservative amino acid substitution" refers to substitution between amino acid residues with similar charge properties or side chain groups, which usually does not affect the normal function of the protein or polypeptide. In some embodiments, the insertion is insertion of a hydrophobic amino acid or a positively charged amino acids. In some embodiments, the deletion is deletion of a hydrophilic amino acid.

In other embodiments, at least one or more of the amino acid substitutions are substitutions with hydrophobic amino acids or positively charged amino acids. In other embodiments, the hydrophobic amino acid is preferably C, A, F, I, L, M, P, V, W, M or Y, more preferably I. In other embodiments, the positively charged amino acid is preferably K, R or H, more preferably K or R. In some embodiments, the hydrophobic amino acid or positively charged amino acid is selected from the group consisting of I, F and R residues. In some embodiments, the hydrophilic amino acid is preferably D, E, Q, S or T.

The polypeptide can also include an amino acid sequence that has at least 60% sequence identity with the amino acids 44-67 sequence of the M2 protein or fragment thereof, as long as the polypeptide has the function of cell-penetrating peptides. For example, the polypeptide can comprise an amino acid sequence that has at least 60%, at least 65%, at least 70%, at least 80%, at least 85%, at least 90%, or at least 95% sequence identity with amino acids 44-67 sequence of the influenza virus M2 protein or the fragment thereof, as long as the polypeptide has the function of cell-penetrating peptides.

The percentage of sequence identity between two sequences can be determined by, for example, using mathematical algorithms. By using programs based on such mathematical algorithms, sequence comparisons (i.e. alignments) for determination of sequence identity can be performed. The programs can be appropriately executed by computers. Examples of such programs include, but are not limited to, CLUSTAL of the PC/Gene program, the ALIGN program (Version 2.0), and GAP, BESTFIT, BLAST, FASTA and TFASTA of the Wisconsin genetics software package. The alignment using these programs can be implemented, for example, by using default parameters.

In any embodiments of the above polypeptides, the influenza virus can be any type of influenza virus. In some embodiments, the influenza virus can be, but is not limited to, H1N1, H5N1, H5N6 or H7N9 influenza virus. It is known in the art that the amino acid sequence of the M2 protein is conserved among different types of influenza viruses.

For example, in some embodiments, the M2 protein of the present invention can be from, but is not limited to, the following influenza virus strains: A/Caledonia/20/1999 (H1N1), A/HongKong/97/98 (H5N1) and A/chicken/Jilin/9/2004 (H5N1).

In another aspect, the present invention relates to a method for engineering cell-penetrating peptides based on the influenza virus M2 protein, and relates to the obtained variant sequences. Specifically, the present invention performed modification based on polypeptides Jilin-2004(H5N1)-M2-44-56, Jilin-2004(H5N1)-M2-45-62 and Jilin-2004(H5N1)-M2-44-65, which have strong cell-penetrating peptide functions, and has obtained variant polypeptide sequences with similar or even stronger cell-penetrating peptide functions.

In one aspect, the present invention relates to a polypeptide, which has the amino acid sequence of the following formula: X1-X2-X3-X4-X5-X6-X7-X8-X9-X10-X11-X12-X13-X14-X15-X16-X17-X18-X19-X20-X2 1-X22-X23-X24; wherein X1 is D, R, L, I or absent; X2 is R, I or S; X3 is L, I, F, A, R or K; X4 is F, R or I; X5 is F, R or I; X6 is K, R or I; X7 is C, I or R; X8 is A, I, F, K or R; X9 is Y, I, F , K or R; X10 is R or S; X11 is R, S or absent; X12 is L, I, F, R or K or absent; X13 is K, R, T or absent; X14 is Y, F, R or K or absent; X15 is G or R or K or absent; X16 is R, L, F or A or absent; X17 is I, K, R or T or absent; X18 is R, I or T or absent; X19 is G, R or K or absent; X20 is P or absent; X21 is S, K or absent; X22 is T, V or absent; X23 is E or absent; X24 is G or absent; or
the polypeptide has an amino acid sequence that has at least 60%, at least 70%, at least 80%, at least 85%, at least 90%, at least 95%, or 100% sequence identity with the amino acid sequence; or
the polypeptide has an amino acid sequence with deletions, insertions or substitutions of one or more amino acid residue(s) in the amino acid sequence.

Correspondingly, in one aspect, the present invention relates to a polypeptide that has an amino acid sequence selected from the group consisting of:
a. amino acid sequences comprising DRLFFKCIYRRLK;
b. amino acid sequences comprising amino acid sequence DRLFFKCIYRRLK with substitution, deletion or insertion of one or more amino acid residue(s), and
c. amino acid sequences comprising an amino acid sequence that has at least 60%, at least 70%, at least 80%, at least 85%, at least 90%, at least 95% or 100% sequence identity with DRLFFKCIYRRLK,
wherein the polypeptide has the function of cell-penetrating peptides.

DRLFFKCIYRRLK corresponds to the amino acids 44-56 sequence of Jilin-2004(H5N1) influenza virus M2 protein (Jilin-2004(H5N1)-M2-44-56), which has a strong cell-penetrating peptide function. Correspondingly, the cell-penetrating peptide of the present invention can comprise this sequence or functional variant sequences thereof.

In some embodiments, the substitution, deletion or insertion of one or more amino acid residue(s) are substitution, deletion or insertion of 1-5 amino acid residues, such as substitution, deletion or insertion of 1, 2, 3, 4, or 5 amino acids. In some embodiments, at least one, at least two, at least three, at least four, or all five substitutions can be substitutions with hydrophobic amino acids or positively charged amino acids, for example, selected from I, F and R residues. In some embodiments, the insertion is insertion of hydrophobic amino acids or positively charged amino acids. In some embodiments, the deletion is deletion of hydrophilic amino acids.

In some embodiments, the polypeptide has an amino acid sequence of formula X1-X2-X3-X4-X5-X6-X7-X8-X9-X10-X11-X12-X13; wherein X1 is D or R; X2 is R; X3 is L, I or F; X4 is F; X5 is F; X6 is K or R; X7 is C or R; X8 is I or F; X9 is Y, I or F; X10 is R; X11 is R; X12 is L, I or F; and X13 is K or R; or the polypeptide has an amino acid sequence that has at least 60%, at least 70%, at least 80%, at least 85%, at least 90%, at least 95% or 100% sequence identity with the amino acid sequences; or an amino acid sequence with deletions, insertions or substitutions of one or more amino acid residue(s) in the amino acid sequence.

In some embodiments, the polypeptide has an amino acid sequence selected from the group consisting of RRLFFKCIYRRLK, RRLFFRCIYRRLK, RRLFFRRIYRRLK, RRLFFRRIYRRLR, DRIFFKCIYRRLK, DRIFFKCIIRRLK, DRIFFKCIIRRIK, DRFFFKCIYRRLK, DRFFFKCFYRRLK, DRFFFKCFFRRLK, DRFFFKCFFRRFK and RRFFFRRFFRRFR.

In another aspect, the present invention relates to a polypeptide, which has an amino acid sequence selected from the group consisting of:
a. amino acid sequences comprising RLFFKCIYRRLKYGLKRG;
b. amino acid sequences comprising amino acid sequence RLFFKCIYRRLKYGLKRG with substitution, deletion or insertion of one or more amino acid residue(s), and
c. amino acid sequences comprising an amino acid sequence that has at least 60%, at least 70%, at least 80%, at least 85%, at least 90%, at least 95% or 100% sequence identity with RLFFKCIYRRLKYGLKRG, wherein the polypeptide has the function of cell-penetrating peptides.

RLFFKCIYRRLKYGLKRG corresponds to the amino acids 45-62 sequence of Jilin-2004(H5N1) influenza virus M2 protein (Jilin-2004(H5N1)-M2-45-62), which has a strong cell-penetrating peptide function. Correspondingly, the cell-penetrating peptide of the present invention can comprise this sequence or functional variant sequences thereof.

In some embodiments, the substitution, deletion or insertion of one or more amino acid residue(s) is/are substitution, deletion or insertion of 1-6 amino acid residues, such as substitution, deletion or insertion of 1, 2, 3, 4, 5 or 6 amino acids. In some embodiments, at least one, at least two, at least three, at least four, at least five, or all six the substitutions can be substitutions with hydrophobic amino acids or positively charged amino acids, for example, selected from I, F and R residues. In some embodiments, the insertion is insertion of hydrophobic amino acids or positively charged amino acids. In some embodiments, the deletion is deletion of hydrophilic amino acids.

In some embodiments, the polypeptide has an amino acid sequence of formula X1-X2-X3-X4-X5-X6-X7-X8-X9-X10-X11-X12-X13-X14-X15-X16-X17-X18; wherein X1 is R or S; X2 is A, L or F; X3 is F or I; X4 is F or I; X5 is R or K; X6 is I, C or R; X7 is A, I or F; X8 is K, Y or F; X9 is R or S; X10 is R or S; X11 is A, L or F; X12 is K, R or T; X13 is Y, F or K; X14 is G, R or K; X15 is L, F or A; X16 is K, R or T; X17 is R or T; and X18 is G, R or K, or the polypeptide has an amino acid sequence that has at least 60%, at least 70%, at least 80%, at least 85%, at least 90%, at least 95% or 100% sequence identity with the amino acid sequences; or an amino acid sequence with deletions, insertions or substitutions of one or more amino acid residue(s) in the amino acid sequence.

In some other embodiments, the polypeptide has an amino acid sequence selected from the group consisting of RLFFRCIYRRLKYGLKRG, RLFFRRIYRRLKYGLKRG, RLFFRRIYRRLRYGLKRG, RLFFRRIYRRLRYRLKRG, RLFFRRIYRRLRYRLRRG, RLFFRRIYRRLRYRLRRR ,RFFFKCIYRRLKYGLKRG, RFFFKCFYRRLKYGLKRG, RFFFKCFFRRLKYGLKRG, RFFFKCFFRRFKYGLKRG, RFFFKCFFRRFKFGLKRG, RFFFKCFFRRFKFGFKRG and RFFFRRFFRRFRFRFRRR

In other embodiments, the present invention relates to single site mutation polypeptide sequences of Jilin-2004(H5N1)-M2-45-62 polypeptide. For example, in some embodiments, the polypeptide has an amino acid sequence selected from the group consisting of

SLFFKCIYRRLKYGLKRG, RAFFKCIYRRLKYGLKRG, RLIFKCIYRRLKYGLKRG,
RLFIKCIYRRLKYGLKRG, RLFFKIIYRRLKYGLKRG, RLFFKCAYRRLKYGLKRG,
RLFFKCIKRRLKYGLKRG, RLFFKCIYSRLKYGLKRG, RLFFKCIYRSLKYGLKRG,
SLFFKCIYRRLKYGLKRG, RLFFKCIYRRAKYGLKRG, RLFFKCIYRRLTYGLKRG,
RLFFKCIYRRLKKGLKRG, RLFFKCIYRRLKYKLKRG, RLFFKCIYRRLKYGAKRG,
RLFFKCIYRRLKYGLTRG, RLFFKCIYRRLKYGLKTG and RLFFKCIYRRLKYGLKRK.

In still another aspect, the present invention relates to a polypeptide, which has an amino acid sequence selected from the group consisting of:
a. amino acid sequences comprising DRLFFKCIYRRLKYGLKRGPST; and
b. amino acid sequences comprising amino acid sequence DRLFFKCIYRRLKYGLKRGPST with substitution, deletion or insertion of one or more amino acid residue(s),
c. amino acid sequences comprising an amino acid sequence that has at least 60%, at least 70%, at least 80%, at least 85%, at least 90%, at least 95% or 100% sequence identity with DRLFFKCIYRRLKYGLKRGPST,
wherein the polypeptide has the function of cell-penetrating peptides.

DRLFFKCIYRRLKYGLKRGPST corresponds to the amino acids 44-65 sequence of Jilin-2004(H5N1) influenza virus M2 protein (Jilin-2004(H5N1)-M2-44-65), which has a strong cell-penetrating peptide function. Correspondingly, the cell-penetrating peptide of the present invention can comprise this sequence or functional variant sequences thereof.

In some embodiments, the substitution, deletion or insertion of one or more amino acid residue(s) are substitution, deletion or insertion of 1-10 amino acid residues, such as substitution, deletion or insertion of 1, 2, 3, 4, 5, 6, 7, 8, 9 or 10 amino acids. In some embodiments, at least one, at least two, at least three, at least four, at least five, at least six, at least seven, at least eight, at least nine, or all ten the substitutions can be substitutions with hydrophobic amino acids or positively charged amino acids, for example, selected from I, F and R residues. In some embodiments, the insertion is insertion of hydrophobic amino acids or positively charged amino acids. In some embodiments, the deletion is deletion of hydrophilic amino acids.

In some embodiments, the polypeptide has an amino acid sequence of formula X1-X2-X3-X4-X5-X6-X7-X8-X9-X10-X11-X12-X13-X14-X15-X16-X17-X18-X19-X20-X2 1-X22; wherein X1 is D or R; X2 is R; X3 is L or F; X4 is F; X5 is F; X6 is K or R; X7 is C or R; X8 is I or F; X9 is Y or F; X10 is R; X11 is R; X12 is L or F; X13 is K or R; X14 is Y or F; X15 is G or R; X16 is L; X17 is K or R; X18 is R; X19 is G or R or K; X20 is P; X21 is S or K; and X22 is T or V; or the polypeptide has an amino acid sequence that has at least 60%, at least 70%, at least 80%, at least 85%, at least 90%, at least 95% or 100% sequence identity with the amino acid sequences; or an amino acid sequence with deletions, insertions or substitutions of one or more amino acid residue(s) in the amino acid sequence.

In some embodiments, the polypeptide has an amino acid sequence selected from the group consisting of DRFFFKCIYRRLKYGLKRGPST, DRFFFKCIYRRLKYGLKRRPST, DRFFFKCIYRRLKFGLKRGPST, DRFFFKCIFRRLKYGLKRGPST, DRFFFKCIFRRLKFGLKRGPST, DRFFFKCFFRRFKFGLKRGPST, RRFFFKCFFRRFKFGLKRGPST, DRFFFKRIYRRLKYRLKRRPST, DRFFFKRIYRRLKYRLKRRPKV, DRFFFKRIFRRLKYRLKRRPST, DRFFFKRIFRRLKFRLKRRPST, DRFFFKRIFRRFKFRLKRRPST, RRFFFKRIFRRFKFRLKRRPST, RRFFFKRIFRRFKFRLKRRPKV, RRLFFKCIYRRLKYGLKRGPST, RRLFFKCIYRRLKYGLKRGPKV, RRFFFKCIYRRLKYGLKRGPST, RRLFFKCIYRRLKYGLKRRPST, RRLFFKCIYRRLKYGLKRKPST, RRLFFRRIYRRLKYGLKRGPST, RRLFFRRIYRRLRYRLRRRPST, DRLFFKRIYRRLKYGLKRGPST, DRLFFRRIYRRLKYGLKRGPST, DRLFFRRIYRRLRYRLRRRPST, DRLFFKCIYRRLKYGLKRRPST, DRLFFKCIYRRLKYRLKRRPST, DRLFFKRIYRRLKYRLKRRPST, RRLFFKRIYRRLKYRLKRRPST and DRLFFKCIYRRLKYGLKRGPKV.

In yet another aspect, the present invention relates to a polypeptide, which has an amino acid sequence selected from the group consisting of:
a. amino acid sequences comprising DRLFFKCIYRRLKYGLKR; and
b. amino acid sequences comprising amino acid sequence of DRLFFKCIYRRLKYGLKR with substitution, deletion or insertion of one or more amino acid residue(s),
c. amino acid sequences containing an amino acid sequence that has at least 60%, at least 70%, at least 80%, at least 85%, at least 90%, at least 95% or 100% sequence identity with DRLFFKCIYRRLKYGLKR,
wherein the polypeptide has the function of cell-penetrating peptides.

In some embodiments, the substitution, deletion or insertion of one or more amino acid residue(s) is/are substitution, deletion or insertion of 1-10 or 1-6, such as 5, 4, 3 or 2 amino acid residue(s).

In some embodiments, at least one or more of the substitutions are substitutions with hydrophobic amino acids or positively charged amino acids. In some embodiments, the insertion is insertion of a hydrophobic amino acid or positively charged amino acid. In some embodiments, the deletion is deletion of a hydrophilic amino acid.

In some embodiments, the polypeptide has an amino acid sequence of formula X1-X2-X3-X4-X5-X6-X7-X8-X9-X10-X11-X12-X13-X14-X15-X16-X17-X18; wherein X1 is D, R, L or I; X2 is R or I; X3 is L, R or K; X4 is F or R; X5 is F or R; X6 is K or I; X7 is C, R or I; X8 is I, K or R; X9 is Y, K or R; X10 is R; X11 is R; X12 is L, R or K; X13 is K; X14 is Y or R; X15 is G; X16 is L or R; X17 is K or I; and X18 is R or I; or the polypeptide has an amino acid sequence that has at least 60%, at least 70%, at least 80%, at least 85%, at least 90%, at least 95% or 100% sequence identity with the amino acid sequences; or an amino acid sequence with deletions, insertions or substitutions of one or more amino acid residue(s) in the amino acid sequence.

In some embodiments, the polypeptide has an amino acid sequence selected from the group consisting of RRLFFKCIYRRLKYGLKR, LRLFFKCIYRRLKYGLKR, DILFFKCIYRRLKYGLKR, DRRFFKCIYRRLKYGLKR, DRLRRKCIYRRLKYGLKR, DRLFFICIYRRLKYGLKR, DRLFFKRIYRRLKYGLKR, DRLFFKIIYRRLKYGLKR, DRLFFKCKKRRLKYGLKR, DRRRRKCRRRRRKYGRKR, DRLFFKCIYRRRKRGLKR, DRLFFKCIYRRLKYGRKR, DRKRRKCKYRRKKYGRKR, DRLFFKCIYRRLKYGLII and IILFFKCIYRRLKYGLKR.

### Conjugates and fusion proteins

The present invention also relates to conjugates and fusion proteins, which comprise the polypeptide of the present invention.

In the embodiments regarding the conjugate, the conjugate comprises the polypeptide of the present invention and a moiety conjugated to the polypeptide. There is not particular limitation on the moiety, which can be a therapeutic moiety, a detectable moiety or a cosmetic moiety, selected from the group consisting of protein, peptide, nucleic acid, antibiotic, anti-inflammatory drug, anti-tumor drug, neuroprotective agent, chemotherapeutic, cytotoxin, radioisotope, fluorescent marker, luminescent substance, chromogenic substance and enzyme.

There is not particular limitation on theselection of the protein, peptide, nucleic acid, antibiotic, anti-inflammatory drug, anti-tumor drug, neuroprotective agent, chemotherapeutic, cytotoxin, radioisotope, fluorescent marker, luminescent substance, chromogenic substance and enzyme, as long as it does not affect the cell-penetrating peptide function of the polypeptide of the present invention.

In some embodiments, the therapeutic moiety is selected from the group consisting of 13-cis-retinoic acid, 2-amino-6-mercaptopurine, 2-CdA, 2-chlorodeoxyadenosine, 5-fluorouracil, 6-thioguanine, 6-mercaptopurine, Accutane, Actinomycin D, adriamycin, Adrucil, Agrylin, Ala-Cort, Aldesleukin, Alemtuzumab, Alitretinoin, Alkaban-AQ, Alkeran, All-trans-retinoicacid, α interferon, Altretamine, Amethopterin, Amifostine, Aminoglutethimide, Anagrelide, Anandron, Anastrozle, Arabinosylcytosine, Aranesp, Aredia, Arimidex, Aromasin, Arsenic trioxide, Asparaginase, ATRA, Avastin, BCG, BCNU, Bevacizumab, Bexarotene, Bicalutamide, BiCNU, Blenoxane, Bleomycin, Bortezomib, Busulfan, Busulfex, C225, Calcium Folinate, Campath, Camptosar, Camptothecin-11, Capecitabine, Carac, Carboplatin, Carmustine, Carmustine Tablet, Casodex, CCNU, CDDP, CeeNU, Daunorubicin, Cetuximab, Chlorambucil, Cisplatin, Citrovorum Factor, Cladribine, Cortisone, Cosmegen, CPT-11, Cyclophosphamide, Cytadren, Cytarabine, Cytarabine Liposome, Cytosar-U, Cytoxan, Dacarbazine, Dactinomycin, Dapomycin α, Daunomycin, Daunorubicin, Daunorubicin Hydrochloride, Daunorubicin Liposome, DaunoXome, Decadron, Delta-Cortef, Deltasone, Denileukin-diftitox, DepoCyt, Dexamethasone, Dexamethasone Acetate, Dexamethasone Sodium Phosphate, Dexasone, Dexrazoxane, DHAD, DIC, Diodex, Docetaxel, Doxil, Doxorubicin, Doxorubicin Liposome, Droxia, DTIC, DTIC-Dome, Duralone, Efudex, Eligard, Ellence, Eloxatin, Elspar, Emcyt, Epirubicin, Epoetin α, Erbitux, Erwinase, Estramusting, Ethyol, Etopophos, Etoposide, Etoposide Phosphate, Eulexin, Evista, Exemestane, Fareston, Faslodex, Femara, Filgrastim, Floxuridine, Fludara, Fludarabine, Fluoroplex, Fluorouracil, Fluoxymesterone, Flutamide, Folinic Acid, FUDR, Fulvestrant, G-CSF, Gefitinib, Gemcitabine, Gemtuzumab, Gemzar, Gleevec, Leuprorelin Acetate, LupronDepot, Matulane, Maxidex, Mechlorethamine, Mechlorethamine Hydrochloride, Medrol, Methylprednisolone, Megace, Megestrol, Megestrol Acetate, Melphalan, Mercaptopurine, Mesna, Mesnex, Methotrexate, Methotrexate Sodium, Methylprednisolone, Mylocel, Letrozole, Neosar, Neulasta, Neumega, Neupogen, Nilandron, Nilutamide, Nitrogen Mustard, Novaldex, Novantrone, Octreotide, Octreotide Acetate, Oncospar, Oncovin, Ontak, Onxal, Oprevelkin, Orapred, Orasone, Oxaliplatin, Paclitaxel, Pamidronate, Panretin, Paraplatin, Pediapred, PEG interferon, Pegaspargase, Pegfilgrastim, PEG-INTRON, PEG-L-Asparaginase, Phenylalanine Mechlorethamine, Platinol, Platinol-AQ, Prednisolone, Prednison, Prelone, Procarbazine, PROCRIT, Proleukin, Polifeprosan 20 with Carmustine Implant, Purinethol, Raloxifene, Rheumatrex, Rituxan, Rituximab, Roveron-A, Rubex, Rubidomycin Hydrochloride, Sandostatin, Sandostatin LAR, Sargramostim, Solu-Cortef, Solu-Medrol, STI-571, Streptozocin, Tamoxifen, Taggretine, Taxol, Taxotere, Temodar, Temozolomide, Teniposide, TESPA, Thalidomide, Thalomid, TheraCys, Thioguanine, Thioguanine Tablet, Thioxophosphamide, Thioplex, Thiotepa, TICE, Toposar, Topotecan, Toremifene, Trastuzumab, Tretinoin, Trexall, Trisenox, TSPA, VCR, Velban, Velcade, Vepesid, Vesanoid, Viadur, Vinblastine, Vinblastine Sulfate, Vincasar Pfs, Vincristine, Vinorelbine, Vinorelbine Tartrate, VLB, VP-16, Vumon, Xeloda, Zanosar, Zevalin, Zinecard, Zoladex, Zoledronic Acid, Zometa, Gliadel, Glivec, GM-CSF, Goserelin, Granulocyte Colony Stimulating Factor, Fluoxymesterone, Herceptin, Hexadrol, Hexalen, Altretamine, HMM, Hycamtin, Hydrea, Hydrocortisone Acetate, Hydrocortisone, Hydrocortisone Sodium Phosphate, Hydrocortisone Sodium Succinate, Hydrocortisone Phosphate, Hydroxyurea, Ibritumomab, Ibritumomab Tiuxetan, Idamycin, Idarubicin, Ifex, Interferon-alpha, Ifosfamide, IL2, IL-11, Imatinib Mesylate, Imidazole Carboxamide, Interferon α, PEG-interferon α, Interleukin 2, Interleukin 11, Leucovorin, Leukeran, Leukine, Leuprorelin, Leurocristine, Leustatin, Liposomal Ara-C, Liquid Pred, Lomustine, L-PAM, L-Sarcolysin, Meticorten, Mitomycin, Mitomycin-C, Mitoxantrone, M-Metacortandracin, MTC, MTX, Mustargen, Mutamycin, Myleran, Iressa, Irinotecan, Isotretinoin, Kidrolase, Lanacort, L-Asparaginasum and LCR, Ganciclovir, Azido Deoxythymidine (AZT), Lamivudine (3TC), Acapolone, acetyl Sodium Sulfone, Alymycin, Alessidine, Mecillinam, Pivmecillinam, Amicycline, Amifloxacin, Amfloxacin Mesylate, Amikacin, Amikacin Sulfate, Aminosalicylic Acid, Sodium Aminosalicylate, Amoxicillin, Amphotericin, Ampicillin, Ampicillin Sodium, Apalcillin Sodium, Apramycin, Aspartate, Aspartate Sulfate, Avilamycin, Avoparcin, Azithromycin, Azlocillin, Azlocillin Sodium, Bacampicillin Hydrochloride, Bacitracin, Bacitracin Methylene Disalicylate, Bacitracin Zinc, Bambermycin, Benzoyl Calcium, Erythromycin Sulfate, Betamycin Sulfate, Biapenem, Beniamycin, Biphenamine Hydrochloride, Biscaptooxypyridine Magnesium Sulfate, Buticacin, Butylosidectin Sulfate, Capreomycin Sulfate, Capadol, Carbenicillin Disodium, Carbenicillin Indene Sodium, Carbenicillin Sodium, Carbenicillin Potassium, Coumarin Sodium, Cefaclor, Cefalexin, Cefamendol, Cefamendole Sodium, Cefamendol Sodium, Cefparo, Ceftriaxone, Cefazoline Sodium, Cefazoline, Cefoperazone, Cefdinir, Cefepime, Cefepime Hydrochloride, Cefanol, Cefoxime, Cefoxime Hydrochloride, Cefmezole, Cefmezole Sodium, Cefniximetam Sodium, Cefonicid Sodium, Cefoperazone Sodium, Cefdinib, Cefotaxime Sodium, Cefotetam, Cefotetam Disodium, Cefotiam Hydrochloride, Cefoxitin, Cefoxitin Sodium, Cefimizole, Cefpiazide Sodium, Cefpiramide, Cefpiramide Sodium, Cefpirome Sulfate, Cefpodoxime Proxetil, Cefprozil, Cefrotidine, Cefsulodine Sodium, Ceftazidime, Ceftibuten, Ceftizoxime Sodium, Ceftriaxone Sodium, Cefuroxime, Cefuroxime Ester, Cefacetonitrile Sodium, Cefexin, Cefexin Hydrochloride, Cefosporin, Cefotaxidine, Cefothiophene Sodium, Cefpirin Sodium, Cefradine, Cetocycline Hydrochloride, Chloramphenicol Acetyl, Chloramphenicol, Chloramphenicol Palmitate, Chloramphenicol Pantothenate Complex, Chloramphenicol Sodium Succinate, Chlorhexidine Aminobenzene Phosphate, Chloroxylphenol, Chlorotetracycline Hydrosulfate, Chlorotetracycline Hydrochloride, Cinoxacin, Ciprofloxacin, Ciprofloxacin Hydrochloride, Cirolemycin, Clarithromycin, Clindfloxacin Hydrochloride, Clindamycin, Clindamycin Hydrochloride, Clindamycin Palmitate Hydrochloride, Clindamycin Phosphate, Clofazimine,Cloxacillin Benzathine, Cloxacillin Sodium, Chlorohydroxyquine, Colistin Mesylate Sodium, Coumarin, Coumarin Sodium, Cyclocillin, Cycloserine, Dafoptine, Dapsone, Datoramycin, Demecycline, Demecycline Hydrochloride, Demecycline, Denofungin, Diaveridine, Dicloxacillin, Dicloxacillin Sodium, Dihydrostreptomycin Sulfate, Dipyrithione, Dierythromycin, Doxycycline, Doxycycline Calcium, Doxycycline Phosphate Complex, Doxycycline Hydrochloride, Droxacin Sodium, Enoxacin, Epicillin, Epitetracycline Hydrochloride, Erythromycin, Erythromycin acistrate, erythromycin estolate, erythromycin ethylsuccinate, erythromycin gluceptate, erythromycin lactobionate, erythromycin propionate, erythromycin stearate, ethambutol hydrochloride, ethionamide, fleroxacin, floxacillin, fludalanine, flumequine, fosfomycin, fosfomycin tromethamine, fumoxicillin, furazolium chloride, furazolium tartrate, fusidate sodium, fusidic acid, gentamicin sulfate, gloximonam, gramicidin, haloprogin, hetacillin, hetacillin potassium, hexedine, ibafloxacin, imipenem, isoconazole, isepamicin, isoniazid, josamycin, kanamycin sulfate, kitasamycin, levofuraltadone, levopropylcillin potassium, lexithromycin, lincomycin, lincomycin hydrochloride, lomefloxacin, Lomefloxacin hydrochloride, lomefloxacin mesylate, loracarbef, mafenide, meclocycline, meclocycline sulfosalicylate, megalomicin potassium phosphate, mequidox, meropenem, methacycline, methacycline hydrochloride, methenamine, methenamine hippurate, methenamine mandelate, methicillin sodium, metioprim, metronidazole hydrochloride, metronidazole phosphate, mezlocillin, mezlocillin sodium, minocycline, minocycline hydrochloride, mirincamycin hydrochloride, monensin, monensin sodiumr, nafcillin sodium, nalidixate sodium, nalidixic acid, natainycin, nebramycin, neomycin palmitate, neomycin sulfate, neomycin undecylenate, netilmicin sulfate, neutramycin, nifuiradene, nifuraldezone, nifuratel, nifuratrone, nifurdazil, nifurimide, nifiupirinol, nifurquinazol, nifurthiazole, nitrocycline, nitrofurantoin, nitromide, norfloxacin, novobiocin sodium, ofloxacin, onnetoprim, oxacillin, oxacillin sodium, oximonam, oximonam sodium, oxolinic acid, oxytetracycline, oxytetracycline calcium, oxytetracycline hydrochloride, paldimycin, parachlorophenol, paulomycin, pefloxacin, pefloxacin mesylate, penamecillin, penicillin G benzathine, penicillin G potassium, penicillin G procaine, penicillin G sodium, penicillin V, penicillin V benzathine, penicillin V hydrabamine, penicillin V potassium, pentizidone sodium, phenyl aminosalicylate, piperacillin sodium, pirbenicillin sodium, piridicillin sodium, pirlimycin hydrochloride, pivampicillin hydrochloride, pivampicillin pamoate, pivampicillin probenate, polymyxin B sulfate, porfiromycin, propikacin, pyrazinamide, pyrithione zinc, quindecamine acetate, quinupristin, racephenicol, ramoplanin, ranimycin, relomycin, repromicin, rifabutin, rifametane, rifamexil, rifamide, rifampin, rifapentine, rifaximin, rolitetracycline, rolitetracycline nitrate, rosaramicin, rosaramicin butyrate, rosaramicin propionate, rosaramicin sodium phosphate, rosaramicin stearate, rosoxacin, roxarsone, roxithromycin, sancycline, sanfetrinem sodium, sarmoxicillin, sarpicillin, scopafungin, sisomicin, sisomicin sulfate, sparfloxacin, spectinomycin hydrochloride, spiramycin, stallimycin hydrochloride, steffimycin, streptomycin sulfate, streptonicozid, sulfabenz, sulfabenzamide, sulfacetamide, sulfacetamide sodium, sulfacytine, sulfadiazine, sulfadiazine sodium, sulfadoxine, sulfalene, sulfamerazine, sulfameter, sulfamethazine, sulfamethizole, sulfamethoxazole, sulfamonomethoxine, sulfamoxole, sulfanilate zinc, sulfanitran, sulfasalazine, sulfasomizole, sulfathiazole, sulfazamet, sulfisoxazole, sulfisoxazole acetyl, sulfisboxazole diolamine, sulfomyxin, sulopenem, sultamricillin, suncillin sodium, talampicillin hydrochloride, teicoplanin, temafloxacin hydrochloride, temocillin, tetracycline, tetracycline hydrochloride, tetracycline phosphate complex, tetroxoprim, thiamphenicol, thiphencillin potassium, ticarcillin cresyl sodium, ticarcillin disodium, ticarcillin monosodium, ticlatone, tiodonium chloride, tobramycin, tobramycin sulfate, tosufloxacin, trimethoprim, trimethoprim sulfate, trisulfapyrimidines, troleandomycin, trospectomycin sulfate, tyrothricin, vancomycin, vancomycin hydrochloride, virginiamycin, or zorbamycin, Silybin.

In some embodiments, the detectable moiety is selected from the group consisting of UV-Vis label, near infrared label, luminescent group, phosphorescent group, magnetic spin resonance label, photosensitizer, photocleavable moiety, chelating center, heavy atom, radioisotope, isotope detectable spin resonance label, paramagnetic moiety, chromophore, luminophore, such as metalloporphyrin; benzoporphyrin; azabenzoporphyrin; naphthoporphyrin, phthalocyanine; polycyclic aromatic hydrocarbon, such as perylene, perylene diimide, pyrene; azo dye; xanthene dye; boron dipyrromethene, aza-boron dipyrromethene, cyanine dye, metal-ligand complex such as bipyridine, bipyridyl, phenanthroline, coumarin and acetylacetonate of ruthenium and iridium; acridine and oxazine derivatives such as benzophenoxazine; aza-annulene, squaraine; 8-hydroxyquinoline, polymethine, luminescence-producing nanoparticles such as quantum dot and nanocrystal; quinolone, terbium conjugate; inorganic phosphor; ionophores such as crown ether attachment or derivatization of dyes; Pd(II) octaethylporphyrin; Pt(II)-octaethylporphyrin; Pd(II) tetraphenylporphyrin; Pt(II) tetraphenylporphyrin; Pd(II) meso-tetraphenylporphyrin tetrabenzomorphine; Pt(II) meso-tetraphenylmethyl benzoporphyrin; Pd(II) octaethylporphyrin; Pt(II) octaethylporphyrin; Pd(II) meso-tetra(pentafluorophenyl)porphyrin; Pt(II) meso-tetra(pentafluorophenyl)porphyrin; Ru(II) tris(4,7-diphenyl-1, 10-phenanthroline)(Ru(dpp)₃); Ru(II)tris(1,10-phenanthroline)(Ru(phen)₃), tris(2,2"-bipyridine) ruthenium(II) chloride hexahydrate (Ru(bpy)₃); erythrosine B; fluorescein; fluorescein isothiocyanate (FITC); eosin; iridium (III) ((N-methyl-benzimidazol-2-yl)-7-(diethylamino)-coumarin)); indium (III) ((benzothiazol-2-y1)-7-(diethylamino)-coumarin)-2-(acetylacetonate); Lumogen dye; Macroflex fluorescent red; Macrolex fluorescent yellow; Texas red; rhodamine B; rhodamine 6G; endorhodamine; m-cresol; thymol blue; xylenol blue; cresol red; chlorophenol blue; bromocresol green; bromocresol red; bromothymol blue; Cy2; Cy3; Cy5; Cy5.5; Cy7; 4-nitrophenol; alizarin; phenolphthalein; o-cresolphthalein; chlorophenol red; calcein; bromoxylenol; phenol red; neutral red; nitrazine; 3,4,5,6-tetrabromophenolphthalein; congo red; fluorescein; eosin; 2',7'-dichlorofluorescein; 5(6)-carboxy-fluorescein; carboxynaphthofluorescein; 8-hydroxystyrene-1,3,6-trisulfonic acid; seminaphthodifluoro; seminaphthofluorescein; tris(4,7-diphenyl-1,10-phenanthroline)ruthenium(II) dichloride; (4,7-diphenyl-1,10-phenanthroline)ruthenium(II) tetraphenyl boron; platinum(II) octaethylporphyrin; dialkyl carbocyanine; dioctadecyl cyclodicarbocyanine; fluorenylmethoxycarbonyl chloride; 7-amino-4-methylcoumarin (Amc); green fluorescent protein (such as GFP, GFP-2, tagGFP, turboGFP, eGFP, Emerald, Azami Green, monomer Azami Green, CopGFP, AceGFP, ZsGreen1), yellow fluorescent protein (such as YFP, eYFP, Citrine, Venus, YPet, PhiYFP, ZsYellowl), blue fluorescent protein (such as eBFP, eBFP2, Azurite, mKalamal, GFPuv, Sapphire, T-sapphire), cyan fluorescent protein (such as eCFP, Cerulean, CyPet, AmCyanl, Midoriishi-Cyan), red fluorescent protein (such as mKate, mKate2, mPlum, DsRed monomer, mCherry, mRFP1, DsRed-Express, DsRed2, DsRed-monomer, HcRed-Tandem, HcRedl, AsRed2, eqFP611, mRaspberry, mStrawberry, Jred), orange fluorescent protein (such as mOrange, mKO, Kusabira-Orange, monomer Kusabira-Orange, mTangerine, tdTomato) and any other suitable fluorescent proteins.

In some embodiments, the cosmetic moiety can be a cosmetic polypeptide, such as palmitoyl pentapeptide 4, palmitoyl tetrapeptide 7, carnosine, acetyl hexapeptide 8, aFGF, bFGF or EGF, botulinus toxin, elastin and hyaluronic acid. In some embodiments, the polypeptide of the present invention is conjugated to one of the moieties. In other embodiments, the polypeptide of the present invention is conjugated to several moieties. In some embodiments, the polypeptide of the present invention is combined with one of the moieties in a non-covalent form. In other embodiments, the polypeptide of the present invention is combined with several moieties in a non-covalent form.

There is not particular limitation on the manner of conjugating the polypeptide of the present invention to the moiety, and the conjugation can be carried out by, for example, physical adsorption or chemical linkage. For example, the polypeptide of the present invention and the moiety can be conjugated non-covalently or covalently.

In the embodiments regarding the fusion protein, the fusion protein comprises the polypeptide of the present invention and the moiety fused to the polypeptide. The moiety can be selected from the group consisting of antigen, antibody or antigen binding fragment thereof, ligand, receptor, cytokine, transcription regulation factor, fluorescent protein and enzyme.

In some embodiments, the moiety fused to the polypeptide is a therapeutic moiety, such as human growth hormone, bovine growth hormone, porcine growth hormone, growth hormone releasing hormone, growth hormone releasing peptide, granulocyte-colony stimulating factor, granulocyte macrophage-colony stimulating factor, macrophage-colony stimulating factor, erythropoietin, bone morphogenetic protein, interferon, insulin, atrial peptide hormone-III, monoclonal antibody, tumor necrosis factor, macrophage activating factor, interleukin, tumor degradation factor, insulin-like growth factor, epidermal growth factor, tissue plasminogen activator and urokinase.

In some embodiments, the moiety fused to the polypeptide is a detectable moiety, for example, green fluorescent protein (such as GFP, GFP-2, tagGFP, turboGFP, eGFP, Emerald, Azami Green, monomer Azami Green, CopGFP, AceGFP, ZsGreen1), yellow fluorescent protein (such as YFP, eYFP, Citrine, Venus, YPet, PhiYFP, ZsYellowl), blue fluorescent protein (such as eBFP, eBFP2, Azurite, mKalamal, GFPuv, Sapphire, T-sapphire), cyan fluorescent protein (such as eCFP, Cerulean, CyPet, AmCyanl, Midoriishi-Cyan), red fluorescent protein (such as mKate, mKate2, mPlum, DsRed monomer, mCherry, mRFP1, DsRed-Express, DsRed2, DsRed-monomer, HcRed-Tandem , HcRedl, AsRed2, eqFP611, mRaspberry, mStrawberry, Jred), orange fluorescent protein (such as mOrange, mKO, Kusabira-Orange, monomer Kusabira-Orange, mTangerine, tdTomato) and any other suitable fluorescent proteins.

In some embodiments, the moiety fused to the polypeptide is a cosmetic moiety, including cosmetic polypeptides, such as palmitoyl pentapeptide 4, palmitoyl tetrapeptide 7, carnosine, acetyl hexapeptide 8, aFGF, bFGF or EGF, botulinus toxin, elastin and hyaluronic acid.

There is not particular limitation on the selection of the antigen, antibody or antigen binding fragment thereof, ligand, receptor, cytokine, transcription regulation factor, fluorescent protein and enzyme, as long as it does not affect the cell-penetrating peptide function of the present polypeptide.

In some embodiments, the polypeptide and the moiety are directly fused. In other embodiments, the polypeptide and the moiety are fused via a linker, for example, via a flexible linker.

In some embodiments, the polypeptide can be fused to the moiety at its N-terminus. In some other embodiments, the polypeptide can be fused to the moiety at its C-terminus. In still other embodiments, the polypeptide can be fused to several moieties at its N-terminus and C-terminus.

### Methods and uses

In one aspect, the present invention relates to a method for allowing a polypeptide to penetrate a cell, which includes the step of incubating the polypeptide, conjugate or fusion protein of the present invention with the cell.

In some embodiments, the cell is a cell line (such as an immortalized cell line), such as an animal or plant cell line, or a microbial cell (such as a bacterial cell or fungal cell), or a primary cell isolated from a subject or a cultured plant cell.

In some embodiments, the cell is selected from the group consisting of tumor cell, fibroblast, epithelial cell, endothelial cell, immune cell, skin cell and nerve cell; or selected from the group consisting of meristematic cell, stone cell, parenchyma cell, germ cell, root hair cell, duct cell, sieve cell, mesophyll cell and guard cell, epidermal cell and pigment cell. In some embodiments, the epithelial cell is selected from digestive tract epithelial cell and respiratory tract epithelial cell.

There is not limitation on the type of the tumor cell, and can be derived from various cancer types, for example, basal cell carcinoma, biliary tract cancer; bladder cancer; bone cancer; brain and CNS cancer; breast cancer; peritoneal cancer; cervical cancer; cholangiocarcinoma; choriocarcinoma; colon and rectal cancer; connective tissue cancer; digestive system cancer; endometrial cancer; esophageal cancer; eye cancer; head and neck cancer; stomach cancer; glioblastoma; liver cancer; hepatic carcinoma; intraepithelial neoplasms; kidney cancer; laryngeal cancer; leukemia; hepatocellular carcinoma; lung cancer; lymphoma, including Hodgkin's lymphoma and non-Hodgkin's lymphoma; melanoma; myeloma; neuroblastoma; oral cancer; ovarian cancer; pancreatic cancer; prostate cancer; retinoblastoma; rhabdomyosarcoma; rectal cancer; respiratory system cancer; salivary gland cancer; sarcoma; skin cancer; squamous cell carcinoma; gastric cancer; teratoma; testicular cancer; thyroid cancer; uterine or endometrial cancer; urinary system cancer; vulvar cancer; and other cancers and sarcomas; and B-cell lymphoma; chronic lymphocytic leukemia (CLL); acute lymphoblastic leukemia (ALL); hairy cell leukemia; chronic myeloblastic leukemia, and the like.

In some embodiments, the cell is selected from, but not limited to, the group consisting of A549 cell, HCT-116 cell, MKN-45 cell, 293T cell, HUVEC cell, ESF-1 cell, HeLa cell and MRC-5 cell.

In any embodiments of the above method for allowing a polypeptide to penetrate a cell, the polypeptide, conjugate, or fusion protein can be incubated with the cell for 5 min to 24 h, such as 10 min to 12 h, 30 min to 6 h, or 1 h to 3 h.

In any embodiments of the above method for allowing a polypeptide to penetrate a cell, the concentration of the polypeptide, conjugate or fusion protein can be 0.01 µM to 100 µM, for example, 0.03 µM to 30 µM, 0.1 µM to 10 µM, or 1 µM to 3 µM.

In another aspect, the present invention relates to a method for introducing a molecule into a cell, the method comprising the step of incubating a mixture of the molecule and the polypeptide, conjugate or fusion protein of the present invention with the cell.

In some embodiments, the molecule is selected from the group consisting of protein, nucleic acid, peptide, lipid, metabolite, drug and small molecule compound.

In some embodiments, the molecule is selected from the group consisting of antibiotic, anti-inflammatory drug, anti-tumor drug, neuroprotective agent, chemotherapeutic, cytotoxin, radioisotope, fluorescent marker, luminescent substance, chromogenic substance, antigen, cytokine, transcription regulation factor and enzyme.

In some embodiments, the molecule is a small RNA, for example selected from siRNA and microRNA.

In some embodiments, the cell is a cell line (such as an immortalized cell line) or a primary cell isolated from a subject.

In some embodiments, the cell is selected from the group consisting of tumor cell, fibroblast, epithelial cell, endothelial cell, immune cell, skin cell and nerve cell. In some embodiments, the epithelial cell is selected from digestive tract epithelial cell and respiratory tract epithelial cell.

In some embodiments, the cell is selected from the group consisting of A549 cell, HCT-116 cell, MKN-45 cell, 293T cell, HUVEC cell, ESF-1 cell, HeLa cell and MRC-5 cell.

In yet another aspect, the present invention relates to use of the polypeptide, conjugate and fusion protein of the present invention in introducing a molecule into a cell.

In some embodiments, the molecule is selected from the group consisting of protein, nucleic acid, peptide, lipid, metabolite, drug and small molecule compound.

In some embodiments, the molecule is selected from the group consisting of antibiotic, anti-inflammatory drug, anti-tumor drug, neuroprotective agent, chemotherapeutic, cytotoxin, radioisotope, fluorescent marker, luminescent substance, chromogenic substance, antigen, cytokine, transcription regulation factor and enzyme.

In some embodiments, the molecule is a small RNA, for example selected from siRNA and microRNA.

In some embodiments, the cell is a cell line or a primary cell isolated from a subject.

In some embodiments, the cell is selected from the group consisting of tumor cell, fibroblast, epithelial cell, endothelial cell, immune cell, skin cell and nerve cell. In some embodiments, the epithelial cell is selected from digestive tract epithelial cell and respiratory tract epithelial cell.

In some embodiments, the cell is selected from the group consisting of A549 cell, HCT-116 cell, MKN-45 cell, 293T cell, HUVEC cell, ESF-1 cell, HeLa cell and MRC-5 cell.

### Treatment and diagnosis of diseases, tracing, preferably intracellular tracing, or cosmetic methods, uses and related compositions

In one aspect, the present invention relates to a method for the treatment and diagnosis of diseases, tracing, preferably intracellular tracing, or cosmetic uses, the method comprising administration of the polypeptide, conjugate or fusion protein of the present invention to a subject in need.

In another aspect, the present invention relates to a method for treatment and diagnosis of diseases, tracing, preferably intracellular tracing, or cosmetic uses, the method comprising administration of the polypeptide, conjugate or fusion protein of the present invention, as well as ingredients selected from the group consisting of antibiotic, anti-inflammatory drug, anti-tumor drug, neuroprotective agent, chemotherapeutic agent, cytotoxin, radioisotope, fluorescent marker, luminescent substance, chromogenic substance, antigen, cytokine, transcription regulation factor, enzyme and nucleic acid, including small RNA, to a subject in need.

In one embodiment of the present invention, the nucleic acid can be a natural or artificial DNA or RNA molecule, which is single-stranded or double-stranded. The nucleic acid molecule can be one or more nucleic acids of the same type (for example, having the same nucleotide sequence), or different types of nucleic acids. The nucleic acid molecule includes, but is not limited to, one or more selected from the group consisting of: DNA, cDNA, decoy DNA, RNA, siRNA, miRNA, shRNA, stRNA, snoRNA, snRNA, PNA, antisense oligomer, plasmid and other modified nucleic acids.

In some embodiments, the small RNA is selected from siRNA and microRNA.

In any embodiments of the method of the present invention for treatment and diagnosis of diseases, tracing, preferably intracellular tracing, or cosmetic uses, the disease can be selected from cancer, viral infection, central nervous system disease, inflammatory disease, autoimmune disease, mitochondrial related disease and metabolic disease.

In one aspect, the present invention relates to the use of the polypeptide, conjugate or fusion protein of the present invention in the treatment of diseases or in cosmetology. In some embodiments, the disease is selected from cancer, viral infection, central nervous system disease, inflammatory disease, autoimmune disease, mitochondrial related disease and metabolic disease.

In another aspect, the present invention relates to the use of the polypeptide, conjugate or fusion protein of the present invention in the preparation of a pharmaceutical composition for the treatment and diagnosis of diseases, tracing, preferably intracellular tracing, or cosmetic uses.

In some embodiments, the pharmaceutical composition further comprises ingredients selected from the group consisting of antibiotic, anti-inflammatory drug, anti-tumor drug, neuroprotective agent, chemotherapeutic, cytotoxin, radioisotope, fluorescent marker, luminescent substance, chromogenic substance, antigen, cytokine, transcription regulation factor, enzyme and small RNA.

In some embodiments, the small RNA is selected from siRNA and microRNA.

In any embodiments of the above uses, the disease can be selected from the group consisting of cancer, viral infection, central nervous system disease, inflammatory disease, autoimmune disease, mitochondrial related disease and metabolic disease.

In yet another aspect, the present invention relates to a nucleic acid molecule, which comprises a nucleotide sequence encoding the polypeptide or fusion protein of the present invention.

In one aspect, the invention relates to a vector, which comprises the nucleic acid molecule of the invention.

In the present invention, cosmetology includes eliminating and delaying skin aging, whitening and anti-wrinkle. In embodiments related to cosmetology, those skilled in the art can conjugate cosmetic substances to the penetrating peptide of the present invention, covalently or non-covalently, so as to deliver them into the cell to exert their effects; wherein the cosmetic substances are for example cosmetic polypeptides, such as palmitoyl pentapeptide-4, palmitoyl tetrapeptide 7, carnosine, acetyl hexapeptide 8, aFGF, bFGF or EGF, botulinus toxin, elastin, hyaluronic acid, and the like.

As used herein, "vector" refers to a nucleic acid delivery vehicle into which polynucleotides can be inserted. The vector is called an expression vector when it can express the protein encoded by the inserted polynucleotide. The vector can be introduced into the host cell by transformation, transduction or transfection, and then the genetic material elements it carries can be expressed in the host cell. The vector is generally acknowledged by those skilled in the art, including but not limited to: (1) plasmid; (2) phagemid; (3) cosmid; (4) artificial chromosome, such as yeast artificial chromosome (YAC), bacterial artificial chromosome (BAC) or PI-derived artificial chromosome (PAC); (5) phage, such as λ phage or M13 phage and (6) animal virus, such as retrovirus (including lentivirus), adenovirus, adeno-associated virus, herpes virus (such as herpes simplex virus), pox virus and baculovirus. A vector can contain a variety of elements to control the expression, including but not limited to promoter sequence, transcription initiation sequence, enhancer sequence, selection element and reporter gene; in addition, the vector can also contain a replication initiation site.

In one aspect, the present invention relates a composition, which comprises the polypeptide, conjugate or fusion protein of the present invention.

In some embodiments, the composition further comprises ingredients selected from the group consisting of antibiotic, anti-inflammatory drug, anti-tumor drug, neuroprotective agent, chemotherapeutic, cytotoxin, radioisotope, fluorescent marker, luminescent substance, chromogenic substance, antigen, cytokine, transcription regulation factor, enzyme and small RNA.

In some embodiments, the small RNA is selected from siRNA and microRNA.

In some embodiments, the composition is a pharmaceutical composition and further comprises one or more pharmaceutically acceptable carriers.

The phrase "pharmaceutically acceptable carrier" refers to a pharmaceutically acceptable material, composition or vehicle, such as liquid or solid filler, diluent, excipient, solvent, media, encapsulating material, manufacturing aid (such as lubricant, magnesium talc, calcium stearate or zinc stearate, or stearic acid) or solvent encapsulating material, which is involved in maintaining the stability, solubility or activity of the LAP binding agent. Each carrier must be "acceptable" in the sense of being compatible with other ingredients of the formulation and not harmful to the patient. Some examples of a material that can serve as a pharmaceutically acceptable carrier include: (1) saccharide, such as lactose, glucose and sucrose; (2) starch, such as corn starch and potato starch; (3) cellulose and derivates thereof, such as sodium carboxymethyl cellulose, methyl cellulose, ethyl cellulose, microcrystalline cellulose and cellulose acetate; (4) powdered tragacanth; (5) malt; (6) gelatin; (7) excipient, such as cocoa butter and suppository wax; (8) oil, such as peanut oil, cottonseed oil, safflower oil, sesame oil, olive oil, corn oil and soybean oil; (9) glycol, such as propylene glycol; (10) polyol, such as glycerol, sorbitol, mannitol and polyethylene glycol (PEG); (11) ester, such as ethyl oleate and ethyl laurate; (12) agar; (13) buffer, such as magnesium hydroxide and aluminium hydroxide; (14) alginic acid; (15) pyrogen-free water; (16) isotonic saline; (17) Ringer's solution; (19) pH buffer solution; (20) polyester, polycarbonate and/or polyanhydrides; (21) filler, such as polypeptide and amino acid; (22) serum component, such as serum albumin, HDL and LDL; (23) C₂-C₁₂ alcohol, such as ethanol; and (24) other non-toxic compatible substance used in pharmaceutical formulations. Releasing agents, coating agents, preservatives and antioxidants can also be present in the pharmaceutical formulation.

### Diseases

As used herein, the term "cancer" refers to malignant neoplasms (Stedman's Medical Dictionary, 25th ed.; Hensyl ed.; Williams & Wilkins: Philadelphia, 1990). Exemplary cancers include, but are not limited to, acoustic neuroma; adenocarcinoma; adrenal carcinoma; rectal cancer; angiosarcoma (such as lymphangiosarcoma, lymphatic endothelial sarcoma and angiosarcoma); cecal cancer; benign monoclonal gammopathy; bile cancer (such as cholangiocarcinoma); bladder cancer; breast cancer (such as adenocarcinoma of the breast, papillary carcinoma of the breast, breast cancer and medullary carcinoma of the breast); brain cancer (such as meningioma, glioblastoma, glioma (such as astrocytoma and oligodendroglioma) and medulloblastoma); bronchial carcinoma; carcinoid tumors; cervical cancer (such as cervical adenocarcinoma); choriocarcinoma; chordoma; craniopharyngioma; colorectal cancer (such as colon cancer, rectal cancer and colorectal adenocarcinoma); connective tissue cancer; epithelial cancer; ependymoma; endothelial sarcoma (such as Kaposi's sarcoma and multiple idiopathic hemorrhagic sarcoma); endometrial cancer (such as uterine cancer and uterine sarcoma); esophageal cancer (such as adenocarcinoma of the esophagus and Barrett's adenocarcinoma); Ewing's sarcoma; eye cancer (such as intraocular melanoma and retinoblastoma); familial eosinophilia; gallbladder cancer; gastric cancer (such as gastric adenocarcinoma); gastrointestinal stromal tumor (GIST); germ cell cancer; head and neck cancer (such as head and neck squamous cell carcinoma, oral cancer (such as oral squamous cell carcinoma), laryngeal cancer (such as laryngeal cancer, pharynx cancer, nasopharyngeal cancer and oropharyngeal cancer)); hematopoietic stem cell cancer (for example, leukemia, such as acute lymphocytic leukemia (ALL) (such as B-cell ALL and T-cell ALL), acute myeloid leukemia (AML) (such as B-cell AML and T-cell AML), chronic myeloid leukemia (CML) (such as B-cell CML and T-cell CML), and chronic lymphocytic leukemia (CLL) (such as B-cell CLL and T-cell CLL); lymphomas, such as Hodgkin's lymphoma (HL) (such as B-cell HL and T-cell HL) and non-Hodgkin's lymphoma (NHL) (such as B-cell NHL, such as diffuse large cell lymphoma (DLCL) (such as diffuse large B-cell lymphoma), follicular lymphoma, chronic lymphocytic leukemia/small lymphocytic lymphoma (CLL/SLL), mantle cell lymphoma (MCL), marginal zone B-cell lymphoma (such as mucous associated lymphoid tissue (MALT) lymphoma, nodular marginal zone B-cell lymphoma and splenic marginal zone B-cell lymphoma), primary mediastinal B-cell lymphoma, Burkitt lymphoma, lymphoplasmacytic lymphoma (i.e. Waldenstrom macroglobulinemia), hairy cell leukemia (HCL), immunoblastic large cell lymphoma, precursor B-lymphoblastic lymphoma and primary central nervous system (CNS) lymphoma; and T-cell NHL, such as precursor T-lymphoblastic lymphoma/leukemia, peripheral T-cell lymphoma (PTCL) (such as cutaneous T-cell lymphoma (CTCL) (such as mycosis fungoides and Sezari syndrome), angioimmunoblastic T-cell lymphoma, extranodal natural killer T-cell lymphoma, enteropathic T-cell lymphoma, subcutaneous panniculitis-like T-cell lymphoma and anaplastic large cell lymphoma); a mixture of one or more of the above leukemias/lymphomas; and multiple myeloma (MM), heavy chain diseases (such as α chain disease, γ chain disease and µ chain disease); hemangioblastoma; hypopharyngeal carcinoma; inflammatory myofibroblastoma; immune cell amyloidosis; renal cancer (such as nephroblastoma, also known as Wilms' tumor, and renal cell carcinoma); liver cancer (such as hepatocellular carcinoma (HCC) and malignant liver cancer); lung cancer (such as bronchial carcinoma, small cell lung cancer (SCLC), non-small cell lung cancer (NSCLC) and lung adenocarcinoma); leiomyosarcoma (LMS); mastocytosis (for example, systemic mastocytosis); muscle cancer; myelodysplastic syndrome (MDS); mesothelioma; myelodysplastic disease (MPD) (such as polycythemia vera (PV), idiopathic thrombocytosis (ET), unexplained myeloid metaplasia (AMM), also known as myelofibrosis (MF), chronic idiopathic myelofibrosis, chronic myeloid leukemia (CML), chronic neutrophilic leukemia (CNL) and hypereosinophilic syndrome (HES)); neuroblastoma; neurofibromatosis (such as neurofibromatosis (NF) type 1 or 2 and schwannoma); neuroendocrine cancer (such as gastroenteropancreatic neuroendocrine tumor (GEP-NET) and carcinoid tumor); osteosarcoma (such as bone cancer); ovarian cancer (such as cystadenocarcinoma, ovarian embryonic carcinoma and ovarian gland carcinoma); papillary adenocarcinoma; pancreatic cancer (such as pancreatic cancer, intraductal papillary mucin-like neoplasm (IPMN) and islet cell tumor); penile cancer (such as Paget's disease of the penis and scrotum); pinealoma; primary neuroectodermal tumor (PNT); plasmacytoma; tumor-like syndrome; intraepithelial neoplasia; prostate cancer (such as adenocarcinoma of the prostate); rectal cancer; rhabdomyosarcoma; salivary gland cancer; skin cancer (such as squamous cell carcinoma (SCC), keratoacanthoma (KA), melanoma and basal cell carcinoma (BCC)); small intestine cancer (such as cecal cancer); soft tissue sarcoma (such as malignant fibrous histiocytoma (MFH), liposarcoma, malignant peripheral nerve sheath tumor (MPNST), chondrosarcoma, fibrosarcoma and myxosarcoma); sebaceous carcinoma; small bowel carcinoma; sweat gland carcinoma; synovial tumor; testicular carcinoma (such as seminoma and testicular embryonic cancer); thyroid cancer (such as papillary thyroid cancer, papillary thyroid cancer (PTC) and medullary thyroid cancer); urethral cancer; vaginal cancer; and vulvar cancer (such as Paget's disease of the vulva).

In the present disclosure, viral infection includes, but is not limited to, poxviridae viral disease, herpesviridae viral disease, adenoviridae viral disease, papillomaviridae and polyomaviridae viral disease, parvoviridae viral disease, hepatotropic DNA viral disease, retroviral viral disease, reoviridae viral disease, bornaviridae viral disease, rhabdoviridae viral disease, filoviridae viral disease, Paramyxoviridae viral disease, orthomyxoviridae viral disease, bunyaviridae viral disease, arenaviridae viral disease, picornaviridae viral disease, caliciviridae viral disease, astroviridae viral disease, coronaviridae viral disease, togaviridae viral disease, flaviviridae viral disease, unspecified viral disease and lentiviral infections. For example, viral infection is infection caused by the following viruses: hepatitis A, B, C virus, influenza virus, varicella virus, herpes simplex virus type I (HSV-I), herpes simplex virus type II (HSV-II), rinderpest virus, respiratory syncytial virus, cytomegalovirus, sea urchin virus, arbovirus, hantavirus, mumps virus, measles virus, rubella virus, human immunodeficiency virus type I (HIV-1), human immunity Defective virus type II (HIV-2), any togaviridae virus (such as dengue virus), alphavirus, flavivirus, coronavirus, rabies virus, green monkey virus, ebola virus, parainfluenza virus, orthomyxovirus, arenavirus, human T-cell leukemia virus type I, human T-cell leukemia virus type II, simian immunodeficiency virus, lentivirus, Epstein-Barr virus, human herpes virus, cercopithecine herpes virus 1 (B virus) and pox virus.

In the present disclosure, central nervous system disease includes, but is not limited to, neurodegenerative disease, stroke, epilepsy, traumatic brain injury, shock, HIV dementia, glaucoma, multiple sclerosis, and the like. Stroke is divided into hemorrhagic and ischemic stroke. The neurodegenerative disease includes Alzheimer's disease, cerebellar atrophy, multiple sclerosis, primary lateral sclerosis, spinal muscular atrophy, Parkinson's disease, Huntington's disease, Creutzfeldt-Jakob disease, bovine spongiform encephalopathy, ataxia telangiectasia and amyotrophic lateral sclerosis.

As used herein, "inflammatory disease" refers to a disease caused by inflammation, derived from inflammation, or which causes inflammation. The term "inflammatory disease" also refers to a dysregulated inflammatory response, which results in an excessive response of macrophages, granulocytes and/or T-lymphocytes, which leads to normal tissue damage and/or cell death. The inflammatory disease can be an acute or chronic inflammatory condition, and can be caused by infection or non-infectious causes. The inflammatory disease includes, but is not limited to, atherosclerosis, arteriosclerosis, autoimmune disorder, multiple sclerosis, systemic lupus erythematosus, polymyalgia rheumatica (PMR), gouty arthritis, degenerative arthritis, tendinitis, bursitis, psoriasis, cystic fibrosis, arthrosteitis, rheumatoid arthritis, inflammatory arthritis, Sjogren's syndrome, giant cell arteritis, progressive systemic sclerosis (scleroderma), ankylosing spondylitis, polymyositis, dermatomyositis, pemphigus, pemphigoid, diabetes (such as type I), myasthenia gravis, Hashimoto's thyroiditis, Graves' disease, Goodpasture's disease, mixed connective tissue disease, sclerosing cholangitis, inflammatory bowel disease, Crohn's disease, ulcerative colitis, pernicious anemia, inflammatory skin disease, usual interstitial pneumonia (UIP), asbestosis, silicosis, bronchiectasis, beryllium poisoning, talc disease, pneumoconiosis, sarcomatoid disease, desquamative interstitial pneumonia, lymphoid interstitial pneumonia, giant cell interstitial pneumonia, cellular interstitial pneumonia, exogenous allergies alveolitis, Wegener's granulomatosis and related forms of vasculitis (temporal arteritis and polyarteritis nodosa), inflammatory skin disease, hepatitis, delayed hypersensitivity reaction (such as poison ivy dermatitis), pneumonia, respiratory tract inflammation, adult respiratory distress syndrome (ARDS), encephalitis, immediate hypersensitivity, asthma, hay fever, allergy, acute allergic reaction, rheumatic fever, glomerulonephritis, pyelonephritis, cellulitis, cystitis, chronic cholecystitis, ischemia (ischemic injury), reperfusion injury, allograft rejection, host versus graft, appendicitis, arteritis, blepharitis, bronchiolitis, bronchitis, cervicitis, cholangitis, chorioamnionitis, conjunctivitis, lacrimal gland inflammation, dermatomyositis, endocarditis, endometritis, enteritis, enterocolitis, epicondylitis, epididymitis, fasciitis, fibrositis, gastritis, gastroenteritis, gingivitis, ileitis, iritis, laryngitis, myelitis, myocarditis, nephritis, omphalitis, oophoritis, orchitis, ostitis, otitis, pancreatitis, mumps, pericarditis, pharyngitis, pleurisy, phlebitis, pneumonia, proctitis, prostatitis, rhinitis, salpingitis, sinusitis, stomatitis, synovitis, orchitis, tonsillitis, urethritis, cystitis, uveitis, vaginitis, vascular inflammation, vulvitis, vulvovaginitis, vasculitis, chronic bronchitis, osteomyelitis, optic neuritis, temporal arteritis, transverse myelitis, necrotizing fasciitis and necrotizing enterocolitis.

As used herein, "autoimmune disease" refers to a disease that results from improper immune responses in a subject against substances and tissues normally present in the body. In other words, the immune system mistakes a part of the body as a pathogen and attacks its own cells. This may be restricted to certain tissues (such as in autoimmune thyroiditis) or may include specific tissues at different sites (such as Goodpasture's disease, which may affect the basal membranes of both the lung and kidney). The treatment of autoimmune diseases usually applies immunosuppressive agents, for example, drugs that reduce the immune response. Exemplary autoimmune diseases include, but are not limited to, glomerulonephritis, Goodpasture's syndrome, necrotizing vasculitis, lymphadenitis, nodular periarteritis, systemic lupus erythematosus, rheumatoid disease, arthritis, psoriatic arthritis, systemic lupus erythematosus, psoriasis, ulcerative colitis, systemic sclerosis, dermatomyositis/polymyositis, antiphospholipid antibody syndrome, scleroderma, pemphigus vulgaris, ANCA-related vasculitis (such as Wegener's granulomatosis and polyangiitis under the microscope), uveitis, Shouglen's syndrome, Crohn's disease, Wright's syndrome, ankylosing spondylitis, Lyme arthritis, Gulan-Barre syndrome, Hashimoto's thyroiditis and cardiomyopathy.

As used herein, the metabolic disease includes, but is not limited to, phenylketonuria, xanthoma, amyloidosis, xanthomas, xanthomas, lipomatous fibroma, lipofibroma, lipofibroma, xanthomatosis, hyperlipidemia, hyperlipemia, hyperlipemia, lipomatosis, hyperliposis, amyloidosis, amyloidosis, galactosemia, obesity, hyperlipidemia, osteomalacia, ricket, osteomalacia and ricket, osteoporosis and diabetes. As used herein, "mitochondrial related disease" includes Huntington's disease, amyotrophic lateral sclerosis, mitochondrial myopathy, encephalopathy, lactic acidosis and stroke-like episodes (MELAS); myoclonus epilepsy associated with ragged-red fibers (MERRF); neuromuscular relaxation, disorder; neuropathy, ataxia, and retinitis pigmentosa/maternally inherited Leigh syndrome (NARP/MILS); Leber's hereditary optic neuropathy (LHON); Kearns-Sayre syndrome (KSS); Pearson marrow-pancreas syndrome (PMPS); chronic progressive external ophthalmoplegia (CPEO); Wright's syndrome; Alpers syndrome; multiple mitochondrial DNA deficiency syndrome; mitochondrial DNA deficiency syndrome; complex I defect; complex II (succinate dehydrogenase (SDH)) defect; complex III defect; cytochrome c oxidase (COX, complex IV) defect; complex V defect; adenine nucleotide transporter (ANT) defect; pyruvate dehydrogenase (PDH) defect; ethylmalonate aciduria with lactic acidemia; 3-methylglutaconate aciduria with lactic acidemia; refractoriness epilepsy attenuated during infection; Asperger's syndrome attenuated during infection; autism attenuated during infection; attention deficit hyperactivity disorder (ADHD); cerebral palsy attenuated during infection; dyslexia attenuated during infection; maternally inherited thrombocytopenia; leukemia; MNGIE (mitochondrial myopathy, peripheral and autonomic neuropathy, gastrointestinal dysfunction and epilepsy); MARIAHS syndrome (mitochondrial ataxia, recurrent infections, aphasia, hypouricemia/hypomyelination, seizures, and dicarboxylic aciduria); ND6 dystonia; periodic vomiting attenuated during infection; 3-hydroxyisobutyric acid urine with lactic acidemia; diabetes insipidus with lactic acidemia; uridine-responsive neurological symptoms (URNS); family bilateral striatal necrosis (FBSN); aminoglycosides related hearing loss; relaxation cardiomyopathy; splenic lymphoma; tungsten symptoms; multiple mitochondrial DNA deletion symptoms; and renal tubular acidemia/diabetes insipidus/disorder symptoms.

As used herein, "mitochondrial related disease" involves, but not limited to: Huntington's disease, amyotrophic lateral sclerosis, mitochondrial myopathy, encephalopathy, lactic acidosis and stroke-like episodes (MELAS); myoclonus epilepsy associated with ragged-red fibers (MERRF); neuromuscular relaxation, disorder; neuropathy, ataxia, and retinitis pigmentosa/maternally inherited Leigh syndrome (NARP/MILS); Leber's hereditary optic neuropathy (LHON); Kearns-Sayre syndrome (KSS); Pearson marrow-pancreas syndrome (PMPS); chronic progressive external ophthalmoplegia (CPEO); Wright's syndrome; Alpers syndrome; multiple mitochondrial DNA deficiency syndrome; mitochondrial DNA deficiency syndrome; complex I defect; complex II (succinate dehydrogenase (SDH)) defect; complex III defect; cytochrome c oxidase (COX, complex IV) defect; complex V defect; adenine nucleotide transporter (ANT) defect; pyruvate dehydrogenase (PDH) defect; ethylmalonate aciduria with lactic acidemia; 3-methylglutaconate aciduria with lactic acidemia; refractoriness epilepsy attenuated during infection; Asperger's syndrome attenuated during infection; autism attenuated during infection; attention deficit hyperactivity disorder (ADHD); cerebral palsy attenuated during infection; dyslexia attenuated during infection; maternally inherited thrombocytopenia; leukemia; MNGIE (mitochondrial myopathy, peripheral and autonomic neuropathy, gastrointestinal dysfunction and epilepsy); MARIAHS syndrome (mitochondrial ataxia, recurrent infections, aphasia, hypouricemia/hypomyelination, seizures, and dicarboxylic aciduria); ND6 dystonia; periodic vomiting attenuated during infection; 3-hydroxyisobutyric acid urine with lactic acidemia; diabetes insipidus with lactic acidemia; uridine-responsive neurological symptoms (URNS); family bilateral striatal necrosis (FBSN); aminoglycosides related hearing loss; relaxation cardiomyopathy; splenic lymphoma; tungsten symptoms; multiple mitochondrial DNA deletion symptoms; and renal tubular acidemia/diabetes insipidus/disorder symptoms.

### DESCRIPTION OF THE FIGURES

Figure 1: Cell entry of different rhodamine-labeled polypeptides detected by confocal laser technology.
Figure 2: Cell entry of FITC-labeled polypeptides at different concentrations detected by flow cytometry.
Figure 3: Entry of FITC-labeled polypeptides into A549 cells detected by flow cytometry. Polypeptide concentration: 1 µM.
Figure 4: Entry of FITC-labeled polypeptides into A549 cells detected by flow cytometry. Polypeptide concentration: 3 µM.
Figure 5: Entry of FITC-labeled polypeptides into A549 cells detected by flow cytometry. Polypeptide concentration: 10 µM.
Figure 6: Entry of FITC-labeled polypeptides into A549 cells at different concentrations detected by flow cytometry.
Figure 7: Entry of FITC-labeled polypeptides into cells detected by flow cytometry. Left panel, HeLa cells; polypeptide concentration: 10 µM. Right panel, MRC5 cells; polypeptide concentration: 3 µM.
Figure 8: Entry of FITC-labeled polypeptide fragments into A549 cells detected by flow cytometry.
Figure 9: Entry of FITC-labeled polypeptide fragments into A549 cells detected by flow cytometry. Polypeptide concentration: 1 µM. Incubation duration: 1 h.
Figure 10: Entry of FITC-labeled polypeptide fragments into A549 cells detected by flow cytometry. Polypeptide concentration: 1 µM. Incubation duration: 1 h.
Figure 11: Entry of FITC-labeled polypeptide fragments into A549 cells detected by flow cytometry. Polypeptide concentration: 1 µM. Incubation duration: 3 h.
Figure 12: Entry of FITC-labeled polypeptide fragments into A549 cells detected by flow cytometry. Polypeptide concentration: 1 µM. Incubation duration: 3 h.
Figure 13: Entry of FITC-labeled polypeptide fragments into 293T cells detected by flow cytometry. Polypeptide concentration: 1 µM. Incubation duration: 1 h.
Figure 14: Entry of FITC-labeled polypeptides into 293T cells detected by flow cytometry. Polypeptide concentration: 1 µM. Incubation duration: 1 h.
Figure 15: Entry of FITC-labeled polypeptide fragments into HUVEC cells detected by flow cytometry. Polypeptide concentration: 1 µM. Incubation duration: 1 h.
Figure 16: Entry of FITC-labeled TAT and M2 (44-65) polypeptides into HUVEC cells detected by flow cytometry. Polypeptide concentration: 1 µM. Incubation duration: 1 h.
Figure 17: Entry of FITC-labeled polypeptide fragments into HCT-116 cells detected by flow cytometry. Polypeptide concentration: 1 µM. Incubation duration: 1 h.
Figure 18: Entry of FITC-labeled TAT, M2 (44-65) and M2 (45-62) polypeptides into HCT-116 cells detected by flow cytometry. Polypeptide concentration: 1 µM. Incubation duration: 1 h.
Figure 19: Entry of FITC-labeled polypeptide fragments into MNK-45 cells detected by flow cytometry. Polypeptide concentration: 1 µM. Incubation duration: 1 h.
Figure 20: Entry of FITC-labeled TAT, M2 (44-65) and M2 (45-62) polypeptides into MKN-45 cells detected by flow cytometry. Polypeptide concentration: 1 µM. Incubation duration: 1 h.
Figure 21: Entry of FITC-labeled polypeptide fragments into U937 cells detected by flow cytometry. Polypeptide concentration: 1 µM. Incubation duration: 1 h.
Figure 22: Entry of FITC-labeled TAT, M2 (44-65) and M2 (45-62) polypeptides into U937 cells detected by flow cytometry. Polypeptide concentration: 1 µM. Incubation duration: 1 h.
Figure 23: Entry of FITC-labeled TAT, M2 (44-65) and M2 (44-61) polypeptides into MRC5 cells detected by flow cytometry. Polypeptide concentration: 1 µM. Incubation duration: 1 h.
Figure 24: Entry of FITC-labeled TAT and M2 (44-65) polypeptides into ESF-1 cells detected by flow cytometry. Polypeptide concentration: 1 µM. Incubation duration: 1 h.
Figure 25: Entry of different FITC-labeled polypeptide fragments into ESF-1 cells detected by confocal laser technology. Polypeptide concentration: 3 µM. Incubation duration: 1 h.
Figure 26: Entry of polypeptides into A549 cells with different incubation durations detected by flow cytometry. Polypeptide concentration: 1 µM. Incubation durations: left: 5 min; middle: 10 min; right: 30 min.
Figure 27: Entry of polypeptides into A549 cells with different incubation durations detected by flow cytometry. Polypeptide concentration: 1 µM
Figure 28: Comparison of entry of different cell-penetrating peptides and Jilin-2004(H5N1)-M2-45-62 polypeptide of the present invention into A549 cells detected by flow cytometry. Polypeptide concentration: 1 µM.
Figure 29: Comparison of entry of different cell-penetrating peptides and Jilin-2004(H5N1)-M2-44-61 polypeptide of the present invention into A549 cells detected by flow cytometry. Polypeptide concentration: 1 µM.
Figure 30: Comparison of entry of different cell-penetrating peptides and Jilin-2004(H5N1)-M2-44-65 polypeptide of the present invention into A549 cells detected by flow cytometry. Polypeptide concentration: 1 µM.
Figure 31: Comparison of entry of different cell-penetrating peptides and Jilin-2004(H5N1)-M2-45-62 polypeptide of the present invention into A549 cells detected by flow cytometry. Polypeptide concentration: 3 µM.
Figure 32: Comparison of entry of different cell-penetrating peptides and Jilin-2004(H5N1)-M2-44-61 polypeptide of the present invention into A549 cells detected by flow cytometry. Polypeptide concentration: 3 µM.
Figure 33: Comparison of entry of different cell-penetrating peptides and Jilin-2004(H5N1)-M2-44-65 polypeptide of the present invention into A549 cells detected by flow cytometry. Polypeptide concentration: 3 µM.
Figure 34: Entry of mutant sequences of Jilin-2004(H5N1)-M2-44-56 polypeptide into A549 cells detected by flow cytometry. Polypeptide concentration: 1 µM. Incubation duration: 1 h.
Figure 35: Entry of mutant sequences of Jilin-2004(H5N1)-M2-45-62 polypeptide into A549 cells detected by flow cytometry. Polypeptide concentration: 1 µM. Incubation duration: 1 h.
Figure 36: Entry of Jilin-2004(H5N1)-M2-45-62 polypeptide and its mutant sequences into A549 cells detected by confocal laser technology.
Figure 37: Entry of single site mutation sequences of Jilin-2004(H5N1)-M2-45-62 polypeptide into A549 cells detected by flow cytometry. Polypeptide concentration: 1 µM. Incubation duration: 3 h.
Figure 38: Entry of Jilin-2004(H5N1)-M2-45-62 polypeptide and its single site mutation sequences into A549 cells detected by confocal laser technology.
Figure 39: Entry of mutant sequences of Jilin-2004(H5N1)-M2-44-65 polypeptide into A549 cells detected by flow cytometry. Polypeptide concentration: 1 µM. Incubation duration: 1 h.
Figure 40: Entry of mutant sequences of Jilin-2004(H5N1)-M2-44-65 polypeptide into A549 cells detected by flow cytometry. Polypeptide concentration: 3 µM. Incubation duration: 1 h.
Figure 41: Entry of mutant sequences of Jilin-2004(H5N1)-M2-44-65 polypeptide into 293T cells detected by flow cytometry. Polypeptide concentration: 1 µM. Incubation duration: 1 h.
Figure 42: Entry of mutant sequences of Jilin-2004(H5N1)-M2-44-65 polypeptide into MRC5 cells detected by flow cytometry. Polypeptide concentration: 1 µM. Incubation duration: 1 h.
Figure 43: Entry of mutant sequences of Jilin-2004(H5N1)-M2-44-65 polypeptide into U937 cells detected by flow cytometry. Polypeptide concentration: 1 µM. Incubation duration: 1 h.
Figure 44: Entry of mutant sequences of Jilin-2004(H5N1)-M2-44-65 polypeptide into HCT-116 cells detected by flow cytometry. Polypeptide concentration: 1 µM. Incubation duration: 1 h.
Figure 45: Cell entry of different cell-penetrating peptides carrying small RNA detected by flow cytometry.
Figure 46: Cell entry of Jilin-2004(H5N1)-M2-45-62 polypeptide carrying small RNA detected by confocal laser technology.
Figure 47: Cell entry of Jilin-2004(H5N1)-M2-44-56 polypeptide carrying small RNA detected by confocal laser technology.
Figure 48: Cell entry of Jilin-2004(H5N1)-M2-44-61, Jilin-2004(H5N1)-M2-44-65 and Jilin-2004(H5N1)-M2-44-65 mut8 polypeptides carrying small RNA detected by confocal laser technology.
Figure 49: Function of different cell-penetrating peptides carring PGY-sRNA-6 into cells detected by dual-luciferase reporter assay.
Figure 50: Cell entry of Jilin-2004(H5N1)-M2-44-56 carrying β-galactosidase.
Figure 51: Entry of the mixture of EGFP and cell-penetrating peptides into A549 cells detected by confocal laser technology. Incubation duration: 1 h.
Figure 52: Entry of EGFP-M2(71-85) and EGFP-M2(45-62) fusion proteins into A549 cells at different concentrations detected by flow cytometry. Incubation duration: 1 h.
Figure 53: Entry of different EGFP fusion proteins into A549 cells detected by flow cytometry. Protein concentration: 10 nM. Incubation duration: 1 h.
Figure 54: Entry of different EGFP fusion proteins into A549 cells detected by flow cytometry. Protein concentration: 30 nM. Incubation duration: 1 h.
Figure 55: Entry of different EGFP fusion proteins into A549 cells detected by flow cytometry. Protein concentration: 100 nM. Incubation duration: 1 h.
Figure 56: Entry of different EGFP fusion proteins into A549 cells detected by confocal laser technology. Protein concentration: 30 nM. Incubation duration: 1 h.
Figure 57: Entry of different EGFP fusion proteins into A549 cells detected by confocal laser technology. Protein concentration: 100 nM. Incubation duration: 1 h.
Figure 58: Entry of different EGFP fusion proteins into A549 cells detected by confocal laser technology. Protein concentration: 300 nM. Incubation duration: 1 h.
Figure 59: Entry of mutant sequences of Jilin-2004(H5N1)-M2-44-61 polypeptide into A549 cells detected by flow cytometry. Polypeptide concentration: 1 µM. Incubation duration: 1 h.
Figure 60: Cell entry of Jilin-2004(H5N1)-M2-44-61 and Jilin-2004(H5N1)-M2-44-65 polypeptides carrying small RNA detected by super high resolution microscopy.
Figure 61: Cell entry of different influenza virus M2 polypeptide fragments carrying small RNA detected by fluorescence quantitative PCR.
Figure 62: Entry of Jilin-2004(H5N1)-M2-45-62 polypeptide and its single site mutation sequences carrying small RNA into A549 cells detected by real-time quantitative PCR.
Figure 63: Entry of Jilin-2004(H5N1)-M2-45-62 polypeptide and its mutant sequences carrying small RNA into A549 cells detected by fluorescence quantitative PCR.
Figure 64: Entry of Jilin-2004(H5N1)-M2-44-61 polypeptide and its mutant sequences carrying small RNA into A549 cells detected by fluorescence quantitative PCR.
Figure 65: Entry of Jilin-2004(H5N1)-M2-44-65 polypeptide and its mutant sequences carrying small RNA into A549 cells detected by fluorescence quantitative PCR.
Figure 66: Function of different cell-penetrating peptides carring HJT-sRNA-m7 into cells detected by dual-luciferase reporter assay.
Figure 67: IL-1β expression of LPS-stimulated cells with different cell-penetrating peptides carrying small RNA, detected by enzyme-linked immunosorbent assay.
Figure 68: TNF-α expression of LPS-stimulated cells with different cell-penetrating peptides carrying small RNA, detected by enzyme-linked immunosorbent assay.
Figure 69: Anti-fibrosis function of HJT-sRNA-m7 carried by different cell-penetrating peptides into cells detected by Western blot.
Figure 70: Expression of GFP plasmids carried by Jilin-2004(H5N1)-M2-44-61 and Jilin-2004(H5N1)-M2-45-62 polypeptides into cells detected by confocal laser technology.
Figure 71: Entry of the EGFP-cell-penetrating peptide fusion protein into different murine organs detected by confocal laser technology.
Figure 72: Entry of Jilin-2004(H5N1)-M2-44-61 polypeptide carrying PGY-sRNA-23 into murine organs detected by fluorescence quantitative PCR.
Figure 73: Entry of Jilin-2004(H5N1)-M2-44-61 polypeptide carrying PGY-sRNA-26 into murine organs detected by fluorescence quantitative PCR.
Figure 74: Anti-inflammatory function of PGY-sRNA-6 carried by Jilin-2004(H5N1)-M2-44-61 polypeptide detected by enzyme-linked immunosorbent assay.
Figure 75: Entry of Jilin-2004(H5N1)-M2-44-61 carrying miR-1246 antagomir into murine lung tissues detected by fluorescence quantitative PCR.
Figure 76: Expressions of TNF-α in the alveolar lavage fluid after entry of Jilin-2004(H5N1)-M2-44-61 carrying miR-1246 antagomir into mice with LPS-induced acute lung injury, detected by enzyme-linked immunosorbent assay.
Figure 77: Expressions of IL-6 in the alveolar lavage fluid after entry of Jilin-2004(H5N1)-M2-44-61 carrying small molecule silybin into mice with LPS-induced acute lung injury, detected by enzyme-linked immunosorbent assay.

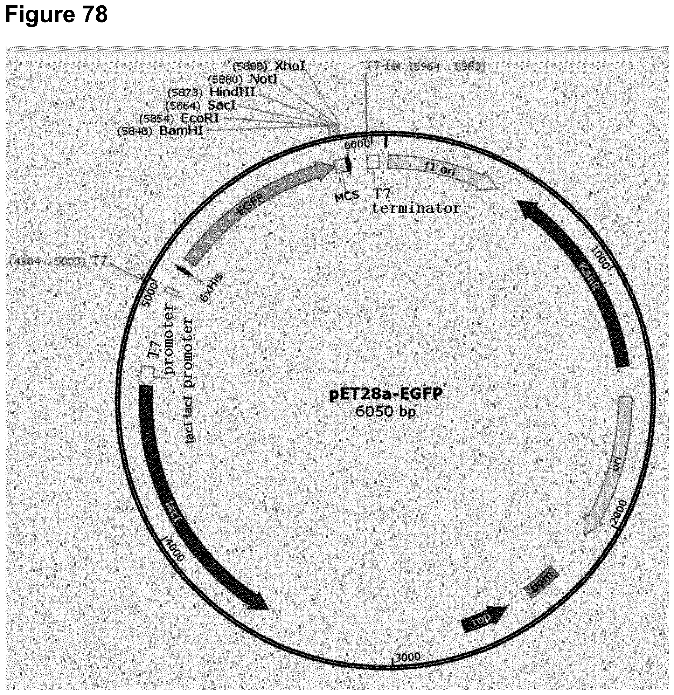

Figure 78: Map of PET-28a-EGFP vector plasmid.

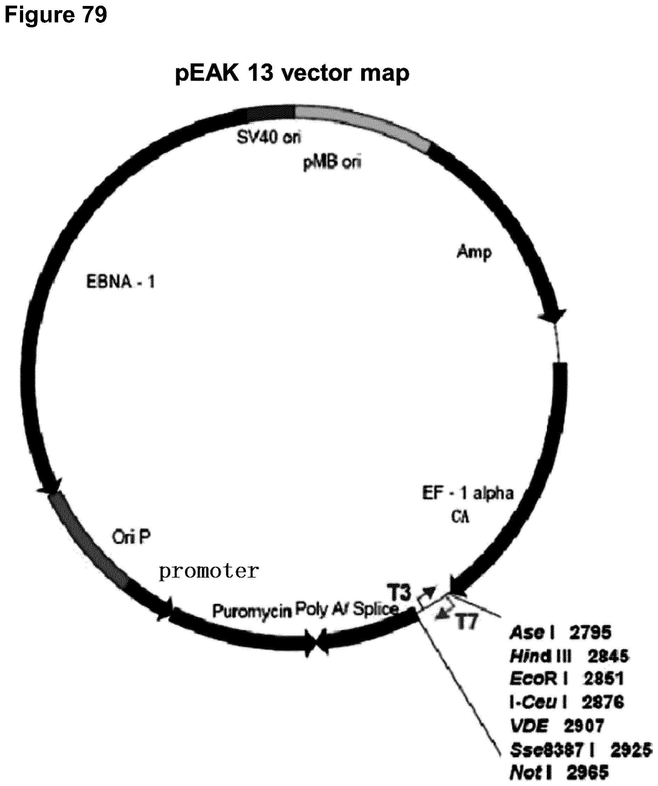

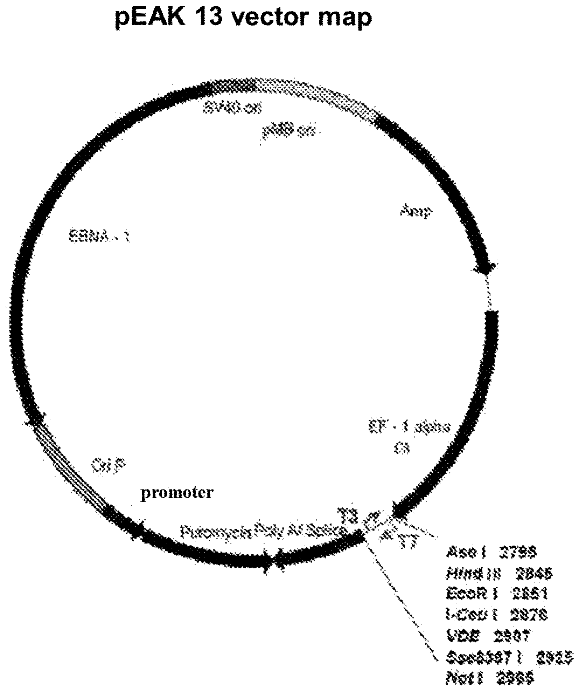

Figure 79: Map of pEAK 13 vector.

### DETAILED DESCRIPTION OF THE INVENTION

The content of the present invention will be further illustrated below with reference to the examples. It should be understood that the following examples are only illustrative and should not be considered as limiting the scope of the present invention.

### Materials

All small RNAs and NS1-GFP plasmids used in the examples:
Small RNA Sequence
PGY-sRNA-23 CCCUCCGCGGCCAGCUUCU
PGY-sRNA-26 UCCGGAAUGAUUGGGCGUAAAGCGU
PGY-sRNA-6 GUUCAGAGUUCUACAGUCCGA

NS1-GFP plasmid is constructed by our laboratory (pEAK13 vector). NS1 is the NS1 protein of influenza virus A/HongKong/97/98(H5N1) (Expressed in the nucleus. NS1-GFP is the N-terminal NS1 directly fused with GFP protein).

EGFP fusion protein: GFP at the N-terminus and the polypeptide is at the C-terminus, directly fused and expressed (PET-28a-EGFP vector).

The coding sequence of EGFP:

Atggtgagcaagggcgaggagctgttcaccggggtggtgcccatcctggtcgagctggacggcgacgtaaacggccacaag
ttcagcgtgtccggcgagggcgagggcgatgccacctacggcaagctgaccctgaagttcatctgcaccaccggcaagctgcccgtg

ccctggcccaccctcgtgaccaccctgacctacggcgtgcagtgcttcagccgctaccccgaccacatgaagcagcacgacttcttcaa
gtccgccatgcccgaaggctacgtccaggagcgcaccatcttcttcaaggacgacggcaactacaagacccgcgccgaggtgaagttc
gagggcgacaccctggtgaaccgcatcgagctgaagggcatcgacttcaaggaggacggcaacatcctggggcacaagctggagta
caactacaacagccacaacgtctatatcatggccgacaagcagaagaacggcatcaaggtgaacttcaagatccgccacaacatcgag
gacggcagcgtgcagctcgccgaccactaccagcagaacacccccatcggcgacggccccgtgctgctgcccgacaaccactacct
gagcacccagtccgccctgagcaaagaccccaacgagaagcgcgatcacatggtcctgctggagttcgtgaccgccgccgggatcac
tctcggcatggacgagctgtacaagtaa (SEQ ID No. 125)

The protein sequence of NS 1 (225 aa):

MDSNTVSSFQVDCFLWHVRKRFADQELGDAPFLDRLRRDQKSLRGRGNTLGLDI
ETATRAGKQIVERILEEESDEALKMPASRYLTDMTLEEMSRDWFMLMPKQKVAGSLC
IKMDQAIMDKTIILKANFSVIFDRLETLILLRAFTEEGAIVGEISPLPSLPGHTGEDVKNAI
GVLIGGLEWNDNTVRVSETIQRFAWRSSDEDGRLPLPPNQKRKMARTIESEV (SEQ ID
No. 126).

### Example 1: General experimental methods

### 1. Preparation of the polypeptide solution

1) Preparation of 1 mM polypeptide stock solution
   The amount of ultrapure water to be added (filtered by a 0.22 µm filter membrane) was calculated according to the mass and relative molecular mass of the synthesized different polypeptides (unlabeled polypeptides, rhodamine-labeled polypeptides and FITC-labeled polypeptides). After dissolution, aliquots of the stock solution were stored in darkness at -80°C.
2) Preparation of different concentrations of polypeptide working solutions
   The polypeptide working solutions were prepared according to the working concentration of different polypeptides, with selection of the corresponding medium as the diluent for different types of cells.

### 2. Entry of polypeptides detected by flow cytometry

### 2.1 Entry of rhodamine- or FITC-labeled polypeptides detected by flow cytometry

1). Cells were digested with 0.05% trypsin and then distributed into the wells of 12-well plates. The plating density was controlled so that the cells grew to about 80%-85% after 24 h. Cells were placed in a 37°C, 5% CO₂ incubator for culture.
2). The cell density was observed after the cells grew for 24 h. The cell culture medium was replaced with the polypeptide working solution prepared with the culture medium. Cells were placed in a 37°C, 5% CO₂ incubator and incubated in the dark for the corresponding durations.
3). The culture medium was discarded. The cells were washed with sterile PBS three times, shaken on a horizontal shaker at 100 rpm for 5 min each time.
4). Cells were digested with 0.05% trypsin, and the trypsin was then discarded. Cells were resuspended in 1 mL sterile PBS and placed in 1.5 mL centrifuge tubes. Cells were centrifuged at 300 g for 10 min at 4°C.
5). The supernatant was discarded. Cells were resuspended in 200 µL sterile PBS.
6). Cell entry of the polypeptides was detected by using a flow cytometer C6.

### 2.2 Cell entry of polypeptides carrying FAM-labeled small RNA detected by flow cytometry

1). A549 cells were digested with 0.05% trypsin and distributed into the wells of 12-well plates. The plating density was controlled so that the cells grew to about 80%-85% after 24 h. Cells were placed in a 37°C, 5% CO₂ incubator for culture.
2). The cell density was observed after the cells grew for 24 h. 30 µM polypeptide and 300 nM FAM-labeled small RNA were mixed and added to the cells. After adding, cells were placed in a 37°C, 5% CO₂ incubator and incubated in the dark for the corresponding durations.
3). The culture medium was discarded. The cells were washed with sterile PBS three times, shaken on a horizontal shaker at 100 rpm for 5 min each time.
4). Cells were digested with 0.05% trypsin, and the trypsin was then discarded. Cells were resuspended in 1 mL sterile PBS and placed in 1.5 mL centrifuge tubes. Cells were centrifuged at 300 g for 10 min at 4°C.
5). The supernatant was discarded. Cells were resuspended in 200 µL sterile PBS.
6). Cell entry of the small RNA was detected by using a flow cytometer C6.

### 2.3. Cell entry of polypeptide-EGFP fusion proteins detected by flow cytometry

1). A549 cells were digested with 0.05% trypsin and distributed into the wells of 12-well plates. The plating density was controlled so that the cells grew to about 80%-85% after 24 h. Cells were placed in a 37°C, 5% CO₂ incubator for culture.
2). The cell density was observed after the cells grew for 24 h. Different concentrations of fusion protein working solutions were prepared with culture medium and added to the cells. After adding, cells were placed in a 37°C, 5% CO₂ incubator and incubated in the dark for the corresponding durations.
3). The culture medium was discarded. The cells were washed with sterile PBS three times, shaken on a horizontal shaker at 100 rpm for 5 min each time.
4). Cells were digested with 0.05% trypsin, and the trypsin was then discarded. Cells were resuspended in 1 mL sterile PBS and placed in 1.5 mL centrifuge tubes. Cells were centrifuged at 300 g for 10 min at 4°C.
5). The supernatant was discarded. Cells were resuspended in 200 µL sterile PBS.
6). Cell entry of the fusion proteins was detected by using a flow cytometer C6.

### 3. Entry of polypeptides detected by confocal laser technology

### 3.1 Cell entry of rhodamine- or FITC-labeled polypeptides detected by confocal laser technology

1). Polylysine-coated cell slides were placed in the wells of 48-well plates and washed twice with the corresponding medium.
2). A549 cells were digested with 0.05% trypsin and distributed into the wells of 48-well plates. The plating density was controlled so that the cells grew to about 80%-85% after 24 h. Cells were placed in a 37°C, 5% CO₂ incubator for culture.
3). The cell density was observed after the cells grew for 24 h. The cell culture medium was replaced to different concentrations of polypeptide working solutions prepared with culture medium. After adding, cells were placed in a 37°C, 5% CO₂ incubator and incubated in the dark for the corresponding durations.
4). The culture medium was discarded. The cells were washed with sterile PBS three times, shaken on a horizontal shaker at 100 rpm for 5 min each time.
5). The PBS was discarded. 4% Paraformaldehyde was added at 200 µL/well and the cells were fixed for 15-20 min at room temperature.
6). The paraformaldehyde was discarded. Cells were washed three times with sterile PBS containing 0.1% triton, shaken on a horizontal shaker at 100 rpm for 5 min each time.
7). The PBS was discarded, a solution containing cytoskeletal F-actin antibody (volume ratio 1:200) and DAPI (volume ratio 1:1000) prepared by using sterile PBS containing 0.1% triton was added at 200 µL/well. The solution was placed in the dark for 30 min at room temperature.
8). The supernatant was discarded. The cells were washed with sterile PBS containing 0.1% triton three times, shaken on a horizontal shaker at 100 rpm for 5 min each time.
9). The slides were mounted and dried. Cell entry of polypeptides was observed by using a Zeiss confocal microscope.

### 3.2 Cell entry of polypeptides carrying Cy3-labeled small RNA detected by confocal laser technology

1). Polylysine-coated cell slides were placed in the wells of 48-well plates and washed twice with the corresponding medium.
2). A549 cells were digested with 0.05% trypsin and distributed into the wells of 48-well plates. The plating density was controlled so that the cells grew to about 80%-85% after 24 h. Cells were placed in a 37°C, 5% CO₂ incubator for culture.
3). After the cells grew for 24 h, the cell culture medium was replaced to a mixed solution of unlabeled polypeptide (30 µM) and Cy3-labeled PGY-6-dsRNA (100 nM) prepared with culture medium. After adding, cells were placed in a 37°C, 5% CO₂ incubator and incubated in the dark for 1 h.
4). The culture medium was discarded. The cells were washed with sterile PBS three times, shaken on a horizontal shaker at 100 rpm for 5 min each time.
5). The PBS was discarded. 4% Paraformaldehyde was added at 200 µL/well and the cells were fixed for 15-20 min at room temperature.
6). The paraformaldehyde was discarded. Cells were washed three times with sterile PBS containing 0.1% triton, shaken on a horizontal shaker at 100 rpm for 5 min each time.
7). The PBS was discarded, a solution containing cytoskeletal F-actin antibody (volume ratio 1:200) and DAPI (volume ratio 1:1000) prepared by using sterile PBS containing 0.1% triton was added at 200 µL/well. The solution was placed in the dark for 30 min at room temperature.
8). The supernatant was discarded. The cells were washed with sterile PBS containing 0.1% triton three times, shaken on a horizontal shaker at 100 rpm for 5 min each time.
9). The slides were mounted and dried. Cell entry of Cy3-labeled small RNA was observed by using a Zeiss confocal microscope.

### 3.3 Cell entry of FITC-labeled polypeptides carrying Cy3-labeled small RNA detected by confocal laser technology

1). Polylysine-coated cell slides were placed in the wells of 48-well plates and washed twice with the corresponding medium.
2). A549 cells were digested with 0.05% trypsin and distributed into the wells of 48-well plates. The plating density was controlled so that the cells grew to about 80%-85% after 24 h. Cells were placed in a 37°C, 5% CO₂ incubator for culture.
3). After the cells grew for 24 h, the cell culture medium was replaced to a mixed solution of FITC-labeled polypeptide (10 µM) and Cy3-labeled PGY-6-dsRNA (400 nM) prepared with culture medium. After adding, cells were placed in a 37°C, 5% CO₂ incubator and incubated in the dark for 1 h.
4). The culture medium was discarded. The cells were washed with sterile PBS three times, shaken on a horizontal shaker at 100 rpm for 5 min each time.
5). The PBS was discarded. 4% Paraformaldehyde was added at 200 µL/well and the cells were fixed for 15-20 min at room temperature.
6). The paraformaldehyde was discarded. Cells were washed three times with sterile PBS containing 0.1% triton, shaken on a horizontal shaker at 100 rpm for 5 min each time.
7). The PBS was discarded, a solution containing DAPI (volume ratio 1:1000) prepared by using sterile PBS containing 0.1% triton was added at 200 µL/well. The solution was placed in the dark for 30 min at room temperature.
8). The supernatant was discarded. The cells were washed with sterile PBS containing 0.1% triton three times, shaken on a horizontal shaker at 100 rpm for 5 min each time.
9). The slides were mounted and dried. Cell entry of FITC-labeled polypeptide and Cy3-labeled small RNA were observed by using a Zeiss confocal microscope.

### 4. Cell entry of polypeptides carrying p-galactosidase observed under the microscope

1). A549 cells were digested with 0.05% trypsin and distributed into the wells of 12-well plates. The plating density was controlled so that the cells grew to about 80%-85% after 24 h. Cells were placed in a 37°C, 5% CO₂ incubator for culture.
2). After the cells grew for 24 h, a mixed solution of polypeptide (50 µM) and β-galactosidase (50 nM) was prepared and added to the cells. Cells were placed for 30 min at 37°C. The cell culture medium was replaced to the mixed solutions prepared with culture medium. After adding, cells were placed in a 37°C, 5% CO₂ incubator and incubated in the dark for 1 h.
3). The staining experiments were performed according to β-galactosidase Staining Kit (Catalog #K802-250) instructions by BioVision.
4). The culture medium was discarded and the cells were washed with sterile PBS once. 500 µL fixing solution was added and the cells were placed for 10-15 min at room temperature.
5). The fixing solution was discarded and the cells were washed with sterile PBS twice.
6). 500 µL staining solution (470 µL staining solution, 5 µL staining supplement, 25 µL 20 mg/ml X-gal dissolved in DMSO) was added. Cells were stained at 37°C overnight.
7). Cell entry of polypeptides carrying β-galactosidase was observed under an inverted microscope.

### 5. Entry of polypeptides carrying small RNA detected by real-time quantitative qPCR

### 5.1 Sample preparation for entry of polypeptide carrying small RNA into A549 cells

1). A549 cells were digested with 0.05% trypsin and distributed into the wells of 12-well plates. The plating density was controlled so that the cells grew to about 80%-85% after 24 h. Cells were placed in a 37°C, 5% CO₂ incubator for culture.
2). After the cells grew for 24 h, a mixed solution of polypeptide (10 µM) and small RNA (100 nM) was prepared in 50 µL DEPC-treated H₂O. Cells were added to each well of 12-well plates, placed in a 37°C, 5% CO₂ incubator and incubated in the dark for 1 h.
3). The culture medium was discarded. The cells were washed with sterile PBS three times, shaken on a horizontal shaker at 100 rpm/min each time.
4). 1 mL TRIzol was added to each well of cells in 12-well plates. Cells were lysed for 10-15 min at room temperature for RNA extraction.

### 5.2 Sample preparation for entry of polypeptide carrying small RNA into murine organs

1). C57 BL/6 male mice (6-8 weeks old and weighed about 20 g) were divided into groups with 4 mice in each group. Each mouse was marked.
2). The amount of ultrapure water to be added (filtered by a 0.22 µm membrane filter) was calculated based on the mass and relative molecular mass of the synthesized polypeptide and the polypeptide was dissolved. Meanwhile, the amount of ultrapure water needed to dissolve the small RNA was calculated based on the number of moles of small RNA. The solution was placed and then mixed well. The next experimental step was carried out after the polypeptide and small RNA were dissolved.
3). The small RNA was mixed at a ratio of 1:1. After mixing, the solution was aliquoted into sterile 1.5 mL EP tubes. Then the polypeptide solution was added to the EP tubes at a P:N ratio of the small RNA to the polypeptide of 1:10. Ultrapure water was added to the mixture to make the injection volume 300 µL per mouse based on the small RNA amount of 3 nmol per mouse. The solution was mixed well and placed for 5 min at room temperature.
4). Mice were sacrificed 1 h after intraperitoneal injection, and the organs were collected, including 8 organs or tissues including brain, heart, liver, spleen, lung, kidney, stomach, small intestine and thymus. The organs were immediately stored in liquid nitrogen.
5). TRIzol LS was added to the mouse blood samples at a ratio of 1:3. The solution was mixed well and placed on ice for lysis. 1 mL TRIzol was added to 2 mL flat-bottom centrifuge tubes, and appropriate amounts of tissues were put into the centrifuge tubes (half of the heart (longitudinally cut), about 0.2 g from porta hepatis of the liver, half of the spleen (cross-cut), the right lobe of the lung, one side of the kidney, half of the brain (longitudinally cut), one side of the testis, all of the thymus, all of the gallbladder, half of the stomach (washed clean) and about 1 cm in length of the small intestine were used for the experiment). The tissues were ground on ice until sufficiently lysed by TRIzol for RNA extraction.

### 5.3 Total RNA extraction of cells or murine tissue samples

1). Chloroform was added to the samples at a volume ratio of 200 µL chloroform/mL TRIzol. The EP tubes were tightly closed and vigorously shaken to mix the samples, placed for 15 min at room temperature and shaken again during this period.
2). The tubes were centrifuged at 13200 g·min⁻¹ for 20 min at 4°C. In the meantime, a new batch of 1.5 mL EP tubes was marked.
3). The upper water phase was slowly pipetted into the corresponding centrifuge tubes. About 540 µL of the upper aqueous phase was drawn from 1 mL TRIzol lysis sample.
4). Pre-cooled isopropanol of 0.8 times the volume of the water phase was added to the upper water phase and mixed well. The solution was placed in a refrigerator for 30 min at -40°C.
5). The tubes were centrifuged at 13200 g·min⁻¹ for 25 min at 4°C. At this time, RNA pellets can be observed adherent to the tube wall. The supernatant was carefully discarded. The lids were opened and the tubes were placed upside down on absorbent paper.
6). 1 mL of 75% ethanol (prepared with DEPC-treated water) was added. The tubes were shaken to suspend the precipitate.
7). The tubes were centrifuged at 13200 g·min⁻¹ for 10 min at 4°C. The supernatant was discarded to the greatest extent. The lids were opened and the tubes were placed upside down on absorbent paper.
8). Steps 6-7 were repeated.
9). Excess liquid at the bottom of the tubes were carefully removed. The tubes were dried at room temperature.
10). The RNA precipitates were dissolved with different volumes of DEPC-treated H₂O according to the size of the RNA precipitates observed. The solutions were thoroughly mixed after the RNA precipitates were completely dissolved.
11). The absorbance value was determined using 1 µL of each RNA sample by NanoDrop 2000 to determine the purity and concentration of the RNA, so as to facilitate the subsequent reverse transcription experiment.

### 5.4 Reverse transcription of RNA to cDNA

1). PCR tubes were labeled according to the number and name of the RNA samples.
2). The reversal transcription system was prepared as follows:

| Components | Volume (µL) |
|---|---|
| 10× RT Buffer | 2.0 |
| 25× dNTP Mix (100 mmol·L⁻¹) | 0.8 |
| Specific primers for small RNA reverse transcription (10 µmol·L⁻¹) | 2.0 |
| MultiScribe™ Reverse Transcriptase | 1.0 |
| RNase Inhibitor | 0.5 |
| Nuclease-free H₂O | Add up to 10 µL |

Specific primers for small RNA (sRNA) reverse transcription should contain all the reverse transcription primers for target sRNAs and U6 as an internal reference. 10 µL of the prepared reverse transcription system was added to each PCR tube, and then 2 µg of the corresponding RNA was added (The volume of RNA added was 10 µL, or was added up to reach 10 µL with nuclease-free H₂O if less than 10 µL, so that the final volume was 20 µL).
4). The procedure below was performed for reverse transcription:

| | |
|---|---|
| 25°C | 10 min |
| 37°C | 120 min |
| 85°C | 5 min |
| 4°C | Pause |

The reverse transcription product cDNA was diluted with sterile H₂O as needed, and then used for fluorescence real-time quantitative PCR experiments.

### 5.5 Small RNA expression detected by real-time quantitative qPCR

1). The real-time fluorescent quantitative PCR reaction system was prepared. Each reaction system should contain the following components:

| Components of the system | Volume |
|---|---|
| Master Mix, 2× conc. | 5 µL |
| Forward primer (10 µmol·L⁻¹ ) | 0.5 µL |
| Reverse primer (10 µmol·L⁻¹ ) | 0.5 µL |
| cDNA Template | 1 µL |
| Sterile H₂O | 3 µL |

2). The total volume required was calculated based on the number of samples. After preparing the mixture, 9 µL of the system was added to each well of the 384-well plate, and then 1 µL cDNA was added.
3). After adding the reaction system to the 384-well plate, the plate was sealed with a sealing film and transiently centrifuged to make the reaction system at the bottom of the wells.
4). The 384-well plate was put on the reaction tray of the instrument. The program template was selected as:

| | | | |
|---|---|---|---|
| a: 95°C | 5 min | | |
| b: 95°C | 10 s | | |
| c: 55°C | 15 s | | |
| d: 72°C | 20 s | fluorescence signal acquisition | b-d cycle for 40 times |

e: Melting point curve reaction program
f: Cooling reaction

5). Information of sample names, genes detected and the like was set when the reaction was in progress. After the reaction was completed, relative quantification mode was selected, and the relative Ct values of the internal reference gene and the target gene were calculated by using the advanced relative compare analysis.
6). Relative expressions of the target genes were calculated according to the formula: Relative gene expression value = 2^{(-(Ct target gene-Ct internal reference)}.

### 6. Functional research of the entry of polypeptides carrying small RNA detected by dual-luciferase reporter system

1). HEK293T cells were digested with 0.05% trypsin and distributed into the wells of 48-well plates. The plating density was controlled so that the cells grew to about 50%-60% after 12 h. Cells were placed in a 37°C, 5% CO₂ incubator for culture.
2). A mixed solution of polypeptide (30 µM) and the two small RNAs (300 nM) respectively (HJT-sRNA-m7 and PGY-sRNA-6) was prepared in 50 µL complete medium. Cells were added to each well of 48-well plates, placed in a 37°C, 5% CO₂ incubator and incubated for 9 h.
3). Wild-type and mutant dual-luciferase reporter gene plasmids with 3' UTR region of *COLLAGEN3A1* gene and 3' UTR region of *RELA* gene were transfected into cells by using Lipo2000 transfection reagent, 0.25 µg/well.
4). The fluorescence intensity of luciferase was detected by Dual-Luciferase Reporter Gene Detection Kit (Promega) 48 h after transfection.

### 7. Functional research of the entry of polypeptides carrying small RNA detected by enzyme-linked immunosorbent assay

### 7.1. Functional research of the cell entry of polypeptides carrying small RNA

1). THP-1 cells were distributed into 12-well plates and placed in a 37°C, 5% CO₂ incubator for culture.
2). A mixed solution of polypeptide (30 µM) and PGY-sRNA-6 (200 nM) was prepared in 50 µL complete medium. Cells were added to each well of 12-well plates, placed in a 37°C, 5% CO₂ incubator and incubated for 24 h.
3). 1 µg/mL lipopolysaccharide (LPS) was administrated to stimulate the cells. Cells were transferred to 1.5 mL EP tubes after 24 h and centrifuged at 1000 rpm for 5min at 4°C. The cell supernatant was transferred to new 1.5 mL EP tubes and 100× cocktail was added. Expression levels of the inflammatory cytokines (TNF-α and IL-1β) were detected by using ELISA.
4). Coating: self-coating ELISA plates (R&D, IL-1β DY201-05, TNF-α DY210-05) were coated with PBS-diluted Capture Antibody (IL-1 and TNF-α) (the dilution ratio was selected according to the instructions) at room temperature overnight (about 16-18 h);
5). Plate washing: the coated ELISA plates were taken. The Capture Antibody solution was discarded and the remaining liquid was pat dry on filter paper. Then 300 µL of the prepared washing solution (PBS+0.1% tween 20) was added for washing and stayed for 1 min each time (using an ELISA plate shaker). The liquid was discarded and the remaining liquid was pat dry on filter paper each time as well (the same below), and the plates were washed 4 times;
6). Blocking: after washing, 300 µL blocking solution (PBS+1% BSA) was added and incubated for 1 h at room temperature;
7). Preparation: the corresponding standards (IL-1, TNF-α) were prepared within 1 h. The highest concentration standard was prepared according to the instructions, and was then diluted according to a concentration gradient of 1/2. The standards were diluted 7 times, and finally the diluting solution was added to the eighth tube as the 0 tube;
8). Plate washing: the plates were incubated for 1 h and washed with washing solution 4 times;
9). Sample addition: the prepared standards were added to the left and right rows of the ELISA plate, and the samples were added to other wells. The plate was incubated for 2 h at room temperature;
10). Primary antibody addition: the plate was washed 4 times with washing solution. 100 µL Detection Antibody was added and the plate was sealed and incubated for 2 h at room temperature;
11). Secondary antibody addition: the plate was washed 4 times with washing solution. 100 µL Avidin-HRP was added and the plate was sealed and incubated for 20 min at room temperature;
12). Substrate addition: the plate was washed 4 times with washing solution. 100 µL TMB Substrate Solution was added in the dark. The plate was then immediately put in a drawer for about 10-20 min in the dark. After the solution color turned blue, 100 µL stop solution was added to stop the reaction and the color turned from blue to yellow;
13). Absorbance measurement was performed within 30 min at 450 nm detection wavelength and 570 nm reference wavelength.

### 7.2. Functional research of entry of polypeptides carrying small RNA into murine organs

1). C57 BL/6 male mice (6-8 weeks old and weighed about 20 g) were divided into groups with 4 mice in each group. Each mouse was marked. The amount of ultrapure water to be added (filtered by a 0.22 µm membrane filter) was calculated based on the mass and relative molecular mass of the synthesized polypeptide and the polypeptide was dissolved. Meanwhile, the amount of ultrapure water needed to dissolve the small RNA was calculated based on the number of moles of small RNA. The solution was placed and then mixed well. The next experimental step was carried out after the polypeptide and small RNA were dissolved.
2). Solutions of PGY-sRNA-6 and the polypeptide were added into EP tubes. Ultrapure water was added to the mixture to obtain the injection volume 300 µL per mouse based on the small RNA amount of 10 nmol per mouse. The solution was mixed well and placed for 5 min at room temperature.
3). After two intraperitoneal injections 24 h in advance and 1 h in advance, poly(I:C) was dissolved in PBS to prepare a stock solution with a concentration of 10 mg/mL under aseptic conditions. Each mouse was administrated a dose of 500 µg poly(I:C), and each aliquot was 50 µL/tube. The acute lung injury model was established by tracheal drip method. Mice were sacrificed after 6 h, and the alveolar lavage fluid was collected for testing.
4). Coating: self-coating ELISA plates (R&D, IL-6 DY406, TNF-α DY410) were coated with PBS-diluted Capture Antibody (IL-6 and TNF-α) (the dilution ratio was selected according to the instructions) at room temperature overnight (about 16-18 h);
5). Plate washing: the coated ELISA plates were taken. The Capture Antibody solution was discarded and the remaining liquid was pat dry on filter paper. Then 300 µL of the prepared washing solution (PBS+0.1% tween 20) was added for washing and stayed for 1 min each time (using an ELISA plate shaker). The liquid was discarded and the remaining liquid was pat dry on filter paper each time as well (the same below), and the plates were washed 4 times;
6). Blocking: after washing, 300 µL blocking solution (PBS+1% BSA) was added and incubated for 1 h at room temperature;
7). Preparation: the corresponding standards (IL-6 and TNF-α) were prepared within 1 h. The highest concentration standard was prepared according to the instructions, and was then diluted according to a concentration gradient of 1/2. The standards were diluted 7 times, and finally the diluting solution was added to the eighth tube as the 0 tube;
8). Plate washing: the plates were incubated for 1 h and washed with washing solution 4 times;
9). Sample addition: the prepared standards were added to the left and right rows of the ELISA plate, and the samples were added to other wells. The plate was incubated for 2 h at room temperature;
10). Primary antibody addition: the plate was washed 4 times with washing solution. 100 µL Detection Antibody was added and the plate was sealed and incubated for 2 h at room temperature;
11). Secondary antibody addition: the plate was washed 4 times with washing solution. 100 µL Avidin-HRP was added and the plate was sealed and incubated for 20 min at room temperature;
12). Substrate addition: the plate was washed 4 times with washing solution. 100 µL TMB Substrate Solution was added in the dark. The plate was then immediately put in a drawer for about 10-20 min in the dark. After the solution color turned blue, 100 µL stop solution was added to stop the reaction and the color turned from blue to yellow;
13). Absorbance measurement was performed within 30 min at 450 nm detection wavelength and 570 nm reference wavelength.

### 8. Functional research of the cell entry of polypeptides carrying small RNA detected by Western blot

### 8.1 Sample collection for entry of polypeptide carrying small RNA into MRC-5 cells

1). MRC-5 cells were distributed into 12-well plates and placed in a 37°C, 5% CO₂ incubator for culture.
2). A mixed solution of polypeptide (10 µM) and HJT-sRNA-m7 (300 nM) was prepared in 50 µL complete medium. Cells were added to each well of 12-well plates, placed in a 37°C, 5% CO₂ incubator and incubated for 24 h.
3). 3 ng/mL transforming growth factor (TGF) β1 was administrated as the stimulant to stimulate MRC-5 cells. After 72 h of incubation, cells were collected by using strong RIPA lysis buffer for the protein expression level detection of related genes.

### 8.2 Western blot

A. Gel preparation: 10% concentration separation gel (lower layer gel) and 5% concentration stacking gel (upper layer gel) were used. Lanes were made by using 15-hole combs, and the same amount of protein sample was loaded to each lane;
B. Protein electrophoresis: electrophoresis buffer was added and the initial voltage of electrophoresis was 80 V; the voltage was elevated to 120 V when the bromophenol blue dye reached the separation gel, and electrophoresis was continued until the bromophenol blue dye reached the bottom of the separation gel or run beyond the gel;
C. Wet transfer: the apparatus was installed following the order of transfer splint (negative)-sponge-filter paper-gel-PVDF membrane-filter paper-sponge-transfer splint (positive); after installation, the entire transfer apparatus was placed in a 4°C cold room; constant current 300 mA, the membrane was subjected to transfer for 120 min;
D. Blocking: after transfer, the membrane was placed in 3% BSA blocking solution and blocked for 1 h at room temperature;
E. Primary antibody incubation: the blocked PVDF membrane was transferred to the hybridization bag. 3% BSA blocking solution containing the corresponding primary antibody (the information of the primary antibody was as follows) was added to the bag, and the air bubbles were removed. The bag was sealed and incubated at 4°C overnight;
   Fibronectin antibody (Sigma F7387)
   Actin antibody (Sigma A5441)
F. Membrane washing: the PVDF membrane was taken and washed 3 times with TBST, 10 min each time;
G. Secondary antibody incubation: TBST was discarded and 3% BSA blocking solution containing horseradish peroxidase (HRP) conjugated goat anti-rabbit or goat anti-mouse secondary antibody (purchased from Hangzhou Multi Sciences Biotechnology Co., Ltd.) (secondary antibody dilution ratio 1:5000) was added and incubated for 1 h at room temperature;
H. Membrane washing: the membrane was washed 3 times with TBST, 10 min each time;
I. Development: Western chromogenic solution (1:1, V/V, Merck Millipore, enhanced chemiluminescence (ECL) solution purchased from Millipore) was prepared and evenly added on the side of membrane-bound protein dropwise; the membrane was wrapped carefully with plastic wrap and observed after the color developed;
J. Analysis: Image J software was used for analysis.

### 9. Cell entry and expression of GFP plasmid carried by polypeptide detected by confocal laser technology

1). Polylysine-coated cell slides were placed in the wells of 48-well plates and washed twice with the corresponding medium.
2). HEK293T cells were digested with 0.05% trypsin and distributed into the wells of 48-well plates. The plating density was controlled so that the cells grew to about 50%-60% after 12 h. Cells were placed in a 37°C, 5% CO₂ incubator for culture.
3) After the cells grew for 12 h, the cell culture medium was replaced to the mixed solution of polypeptide and plasmid (0.25 µg/well) prepared with complete culture medium, N/P = 400:1. After adding, cells were placed in a 37°C, 5% CO₂ incubator and incubated in the dark for 48 h.
4). The culture medium was discarded and the cells were washed with sterile PBS three times.
5). The PBS was discarded. 4% Paraformaldehyde was added at 200 µL/well and the cells were fixed for 15-20 min at room temperature.
6). The paraformaldehyde was discarded and the cells were washed with sterile PBS three times.
7). The PBS was discarded, the slides were mounted, dried and observed for the expression of GFP green fluorescent protein using a Zeiss confocal microscope.

### 10. Functional research of entry of polypeptide-EGFP fusion protein into murine organs detected by immunohistochemistry

1). C57 BL/6 male mice (6-8 weeks old and weighed about 20 g) were divided into groups with 4 mice in each group. Each mouse was marked.
2). The protein dilution ratio was calculated according to the storage concentration and relative molecular weight of the fusion protein, so that each 360 µL protein solution contained 1 mg of EGFP fusion protein.
3). Each mouse was intraperitoneally injected with 360 µL of PBS or PBS containing 1 mg of fusion protein.
4). Mice were sacrificed after 1 h and the murine organs (liver, spleen, kidney and brain) were collected and embedded in paraffin, and paraffin section was performed.
5). Dewaxing
   a. The slices were put on a shelf and rinsed in sequence according to the following methods:
      Xylene: 2 × 3 min
      mixture of 1:1 xylene and absolute ethanol: 3 min
      Absolute ethanol: 2 × 3 min
      95% ethanol: 3 min
      70% ethanol: 3 min
      50% ethanol: 3 min
      Washed by cold running water
   b. Before antigen retrieval, the slices were immersed in water without drying out, as drying out would cause non-specific antibody binding to produce high background.
6). Antigen retrieval
   a. The sections were deparaffinized to water as described above.
   b. The cooking boiler was set and preheated according to the user instructions.
   c. A suitable repair buffer was preheated in a flask and boiled (heating in a microwave oven was more convenient).
   d. The container to hold the slide rack was placed in the cooking boiler.
   e. The thermal remediation buffer was carefully added to the container and then the slide rack was put into the container. A simpler operation may also be used: the thermal remediation buffer was first put into the container and then they were together put into the cooking boiler.
   f. Capping. The container containing the buffer must also be equipped with a lid. The slide rack may lower the temperature of the remediation buffer at the beginning, but it would rise back to 95-100°C within a few minutes.
   g. The temperature was maintained for 20 min after reaching the desired temperature.
   h. After 20 min, the container was taken out and placed in water to cool for 10 min.
7). Blocking
   (1) The slides were rinsed for 2 × 5 min in TBS containing 0.025% Triton X-100 and with gentle shaking.
   (2) The slides were blocked with TBS containing 10% normal serum and 1% BSA for 2 h at room temperature. The blocking solution was drained (not rinsed) and the area around the slide was wiped dry with paper towels.
8). Primary antibody incubation
   a. The primary antibody was diluted with TBS containing 1% BSA and added to the slides.
   b. The slides were incubated at 4 °C overnight.
9). Secondary antibody incubation
   a. The slides were rinsed for 2 × 5 min in TBS containing 0.025% Triton X-100 and with gentle shaking.
   b. The slides were rinsed for 2 × 5 min in TBS containing 0.025% Triton X-100 and with gentle shaking.
   c. The slides were rinsed for 3 × 5 min in TBS.
   d. The slides were mounting by using mounting medium and coverslips.
10). EGFP fluorescence intensity was observed by using a Zeiss confocal microscope, and ImageJ software was used for statistics.

### 11. Functional research of the entry of polypeptides carrying small molecule drug silybin and small RNA antagomir into murine organs detected by enzyme-linked immunosorbent assay

### 11.1. Functional research of the entry of polypeptides carrying small molecules into murine organs

1). C57 BL/6 male mice (6-8 weeks old and weighed about 20 g) were divided into groups with 4 mice in each group. Each mouse was marked. The amount of ultrapure water to be added (filtered by a 0.22 µm membrane filter) was calculated based on the mass and relative molecular mass of the synthesized polypeptide and the polypeptide was dissolved. The small molecule drug was dissolved in DMSO. The solution was placed and then mixed well. The next experimental step was carried out after the peptide and small RNA were dissolved.
2). 10 mg/kg polypeptide solution and small molecule drug silybin (100 mg/kg) were added into EP tubes, and finally ultrapure water was added to the mixture to make the intragastric volume of each mouse 200 µL. The solution was mixed well and placed for 5 min at room temperature.
3). 48 h in advance, 24 h in advance and 1 h in advance, after three intragastric administrations, LPS was dissolved in PBS to prepare a stock solution with a concentration of 1 mg/mL under aseptic conditions. The acute lung injury model was established by tracheal drip method, with a dosage of 50 µL per mouse. Mice were sacrificed after 9 h, and the alveolar lavage fluid was collected for detection.

### 11.2. Functional research of entry of polypeptides carrying small RNA antagomir into murine organs

1). C57 BL/6 male mice (6-8 weeks old and weighed about 20 g) were divided into groups with 4 mice in each group. Each mouse was marked. The amount of ultrapure water to be added (filtered by a 0.22 µm membrane filter) was calculated based on the mass and relative molecular mass of the synthesized polypeptide and the polypeptide was dissolved. The small molecule drug was dissolved in DMSO. The solution was placed and then mixed well. The next experimental step was carried out after the peptide and small RNA were dissolved.
2). 10 mg/kg polypeptide solution and miR-1246 antagomir (20 µg/mouse) were added into EP tubes, and finally ultrapure water was added to the mixture to make the injection volume of each mouse 100 µL. The solution was mixed well and placed for 5 min at room temperature.
3). After two intraperitoneal injections 24 h in advance and 1 h in advance, LPS was dissolved in PBS to prepare a stock solution with a concentration of 1 mg/mL under aseptic conditions. The acute lung injury model was established by tracheal drip method, with a dosage of 50 µL per mouse. Mice were sacrificed after 9 h, and the alveolar lavage fluid was collected for detection.

### Example 2: Cell entry of polypeptide sequences derived from the influenza virus M2 protein

Based on the sequence of the M2 protein of the Caledonia/20/1999 (H1N1) and A/HongKong/97/98 (H5N1) strains of influenza viruses, the peptide sequences listed in Table 1 were synthesized, and TAT HIV was used as a positive control of cell-penetrating peptides. In order to facilitate the detection of the cell entry of the polypeptides, rhodamine tags were used to label the polypeptides at the N-terminus.

**Table 1. Rhodamine-labeled polypeptide sequences**

| Polypeptide | Sequence |
|---|---|
| NC (negative control) | Rhodamine B-ERGLQRRRFVQNALN (Seq ID No. 1) |
| TAT HIV (positive control) | Rhodamine B-GRKKRRQRRRPPQ (Seq ID No. 2) |
| Caledonia/20/1999(H1N1)-M2-N-M(44-97) | B-DRLFSKSIYRIFKHGLKRGPSTEGVPESMREEYRE EQQNAVDADDGHFVSIELK (Seq ID No. 3) Rhodamine |
| Caledonia/20/1999 (H1N1)-M2-N-M-AH(45-62) | Rhodamine B-RLFSKSIYRIFKHGLKRG (Seq ID No. 4) |
| Caledonia/20/1999 (H1N1)-M2-N-M-AH(60-82) | Rhodamine B-KRGPSTEGVPESMREEYREEQQN (Seq ID No.5) |
| A/HongKong/97/98(H5N1)-M2-N-M-(44-97) | B-DRLFFKCIYRRLKYGLKRGPSTAGVPESMREEYR QEQQNAVDVDDGHFVNIELE (Seq ID No. 6) Rhodamine |
| A/HongKong/97/98(H5N1)-M2-N-M-AH(4 5-62) | Rhodamine B-RLFFKCIYRRLKYGLKRG (Seq ID No. 7) |
| A/HongKong/97/98(H5N1)-M2-N-M-AH(6 0-82) | Rhodamine B-KRGPSTAGVPESMREEYRQEQQN (Seq ID No. 8) |

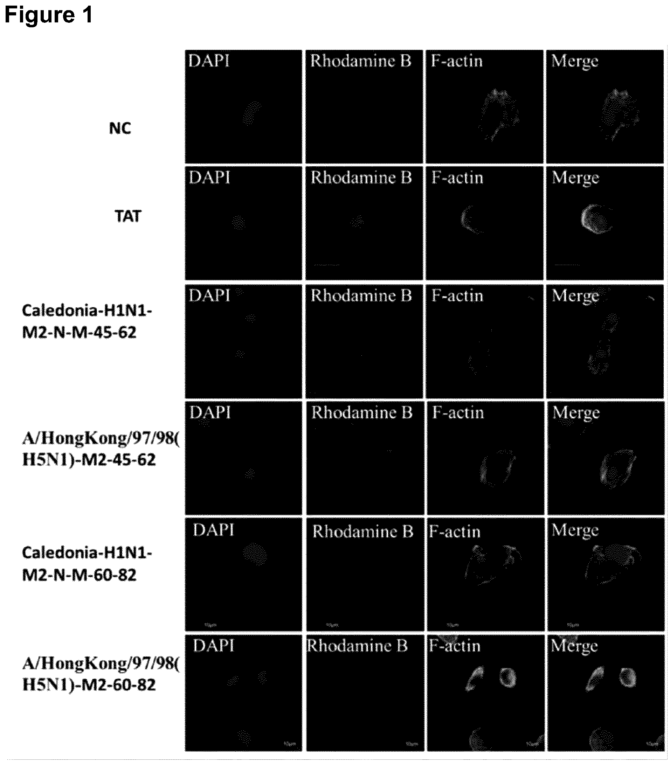

The results of the confocal laser experiment of cell entry of polypeptides are shown in Figure 1. Rhodamine-labeled Caledonia/20/1999(H1N1)-M2-N-M-AH(45-62) and A/HongKong/97/98(H5N1)-M2-N-M-AH(45-62) polypeptides and the positive control TAT polypeptide could all enter A549 cells at a concentration of 30 µM. Meanwhile, rhodamine-labeled Caledonia/20/1999(H1N1)-M2-N-M-AH(60-82) and A/HongKong/97/98(H5N1)-M2-N-M-AH(60-82) peptides did not show detectable signal in A549 cells at a concentration of 30 µM, i.e. they did not enter the cells. The above results showed that the influenza virus M2 protein had the function of a cell-penetrating peptide, and its cell-penetrating peptide function was related to the amino acids 45-62 fragment.

### Example 3: Identification of the functional fragment of cell-penetrating peptide of the M2 protein

In order to determine the preferred peptide range of the cell-penetrating peptide properties of the M2 protein, the polypeptide sequences shown in Table 2 were synthesized.based on the M2 protein sequence of A/chicken/Jilin/9/2004 (H5N1) (which is the highly conserved with the A/HongKong/97/98(H5N1)-M2 used in Example 2). Its sequence consisting of 97 amino acids was synthesized in sections according to the structure of the M2 protein. In order to facilitate the detection of cell entry of the polypeptides, FITC was used to label the polypeptides at the N-terminus.

**Table 2. FITC-labeled polypeptide sequences based on M2 protein**

| Polypeptide | Sequence |
|---|---|
| FITC-NC (negative control) | FITC-Ahx-ERGLQRRRFVQNALN (Seq ID No. 1) |
| FITC-TAT (positive control) | FITC-Ahx-GRKKRRQRRRPPQ (Seq ID No. 2) |
| FITC-Jilin-2004(H5N1)-M2-45 -62 | FITC-Ahx-RLFFKCIYRRLKYGLKRG (Seq ID No. 7) |
| FITC-Jilin-2004(H5N1)-M2-60-82 | FITC-Ahx-KRGPSTEGVPESMREEYRQEQQS(Seq ID No. 9) |
| FITC-Jilin-2004(H5N1)-M2-1-24 | FITC-Ahx-MSLLTEVETPTRNGWECRCSDSSD (Seq ID No. 10) |
| FITC-Jilin-2004(H5N1)-M2-25 -43 | FITC-Ahx-PLVVAASIIGILHLILWIL (Seq ID No. 11) |
| FITC-Jilin-2004(H5N1)-M2-44-61 | FITC-Ahx-DRLFFKCIYRRLKYGLKR (Seq ID No. 12) |
| FITC-Jilin-2004(H5N1)-M2-47-64 | FITC-Ahx-FFKCIYRRLKYGLKRGPS (Seq ID No.l3) |
| FITC-Jilin-2004(H5N1)-M2-5 0-67 | FITC-Ahx-CIYRRLKYGLKRGPSTEG (Seq ID No. 14) |
| FITC-Jilin-2004(H5N1)-M2-53-70 | FITC-Ahx-RRLKYGLKRGPSTEGVPE (Seq ID No. 15) |
| FITC-Jilin-2004(H5N1)-M2-5 6-73 | FITC-Ahx-KYGLKRGPSTEGVPESMR (Seq ID No. 16) |
| FITC-Jilin-2004(H5N1)-M2-59-76 | FITC-Ahx-LKRGPSTEGVPESMREEY (Seq ID No. 17) |
| FITC-Jilin-2004(H5N1)-M2-62-79 | FITC-Ahx-GPSTEGVPESMREEYRQE (Seq ID No. 18) |
| FITC-Jilin-2004(H5N1)-M2-65-82 | FITC-Ahx-TEGVPESMREEYRQEQQS (Seq ID No. 19) |

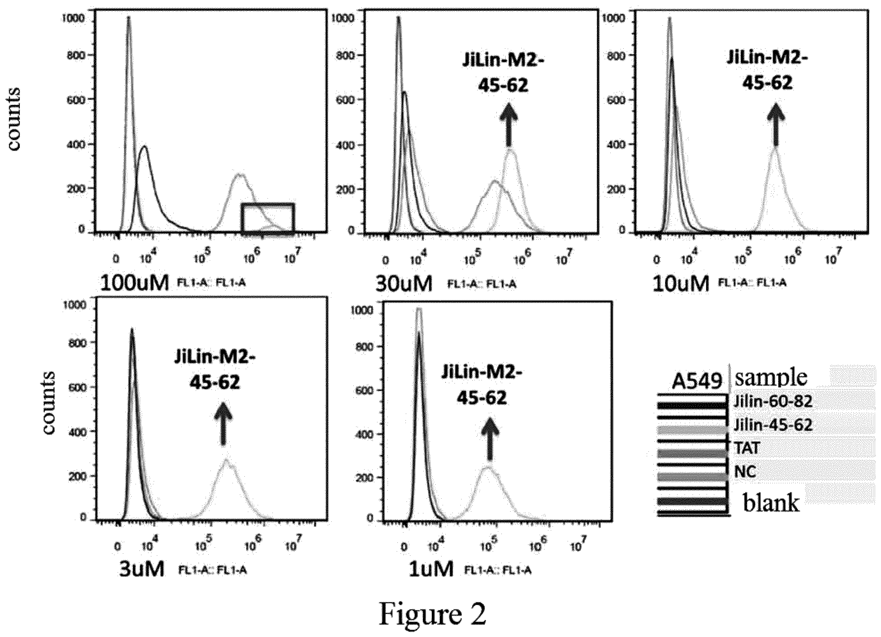

First, the cell entry function of FITC-labeled Jilin-2004(H5N1)-M2-45-62 polypeptide at different concentrations (100 µM, 30 µM, 10 µM, 3 µM and 1 µM) was tested. The results are shown in Figure 2. From these results, it can be seen that the Jilin-2004(H5N1)-M2-45-62 polypeptide could achieve significant A549 cell entry at all concentrations tested, and its entry effect was much stronger than FITC-labeled TAT polypeptide (positive control). It is noticed that the TAT polypeptide did not show cell entry at a concentration of 3 µM, while the FITC-labeled Jilin-2004(H5N1)-M2-45-62 polypeptide still had a significant cell entry effect at a concentration as low as 1 µM. The above results showed that the Jilin-2004(H5N1)-M2-45-62 polypeptide based on influenza virus M2 protein had a much stronger cell penetration function than the positive control TAT, enabling it to achieve cell entry even at low concentrations.

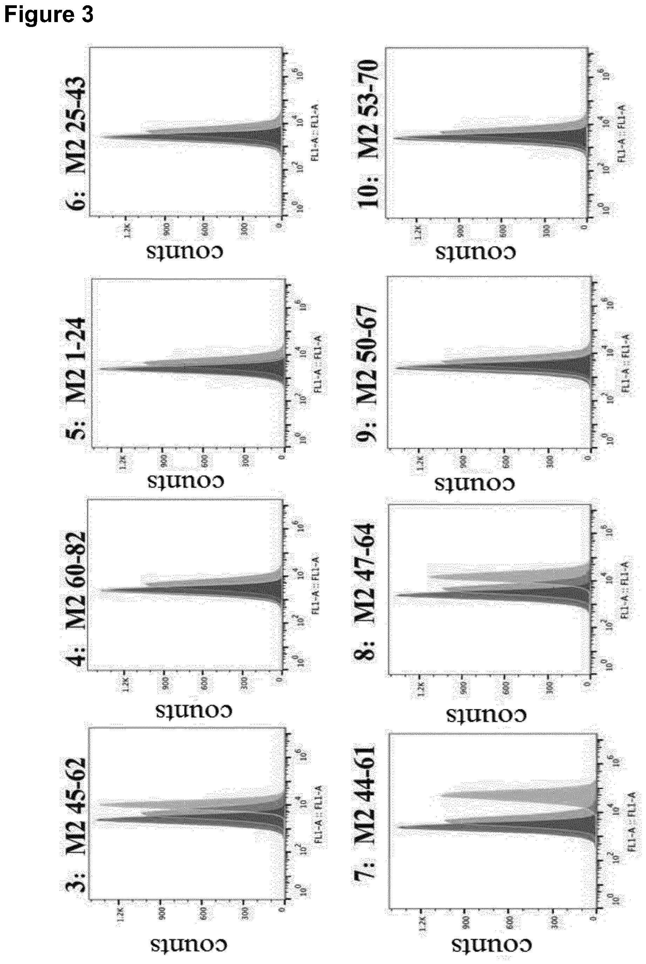

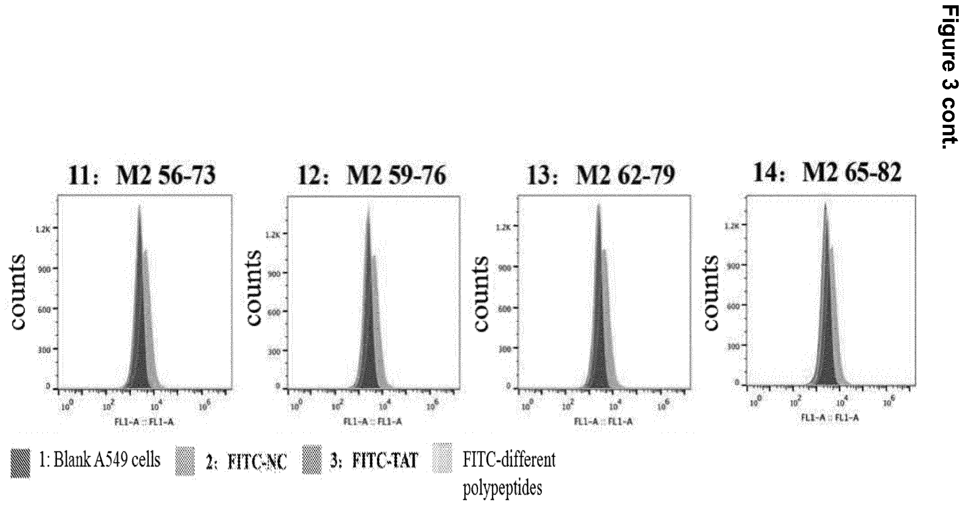

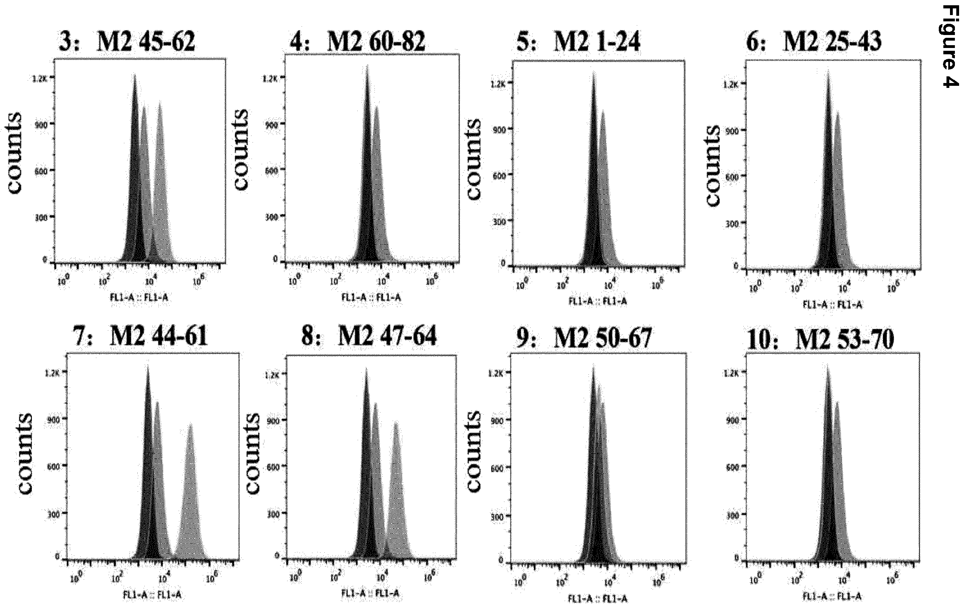

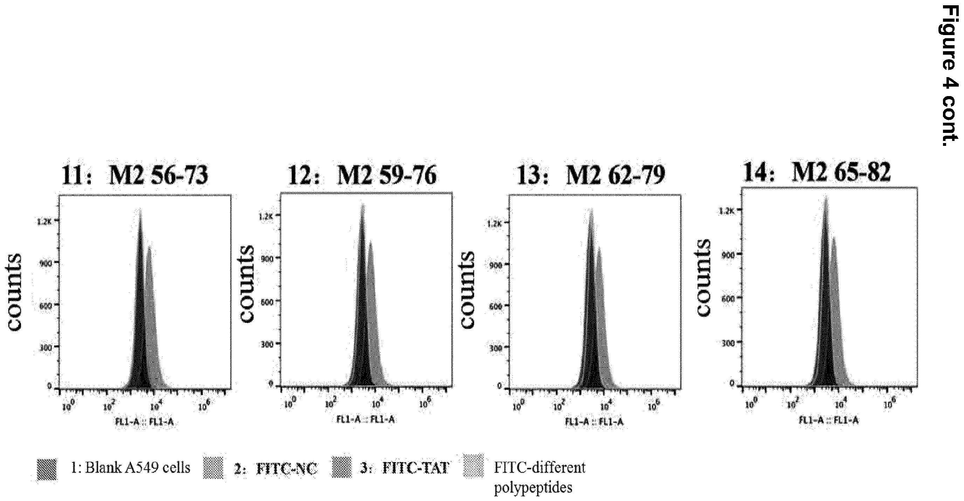

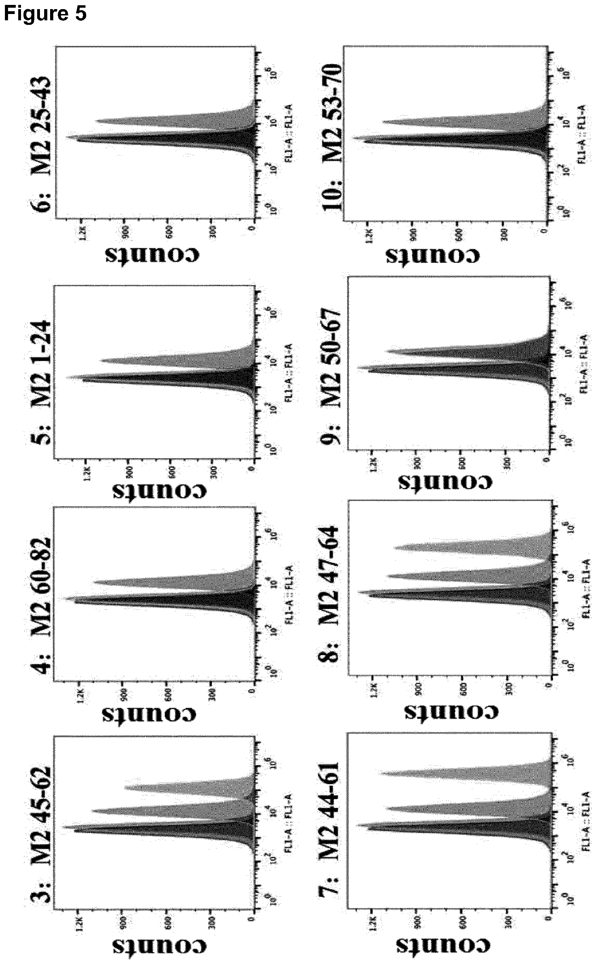

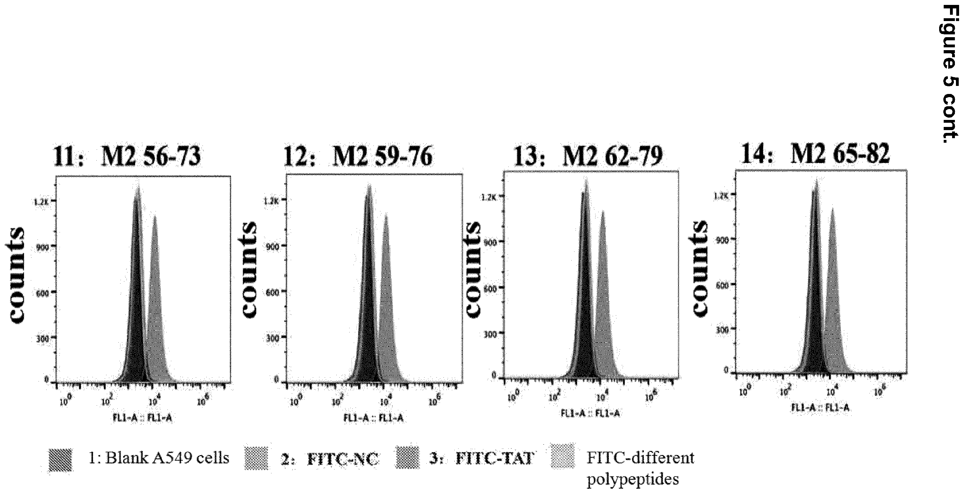

The cell entry function of all synthetic FITC-labeled polypeptides in Table 2 was tested. The peptide concentrations used were 1 µM, 3 µM, and 10 µM, and the flow cytometry results are shown in Figure 3, Figure 4 and Figure 5, respectively. Among them, when using a peptide with a concentration of 1 µM, the results are shown in Figure 3. Jilin-2004(H5N1)-M2-45-62, Jilin-2004(H5N1)-M2-44-61 and Jilin-2004(H5N1) -M2-47-64 entered A549 cells after 1 h of incubation. When using a peptide with a concentration of 3 µM or 10 µM, the results are shown in Figure 4 and Figure 5, respectively. Jilin-2004(H5N1)-M2-45-62, Jilin-2004(H5N1)-M2-44-61, Jilin-2004(H5N1)-M2-47-64 and Jilin-2004(H5N1)-M2-50-67 entered A549 cells after 1 hour of incubation. In addition, it was also observed that except for Jilin-2004(H5N1)-M2-50-67, the cell entry effects of the other three polypeptides were stronger than that of the TAT polypeptide as a positive control. The above results showed that the cell-penetrating peptide function of the M2 protein was related to amino acids 44-67.

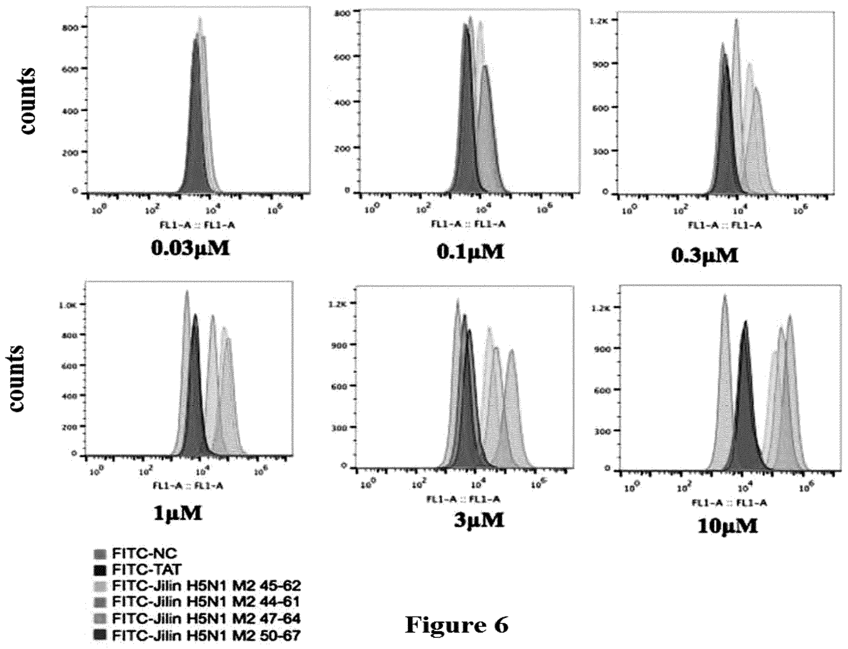

The cell entry effects of the above four polypeptides with cell penetrating function (Jilin-2004(H5N1)-M2-45-62, Jilin-2004(H5N1)-M2-44-61, Jilin-2004(H5N1)-M2-47-64 and Jilin-2004(H5N1)-M2-50-67) at lower concentrations were further tested. Concentrations of 0.03 µM, 0.1 µM, 0.3 µM, 1 µM, 3 µM and 10 µM were used for detection, and the results are shown in Figure 6. These result showed that when the concentration of the polypeptides was reduced to 0.1 µM and 0.03 µM, the polypeptides could still enter A549 cells, and the Jilin-2004(H5N1)-M2-44-61 polypeptide was a polypeptide with even better cell entry effect.

In addition, when the N-terminal polypeptide Jilin-2004(H5N1)-M2-1-24 and the cross-terminal polypeptide Jilin-2004(H5N1)-M2-25-43 were used, no cell entry signal was detected. In addition, the intracellular peptide fragment of the M2 protein FITC-Jilin-2004(H5N1)-M2-53-70, FITC-Jilin-2004(H5N1)-M2-56-73, FITC-Jilin-2004(H5N1)-M2-59-76, FITC-Jilin-2004(H5N1)-M2-62-79 and FITC-Jilin-2004(H5N1)-M2-65-82 all did not show cell entry properties.

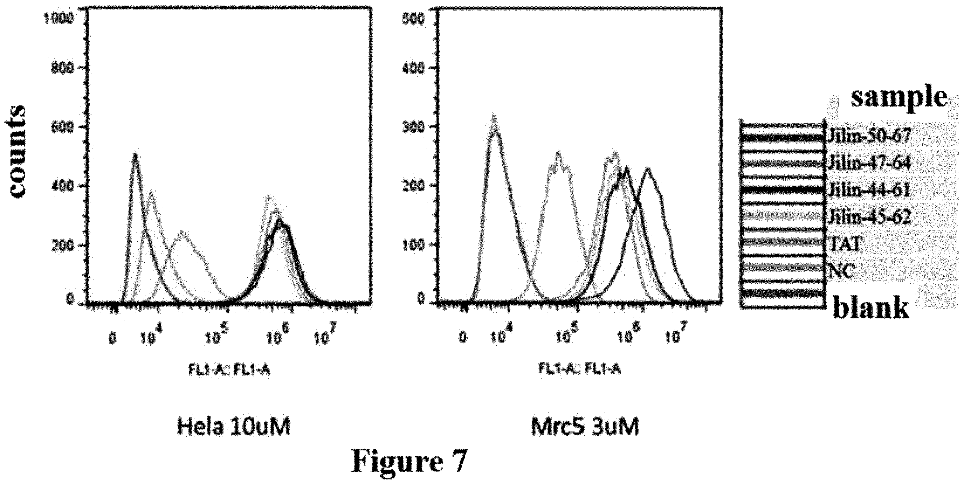

In order to verify whether M2 related polypeptides have cell penetrating properties in other cell types, HeLa cells and MRC5 cells were used to test the cell penetration properties of Jilin-2004(H5N1)-M2-45-62, Jilin-2004(H5N1)-M2-44-61, Jilin-2004(H5N1)-M2-47-64 and Jilin-2004(H5N1)-M2-50-67 polypeptides. The results are shown in Figure 7. The above results are consistent with the results obtained using A549 cells, indicating that the cell-penetrating peptide function of M2 related polypeptides is not limited to specific cells, but has a certain generality.

### Example 4: Identification of preferred polypeptide fragments

According to the results in Example 2 and Example 3, the cell-penetrating peptide properties of the M2 protein was mainly focused in fragment 44-67. Therefore, the sequentially truncated N-terminal and C-terminal fragments of the above fragments were further synthesized (Table 3). The preferred polypeptide fragments were identified by detecting the cell entry function of the polypeptide fragments shown in Table 3.

**Table 3: Truncated fragments of the M2 protein 44-67 sequence**

| Polypeptide | Sequence |
|---|---|
| FITC-Jilin-2004(H5N1)-M2-48-67 | FITC-Ahx-FKCIYRRLKYGLKRGPSTEG (Seq ID No. 20) |
| FITC-Jilin-2004(H5N1)-M2-47-67 | FITC-Ahx-FFKCIYRRLKYGLKRGPSTEG (Seq ID No. 21) |
| FITC-Jilin-2004(H5N1)-M2-46-67 | FITC-Ahx-LFFKCIYRRLKYGLKRGPSTEG (Seq ID No. 22) |
| FITC-Jilin-2004(H5N1)-M2-45 -67 | FITC-Ahx-RLFFKCIYRRLKYGLKRGPSTEG (Seq ID No. 23) |
| FITC-Jilin-2004(H5N1)-M2-44-67 | FITC-Ahx-DRLFFKCIYRRLKYGLKRGPSTEG (Seq ID No. 24) |
| FITC-Jilin-2004(H5N1)-M2-44-66 | FITC-Ahx-DRLFFKCIYRRLKYGLKRGPSTE (Seq ID No. 25) |
| FITC-Jilin-2004(H5N1)-M2-44-65 | FITC-Ahx-DRLFFKCIYRRLKYGLKRGPST (Seq ID No. 26) |
| FITC-Jilin-2004(H5N1)-M2-44-64 | FITC-Ahx-DRLFFKCIYRRLKYGLKRGPS (Seq ID No. 27) |
| FITC-Jilin-2004(H5N1)-M2-44-63 | FITC-Ahx-DRLFFKCIYRRLKYGLKRGP (Seq ID No. 28) |
| FITC-Jilin-2004(H5N1)-M2-44-62 | FITC-Ahx-DRLFFKCIYRRLKYGLKRG (Seq ID No. 29) |
| FITC-Jilin-2004(H5N1)-M2-44-61 | FITC-Ahx-DRLFFKCIYRRLKYGLKR (Seq ID No. 30) |
| FITC-Jilin-2004(H5N1)-M2-44-60 | FITC-Ahx-DRLFFKCIYRRLKYGLK (Seq ID No. 31) |
| FITC-Jilin-2004(H5N1)-M2-44-59 | FITC-Ahx-DRLFFKCIYRRLKYGL (Seq ID No. 32) |
| FITC-Jilin-2004(H5N1)-M2-44-58 | FITC-Ahx-DRLFFKCIYRRLKYG (Seq ID No. 33) |
| FITC-Jilin-2004(H5N1)-M2-44-57 | FITC-Ahx-DRLFFKCIYRRLKY (Seq ID No. 34) |
| FITC-Jilin-2004(H5N1)-M2-44-56 | FITC-Ahx-DRLFFKCIYRRLK (Seq ID No. 35) |
| FITC-Jilin-2004(H5N1)-M2-44-55 | FITC-Ahx-DRLFFKCIYRRL (Seq ID No. 36) |
| FITC-Jilin-2004(H5N1)-M2-44-54 | FITC-Ahx-DRLFFKCIYRR (Seq ID No. 37) |
| FITC-Jilin-2004(H5N1)-M2-44-53 | FITC-Ahx-DRLFFKCIYR (Seq ID No. 38) |

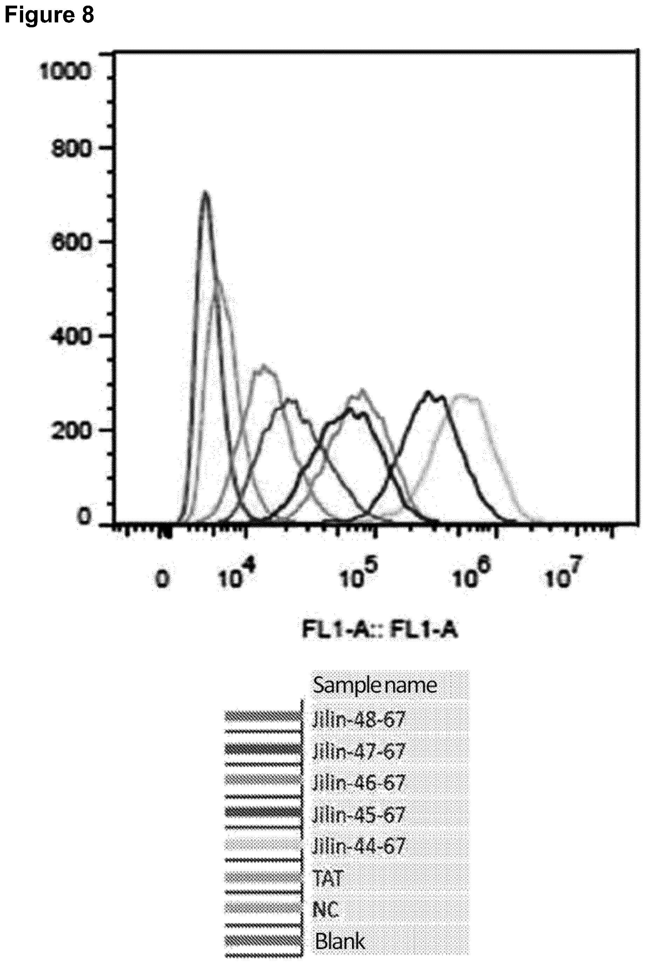

Cell entry properties of the above polypeptides were detected by using flow cytometry. The data of polypeptides truncated sequentially from the N-terminus is shown in Figure 8. These results showed that the sequential removal of the 44^{th} amino acid to the 48^{th} amino acid from the N-terminal, the cell entry effect of the polypeptide gradually attenuated, and the cell entry effect was better when the 44^{th} amino acid is present.

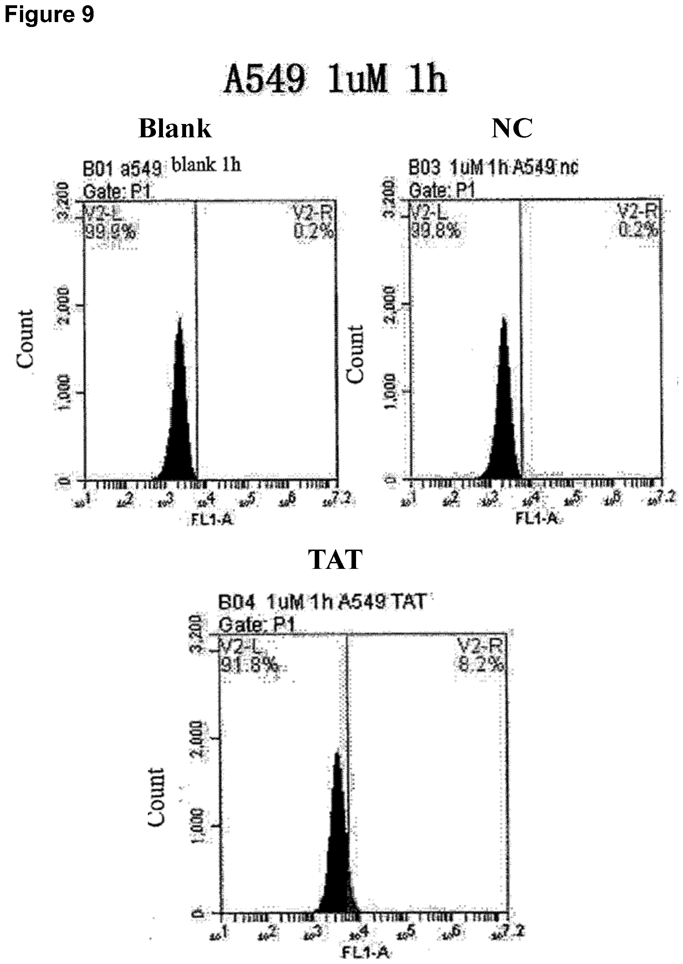

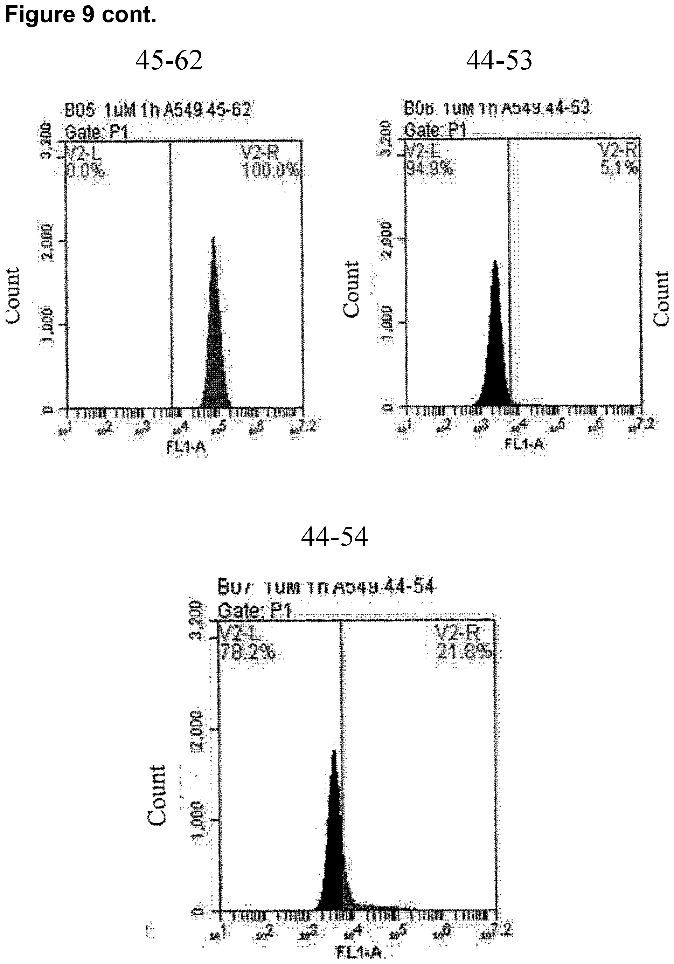

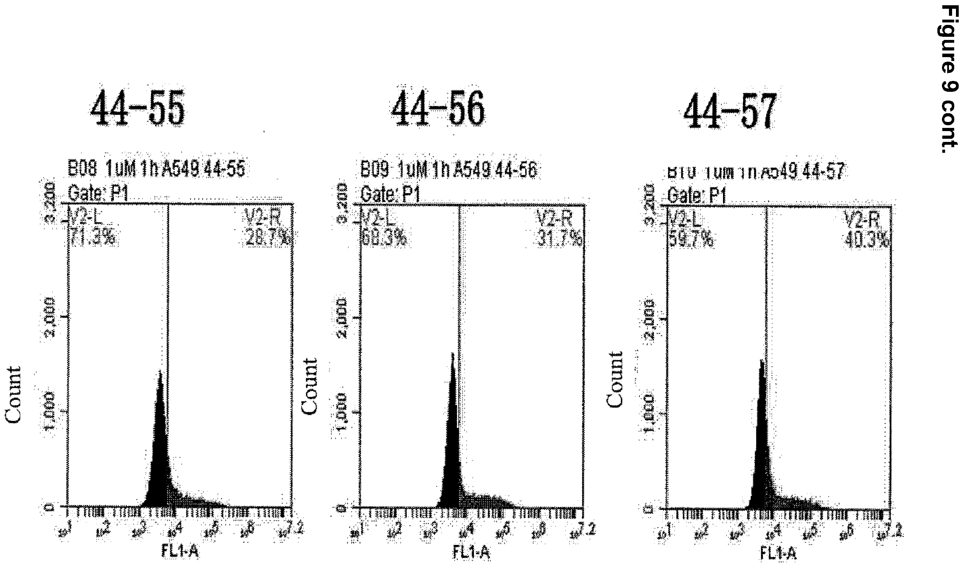

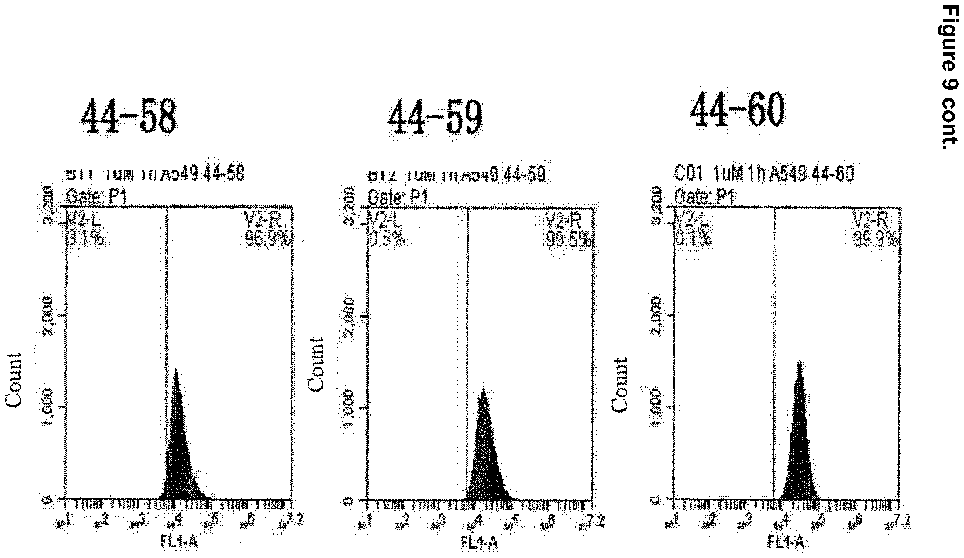

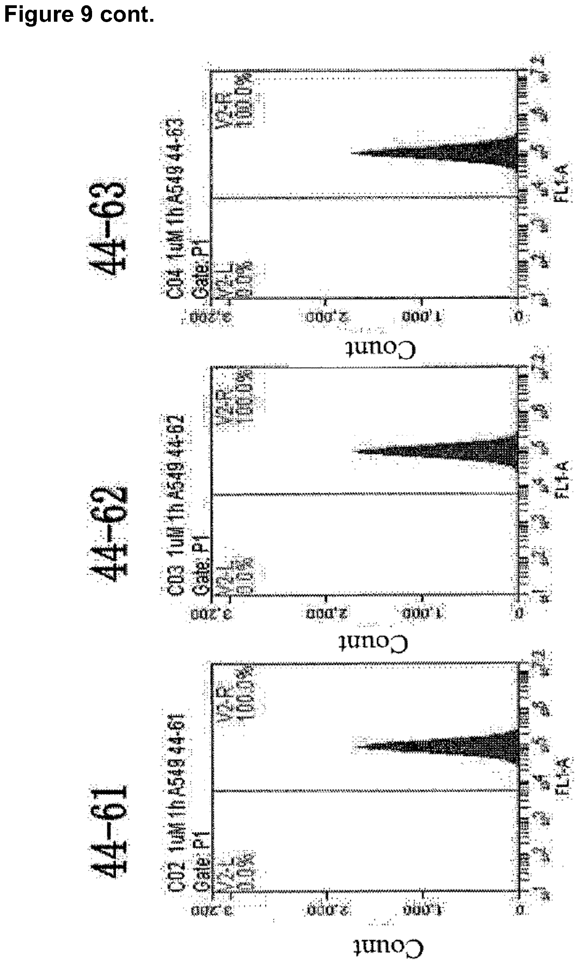

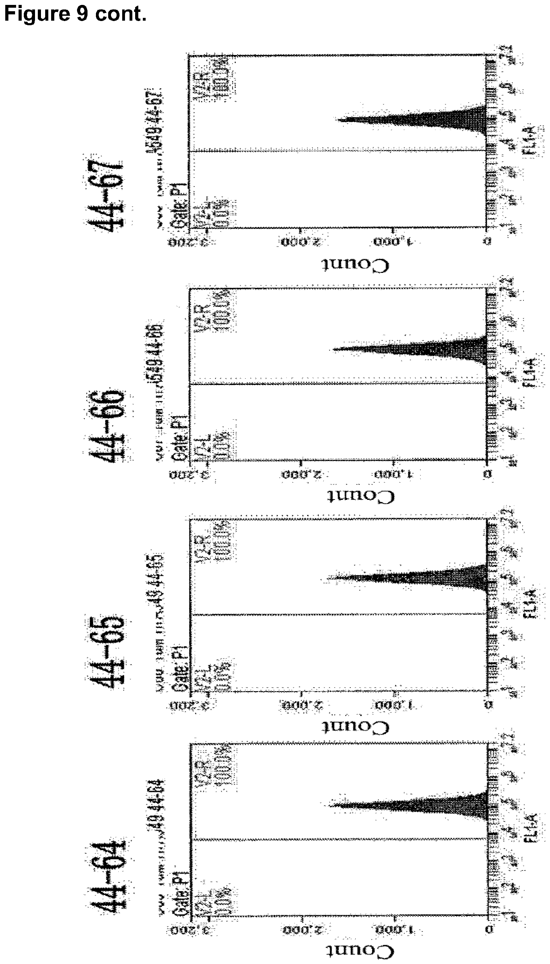

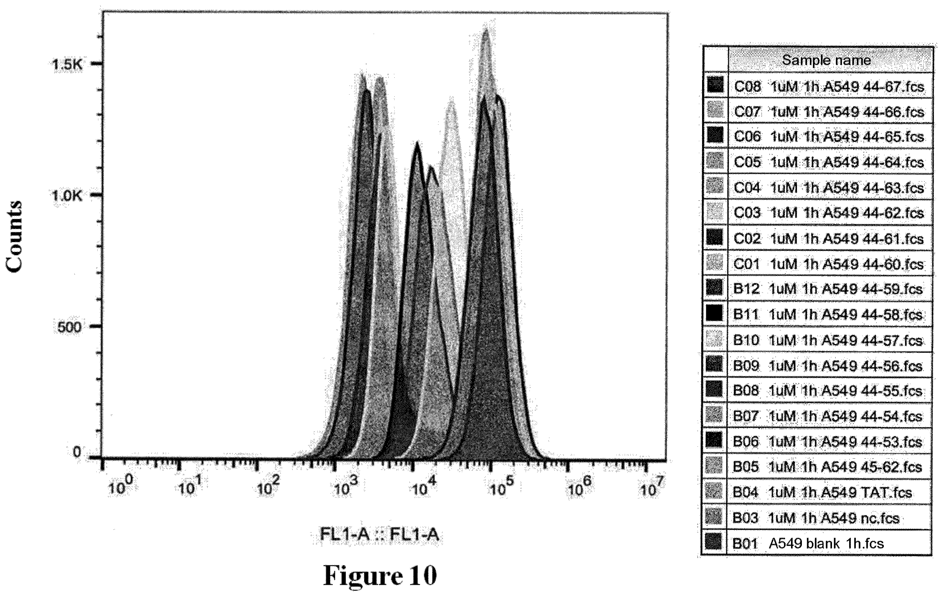

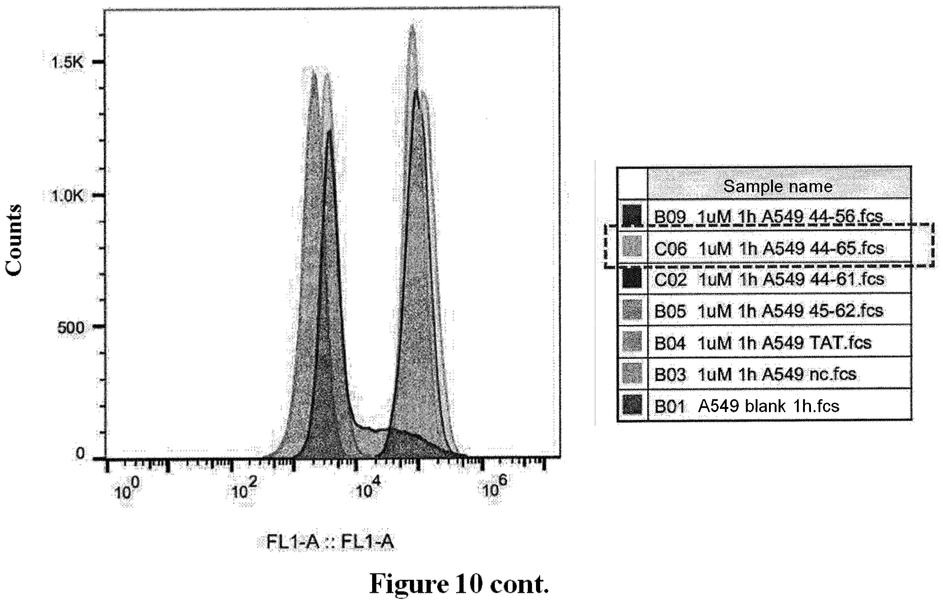

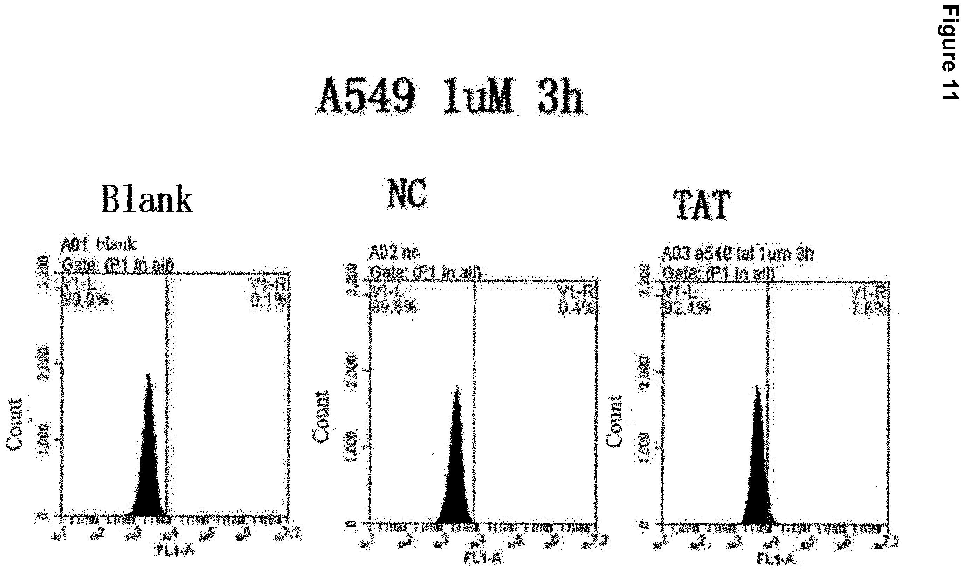

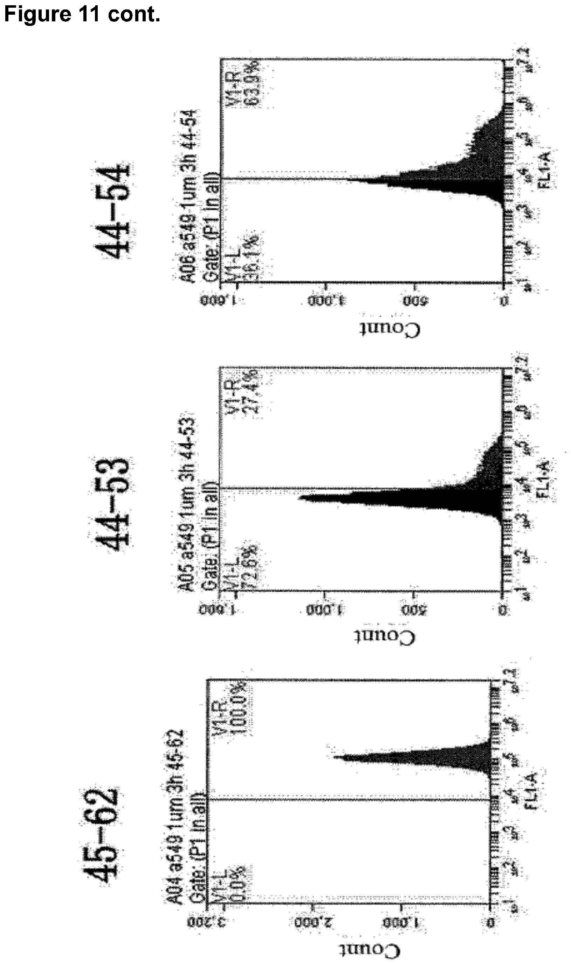

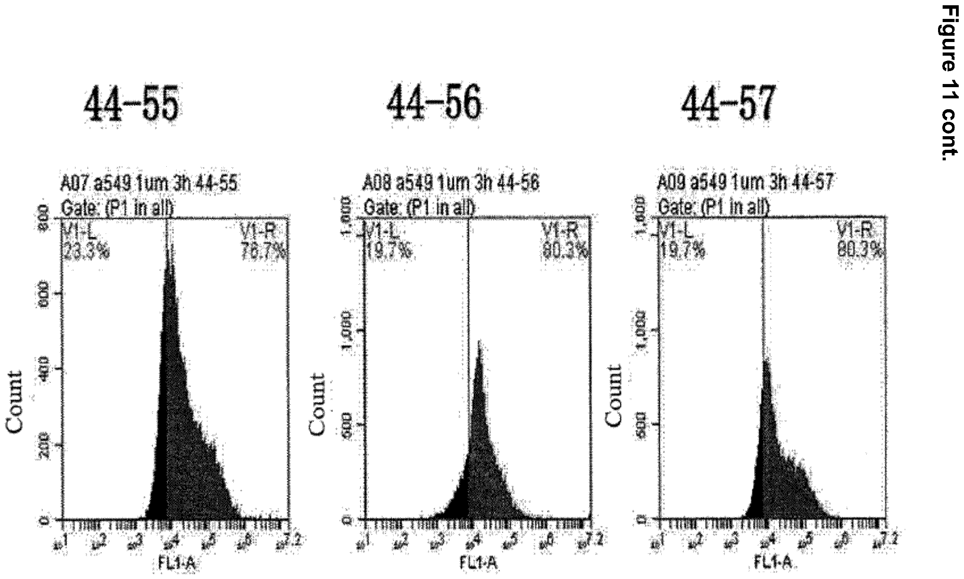

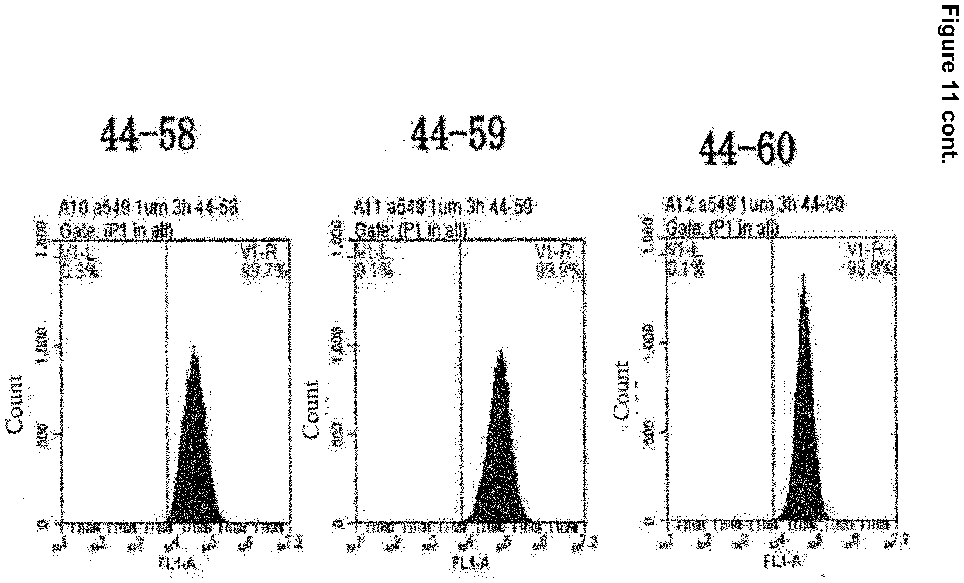

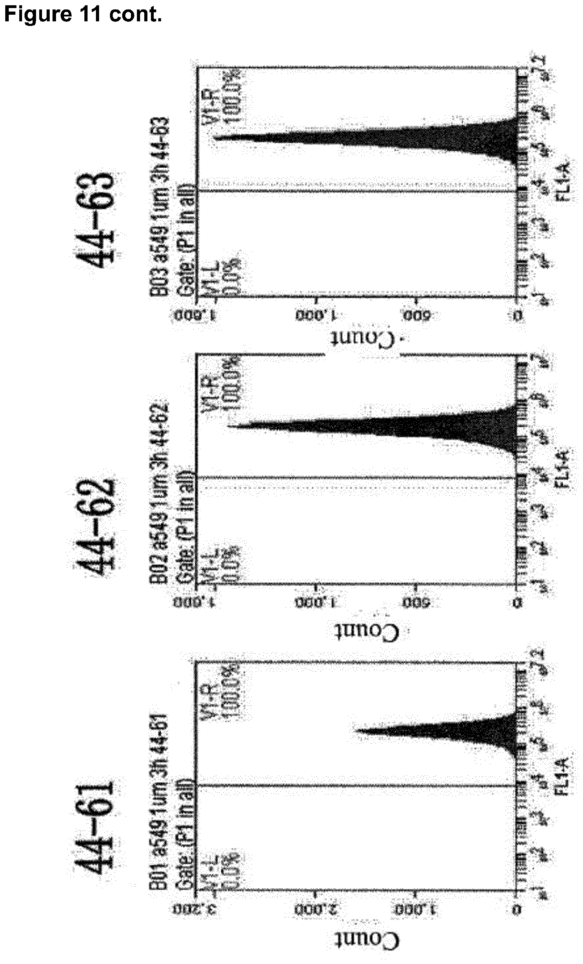

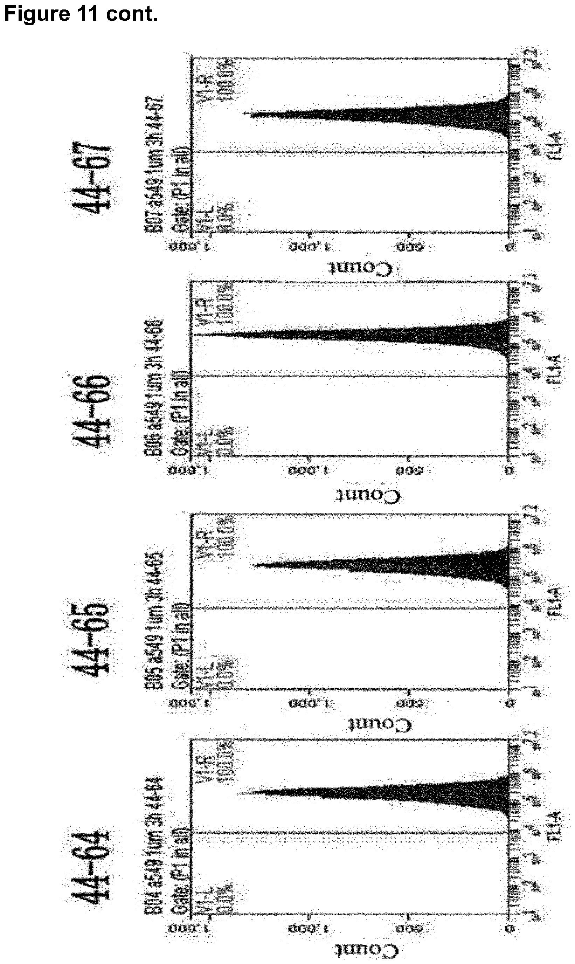

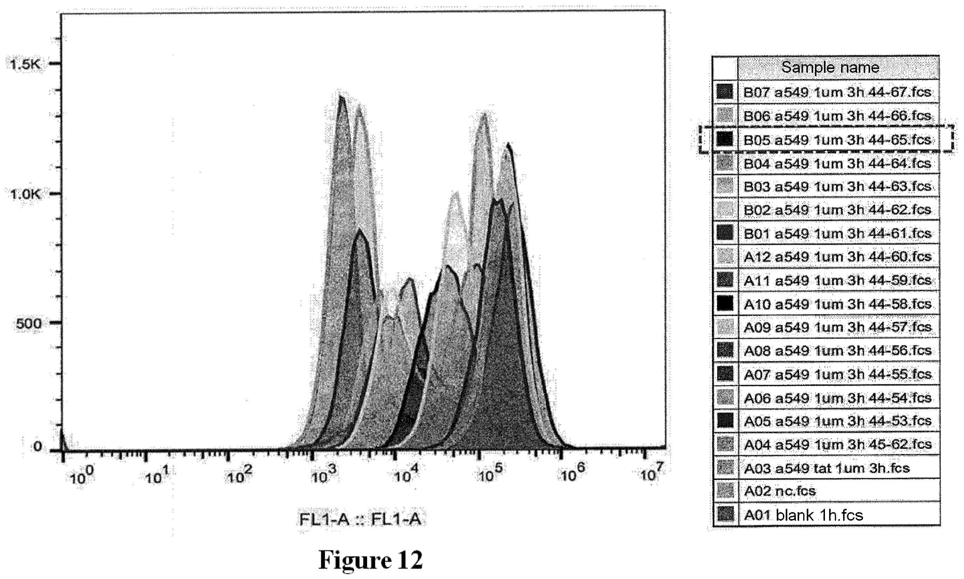

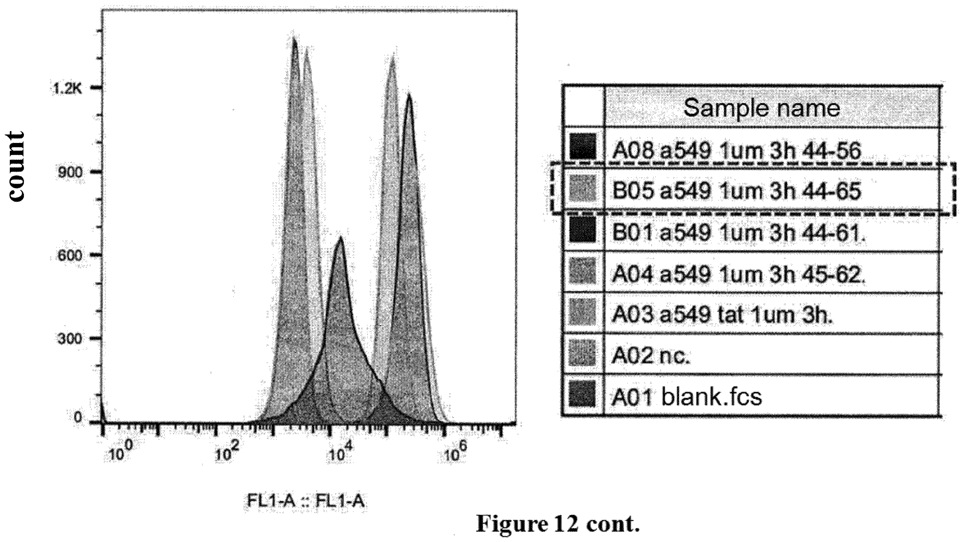

The results of flow cytometry detection of peptides truncated sequentially from the C-terminus (amino acid at position 67) are shown in Figure 9 and Figure 10, in which the peptide concentration used was 1 µM and the incubation duration was 1 h. These result showed that Jilin-2004(H5N1)-M2-44-65 and Jilin-2004(H5N1)-M2-44-61 polypeptides had even better entry effects than other polypeptides. The results of cell entry after 3 h of incubation are shown in Figure 11 and Figure 12, which are consistent with those in Figure 9 and Figure 10, indicating that Jilin-2004(H5N1)-M2-44-65 and Jilin-2004(H5N1)-M2-44-61 polypeptides have even better entry effects. Jilin-2004(H5N1)-M2-45-62 polypeptide is also shown to have a good cell-penetrating peptide effect.

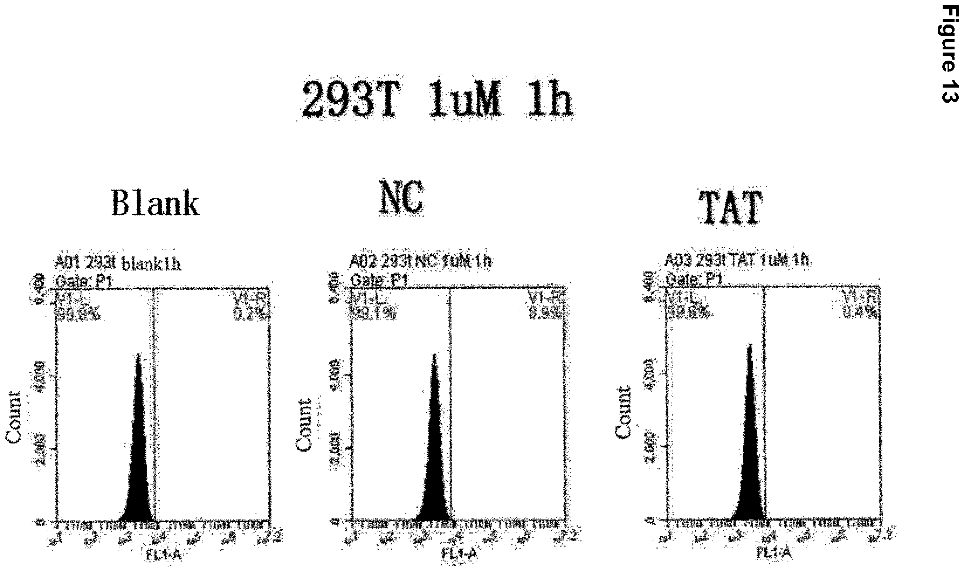

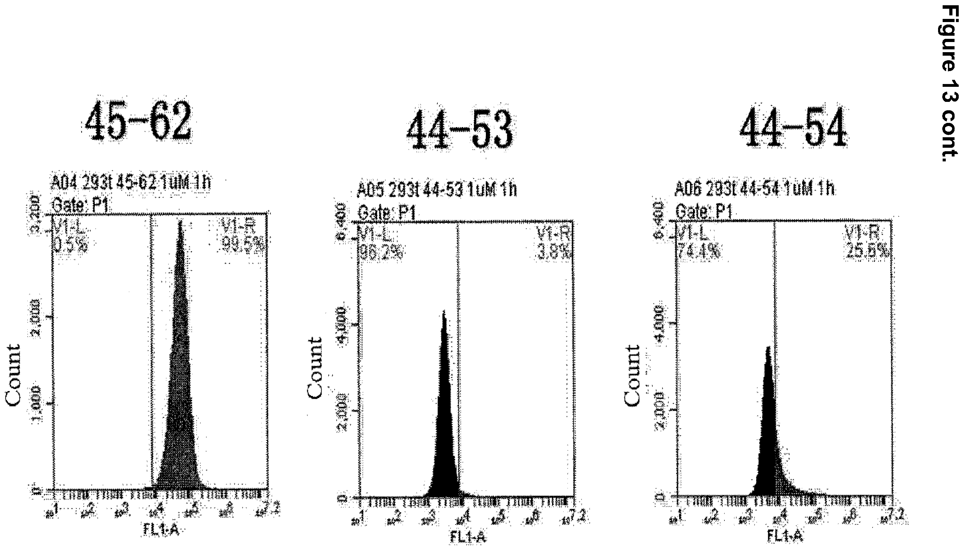

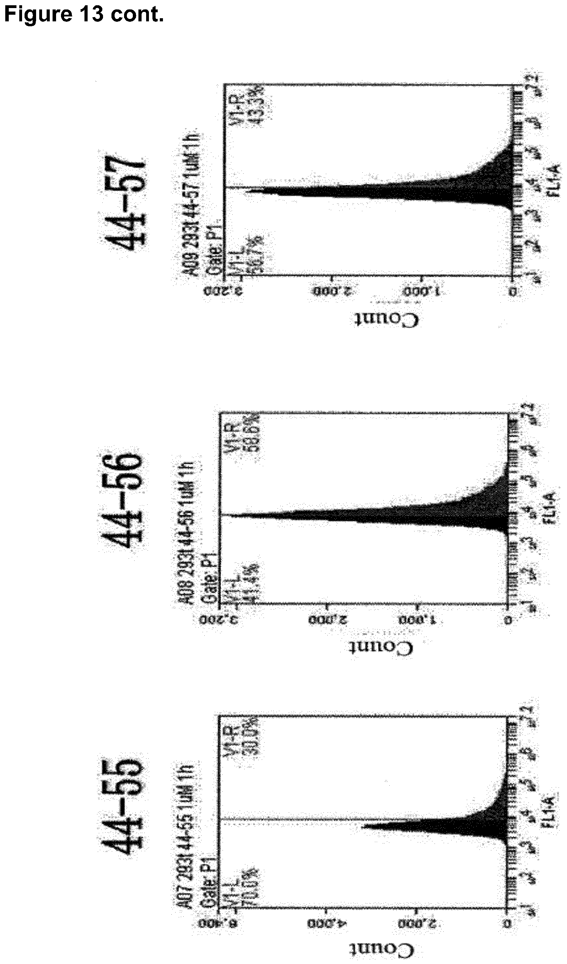

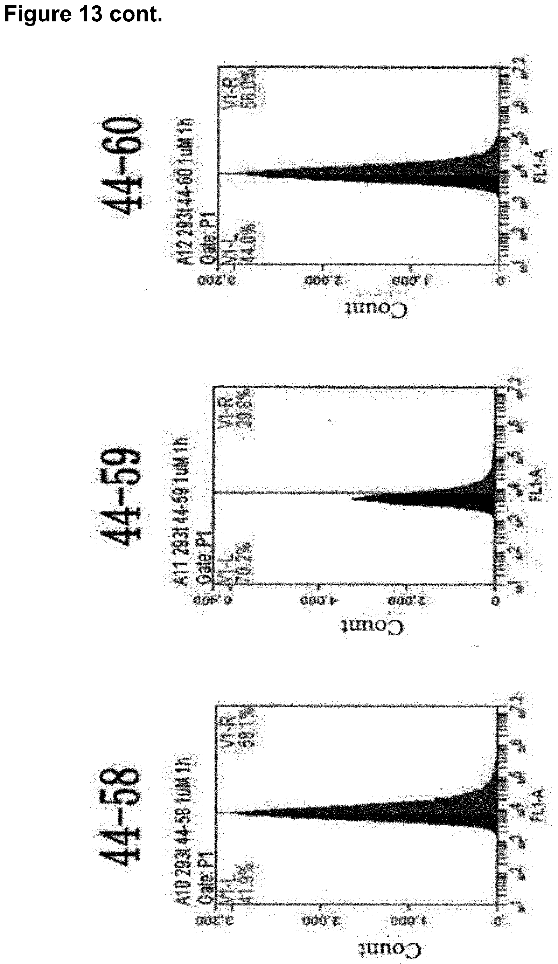

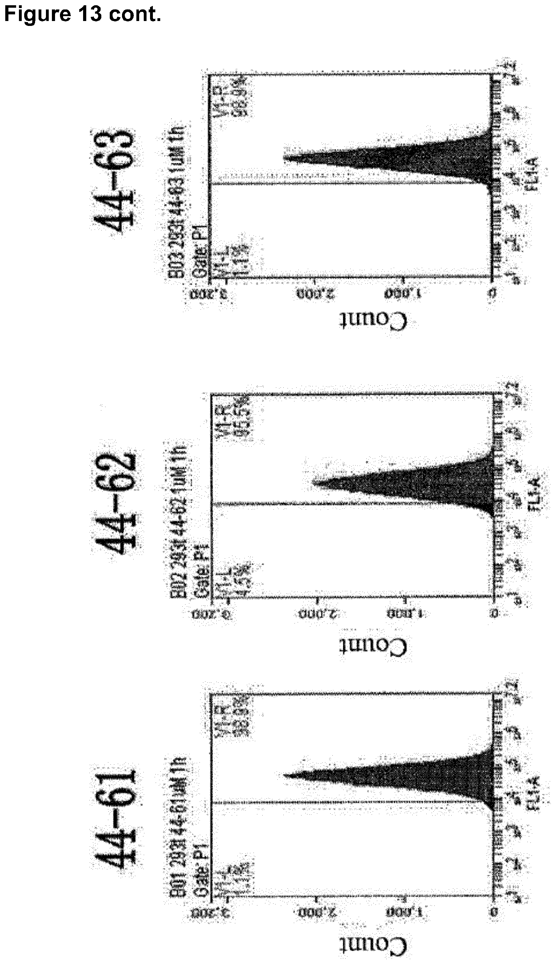

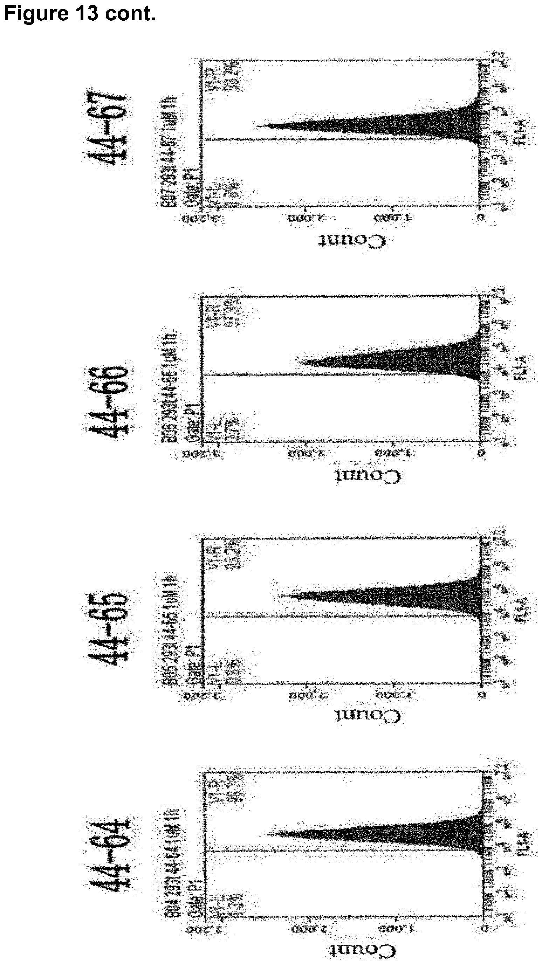

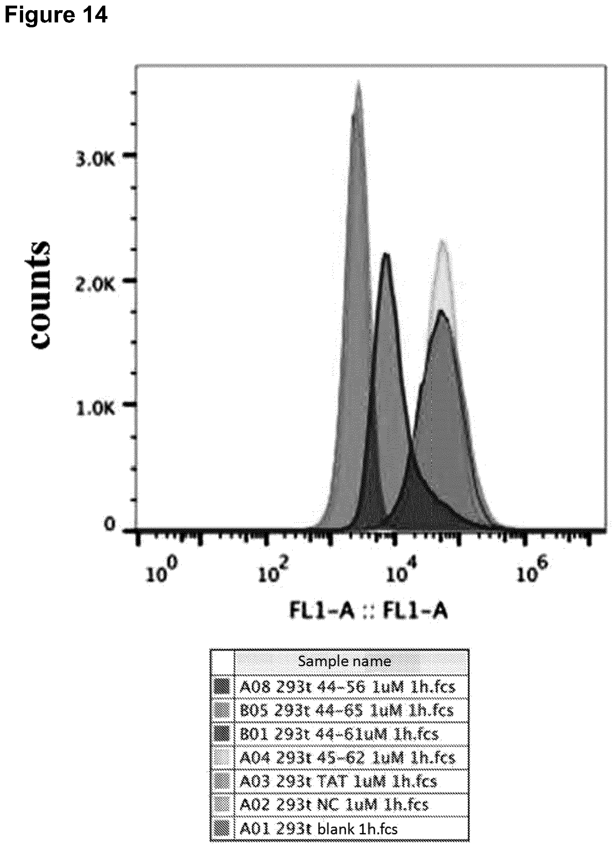

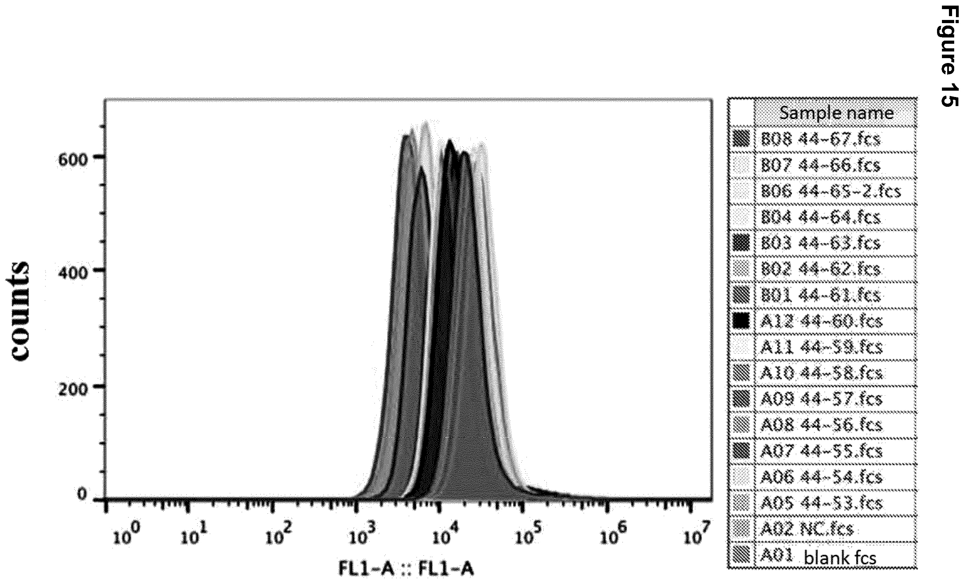

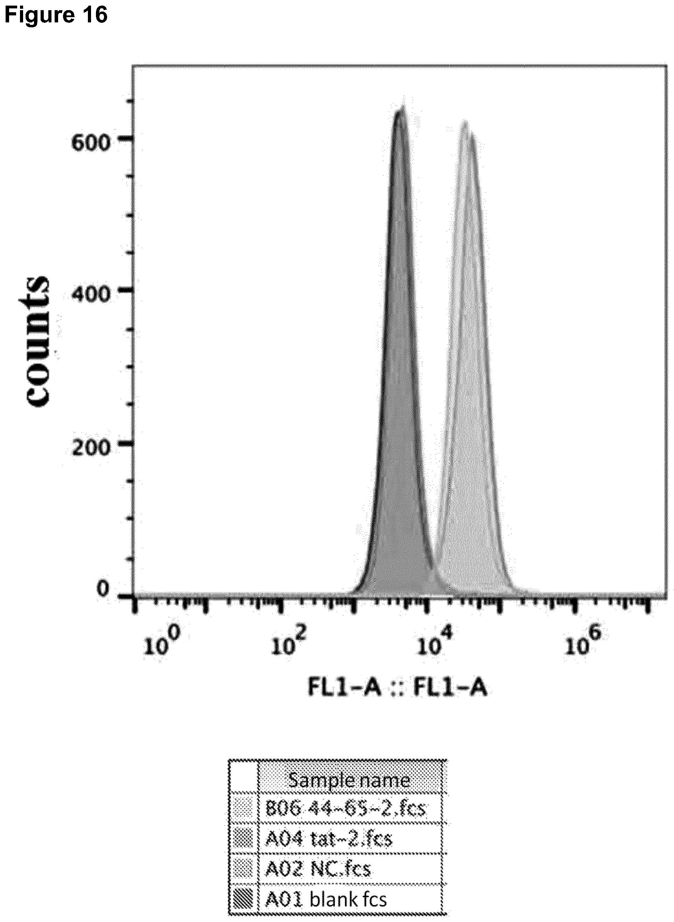

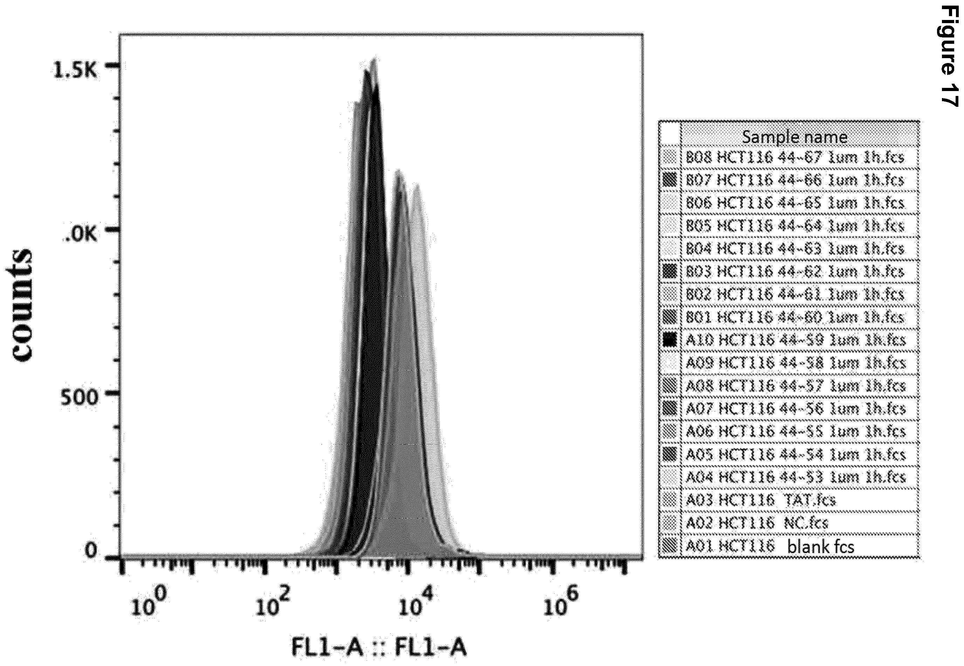

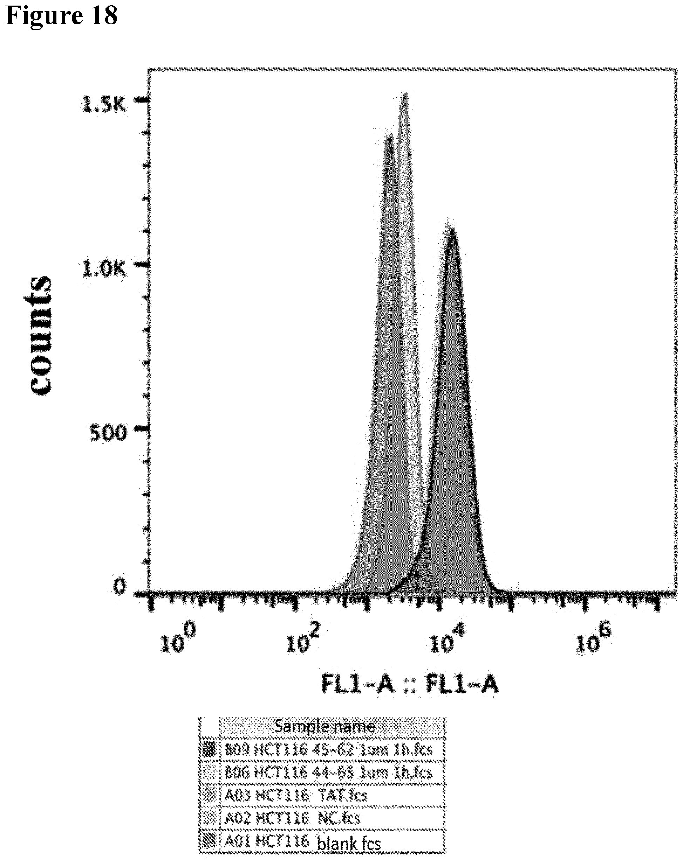

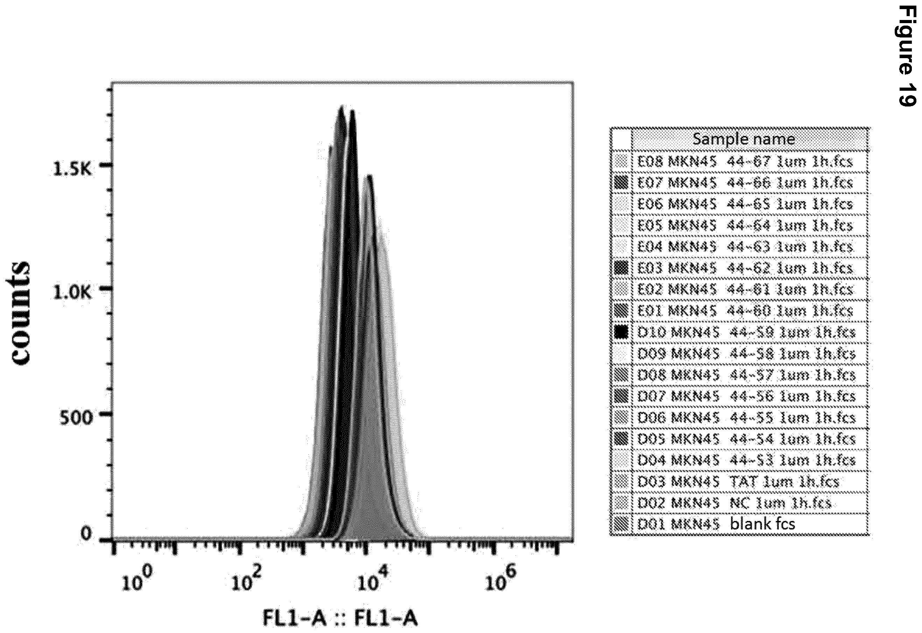

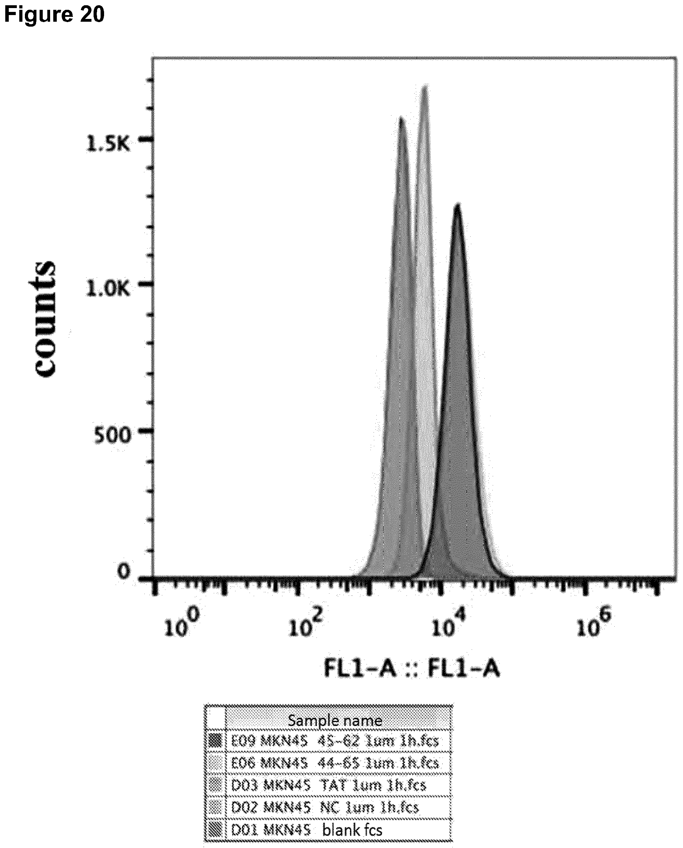

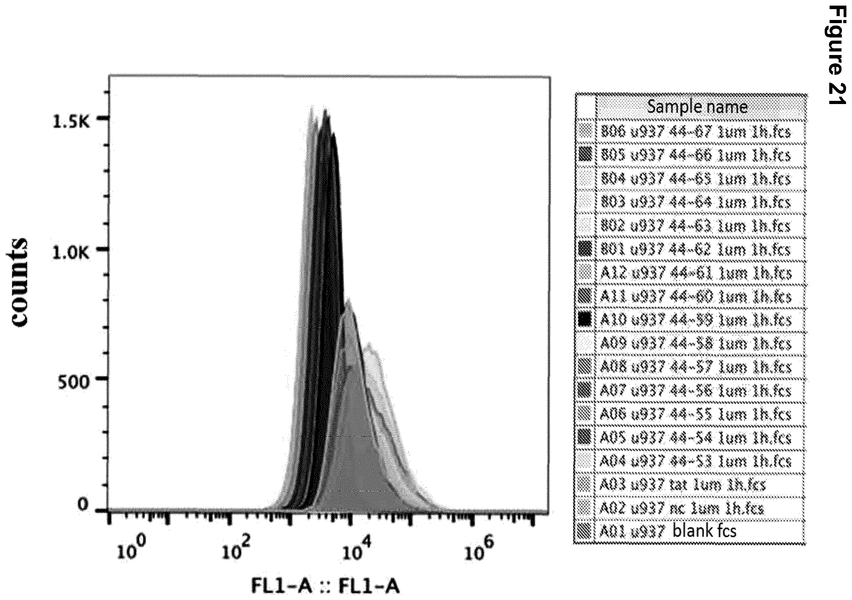

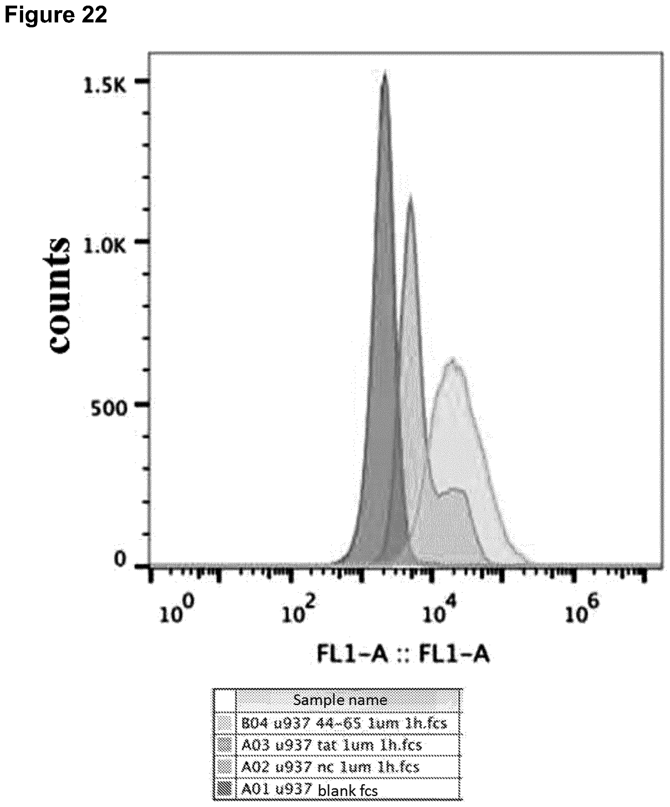

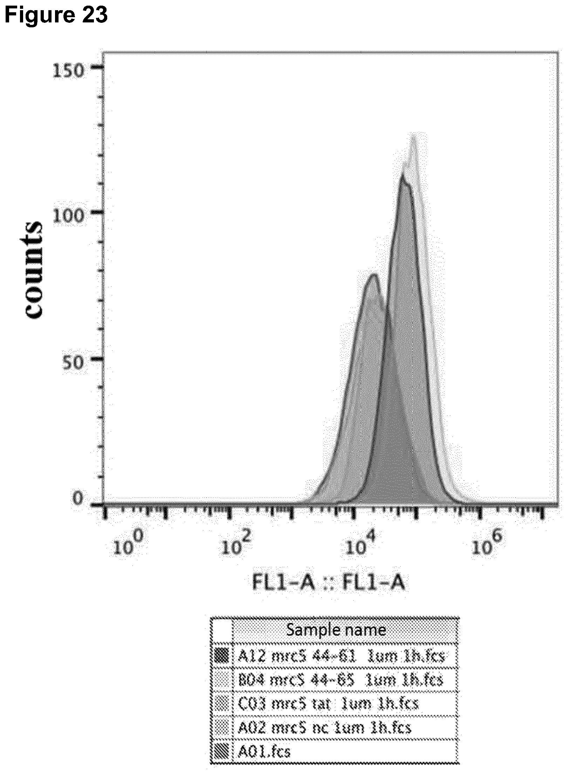

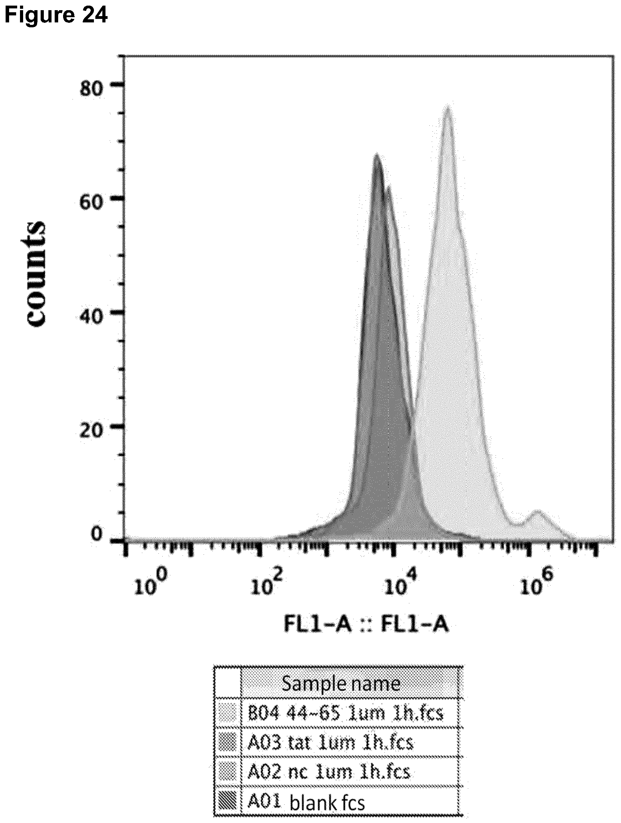

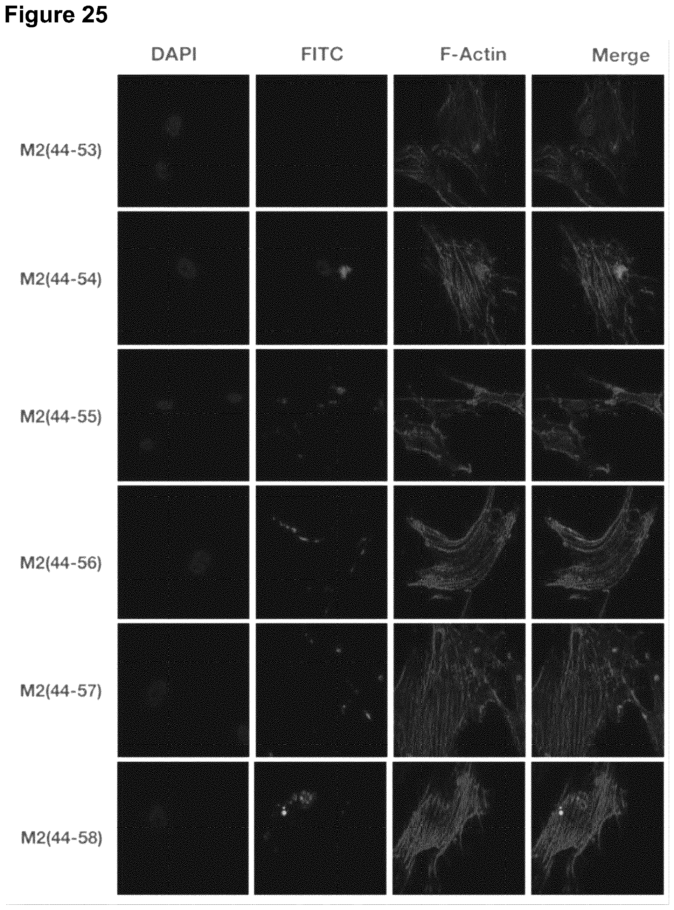

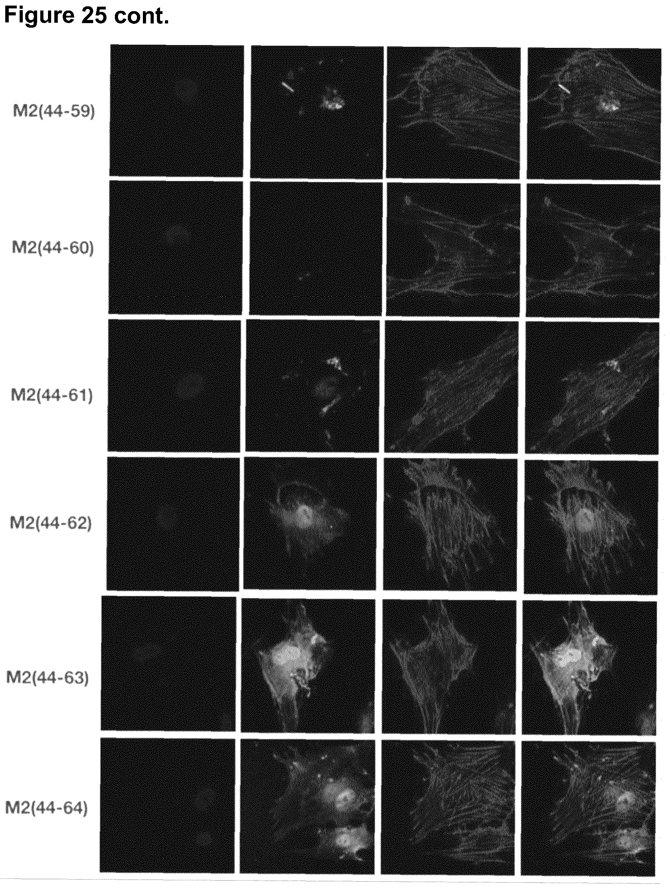

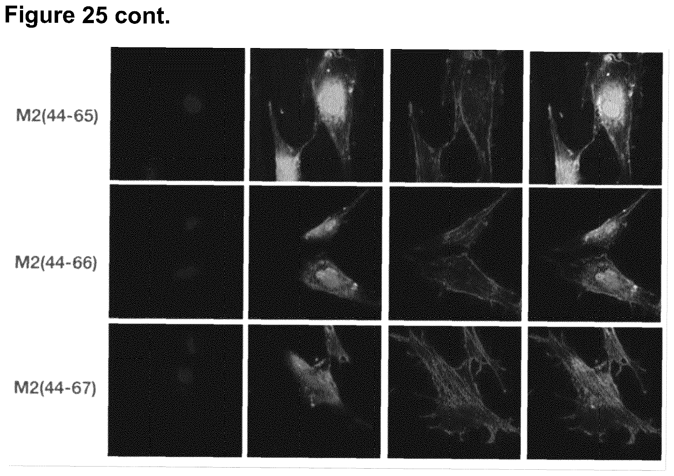

In order to prove that the cell entry function of M2 related peptides is universal without being limited by specific cell types, the cell penetration properties of amino acids 44-67 polypeptide of the M2 protein and truncated fragments thereof were tested in different types of cells, including 293T cell, HUVEC cell, HCT-116 cell, MKN-45 cell, MRC5 cell, U397 cell and ESF-1 cell. The results are shown in Figure 13 to Figure 25. Among them, Figure 13 and Figure 14 show the results using 293T cells; Figure 15 and Figure 16 show the results using HUVEC cells; Figure 17 and Figure 18 show the results using HCT-116 cells; Figure 19 and Figure 20 show the results using MKN-45 cells; Figure 21 and Figure 22 show the results using U937 cells; Figure 23 shows the results of using MRC5 cells; and Figure 24 and Figure 25 show the results of using ESF-1 cells. The same results as those in A549 cells were observed in the above different cells, i.e. the polypeptide based on amino acids 44-67 of the M2 protein and truncated fragments thereof all had cell-penetrating peptide properties in different cell lines, and Jilin-2004 (H5N1)-M2-44-65, Jilin-2004(H5N1)-M2-44-61 and Jilin-2004(H5N1)-M2-45-62 polypeptides had even better entry effects.

### Example 5: Influence of incubation duration on cell entry of polypeptides

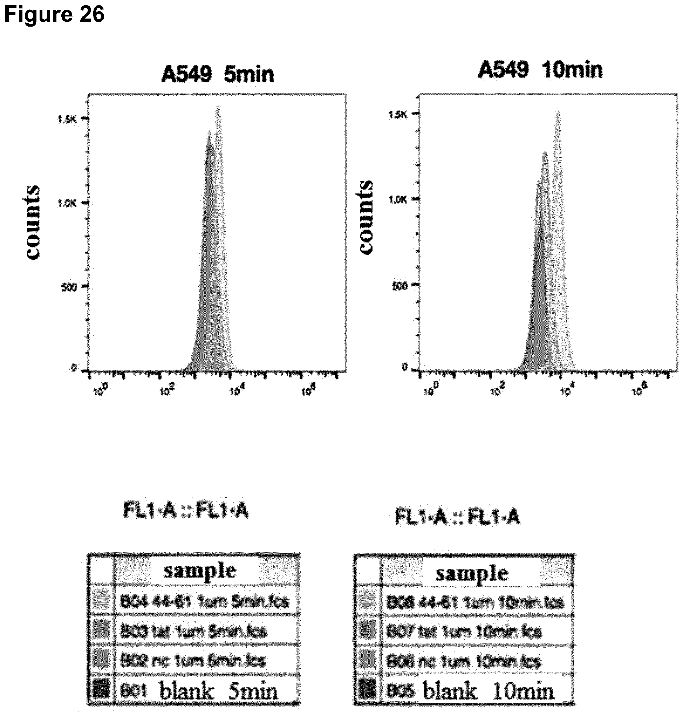

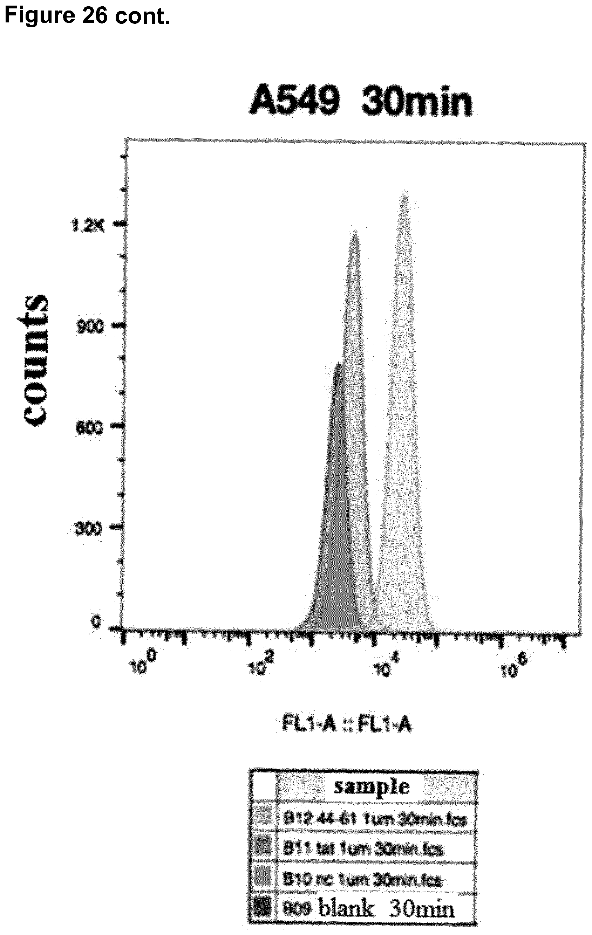

The Jilin-2004(H5N1)-M2-44-61 polypeptide was selected, and A549 cells were used to study the effect of the incubation duration of the polypeptide and the cell on the cell entry of the polypeptide. Blank A549 cells, NC and TAT were used as blank control, negative control and positive control, respectively. First, the cell entry was detected at three time points, 5 min, 10 min and 30 min. The results are shown in Figure 26. These results showed that when 1 µM Jilin-2004(H5N1)-M2-44-61 polypeptide was used, its cell entry could be detected by incubating with A549 cells for 5 min, and the amount of its cell entry increased as the incubation duration increased.

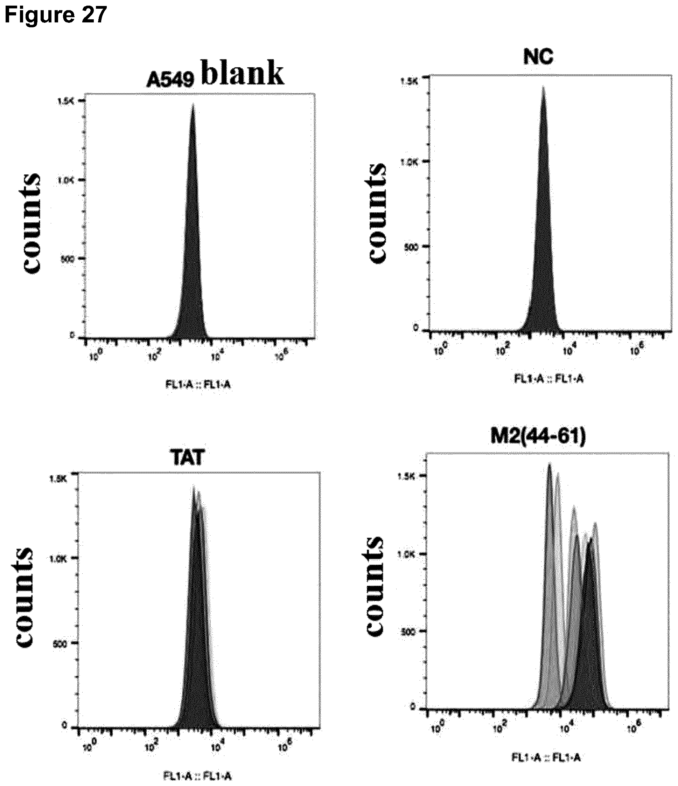

The incubation duration was further extended to 5 min, 10 min, 30 min, 1 h, 2 h, 3 h and 6 h, and the cell entry effect of the polypeptide was tested. As shown in the results in Figure 27, when 1 µM NC negative control polypeptide was used, no entry of polypeptides was observed at all time points. When 1 µM positive control TAT polypeptide was used, the entry into the cell was observed at 5 min, and the effect was obvious at the time point of 1 h. For 1 µM Jilin-2004(H5N1)-M2-44-61 polypeptide, the entry into the cell was observed at 5 min, and had a strong entry effect at 30 min, and there was little difference in entry effects at three time points 1 h, 3 h and 6 h. It is noticed that the cell entry effect of Jilin-2004(H5N1)-M2-44-61 polypeptide was much stronger than that of the positive control TAT polypeptide at the same time points. Based on the above results, the shortest time for the polypeptide entry effect, namely 1 hour, was selected as the incubation duration for the following studies.

### Example 6: Comparison of cell entry effects of cell-penetrating peptides based on M2 protein and existing cell-penetrating peptides

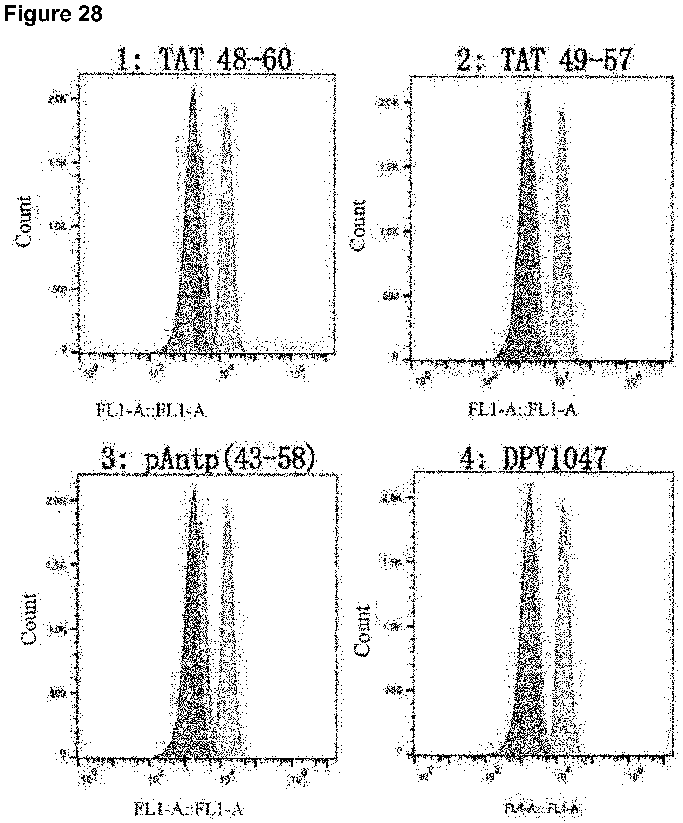

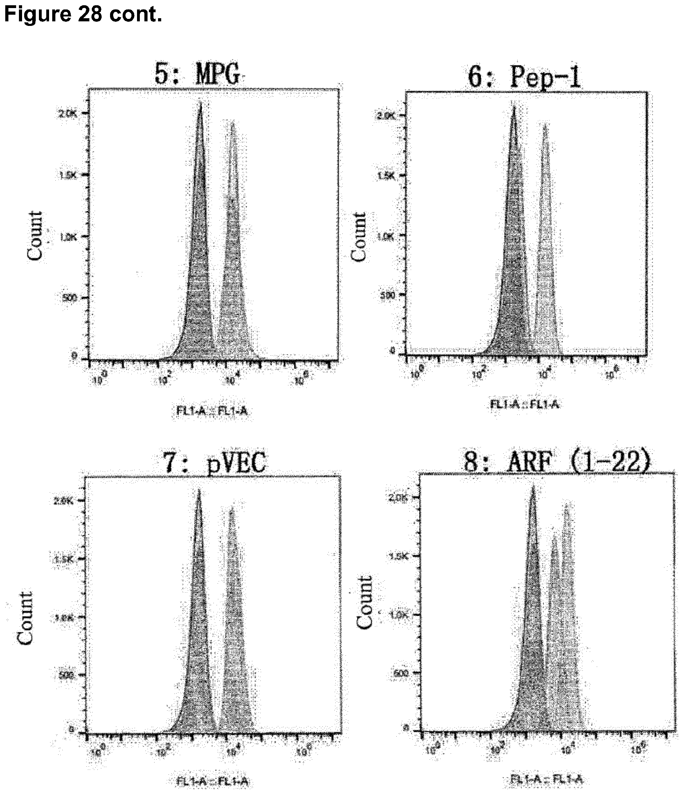

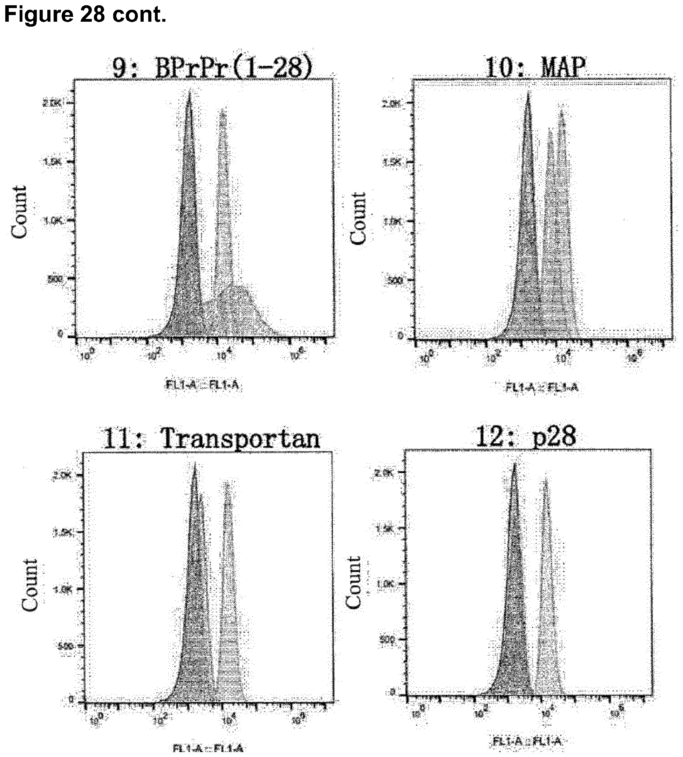

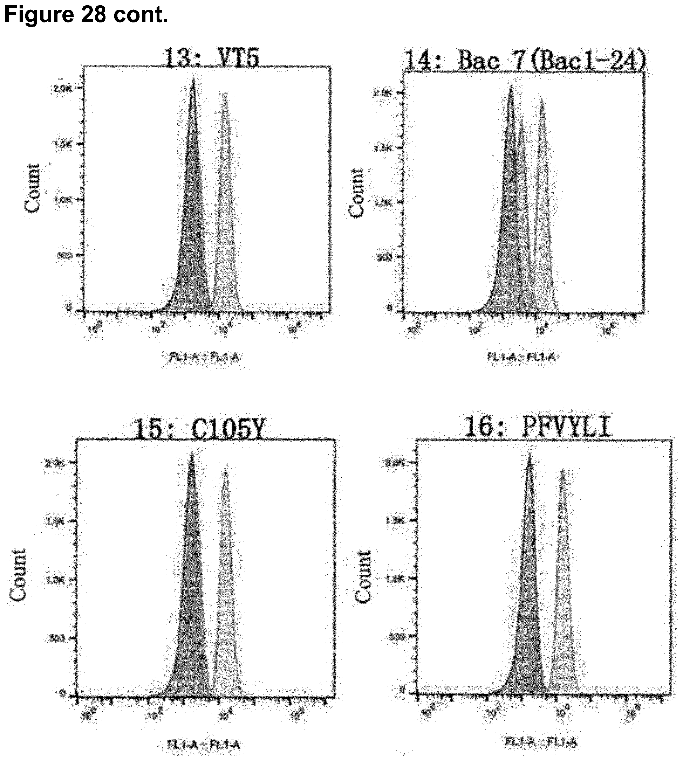

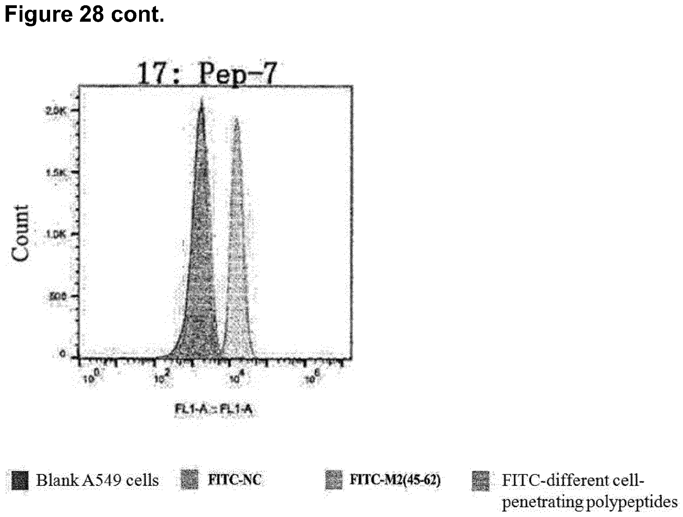

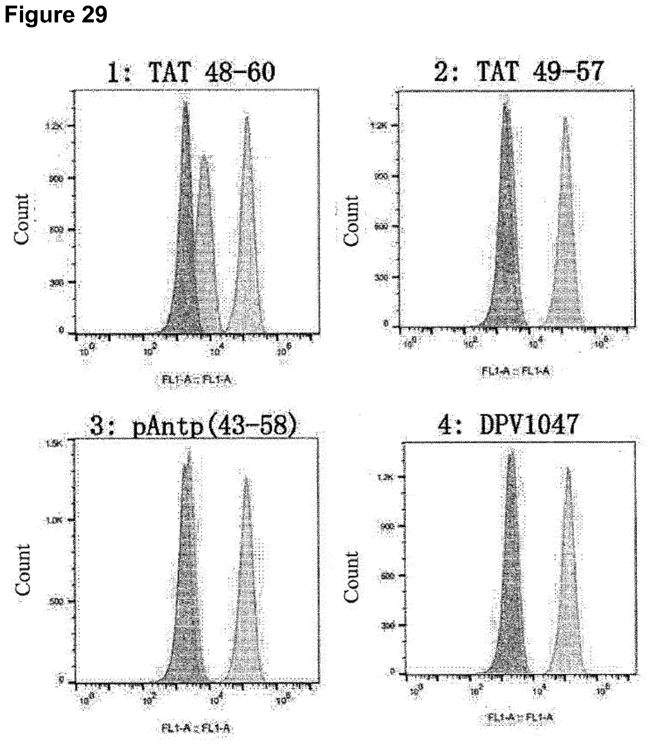

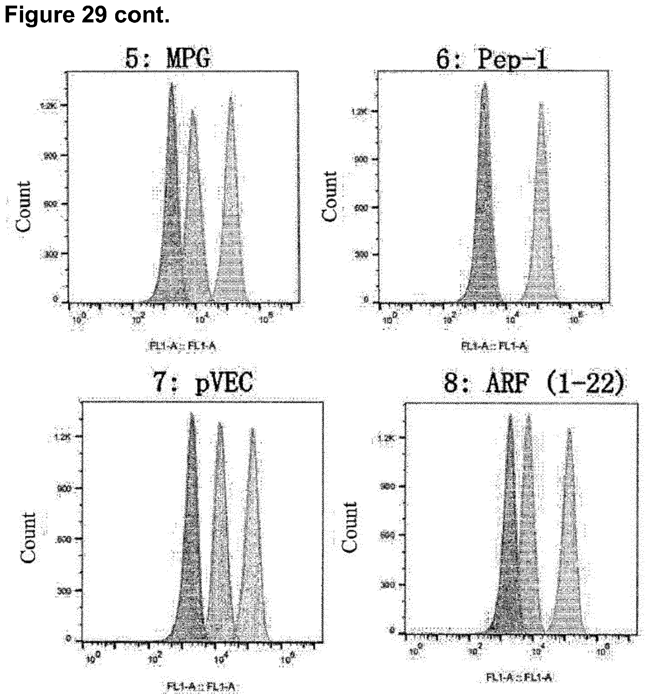

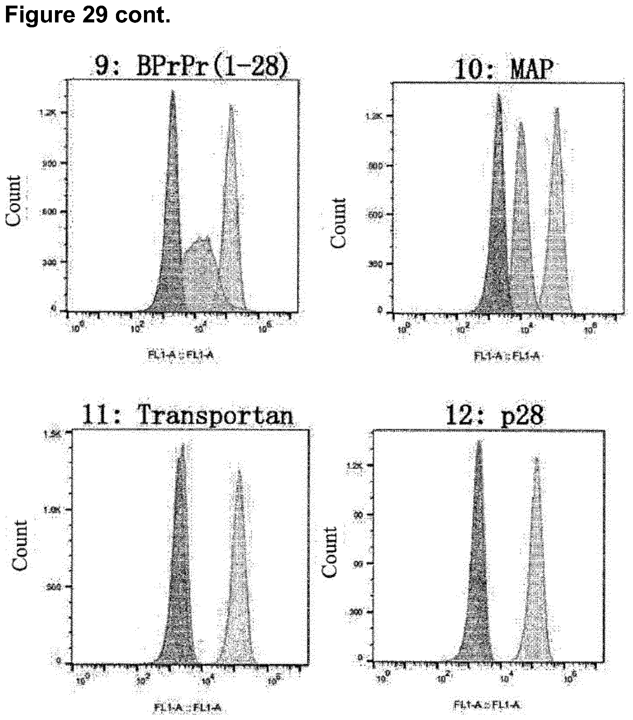

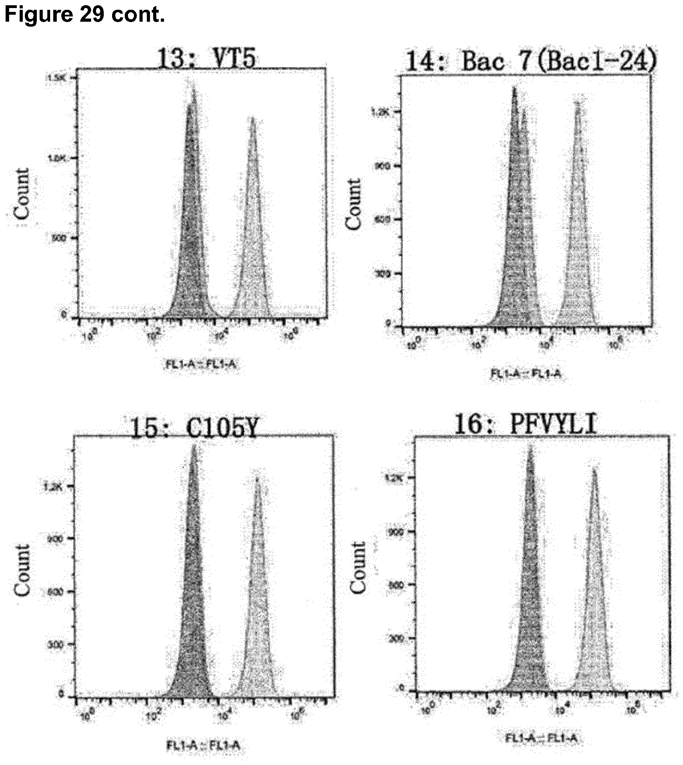

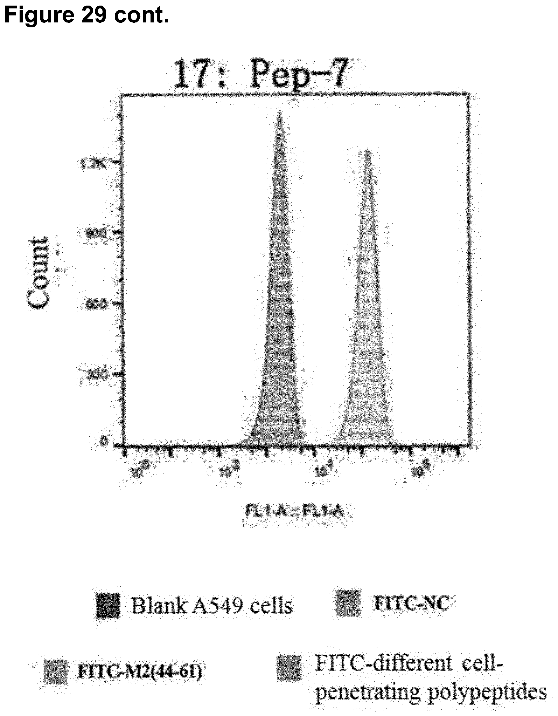

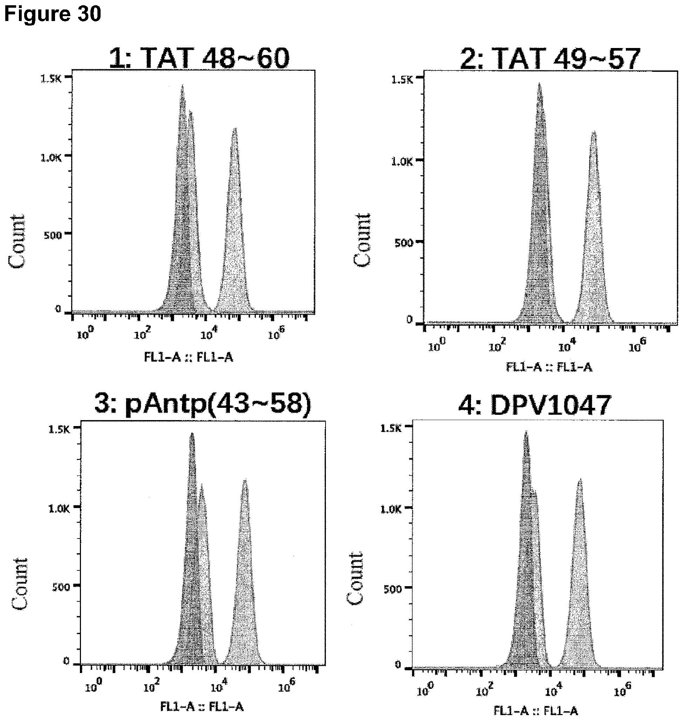

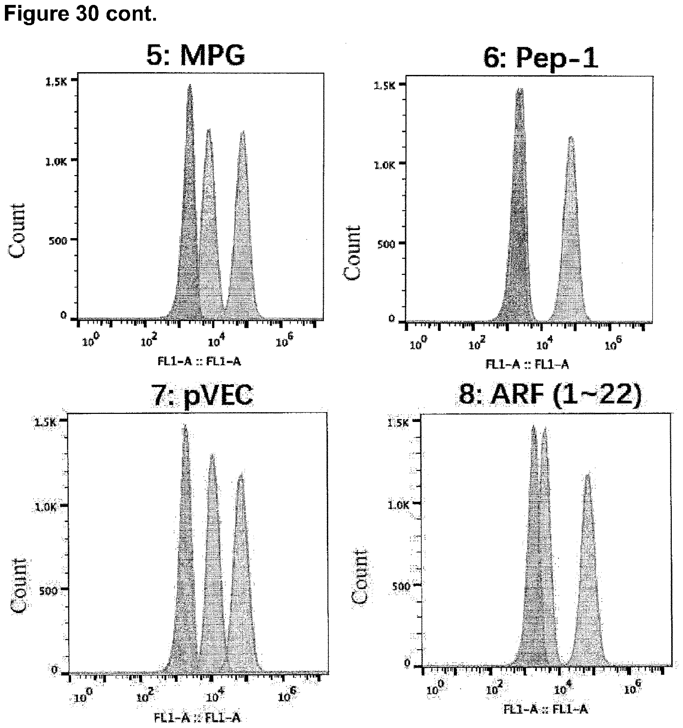

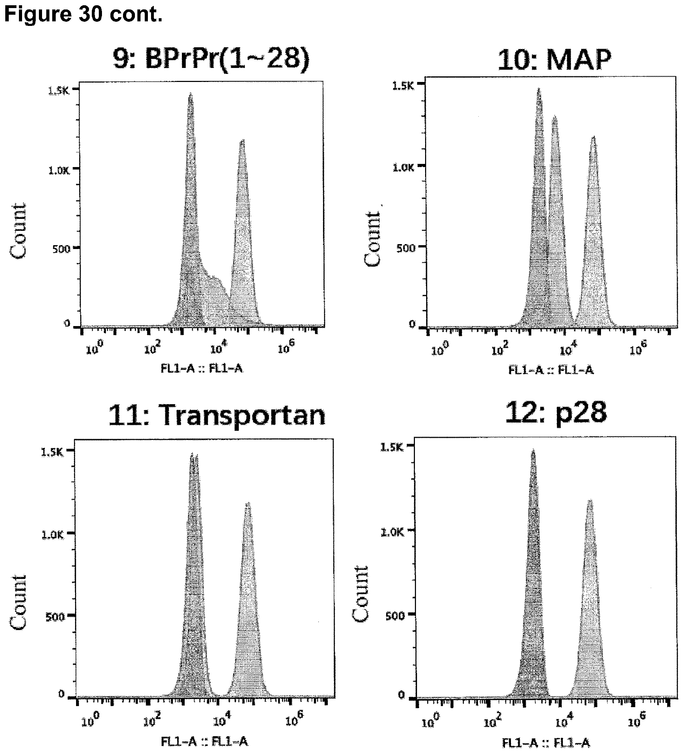

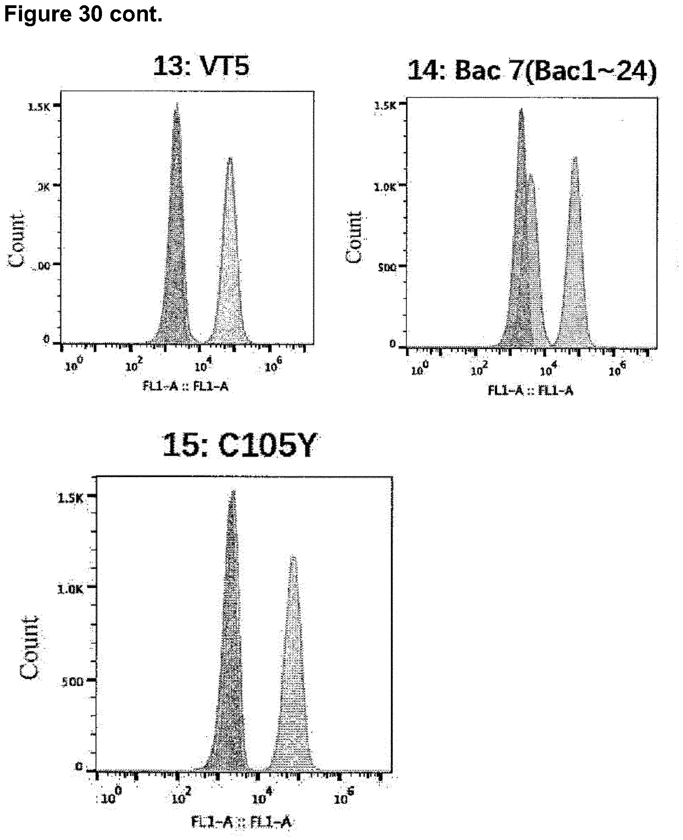

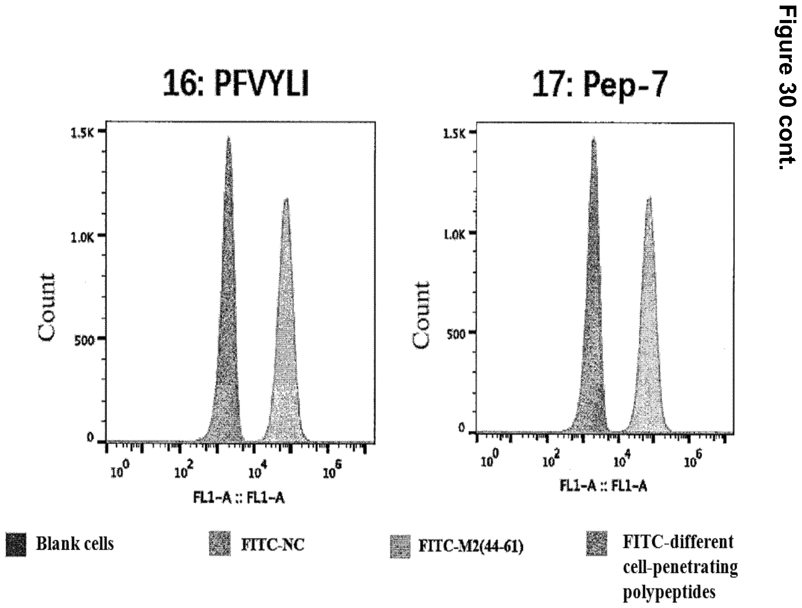

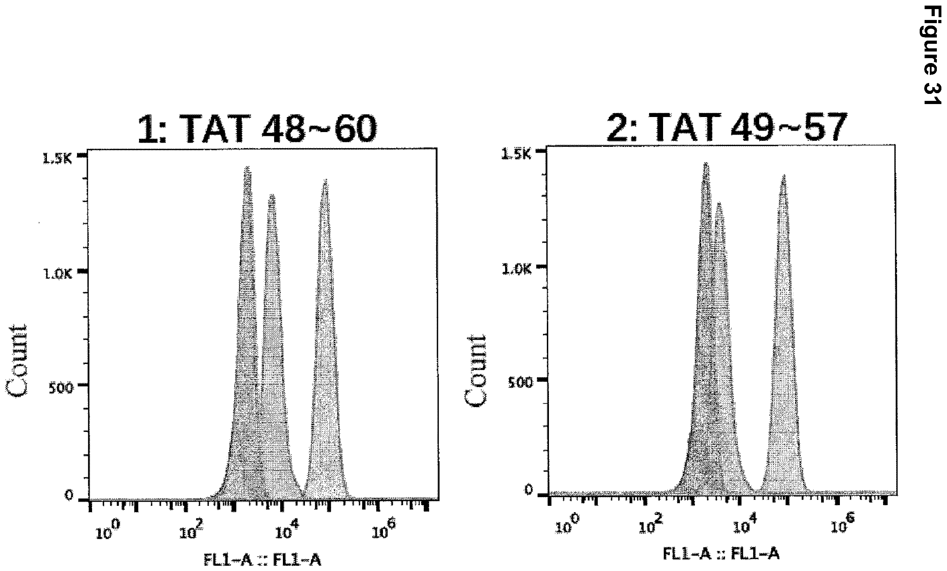

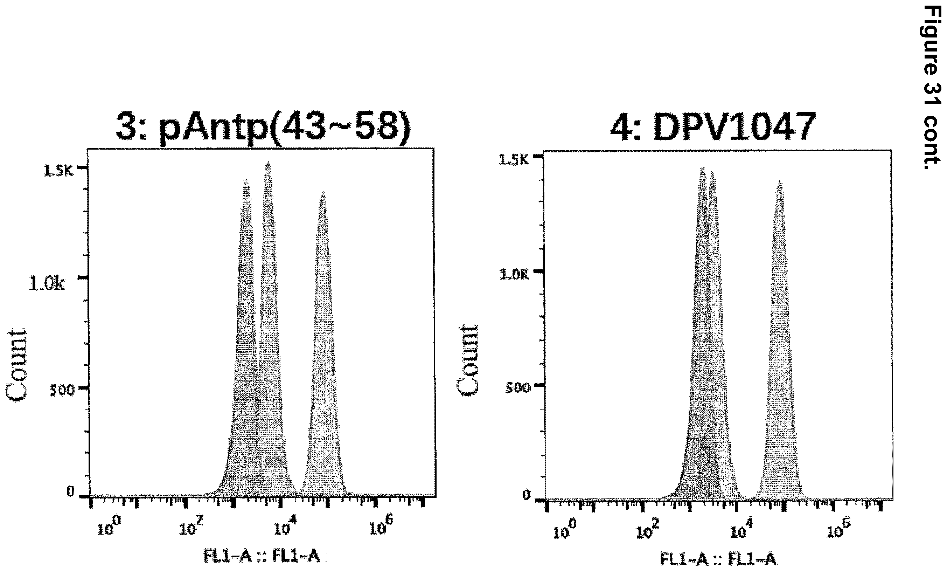

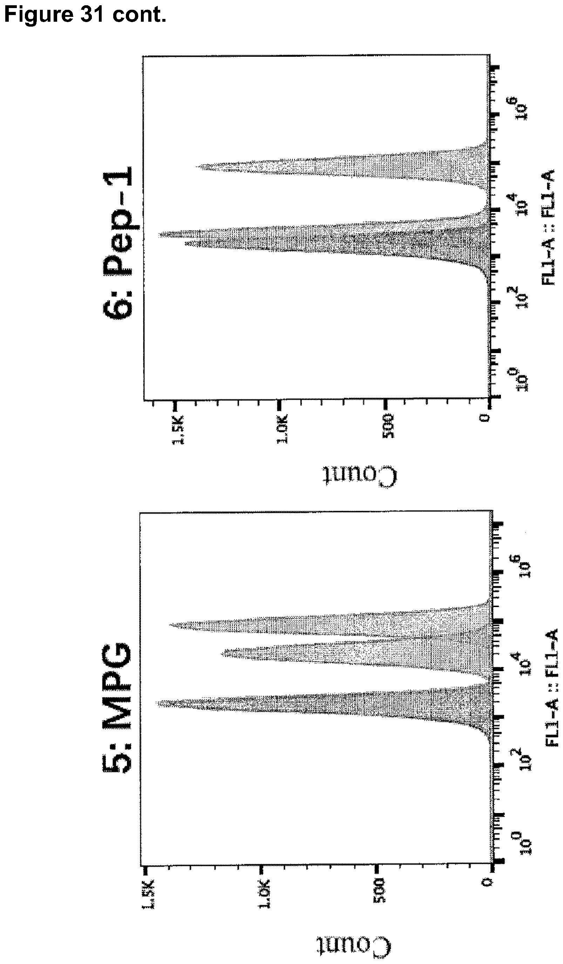

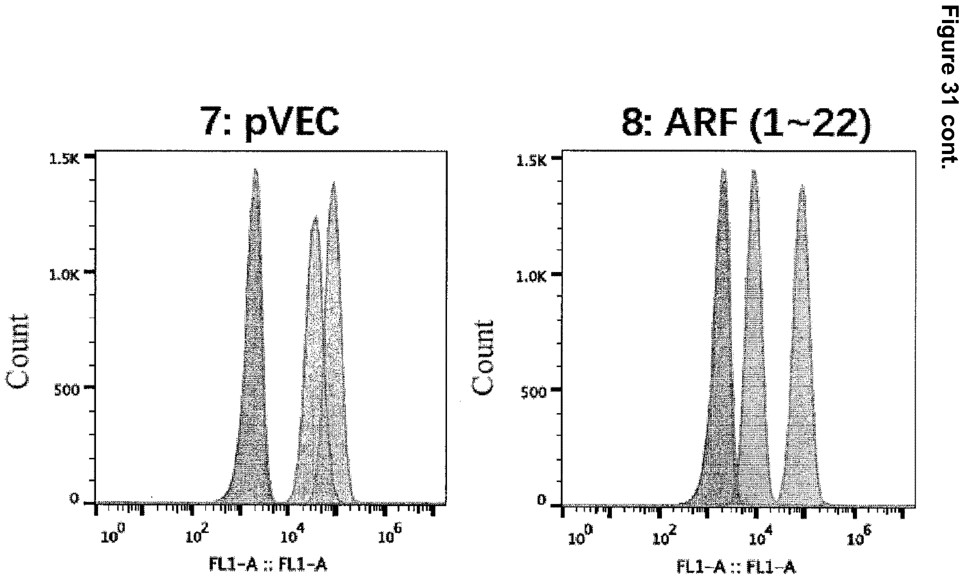

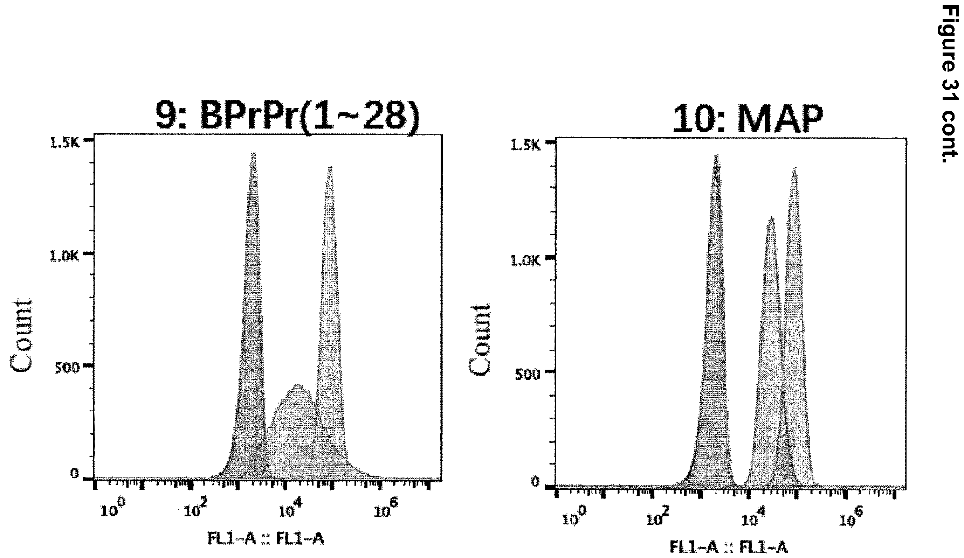

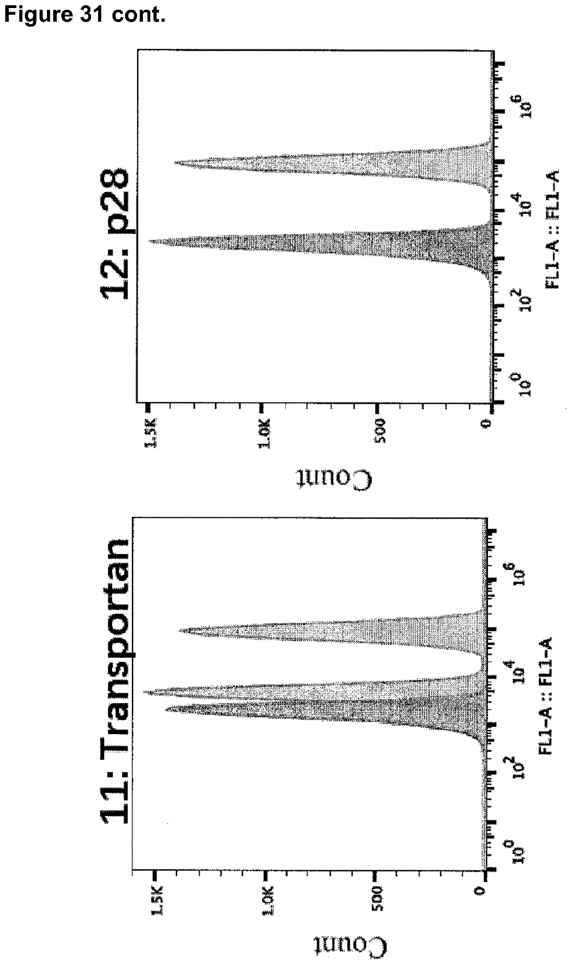

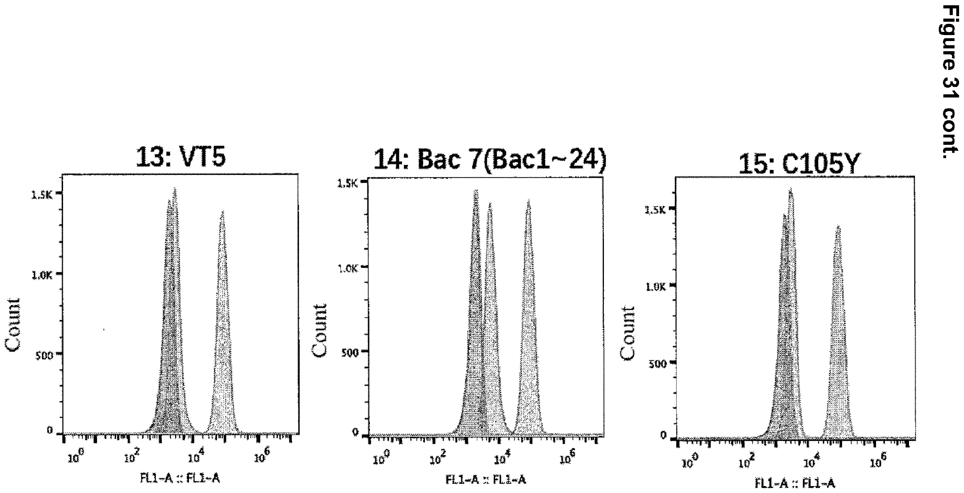

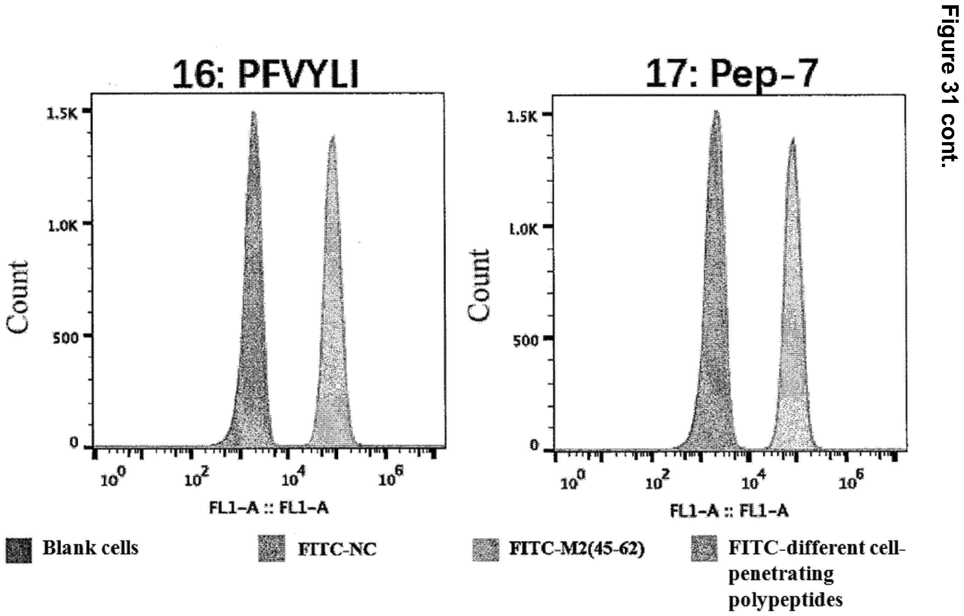

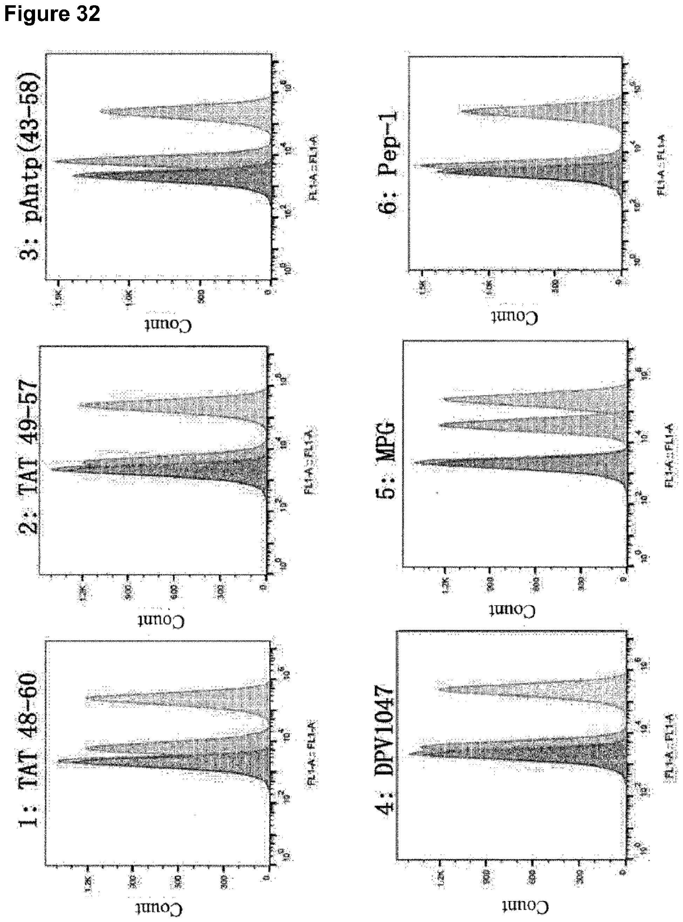

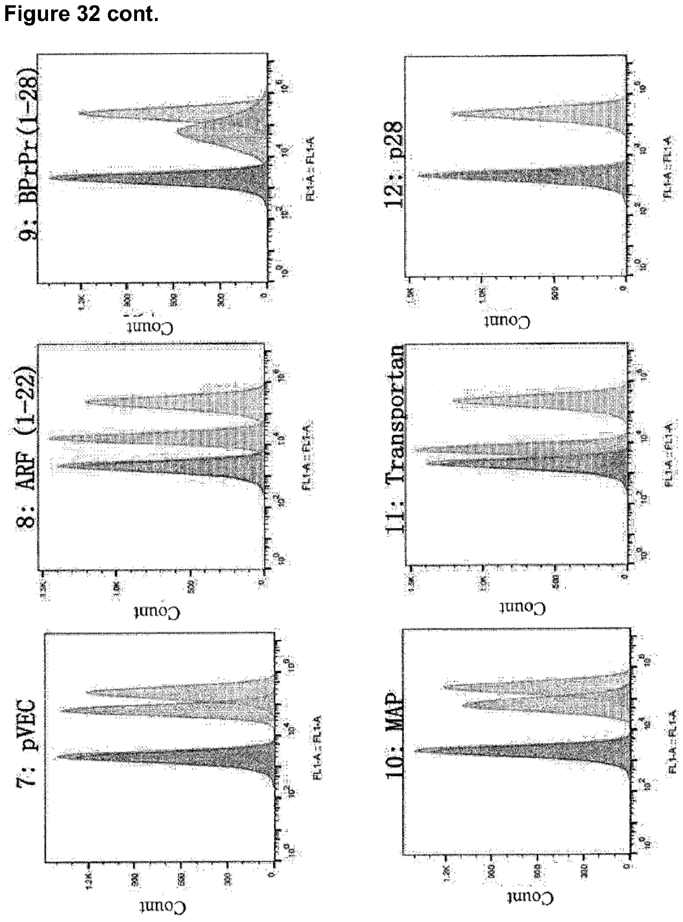

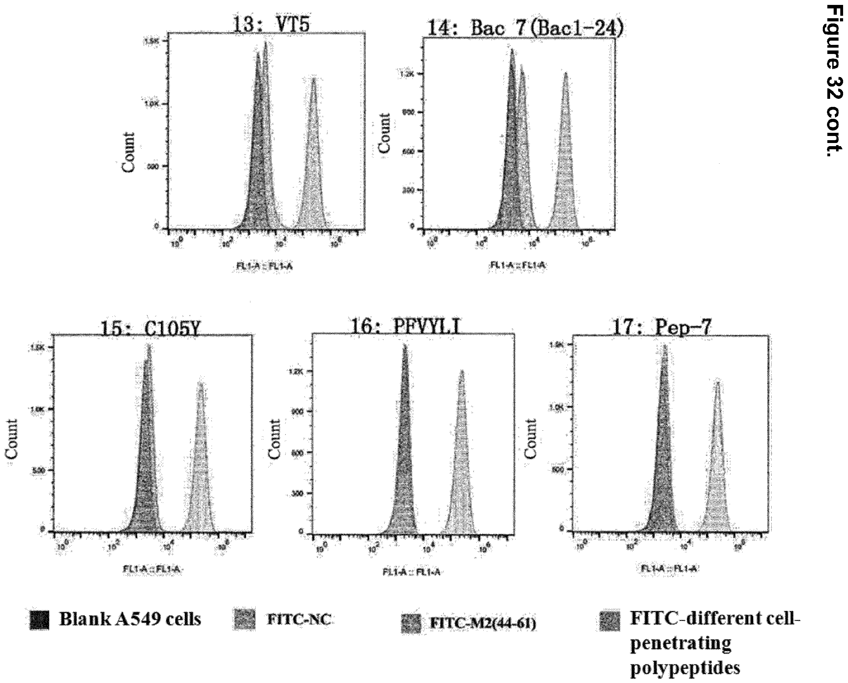

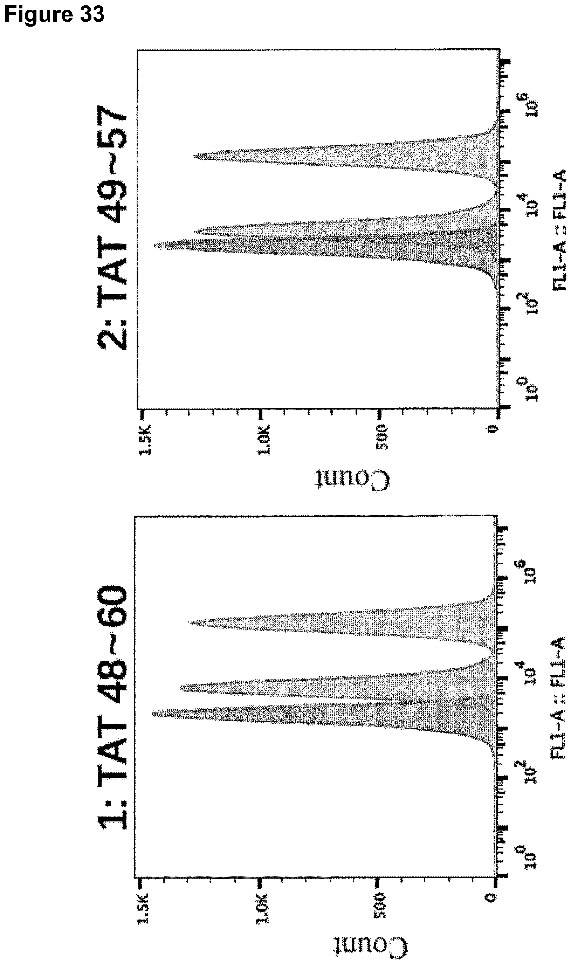

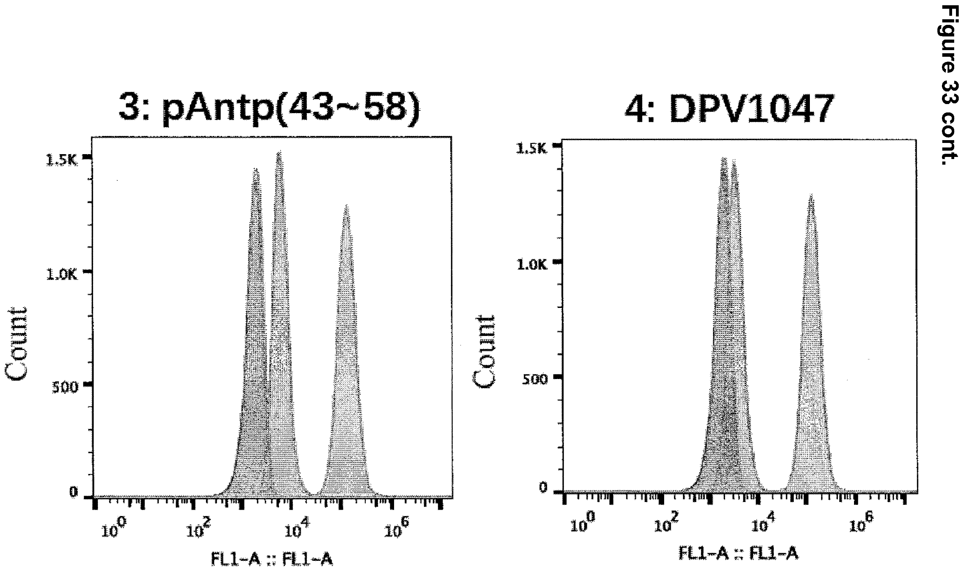

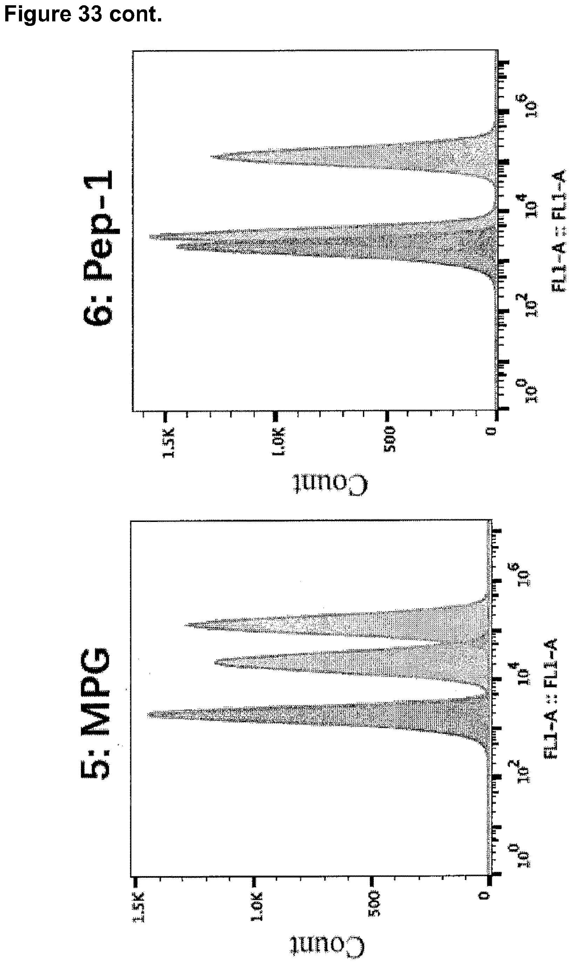

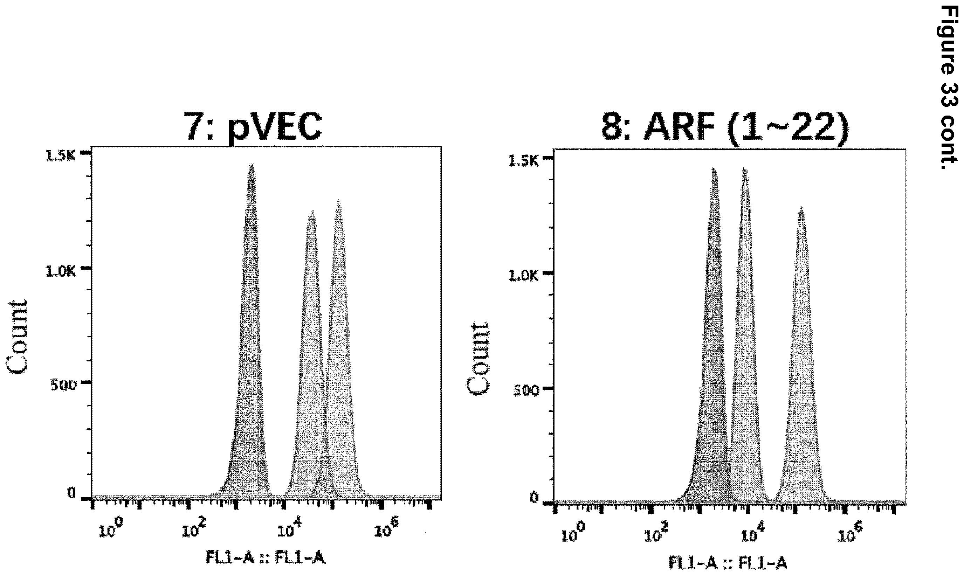

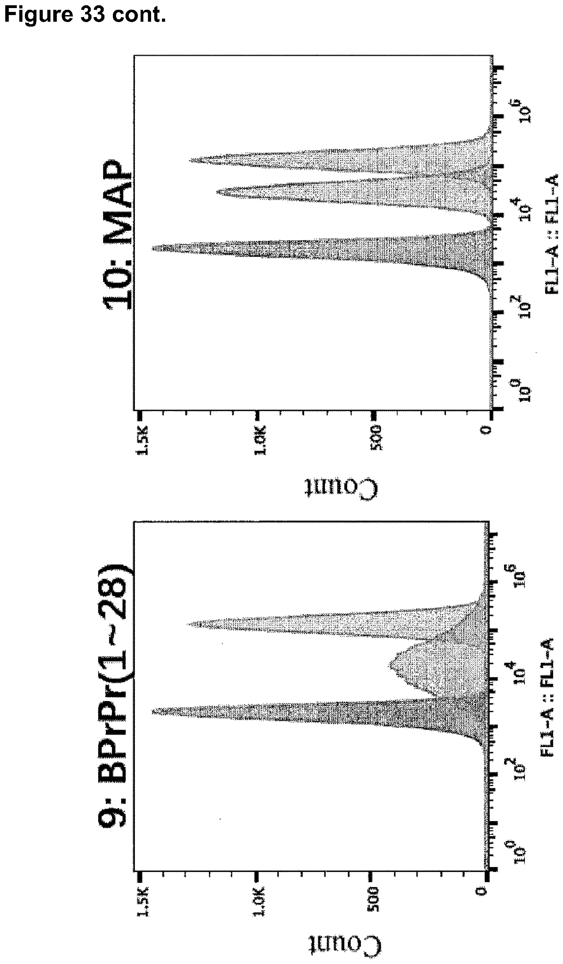

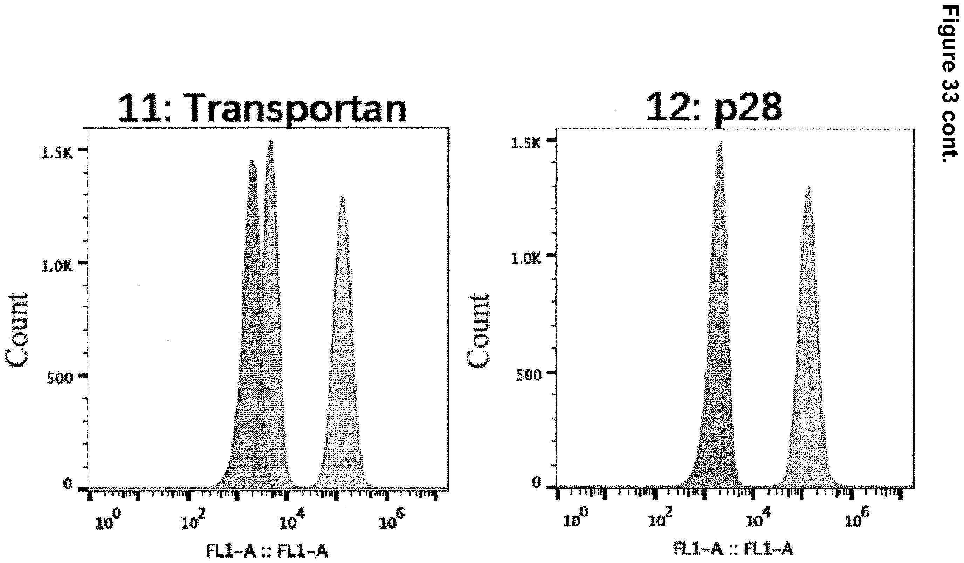

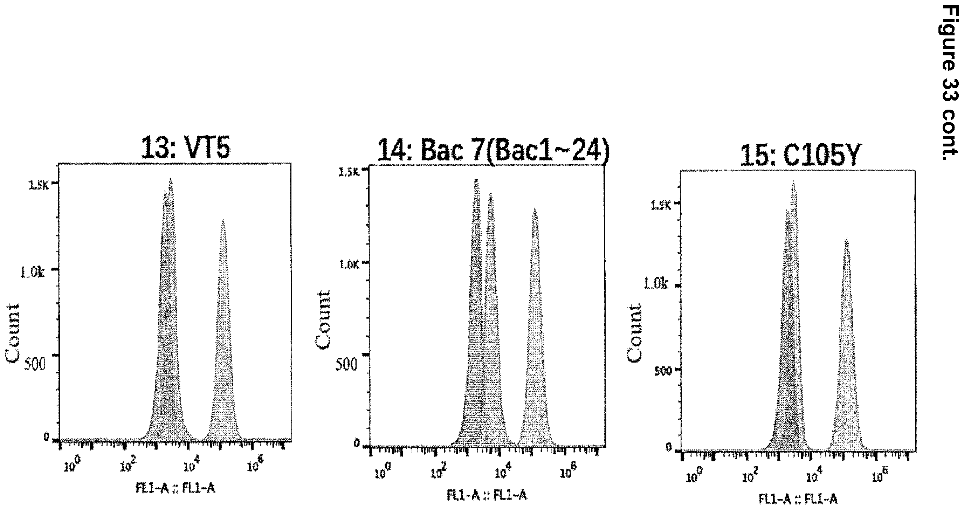

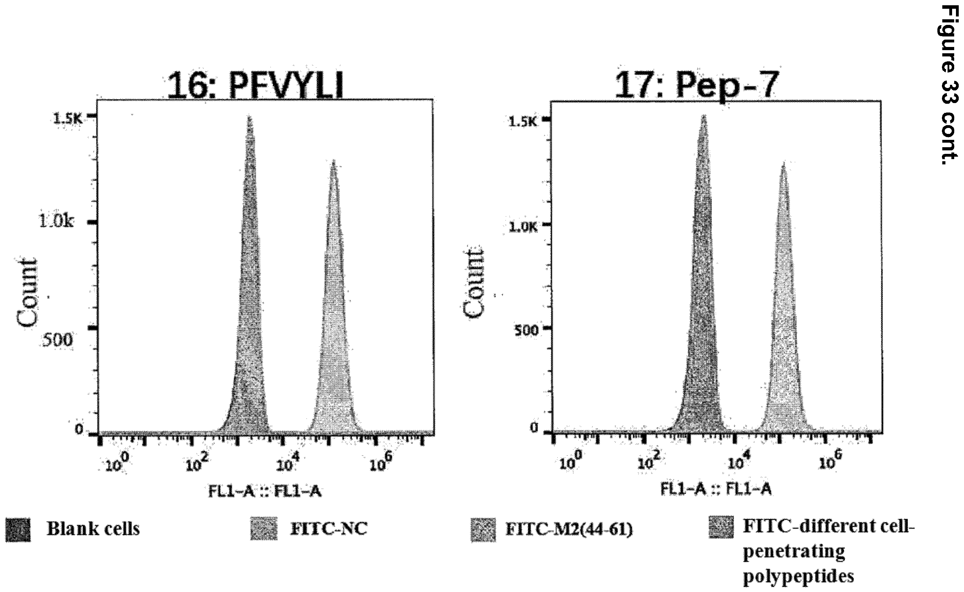

17 FITC-labeled cell-penetrating peptides that have been reported were synthesized (see Table 4). Cell entry effects of Jilin-2004(H5N1)-M2-45-62, Jilin-2004(H5N1)-M2-44-61 and Jilin-2004(H5N1)-M2-44-65 polypeptides based on influenza virus M2 protein and the above 17 cell-penetrating peptides were compared using A549 cells under the conditions of 1 µM of polypeptide concentration and 1 h of incubation duration. As shown in Figure 28, Figure 29 and Figure 30, Jilin-2004(H5N1)-M2-45-62, Jilin-2004(H5N1)-M2-44-61 and Jilin-2004(H5N1)-M2-44-65 polypeptides had significantly better cell entry effects than existing cell-penetrating peptides. Similarly, comparison was performed using A549 cells under the conditions of 3 µM of polypeptide concentration and 1 h of incubation duration. As shown in the results in Figure 31, Figure 32 and Figure 33, Jilin-2004(H5N1)-M2-45-62, Jilin-2004(H5N1)-M2-44-61 and Jilin-2004(H5N1)-M2-44-65 polypeptides had significantly better cell entry effects than existing cell-penetrating peptides.

**Table 4. Sequences of existing cell-penetrating peptides**

| Polypeptide | Sequence |
|---|---|
| HIV-1 TAT48-60 | FITC-Ahx-GRKKRRQRRRPPQ |
| HIV-1 TAT49-57 | FITC-Ahx-RKKRRQRRR |
| Penetratin, pAntp(43-58) | FITC-Ahx-RQIKIWFQNRRMKWKK |
| DPV1047 | FITC-Ahx-VKRGLKLRHVRPRVTRMDV |
| MPG | FITC-Ahx-GALFLGFLGAAGSTMGAWSQPKKKRKV |
| Pep-1 | FITC-Ahx-KETWWETWWTEWSQPKKKRKV |
| pVEC | FITC-Ahx-LLIILRRRIRKQAHAHSK |
| ARF(1-22) | FITC-Ahx-MVRRFLVTLRIRRACGPPRVRV |
| BPrPr(1-28) | FITC-Ahx-MVKSKIGSWILVLFVAMWSDVGLCKKRP |
| MAP | FITC-Ahx-KLALKLALKALKAALKLA |
| Transportan | FITC-Ahx-GWTLNSAGYLLGKINLKALAALAKKIL |
| p28 | FITC-Ahx-LSTAADMQGVVTDGMASGLDKDYLKPDD |
| VT5 | FITC-Ahx-DPKGDPKGVTVTVTVTVTGKGDPKPD |
| Bac 7(Bac 1-24) | FITC-Ahx-RRIRPRPPRLPRPRPRPLPFPRPG |
| C105Y | FITC-Ahx-CSIPPEVKFNKPFVYLI |
| PFVYLI | FITC-Ahx-PFVYLI |
| Pep-7 | FITC-Ahx-SDLWEMMMVSLACQY |

### Example 7: Modification on Jilin-2004(H5Nl)-M2-44-56 polypeptide

Jilin-2004(H5N1)-M2-44-56, Jilin-2004(H5N1)-M2-45-62 and Jilin-2004(H5N1)-M2-44-65 polypeptides, which had significant cell penetrating effects, were further engineered. One or more amino acid residue(s) were mutated in the polypeptide sequence, and the effects of the amino acid mutations on the cell-penetrating peptide function were tested. The polypeptide sequences of Jilin-2004(H5N1)-M2-44-56 and its mutations are shown in Table 5.

**Table 5. Jilin-2004(H5N1)-M2-44-56 and mutant polypeptide sequences thereof**

| Polypeptide | Sequence |
|---|---|
| FITC-JL-2004(H5N1)-M2-44-56 | FITC-Ahx-DRLFFKCIYRRLK (Seq ID No. 35) |
| FITC-JL-M2-44-56-mutl | FITC-Ahx-RRLFFKCIYRRLK (Seq ID No. 39) |
| FITC-JL-M2-44-56-mut2 | FITC-Ahx-RRLFFRCIYRRLK (Seq ID No. 40) |
| FITC-JL-M2-44-56-mut3 | FITC-Ahx-RRLFFRRIYRRLK (Seq ID No. 41) |
| FITC-JL-M2-44-56-mut4 | FITC-Ahx-RRLFFRRIYRRLR (Seq ID No. 42) |
| FITC-JL-M2-44-56-mut5 | FITC-Ahx-DRIFFKCIYRRLK (Seq ID No. 43) |
| FITC-JL-M2-44-56-mut6 | FITC-Ahx-DRIFFKCIIRRLK (Seq ID No. 44) |
| FIT C-JL-M2-44-56-mut7 | FITC-Ahx-DRIFFKCIIRRIK (Seq ID No. 45) |
| FITC-JL-M2-44-56-mut8 | FITC-Ahx-DRFFFKCIYRRLK (Seq ID No. 46) |
| FITC-JL-M2-44-56-mut9 | FITC-Ahx-DRFFFKCFYRRLK (Seq ID No. 47) |
| FITC-JL-M2-44-56-mut10 | FITC-Ahx-DRFFFKCFFRRLK (Seq ID No. 48) |
| FITC-JL-M2-44-56-mut11 | FITC-Ahx-DRFFFKCFFRRFK (Seq ID No. 49) |
| FITC-JL-M2-44-56-mut12 | FITC-Ahx-RRFFFRRFFRRFR (Seq ID No. 50) |

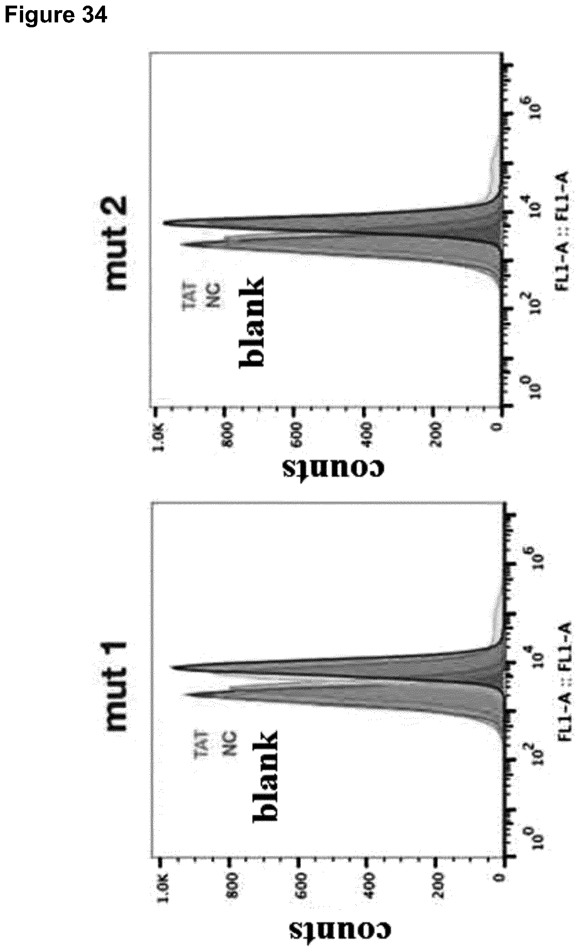

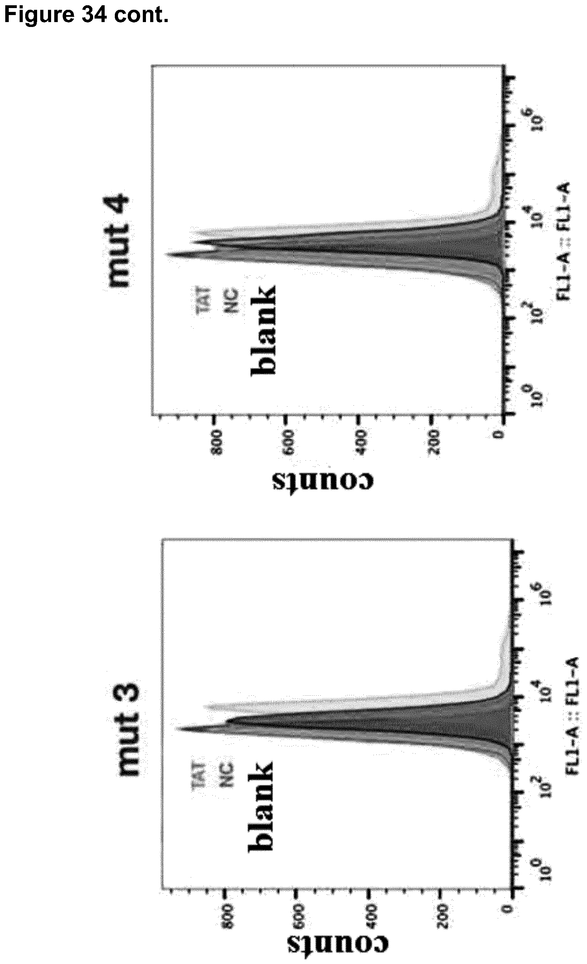

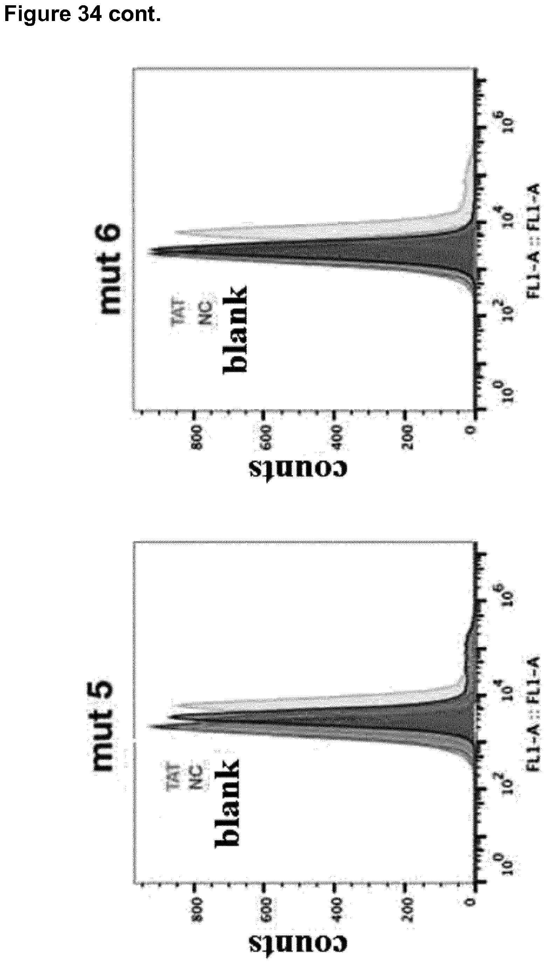

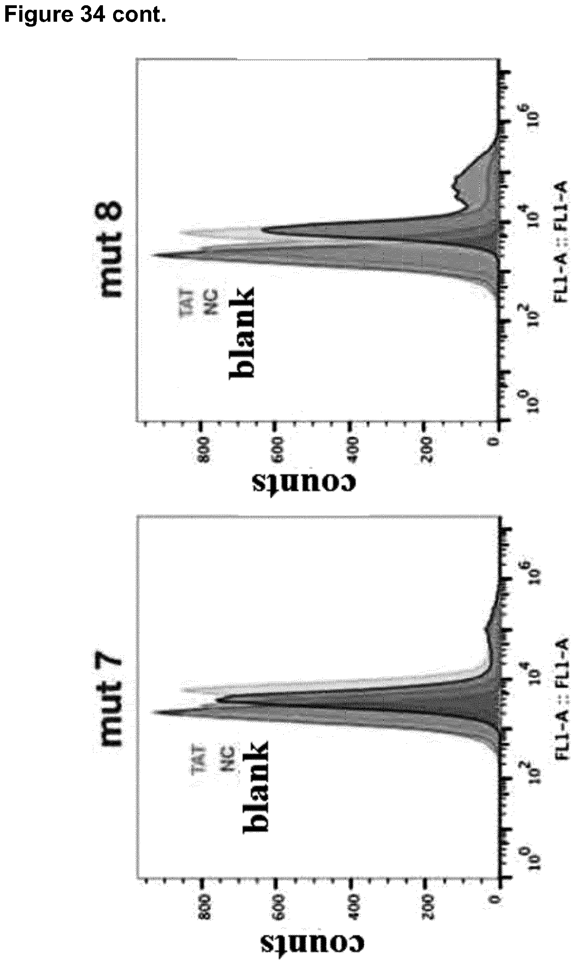

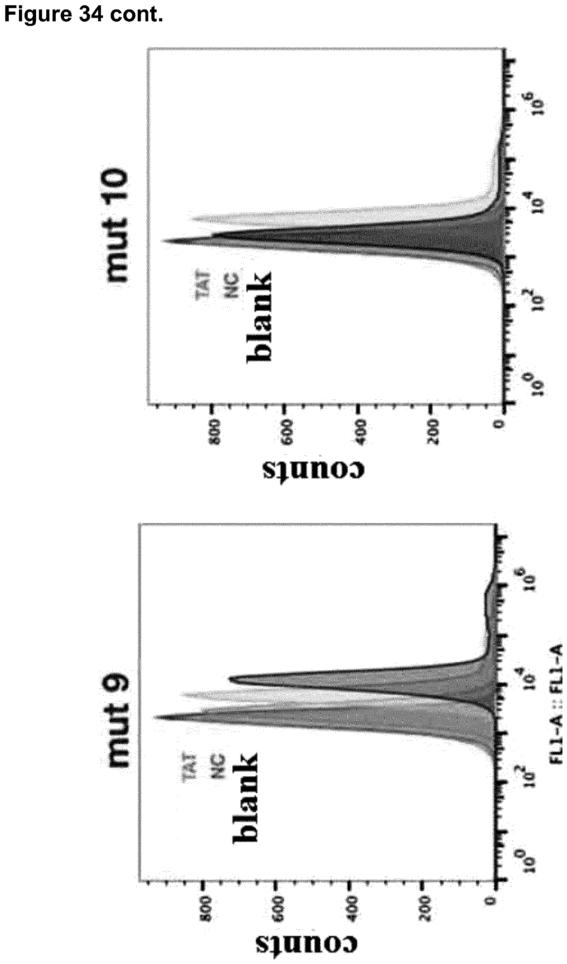

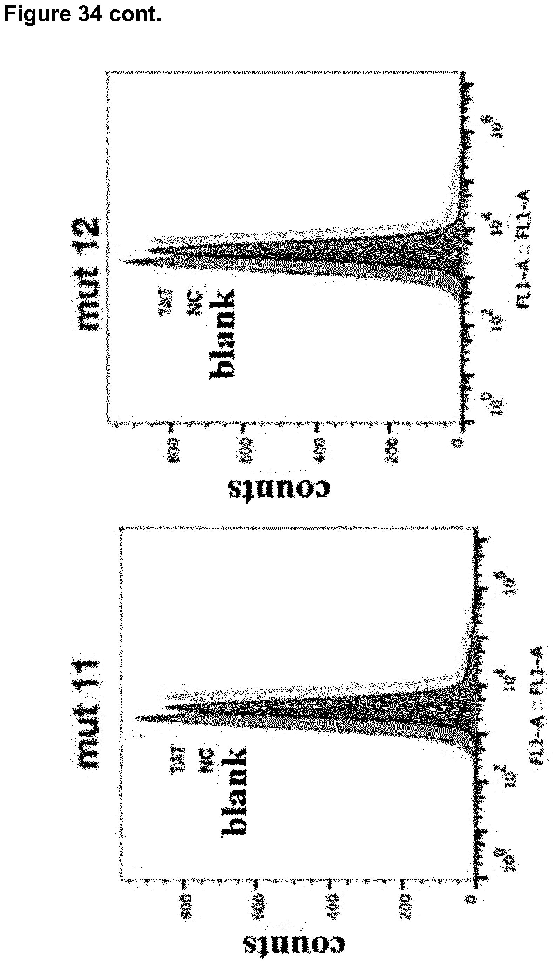

Comparison of the cell-penetrating peptide functions among 12 Jilin-2004(H5N1)-M2-44-56 mutant polypeptide sequences and the positive control TAT polypeptide and the parent Jilin-2004(H5N1)-M2-44-56 was tested by flow cytometry. According to the results shown in Figure 34, Jilin-2004(H5N1)-M2-44-56 and its 12 mutant polypeptide sequences all had cell-penetrating peptide functions, which were all stronger than that of the positive control TAT. Among them, the cell-penetrating peptide function of the mut9 mutant was enhanced compared to the parent polypeptide.

### Example 8-1: modification on Jilin-2004(H5Nl)-M2-45-62 polypeptide

Mutant polypeptide sequences based on Jilin-2004(H5N1)-M2-45-62 polypeptide were synthesized as shown in Table 6.

**Table 6. Jilin-2004(H5N1)-M2-45-62 and mutant polypeptide sequences thereof**

| Polypeptide | Sequence |
|---|---|
| FITC-JL-2004(HSN1)-M2-45-62 | FITC-Ahx-RLFFKCIYRRLKYGLKRG (Seq ID No. 7) |
| FITC-JL-2004(H5N1)-M2-45-62 mut1 | FITC-Ahx-RLFFRCIYRRLKYGLKRG (Seq ID No.51) |
| FITC-JL-2004(H5N1)-M2-45-62 mut2 | FITC-Ahx-RLFFRRIYRRLKYGLKRG (Seq ID No.52) |
| FITC-JL-2004(H5N1)-M2-45-62 mut3 | FITC-Ahx-RLFFRRIYRRLRYGLKRG (Seq ID No. 53) |
| FITC-JL-2004(H5N1)-M2-45-62 mut4 | FITC-Ahx-RLFFRRIYRRLRYRLKRG (Seq ID No. 54) |
| FITC-JL-2004(H5N1)-M2-45-62 mut5 | FITC-Ahx-RLFFRRIYRRLRYRLRRG (Seq ID No. 55) |
| FITC-JL-2004(H5N1)-M2-45-62mut6 | FITC-Ahx-RLFFRRIYRRLRYRLRRR (Seq ID No. 56) |
| FITC-JL-2004(H5N1)-M2-45-62 mut7 | FITC-Ahx-RFFFKCIYRRLKYGLKRG (Seq ID No. 57) |
| FITC-JL-2004(H5N1)-M2-45-62mut8 | FITC-Ahx-RFFFKCFYRRLKYGLKRG (Seq ID No. 58) |
| FITC-JL-2004(H5N1)-M2-45-62 mut9 | FITC-Ahx-RFFFKCFFRRLKYGLKRG (Seq ID No. 59) |
| FITC-JL-2004(H5N1)-M2-45-62 mut10 | FITC-Ahx-RFFFKCFFRRFKYGLKRG (Seq ID No. 60) |
| FITC-JL-2004(H5N1)-M2-45-62 mut11 | FITC-Ahx-RFFFKCFFRRFKFGLKRG (Seq ID No. 61) |
| FITC-JL-2004(H5N1)-M2-45-62 mutl2 | FITC-Ahx-RFFFKCFFRRFKFGFKRG (Seq ID No. 62) |
| FITC-JL-2004(H5N1)-M2-45-62 mutl3 | FITC-Ahx-RFFFRRFFRRFRFRFRRR (Seq ID No. 63) |

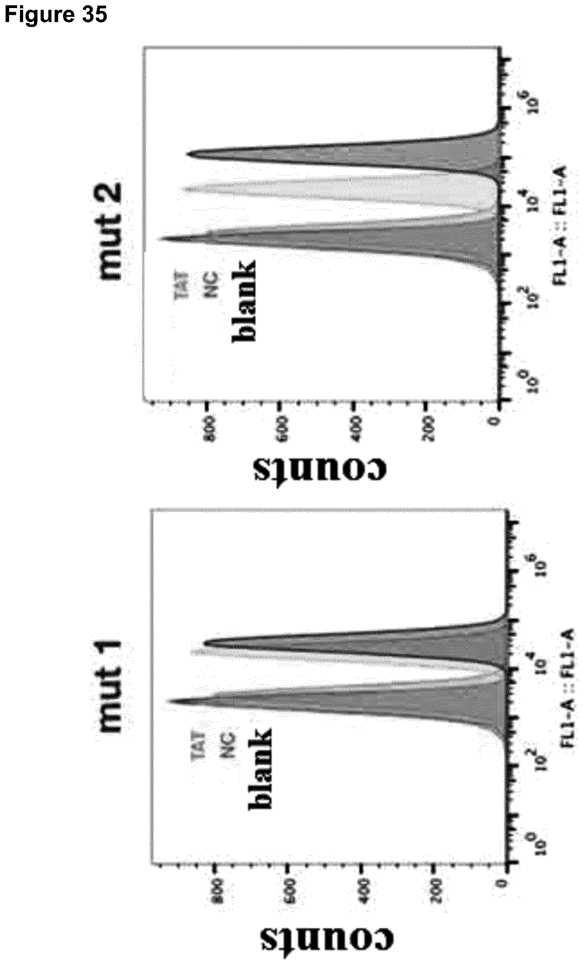

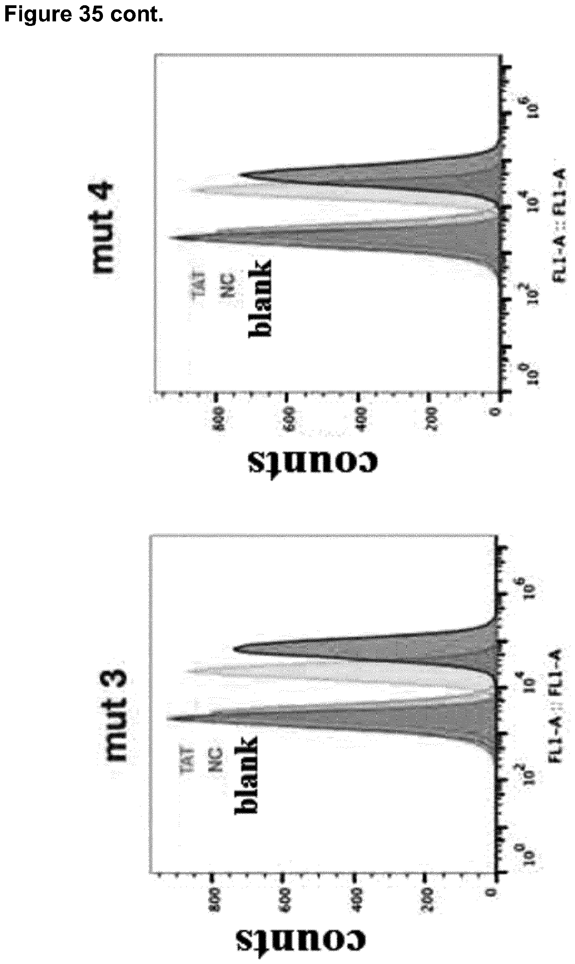

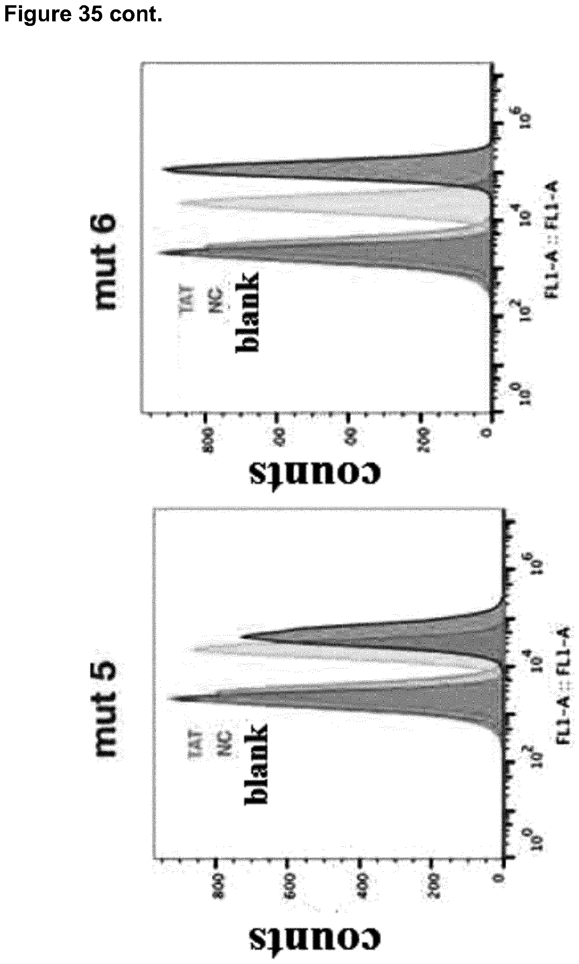

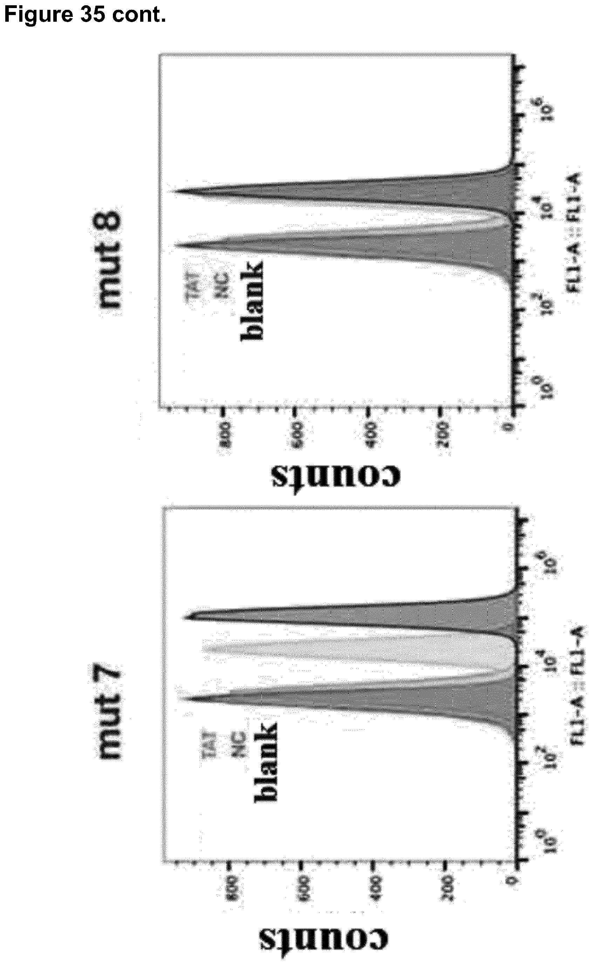

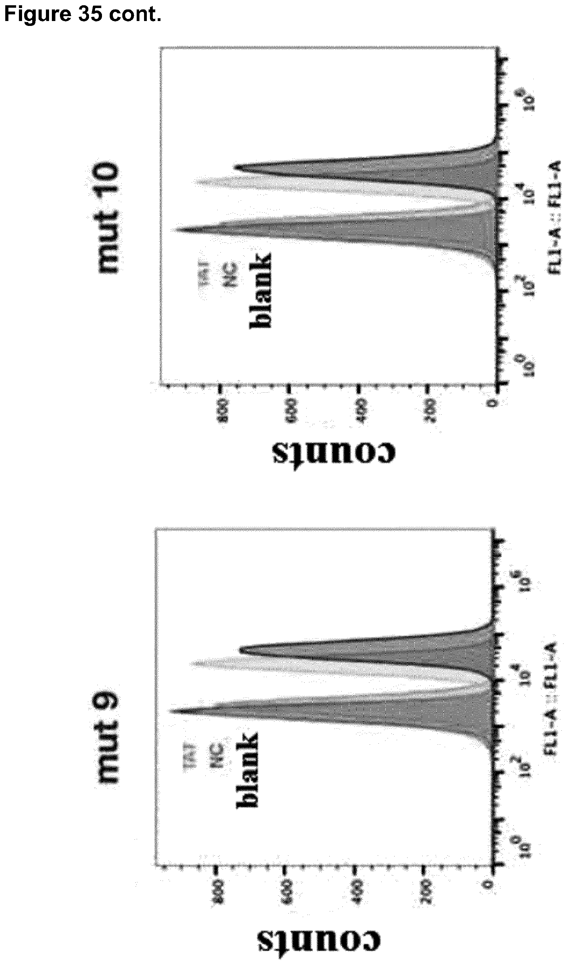

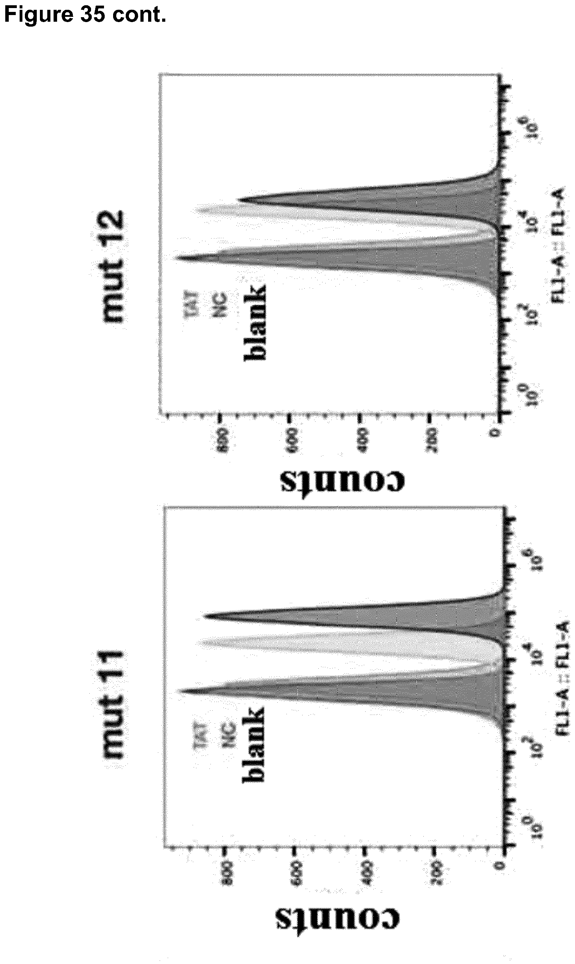

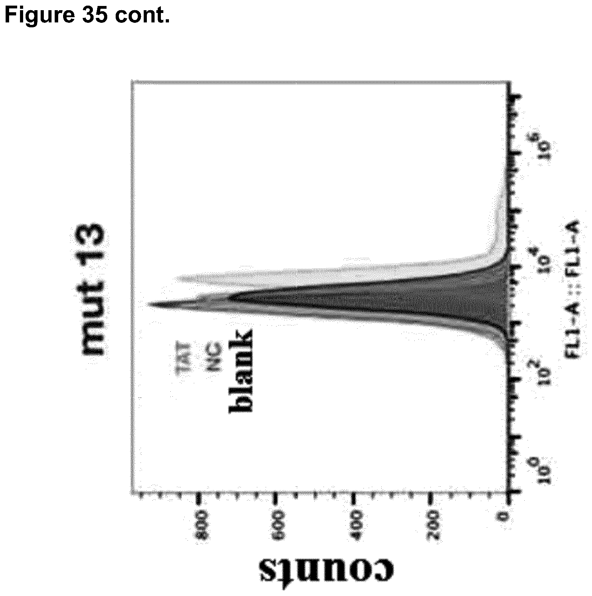

Comparison of the cell-penetrating peptide functions among 13 Jilin-2004(H5N1)-M2-45-62 mutant polypeptide sequences and the positive control TAT polypeptide and the parent Jilin-2004(H5N1)-M2-45-62 was tested by flow cytometry. According to the results shown in Figure 35, Jilin-2004(H5N1)-M2-45-62 and its 13 mutant polypeptide sequences all had cell-penetrating peptide functions, which were all stronger than that of the positive control TAT. It is noticed that the cell-penetrating peptide functions of all 13 Jilin-2004(H5N1)-M2-45-62 mutant polypeptides were stronger than that of the parent Jilin-2004(H5N1)-M2-45-62 polypeptide.

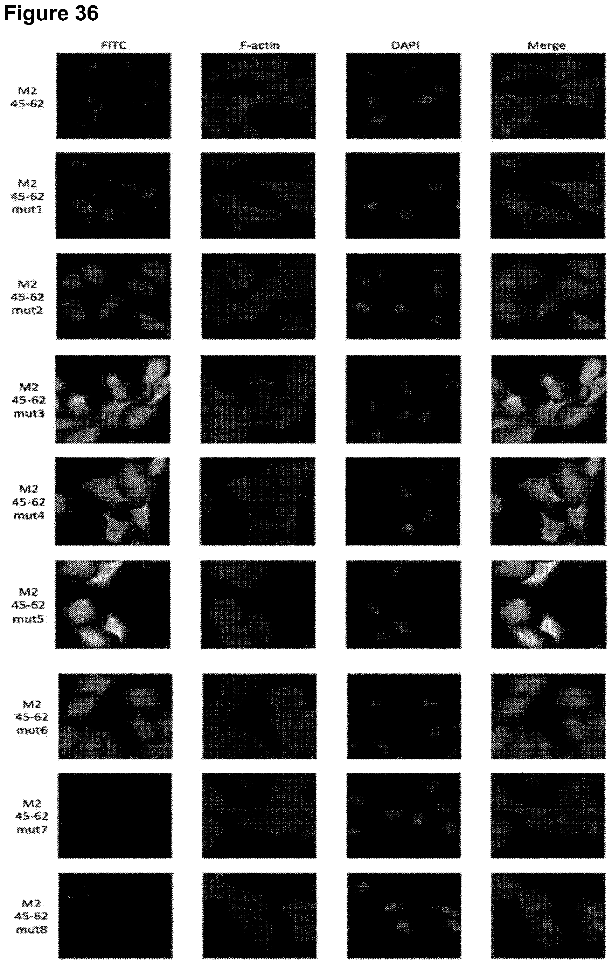

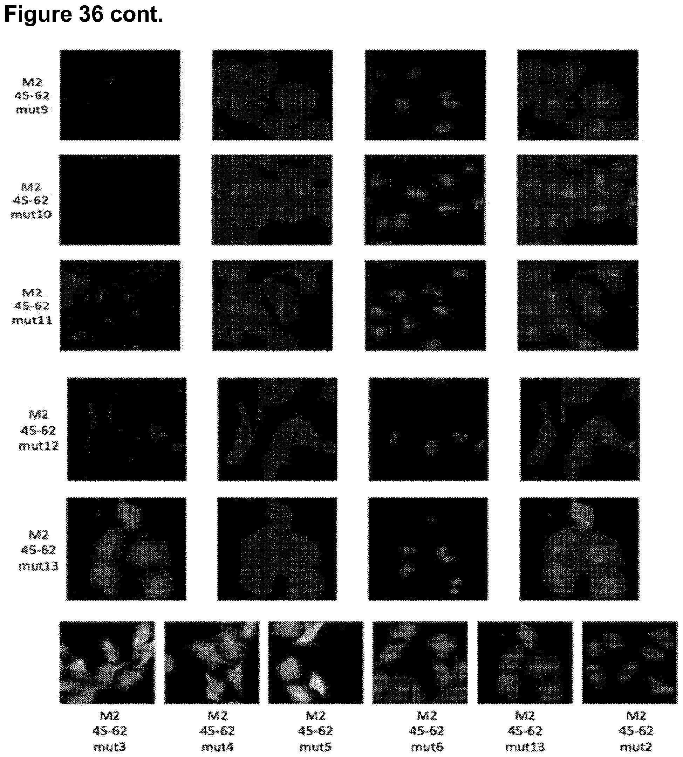

The cell-penetrating peptide functions of Jilin-2004(H5N1)-M2-45-62 and its mutants were tested by imaging using confocal laser technology. The results are shown in Figure 36. Jilin-2004(H5N1)-M2-45-62 and its 13 mutant polypeptide sequences all had cell-penetrating peptide functions. And the cell-penetrating peptide functions of mut2, mut3, mut4, mut5, mut6 and mut13 were stronger than that of theparent Jilin-2004(H5N1)-M2-45-62 polypeptide.

In addition, single site mutant polypeptide sequences based on Jilin-2004(H5N1)-M2-45-62 polypeptide were synthesized as shown in Table 7, and their cell-penetrating peptide functions were tested.

**Table 7. Jilin-2004(H5N1)-M2-45-62 and single site mutant polypeptide sequences thereof**

| Polypeptide | Sequence |
|---|---|
| FITC-JL-2004(HSN1)-M2-45 -62 | FITC-Ahx-RLFFKCIYRRLKYGLKRG (Seq ID No. 7) |
| FITC-JL-2004(H5N1)-M2-45-62 mut45 | FITC-Ahx-SLFFKCIYRRLKYGLKRG (Seq ID No. 64) |
| FITC-JL-2004(H5N1)-M2-45-62 mut46 | FITC-Ahx-RAFFKCIYRRLKYGLKRG (Seq ID No. 65) |
| FITC-JL-2004(H5N1)-M2-45-62 mut47 | FITC-Ahx-RLIFKCIYRRLKYGLKRG (Seq ID No. 66) |
| FITC-JL-2004(H5N1)-M2-45-62 mut48 | FITC-Ahx-RLFIKCIYRRLKYGLKRG (Seq ID No. 67) |
| FITC-JL-2004(H5N1)-M2-45-62 mut49 | FITC-Ahx-RLFFRCIYRRLKYGLKRG (Seq ID No. 51) |
| FITC-JL-2004(H5N1)-M2-45-62 mut50 | FITC-Ahx-RLFFKIIYRRLKYGLKRG (Seq ID No. 69) |
| FITC-JL-2004(H5N1)-M2-45-62mut51 | FITC-Ahx-RLFFKCAYRRLKYGLKRG (Seq ID No. 70) |
| FITC-JL-2004(H5N1)-M2-45-62 mut52 | FITC-Ahx-RLFFKCIKRRLKYGLKRG (Seq ID No. 71) |
| FITC-JL-2004(H5N1)-M2-45-62 mut53 | FITC-Ahx-RLFFKCIYSRLKYGLKRG (Seq ID No. 72) |
| FITC-JL-2004(H5N1)-M2-45-62 mut54 | FITC-Ahx-RLFFKCIYRSLKYGLKRG (Seq ID No. 73) |
| FITC-JL-2004(H5N1)-M2-45-62 mut55 | FITC-Ahx-RLFFKCIYRRAKYGLKRG (Seq ID No. 74) |
| FITC-JL-2004(H5N1)-M2-45-62 mut56 | FITC-Ahx-RLFFKCIYRRLTYGLKRG (Seq ID No. 75) |
| FITC-JL-2004(H5N1)-M2-45-62 mut57 | FITC-Ahx-RLFFKCIYRRLKKGLKRG (Seq ID No. 76) |
| FITC-JL-2004(HSN1)-M2-45-62 mut58 | FITC-Ahx-RLFFKCIYRRLKYKLKRG (Seq ID No. 77) |
| FITC-JL-2004(HSN1)-M2-45-62 mut59 | FITC-Ahx-RLFFKCIYRRLKYGAKRG (Seq ID No. 78) |
| FITC-JL-2004(HSN1)-M2-45-62 mut60 | FITC-Ahx-RLFFKCIYRRLKYGLTRG (Seq ID No. 79) |
| FITC-JL-2004(HSN1)-M2-45-62 mut61 | FITC-Ahx-RLFFKCIYRRLKYGLKTG (Seq ID No. 80) |
| FITC-JL-2004(HSN1)-M2-45-62 mut62 | FITC-Ahx-RLFFKCIYRRLKYGLKRK (Seq ID No. 81) |

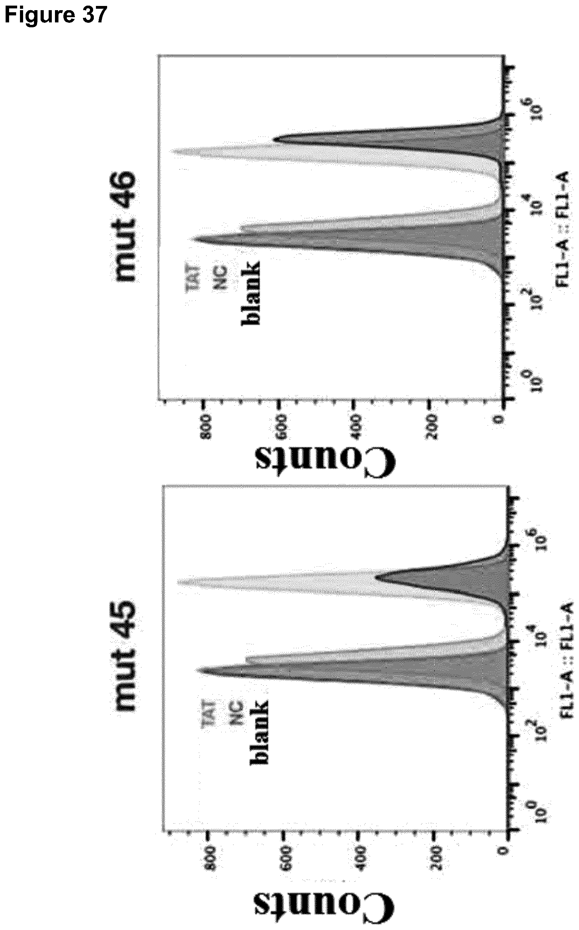

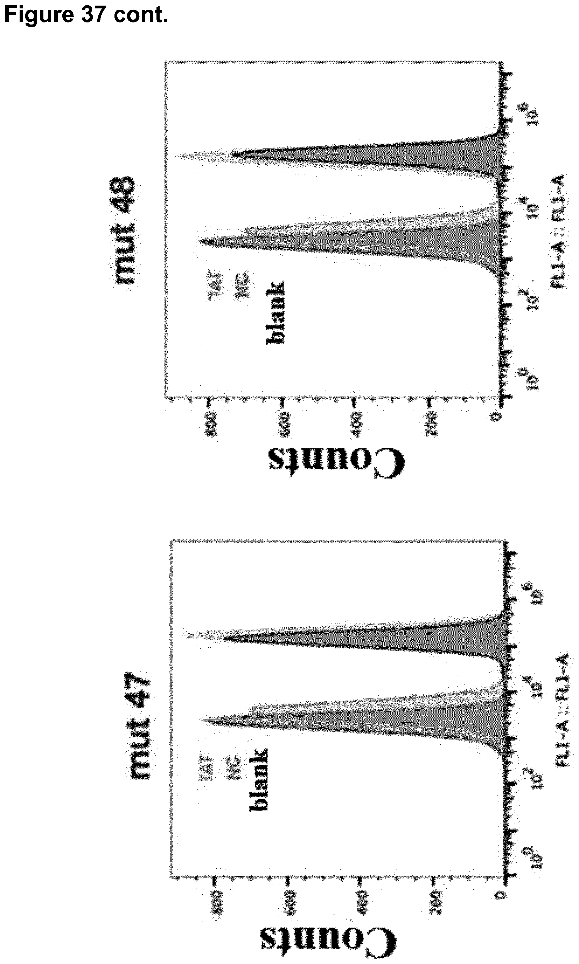

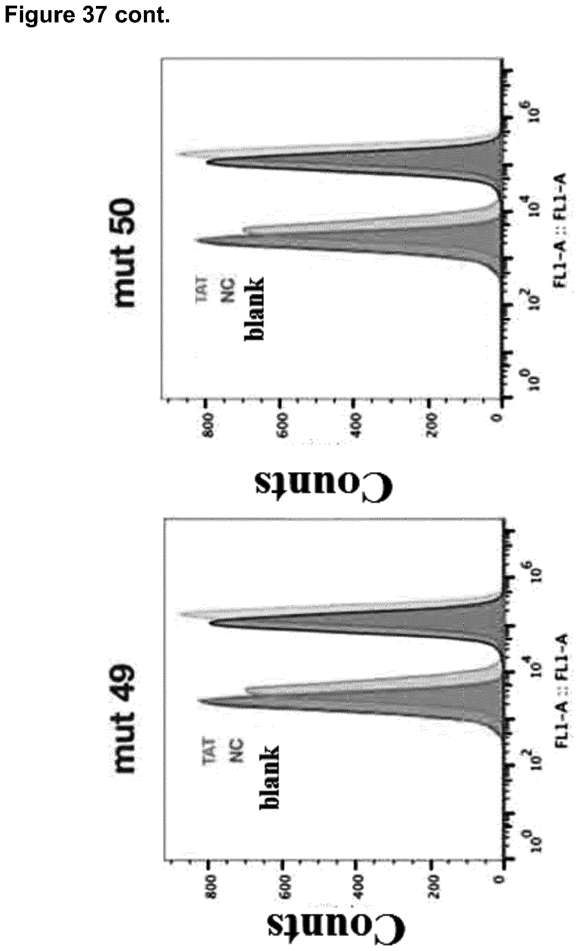

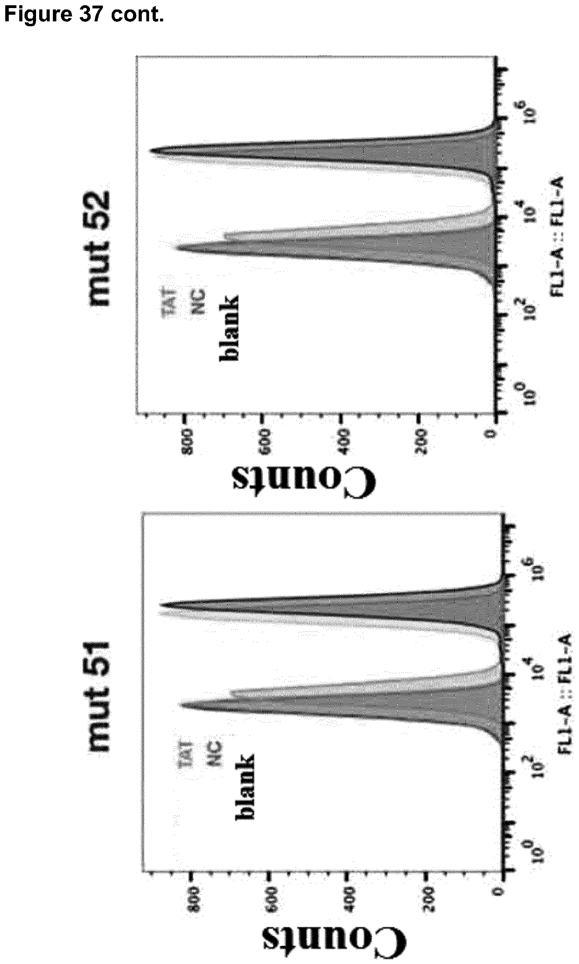

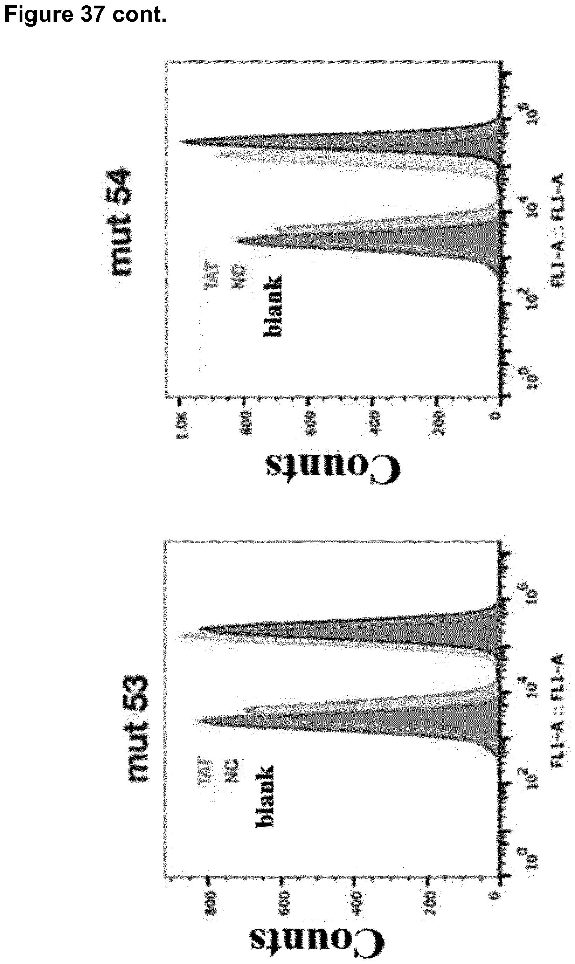

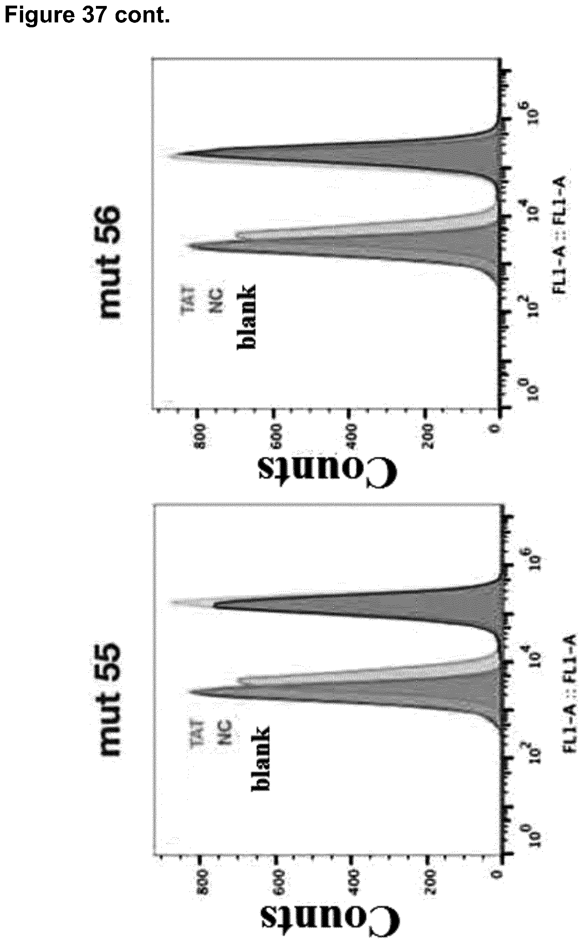

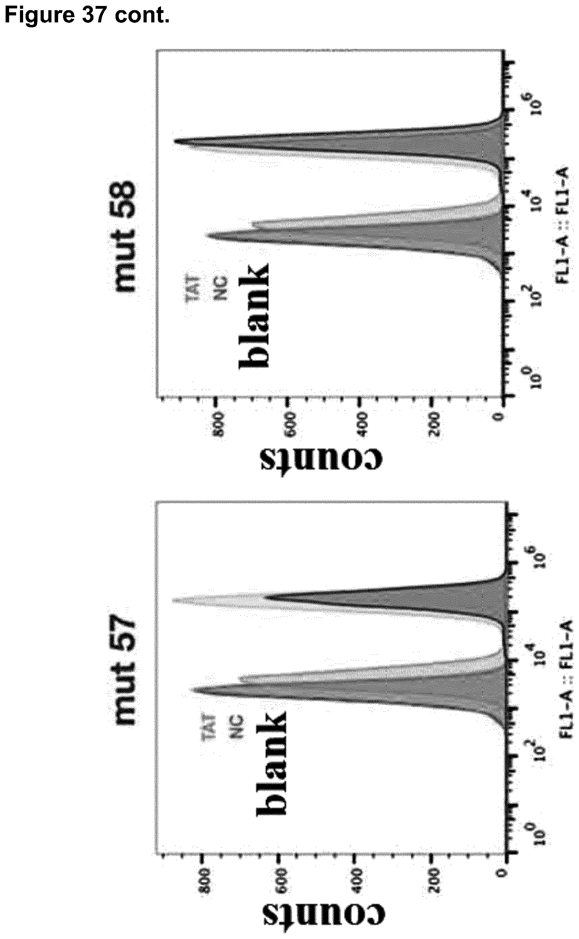

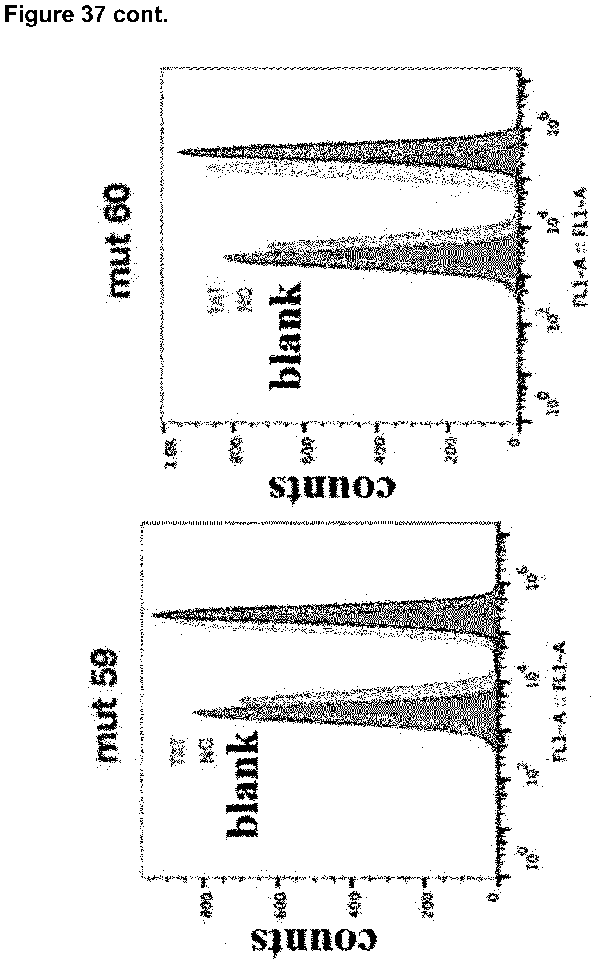

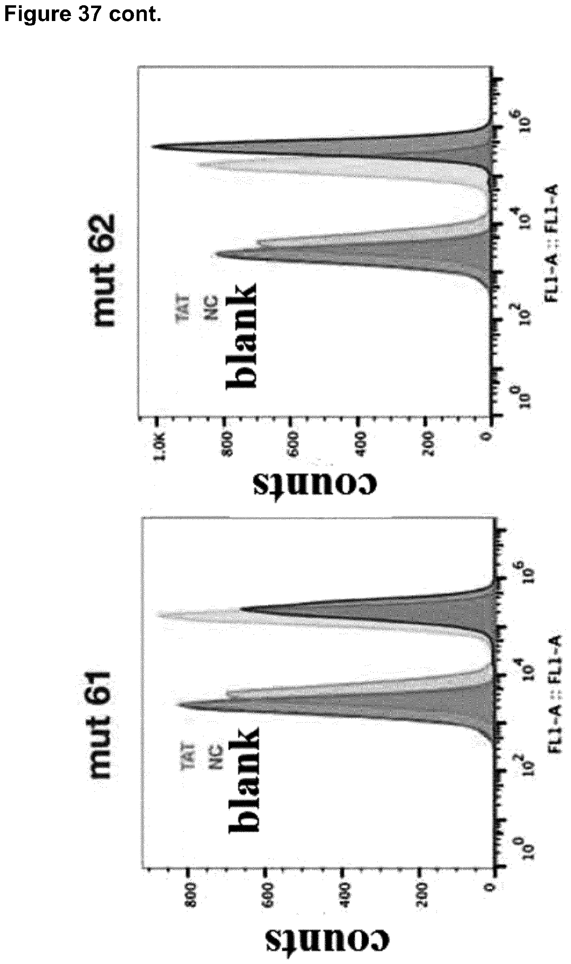

Comparison of the cell-penetrating peptide functions among 18 Jilin-2004(H5N1)-M2-45-62 single site mutant polypeptide sequences and the positive control TAT polypeptide and the parent Jilin-2004(H5N1)-M2-45-62 was tested by flow cytometry. According to the results shown in Figure 37, Jilin-2004(H5N1)-M2-45-62 and its 18 mutant polypeptide sequences all had cell-penetrating peptide functions, which were all stronger than that of the positive control TAT. It is noticed that the cell-penetrating peptide functions of mut46, mut51, mut52, mut53, mut54, mut58, mut59, mut60 and mut62 single site mutant sequences were all stronger than that of the parent Jilin-2004(H5N1)-M2-45-62 polypeptide.

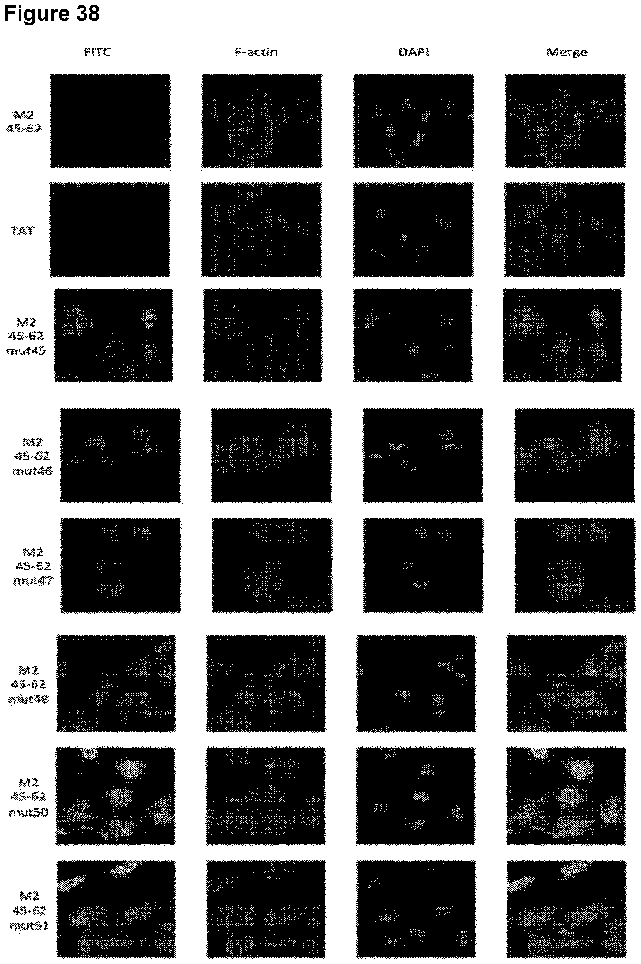

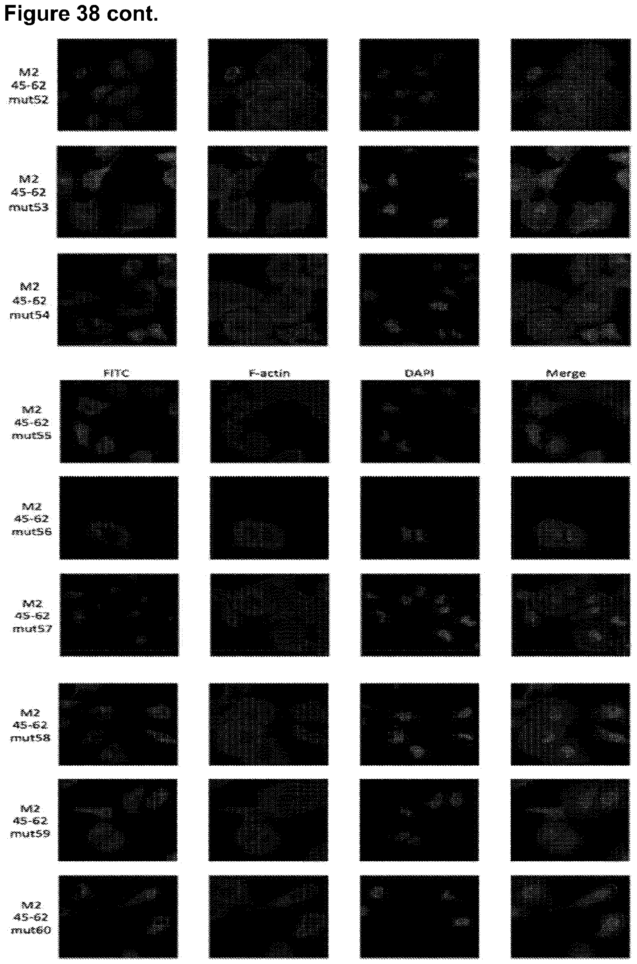

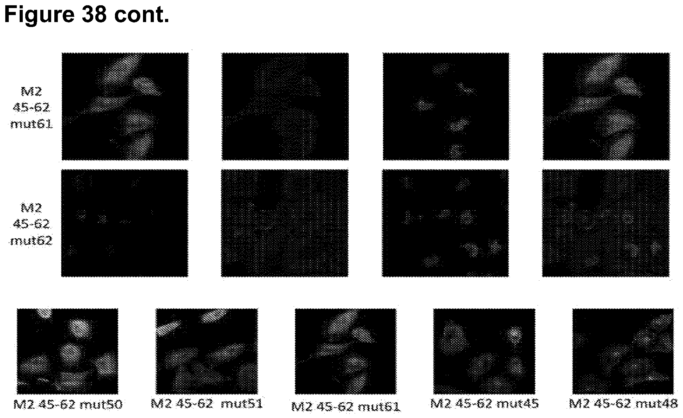

The cell-penetrating peptide functions of Jilin-2004(H5N1)-M2-45-62 and its single site mutants were tested by imaging using confocal laser technology. The results are shown in Figure 38. Jilin-2004(H5N1)-M2-45-62 and its 18 single site mutant polypeptide sequences all had cell-penetrating peptide functions. And the cell-penetrating peptide functions of mut45, mut48, mut50, mut51 and mut61 were stronger than that of the parent Jilin-2004(H5N1)-M2-45-62 polypeptide.

### Example 8-2: modification on Jilin-2004(H5Nl)-M2-44-61 polypeptide

Mutant polypeptide sequences based on Jilin-2004(H5N1)-M2-44-61 polypeptide were synthesized as shown in Table 8.

**Table 8. Jilin-2004(H5N1)-M2-44-61 and mutant polypeptide sequences thereof**

| Polypeptide | Sequence |
|---|---|
| FITC-JL-2004(H5N1)-M2-44-61 | FITC-Ahx-DRLFFKCIYRRLKYGLKR (Seq ID No. 12) |
| FITC-JL-2004(H5N1)-M2-44-61 mut1 | FITC-Ahx-RRLFFKCIYRRLKYGLKR (Seq ID No. 82) |
| FITC-JL-2004(H5N1)-M2-44-61 mut2 | FITC-Ahx-LRLFFKCIYRRLKYGLKR (Seq ID No. 83) |
| FITC-JL-2004(H5N1)-M2-44-61 mut3 | FITC-Ahx-DILFFKCIYRRLKYGLKR (Seq ID No. 84) |
| FITC-JL-2004(H5N1)-M2-44-61 mut4 | FITC-Ahx-DRRFFKCIYRRLKYGLKR (Seq ID No. 85) |
| FITC-JL-2004(H5N1)-M2-44-61 mut5 | FITC-Ahx-DRLRRKCIYRRLKYGLKR (Seq ID No. 86) |
| FITC-JL-2004(H5N1)-M2-44-61 mut6 | FITC-Ahx-DRLFFICIYRRLKYGLKR (Seq ID No. 87) |
| FITC-JL-2004(H5N1)-M2-44-61 mut7 | FITC-Ahx-DRLFFKRJYRRLKYGLKR (Seq ID No. 88) |
| FITC-JL-2004(H5N1)-M2-44-61 mut8 | FITC-Ahx-DRLFFKIIYRRLKYGLKR (Seq ID No. 89) |
| FITC-JL-2004(H5N1)-M2-44-61 mut9 | FITC-Ahx-DRLFFKCKKRRLKYGLKR (Seq ID No. 90) |
| FITC-JL-2004(H5N1)-M2-44-61 mut10 | FITC-Ahx-DRRRRKCRRRRRKYGRKR (Seq ID No. 91) |
| FITC-JL-2004(H5N1)-M2-44-61 mut12 | FITC-Ahx-DRLFFKCIYRRRKRGLKR (Seq ID No. 92) |
| FITC-JL-2004(H5N1)-M2-44-61 mut13 | FITC-Ahx-DRLFFKCIYRRLKYGRKR (Seq ID No. 93) |
| FITC-JL-2004(H5N1)-M2-44-61 mut14 | FITC-Ahx-DRKRRKCKYRRKKYGRKR (Seq ID No. 94) |
| FITC-JL-2004(H5N1)-M2-44-61 mut16 | FITC-Ahx-DRLFFKCIYRRLKYGLII (Seq ID No. 95) |
| FITC-JL-2004(H5N1)-M2-44-61 mut20 | FITC-Ahx-IILFFKCIYRRLKYGLKR (Seq ID No. 96) |

Comparison of the cell-penetrating peptide functions among 15 Jilin-2004(H5N1)-M2-44-61 mutant polypeptide sequences and the positive control TAT polypeptide and the parent Jilin-2004(H5N1)-M2-44-61 was tested by flow cytometry.

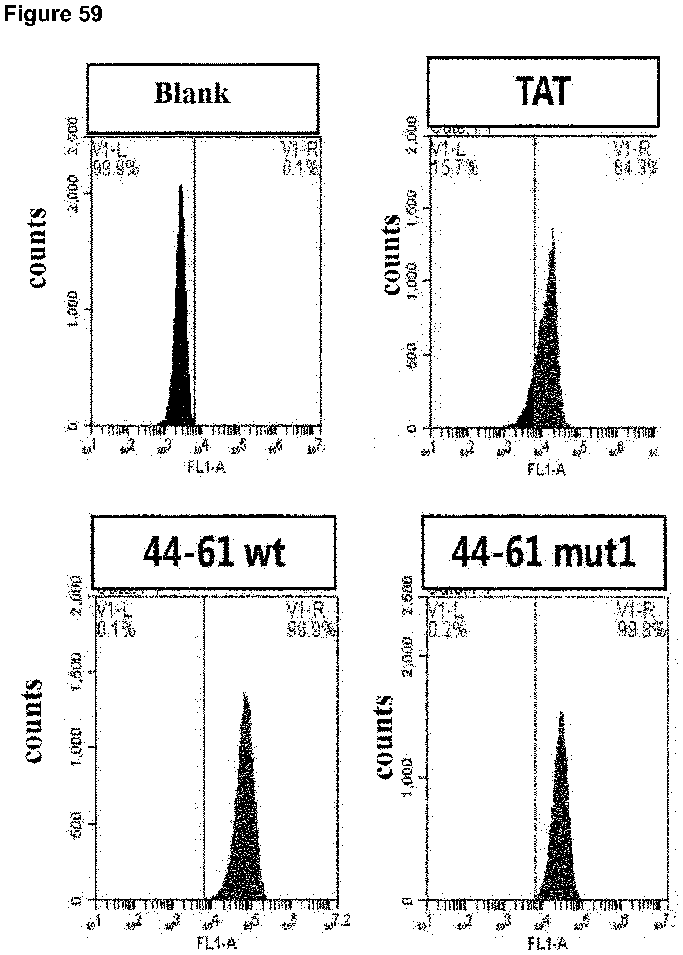

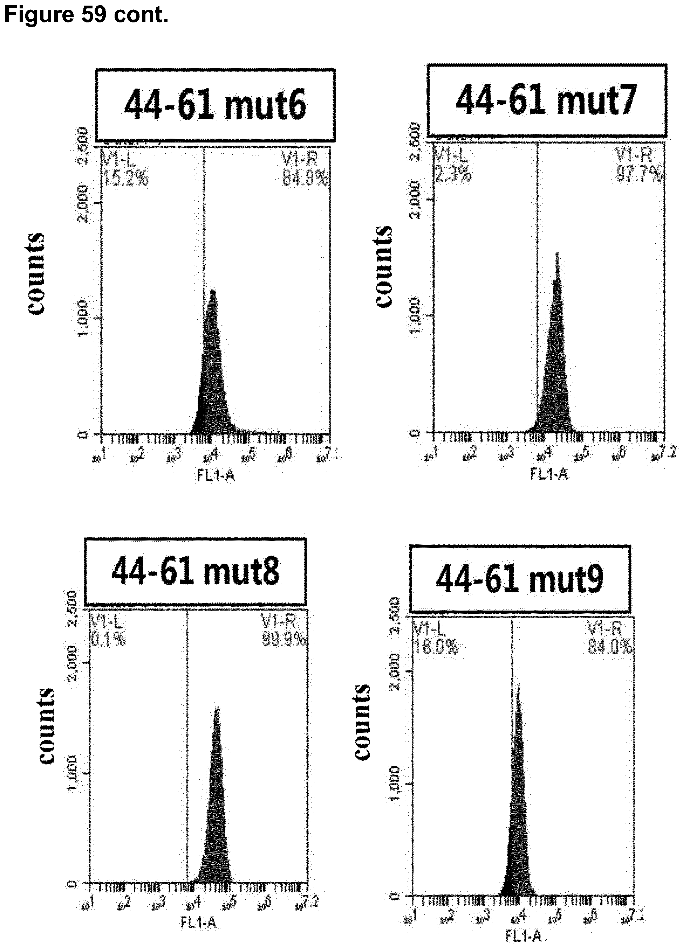

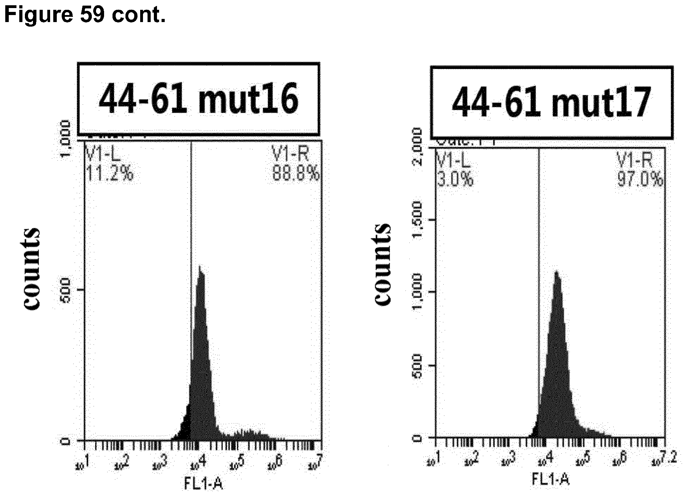

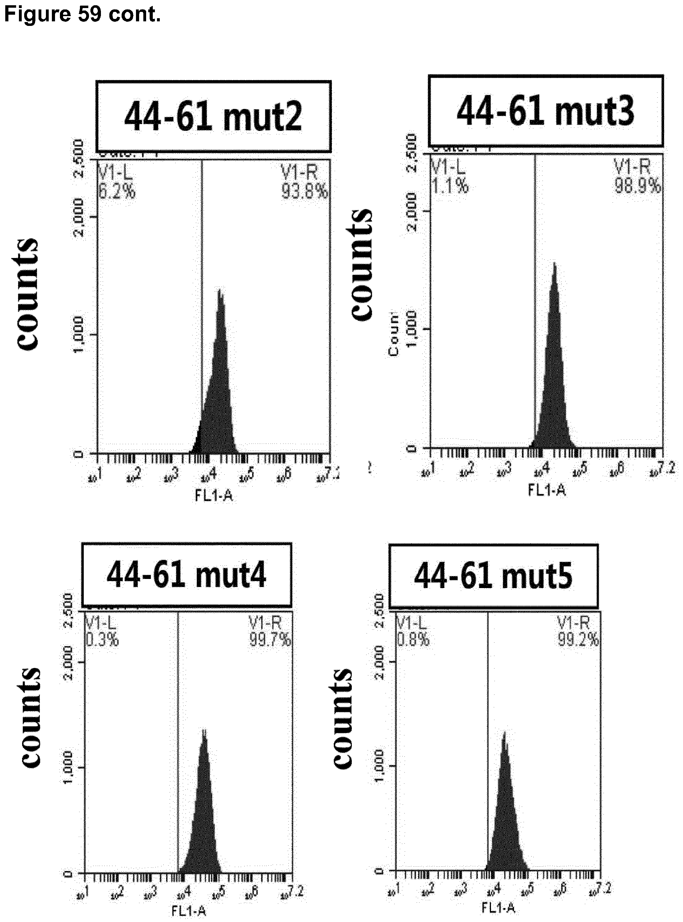

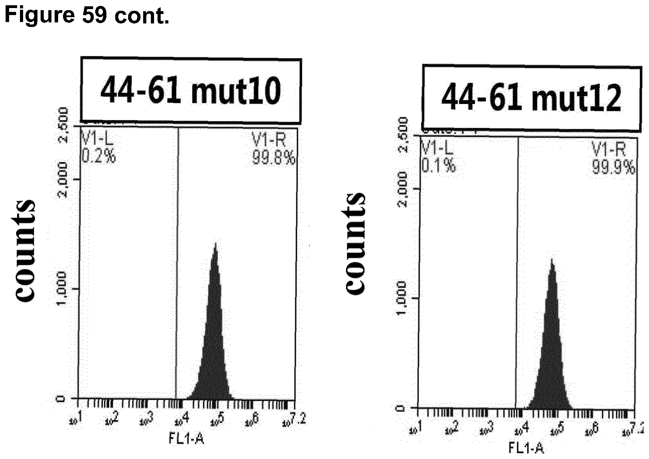

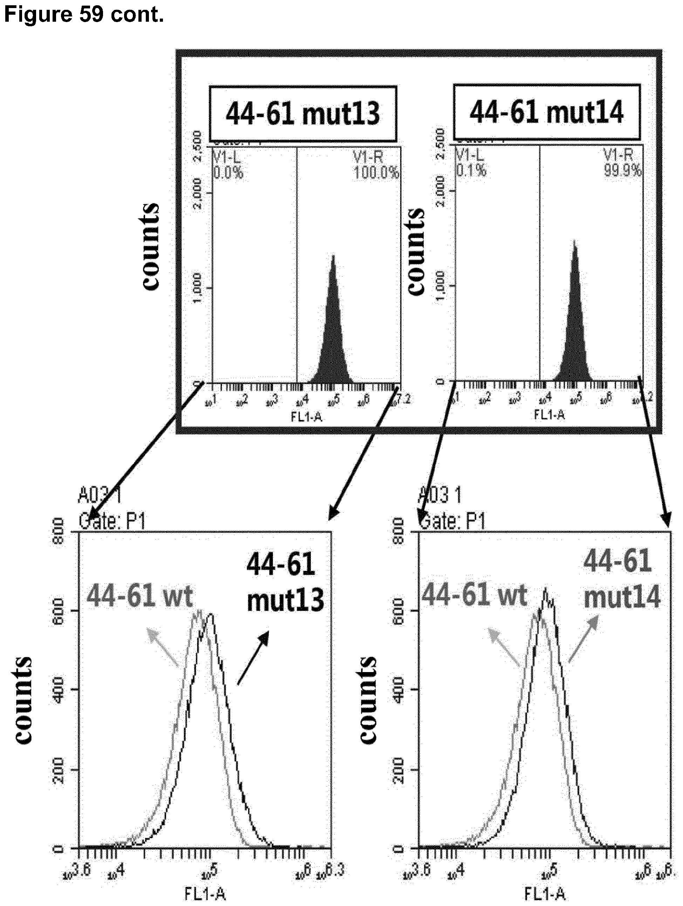

As shown in Figure 59, when using A549 cells and at a polypeptide concentration of 1 µM, Jilin-2004(H5N1)-M2-44-61 polypeptide and its 15 mutants all had cell-penetrating peptide functions, which were all stronger than that of the positive control TAT. Among them, the cell-penetrating peptide functions of mut13 and mut14 were stronger than that of the parent Jilin-2004(H5N1)-M2-44-61 polypeptide.

### Example 9: modification on Jilin-2004(H5N1)-M2-44-65 polypeptide

Mutant polypeptide sequences based on Jilin-2004(H5N1)-M2-44-65 polypeptide were synthesized as shown in Table 9.

**Table 9. Jilin-2004(H5N1)-M2-44-65 and mutant polypeptide sequences thereof**

| Polypeptide | Sequence |
|---|---|
| FITC-JL-2004(H5N1)-M2-44-65 | FITC-Ahx-DRLFFKCIYRRLKYGLKRGPST (Seq ID No. 26) |
| FITC-JL-2004(H5N1)-M2-44-65 mut1 | FITC-Ahx-DRFFFKCIYRRLKYGLKRGPST (Seq ID No. 97) |
| FITC-JL-2004(H5N1)-M2-44-65 mut2 | FITC-Ahx-DRFFFKCIYRRLKYGLKRRPST (Seq ID No. 98) |
| FITC-JL-2004(H5N1)-M2-44-65 mut3 | FITC-Ahx-DRFFFKCIYRRLKFGLKRGPST (Seq ID No. 99) |
| FITC-JL-2004(H5N1)-M2-44-65 mut4 | FITC-Ahx-DRFFFKCIFRRLKYGLKRGPST (Seq ID No. 100) |
| FITC-JL-2004(H5N1)-M2-44-65 mut5 | FITC-Ahx-DRFFFKCIFRRLKFGLKRGPST (Seq ID No. 101) |
| FITC-JL-2004(H5N1)-M2-44-65 mut6 | FITC-Ahx-DRFFFKCFFRRFKFGLKRGPST (Seq ID No. 102) |
| FITC-JL-2004(H5N1)-M2-44-65 mut7 | FITC-Ahx-RRFFFKCFFRRFKFGLKRGPST (Seq ID No. 103) |
| FITC-JL-2004(H5N1)-M2-44-65 mut8 | FITC-Ahx-DRFFFKRIYRRLKYRLKRRPST (Seq ID No. 104) |
| FITC-JL-2004(H5N1)-M2-44-65 mut9 | FITC-Ahx-DRFFFKRIYRRLKYRLKRRPKV (Seq ID No. 105) |
| FITC-JL-2004(H5N1)-M2-44-65 mut10 | FITC-Ahx-DRFFFKRIFRRLKYRLKRRPST (Seq ID No. 106) |
| FITC-JL-2004(H5N1)-M2-44-65 mut11 | FITC-Ahx-DRFFFKRIFRRLKFRLKRRPST (Seq ID No. 107) |
| FITC-JL-2004(H5N1)-M2-44-65 mut12 | FITC-Ahx-DRFFFKRIFRRFKFRLKRRPST (Seq ID No. 108) |
| FITC-JL-2004(H5N1)-M2-44-65 mut13 | FITC-Ahx-RRFFFKRIFRRFKFRLKRRPST (Seq ID No. 109) |
| FITC-JL-2004(H5N1)-M2-44-65 mut14 | FITC-Ahx-RRFFFKRIFRRFKFRLKRRPKV (Seq ID No. 110) |
| FITC-JL-2004(H5N1)-M2-44-65 mut15 | FITC-Ahx-RRLFFKCIYRRLKYGLKRGPST (Seq ID No. 111) |
| FITC-JL-2004(H5N1)-M2-44-65 mut16 | FITC-Ahx-RRLFFKCIYRRLKYGLKRGPKV (Seq ID No. 112) |
| FITC-JL-2004(H5N1)-M2-44-65 mut17 | FITC-Ahx-RRFFFKCIYRRLKYGLKRGPST (Seq ID No. 113) |
| FITC-JL-2004(H5N1)-M2-44-65 mut18 | FITC-Ahx-RRLFFKCIYRRLKYGLKRRPST (Seq ID No. 114) |
| FITC-JL-2004(H5N1)-M2-44-65 mut19 | FITC-Ahx-RRLFFKCIYRRLKYGLKRKPST (Seq ID No. 115) |
| F1TC-JL-2004(H5N1)-M2-44-65 mut20 | FITC-Ahx-RRLFFRRIYRRLKYGLKRGPST (Seq ID No. 116) |
| FITC-JL-2004(H5N1)-M2-44-65 mut21 | FITC-Ahx-RRLFFRRIYRRLRYRLRRRPST (Seq ID No. 117) |
| FITC-JL-2004(H5N1)-M2-44-65 mut22 | FITC-Ahx-DRLFFKRIYRRLKYGLKRGPST (Seq ID No. 118) |
| FITC-JL-2004(H5N1)-M2-44-65 mut23 | FITC-Ahx-DRLFFRRIYRRLKYGLKRGPST (Seq ID No. 119) |
| FITC-JL-2004(H5N1)-M2-44-65 mut24 | FITC-Ahx-DRLFFRRIYRRLRYRLRRRPST (Seq ID No. 120) |
| F1TC-JL-2004(H5N1)-M2-44-65 mut25 | FITC-Ahx-DRLFFKCIYRRLKYGLKRRPST (Seq ID No. 121) |
| FITC-JL-2004(H5N1)-M2-44-65 mut26 | FITC-Ahx-DRLFFKCIYRRLKYRLKRRPST (Seq ID No. 122) |
| FITC-JL-2004(H5N1)-M2-44-65 mut27 | FITC-Ahx-DRLFFKRIYRRLKYRLKRRPST (Seq ID No. 123) |
| FITC-JL-2004(H5N1)-M2-44-65 mut28 | FITC-Ahx-RRLFFKRIYRRLKYRLKRRPST (Seq ID No. 124) |
| FITC- JL-2004(H5N1)-M2-44-65 mut29 | FITC-Ahx-DRLFFKCIYRRLKYGLKRGPKV (Seq ID No. 68) |

Comparison of the cell-penetrating peptide functions among 29 Jilin-2004(H5N1)-M2-44-65 mutant polypeptide sequences and the positive control TAT polypeptide and the parent Jilin-2004(H5N1)-M2-44-65 in A549 cell, 293T cell, MRC5 cell, U937 cell and HCT-116 cell was tested by flow cytometry.

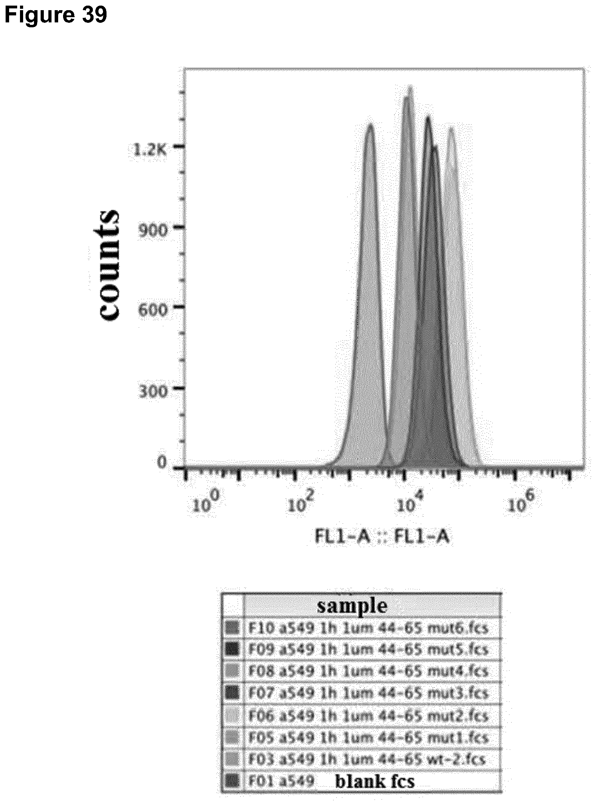

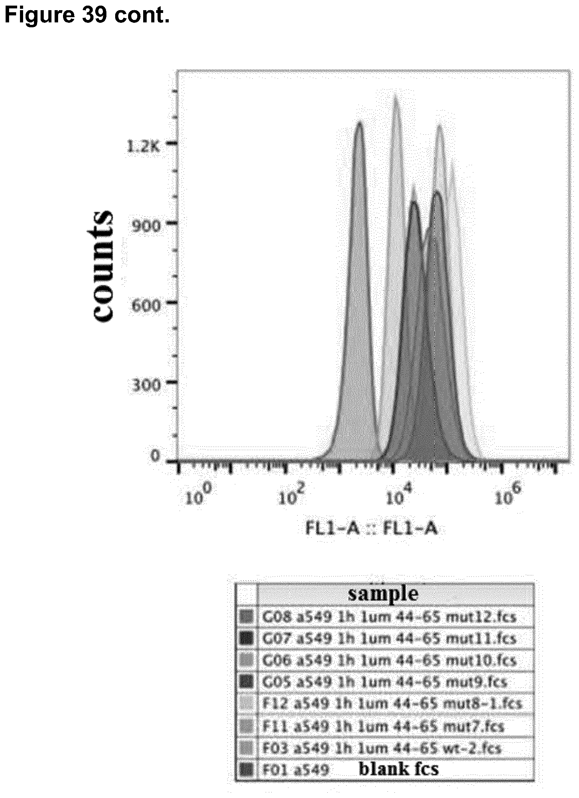

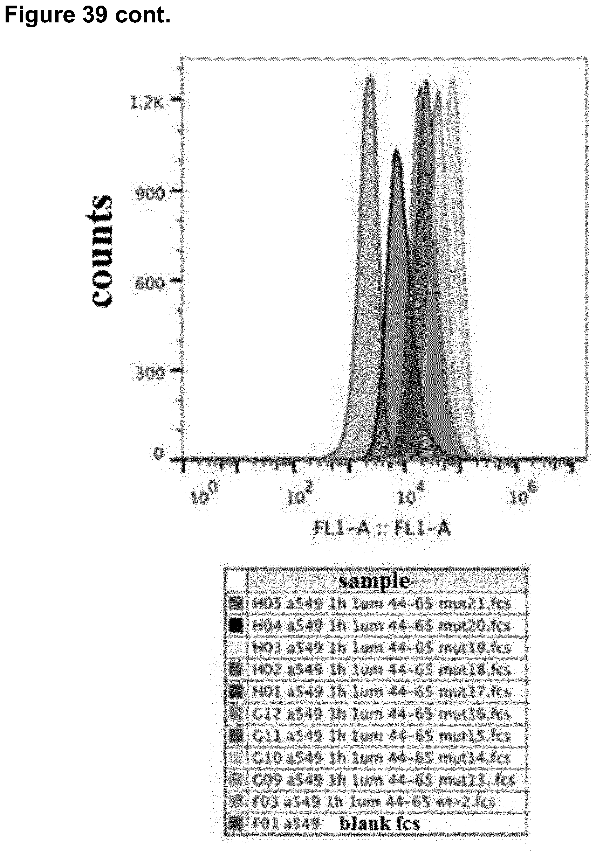

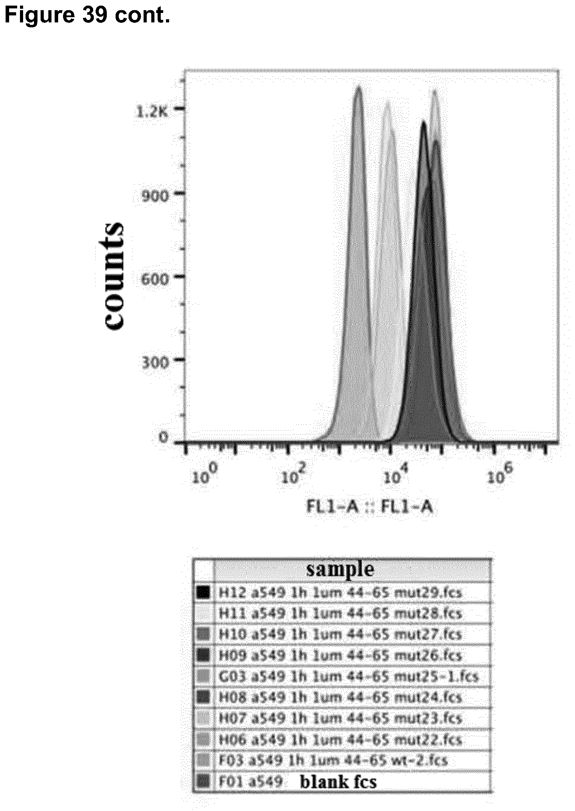

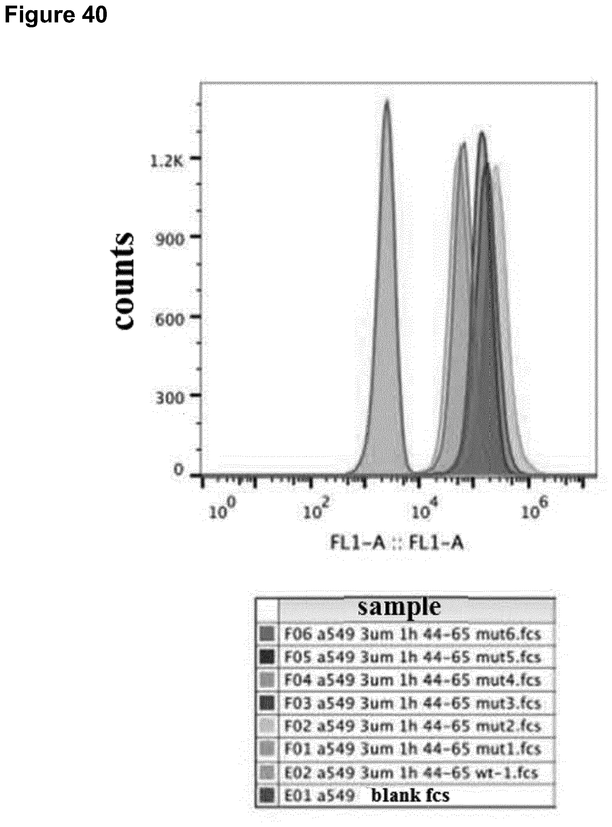

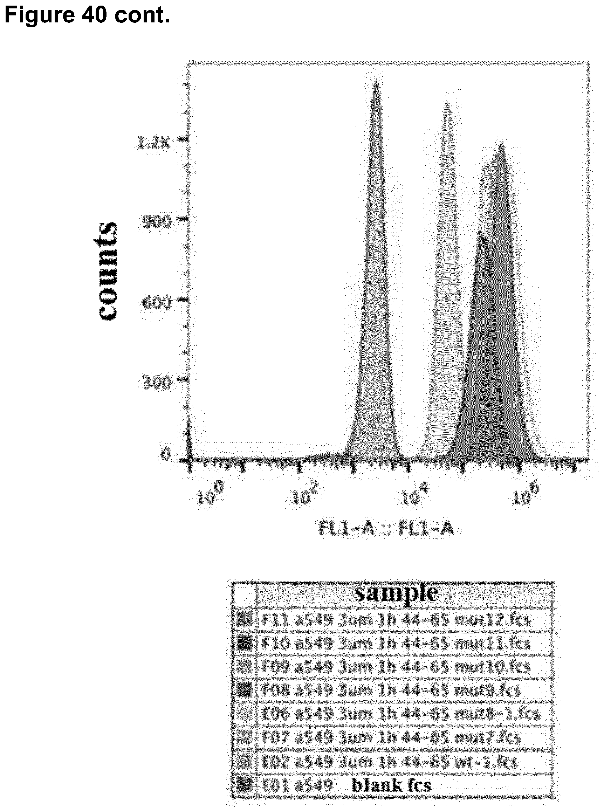

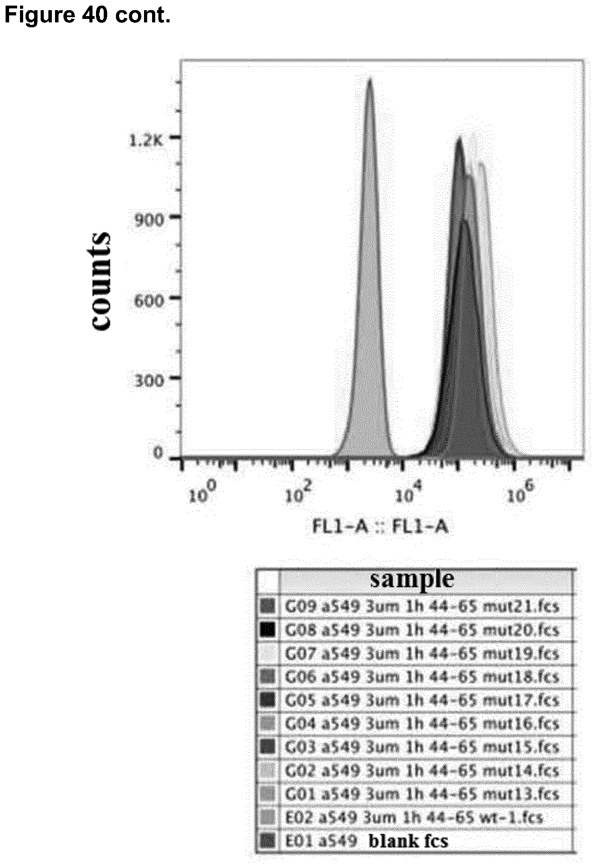

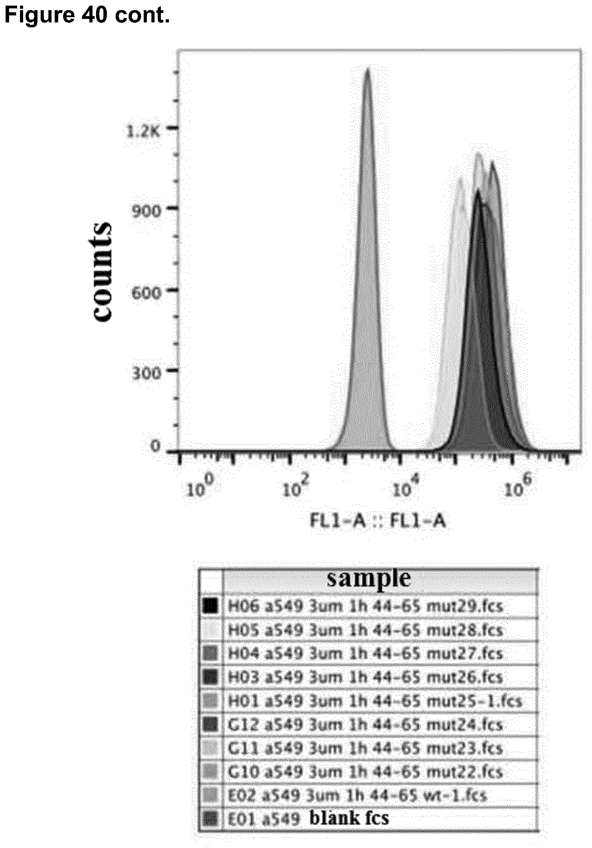

As shown in Figure 39, when using A549 cells and at a polypeptide concentration of 1 µM, Jilin-2004(H5N1)-M2-44-65 polypeptide and its 28 mutants all had cell-penetrating peptide functions, which were all stronger than that of the positive control TAT. Among them, the cell-penetrating peptide functions of mut8, mut25 and mut26 were stronger than that of the parent Jilin-2004(H5N1)-M2-44-65 polypeptide. Besides, as shown in Figure 40, similar results were obtained using a polypeptide concentration of 3 µM, in which Jilin-2004(H5N1)-M2-44-65 polypeptide and its 28 mutants all had stronger cell-penetrating peptide functions than that of the positive control TAT. Among them, the cell-penetrating peptide functions of mut8, mut9, mut10, mut12, mut24, mut25 and mut26 were stronger than that of the parent Jilin-2004(H5N1)-M2-44-65 polypeptide.

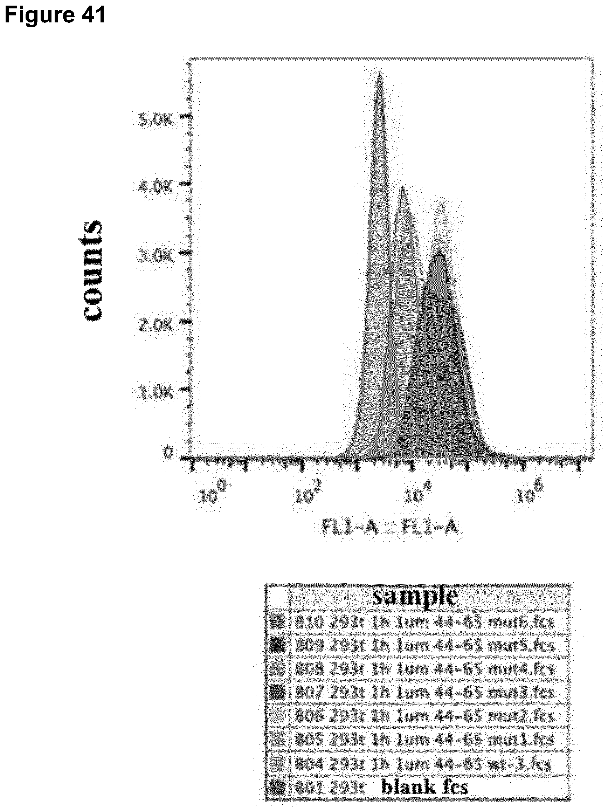

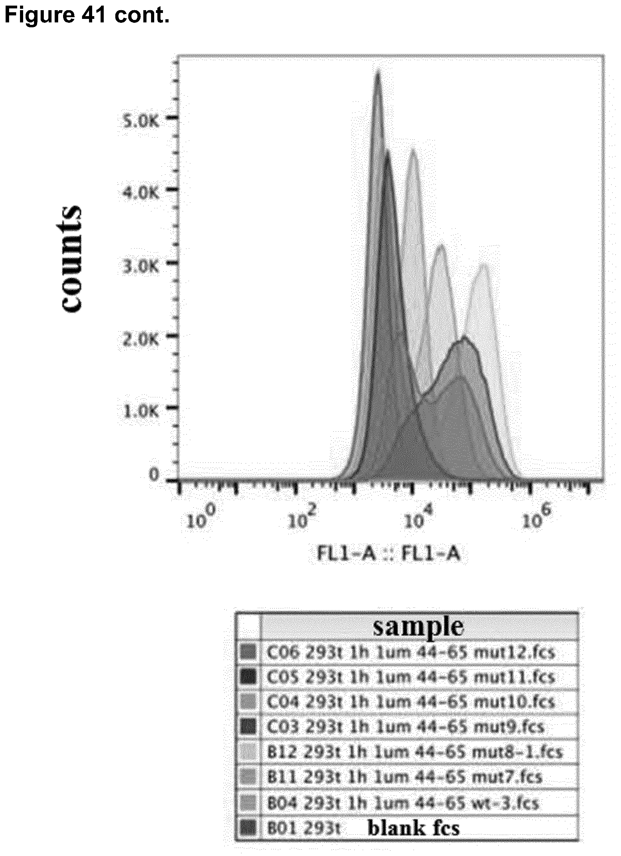

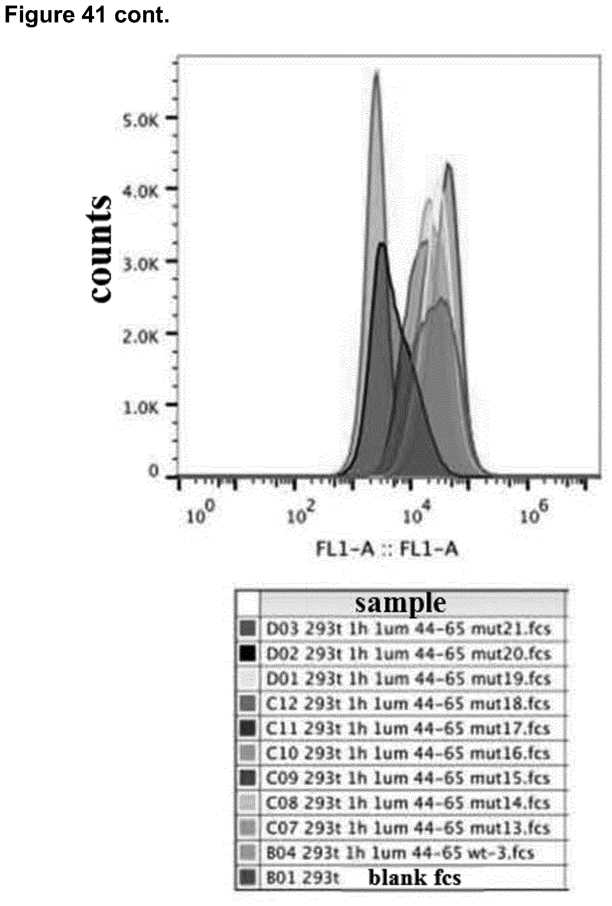

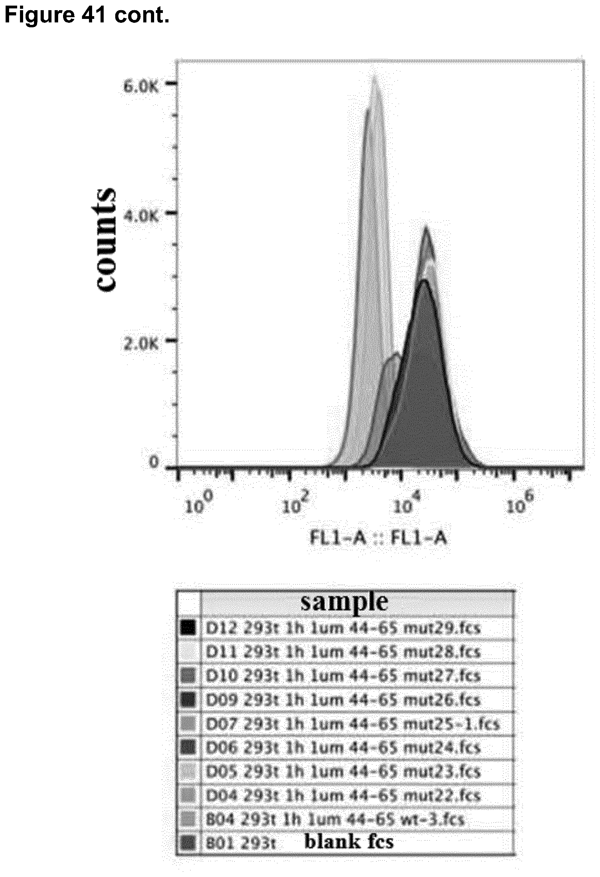

The detected results using 293T cells are shown in Figure 41. Among them, Jilin-2004(H5N1)-M2-44-65 polypeptide and its 28 mutants all had cell-penetrating peptide functions, which were all stronger than that of the positive control TAT. The cell-penetrating peptide functions of mut8, mut9, mut12, mut17, mut25, mut26 and mut27 were stronger than that of the parent Jilin-2004(H5N1)-M2-44-65 polypeptide.

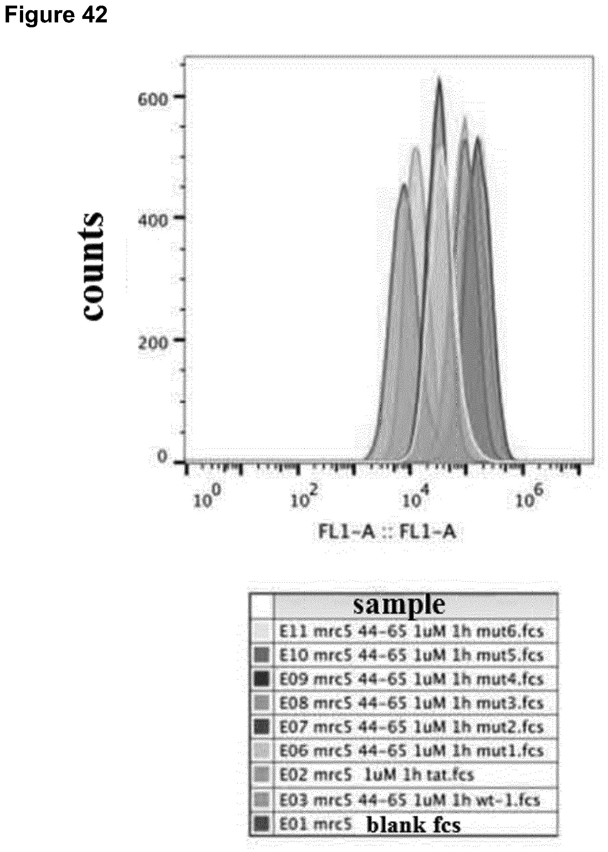

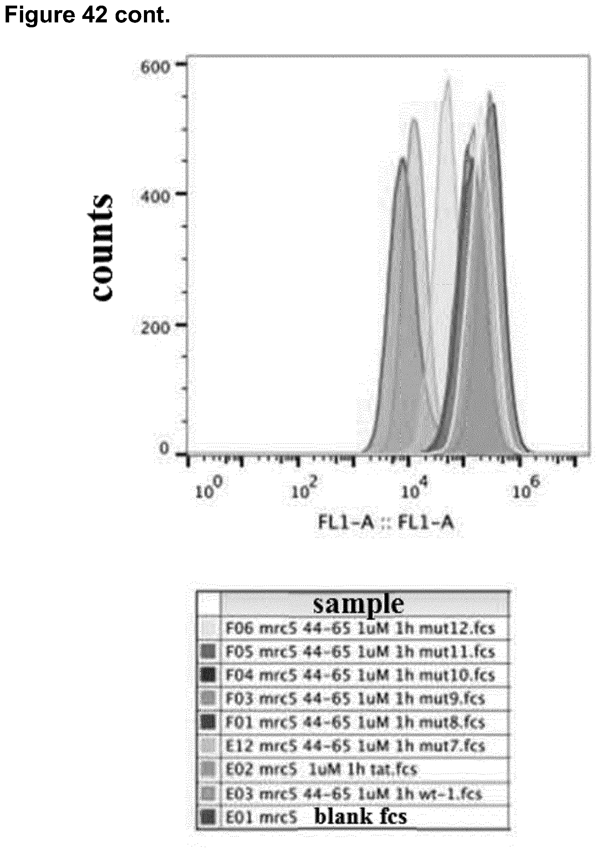

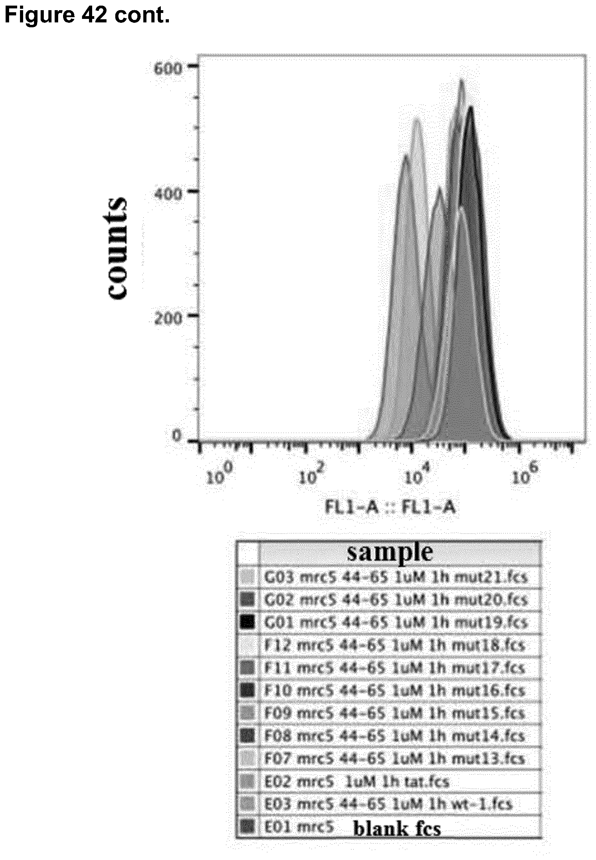

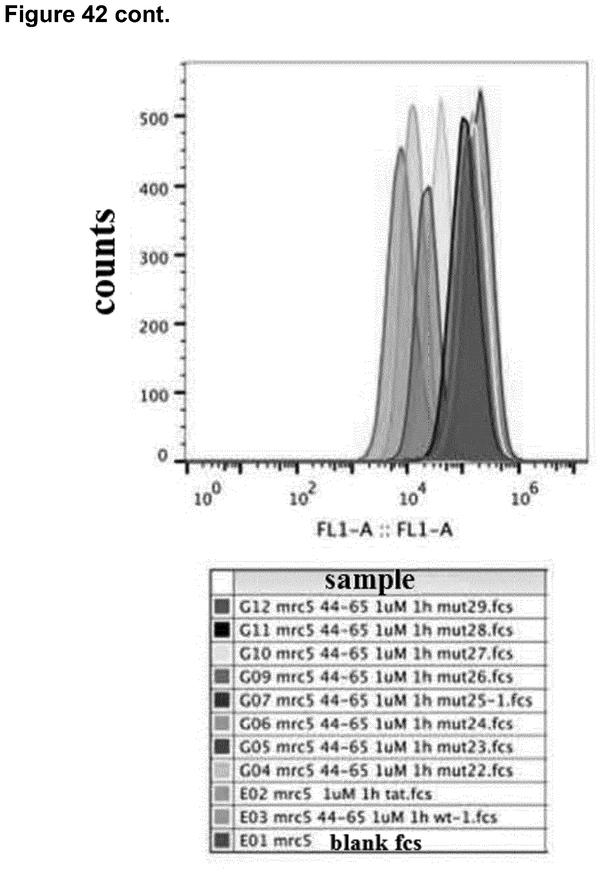

The detected results using MRC5 cells are shown in Figure 42. Among them, Jilin-2004(H5N1)-M2-44-65 polypeptide and its 28 mutants all had cell-penetrating peptide functions, which were all stronger than that of the positive control TAT. The cell-penetrating peptide functions of mut8, mut9, mut12 and mut25 were stronger than that of the parent Jilin-2004(H5N1)-M2-44-65 polypeptide.

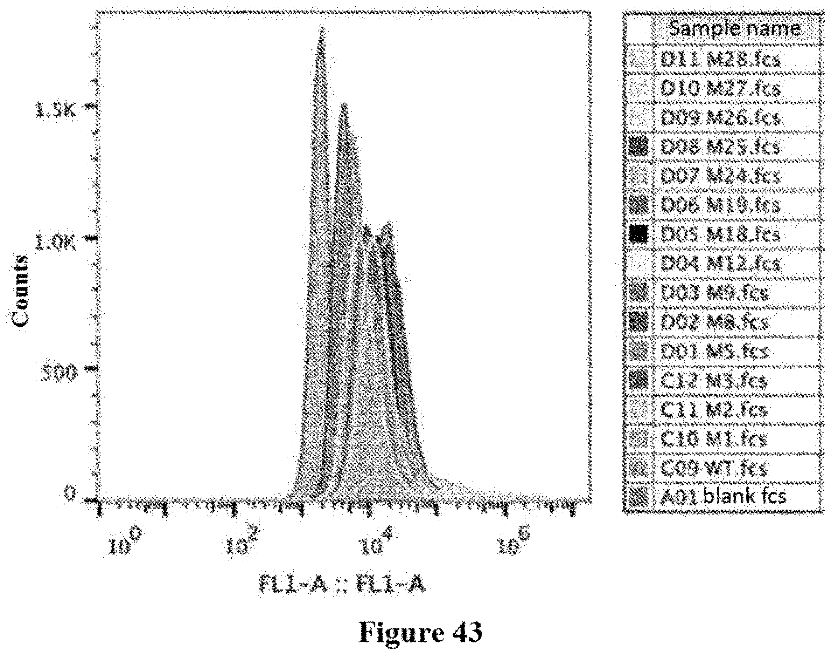

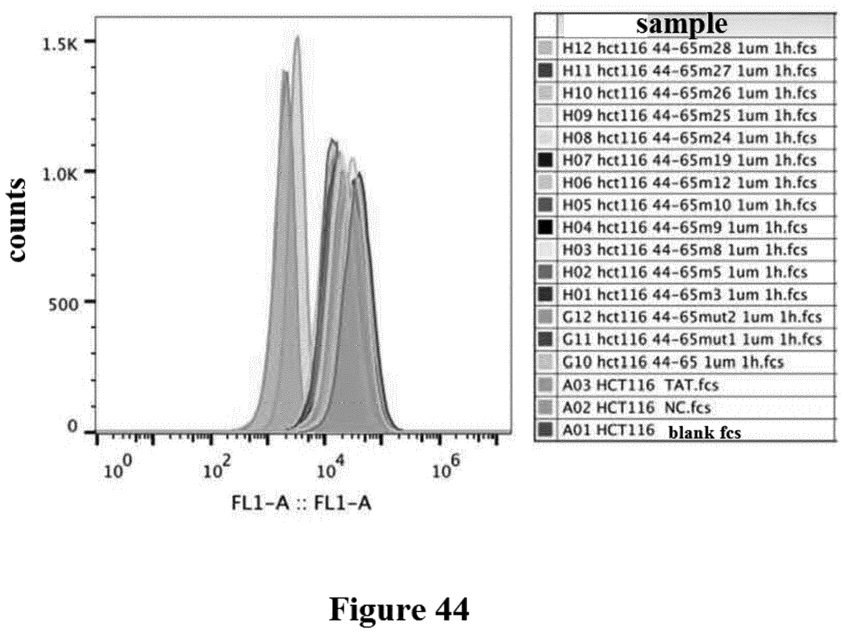

In addition, for some mutant polypeptides in A549 cells, 293T cells and MRC5 cells, the cell-penetrating peptide effects were verified in U937 cells and HCT-116 cells. The results are shown in Figure 43 and Figure 44, respectively, in which Figure 43 shows the results using U937 cells, and Figure 44 shows the results using HCT-116 cells. When using a polypeptide concentration of 1 µM, the above mutant polypeptides all had cell-penetrating peptide functions, which were all stronger than that of the positive control TAT.

### Example 10: Cell entry of cell-penetrating peptides carrying small RNA

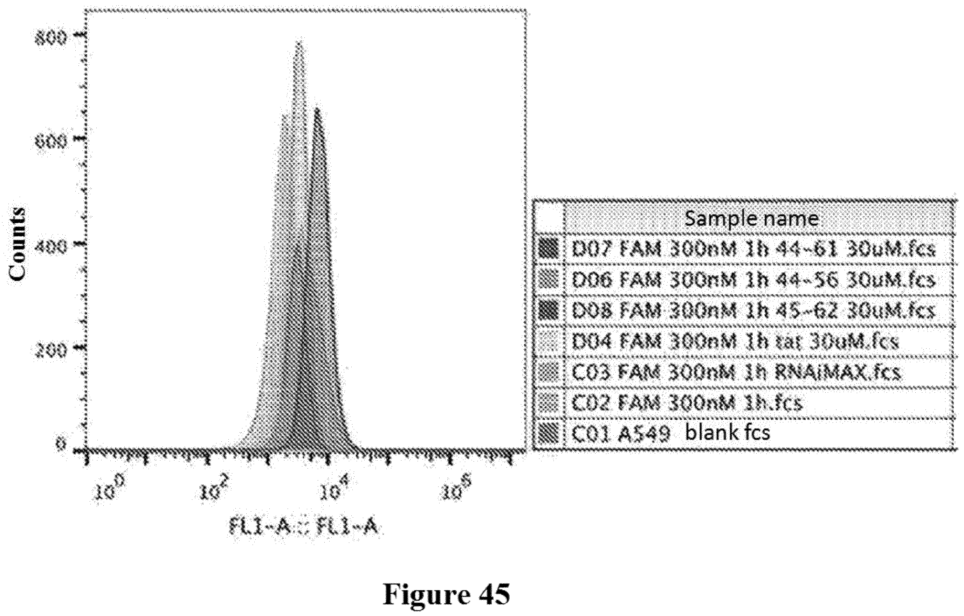

Previous studies have shown that most cell-penetrating peptides can carry biologically active molecules such as small RNA into cells. In order to verify the above properties of the cell-penetrating peptide of the present invention, Jilin-2004(H5N1)-M2-45-62, Jilin-2004(H5N1)-M2-44-61 and Jilin-2004(HSN1)-M2-44-56 polypeptides of the present invention were used to study their effects of carrying small RNA into cells. Experiments were performed in A549 cells using FAM-labeled small RNA. As shown in Figure 45, when 30 µM polypeptide and 300 nM small RNA were used, the above three polypeptides can all carry small RNA into cells within 1 h, and compared with the positive control TAT at the same concentration, their ability to carry small RNA was significantly stronger. It is noticed that the ability of Jilin-2004(H5N1)-M2-44-61 to carry FAM-labeled small RNA was stronger than that of the transfection reagent RNAiMax.

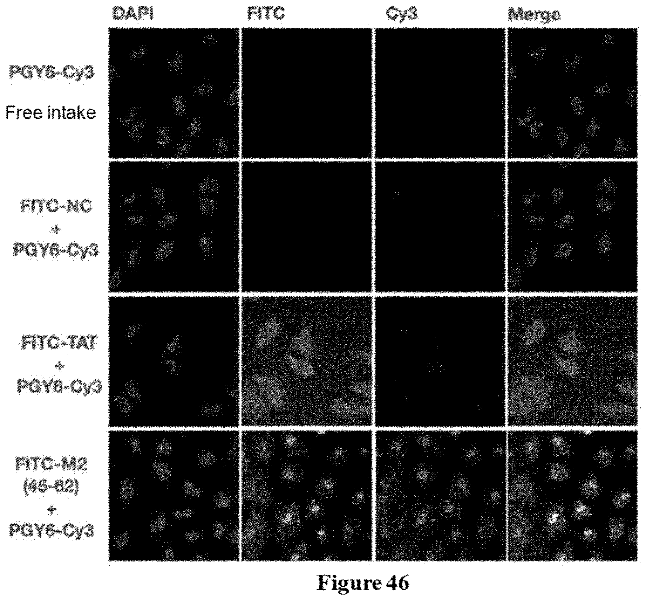

Subsequently, each of the above polypeptides was further verified. For Jilin-2004(H5N1)-M2-45-62, FITC-labeled polypeptide and Cy3-labeled small RNA were used for experiments in A549 cells. As shown in Figure 46, when 10 µM polypeptide and 200 nM small RNA were used, Jilin-2004(H5N1)-M2-45-62 can carry small RNA into cells within 1 h, and compared with the positive control TAT at the same concentration, its ability to carry small RNA was significantly stronger.

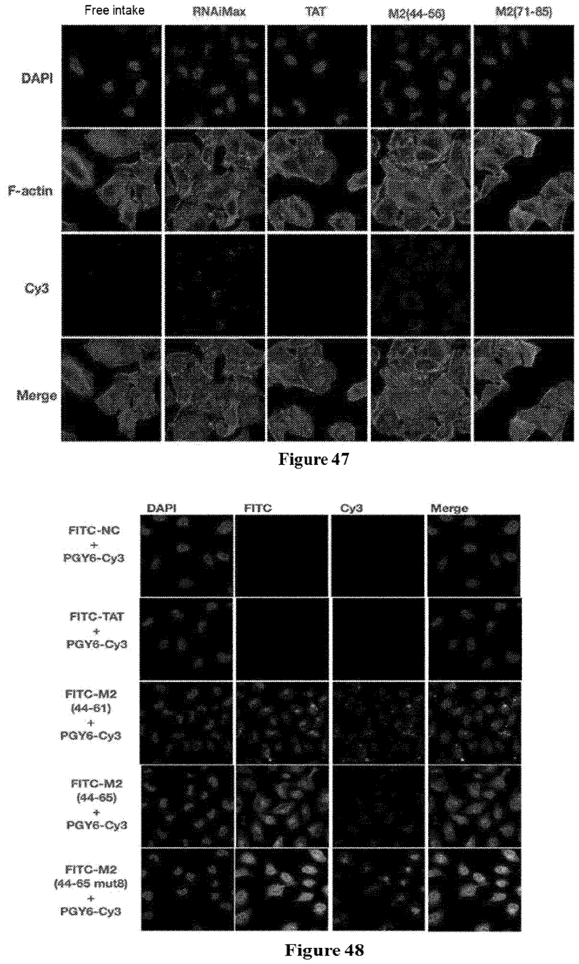

For Jilin-2004(HSN1)-M2-44-56 polypeptide, when 30 µM polypeptide and 100 nM Cy3-labeled small RNA were mixed and incubated with A549 cells, as shown in the results in Figure 47, the polypeptide can carry small RNA into cells within 1 h, and its effect was stronger than that of the transfection reagent RNAiMax.

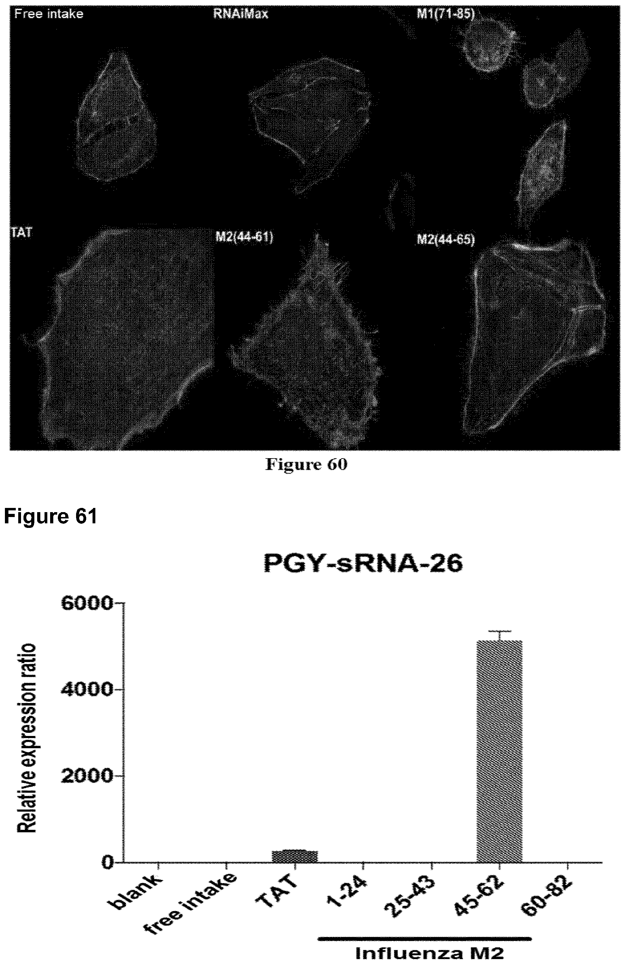

Besides, the ability of Jilin-2004(H5N1)-M2-44-61, Jilin-2004(H5N1)-M2-44-65 polypeptides and its mutant mut8 (Jilin-2004(H5N1)-M2-44-65 mut8) to carry small RNA was tested. When 10 µM polypeptide and 200 nM Cy3-labeled small RNA were mixed and incubated with A549 cells, as shown in the results in Figure 48, the polypeptide can carry small RNA into cells within 1 h, and its effect was stronger than that of the transfection reagent RNAiMax. Among them, the ability of Jilin-2004(H5N1)-M2-44-65 mut8 mutant to carry small RNA into cells was even stronger. When 10 µM polypeptide and 200 nM Cy3-labeled small RNA were mixed and incubated with A549 cells, as shown in the results in Figure 60, the Jilin-2004(H5N1)-M2-44-61 and Jilin-2004(H5N1)-M2-44-65 polypeptides can carry Cy3-labeled small RNA into A549 cells, and their effects were stronger than that of the transfection reagent RNAiMax or of TAT, as observed under a super high resolution microscope.

In the above experiments, confocal laser experiments were used to detect the entry of polypeptides carrying small RNA. We also used real-time quantitative qPCR to detect the relative expression abundance of small RNA carried by polypeptides in the cells, so as to determine the ability of polypeptides to carry small RNA. As shown in Figure 61, according to the cell penetration function of different peptide fragments of influenza virus M2, the control group blank cells, a mixture of polypeptide solution and 100 nM PGY-sRNA-26, and 10 µM Jilin-2004(H5NI)-M2-1-24, Jilin-2004(H5N1)-M2-25-43, Jilin-2004(H5N1)-M2-45-62, Jilin-2004(H5N1)-M2-60-82 polypeptides and control cell-penetrating peptide TAT mixed with 100 nM PGY-sRNA-26 respectively and incubated with A549 cells for 1 h, were tested respectively. Only the polypeptide Jilin-2004(H5N1)-M2-45-62 had the ability to carry small RNA into the cells. Under these experimental conditions, the ability of the control cell-penetrating peptide TAT to carry small RNA was very weak.

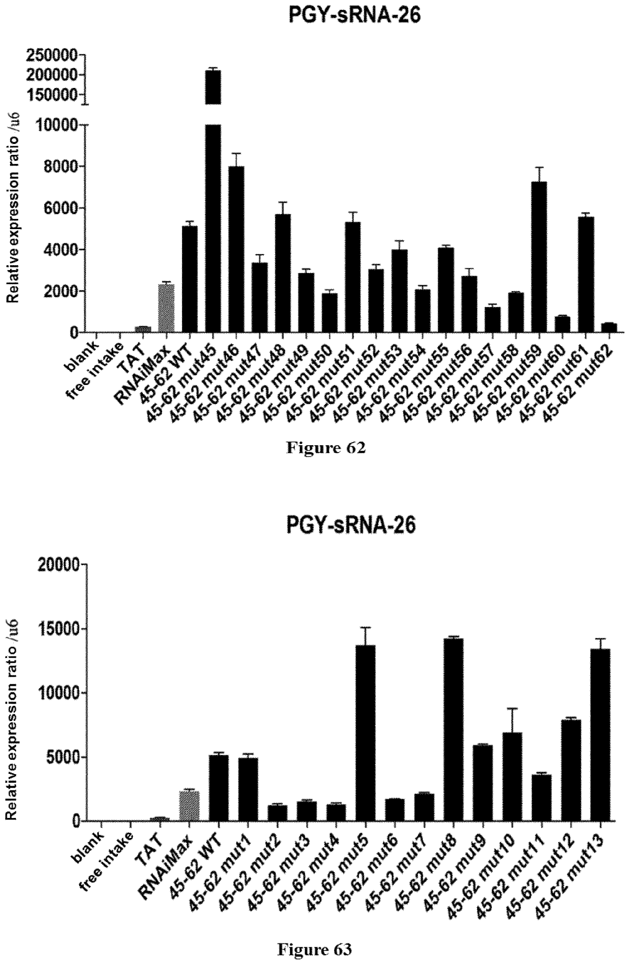

As shown in Figure 62, Jilin-2004(H5N1)-M2-45-62 and its single site mutant polypeptide sequences all had the ability to carry small RNA into cells, and the ability of wild-type Jilin-2004(H5N1)-M2-45-62, Jilin-2004(H5N1)-M2-45-62 mut45, Jilin-2004(H5N1)-M2-45-62 mut46, Jilin-2004(H5N1)-M2-45-62 mut47, Jilin-2004(H5N1)-M2-45-62 mut48, Jilin-2004(H5N1)-M2-45-62 mut49, Jilin-2004(H5N1)-M2-45-62 mut51, Jilin-2004(H5N1)-M2-45-62 mut52, Jilin-2004(H5N1)-M2-45-62 mut53, Jilin-2004(H5N1)-M2-45-62 mut55, Jilin-2004(H5N1)-M2-45-62 mut56, Jilin-2004(H5N1)-M2-45-62 mut59 and Jilin-2004(H5N1)-M2-45-62 mut61 to carry small RNA into cells were all stronger than the transfection reagent RNAiMax group.

Figure 63 showed that Jilin-2004(H5N1)-M2-45-62 and its mutant polypeptide sequences all had the ability to carry small RNA into cells, and the ability of wild-type Jilin-2004(H5N1)-M2-45-62, Jilin-2004(H5N1)-M2-45-62 mut1, Jilin-2004(H5N1)-M2-45-62 mut5, Jilin-2004(H5N1)-M2-45-62 mut8 and Jilin-2004(H5N1)-M2-45-62 mut23 to carry small RNA into cells were all stronger than the transfection reagent RNAiMax group.

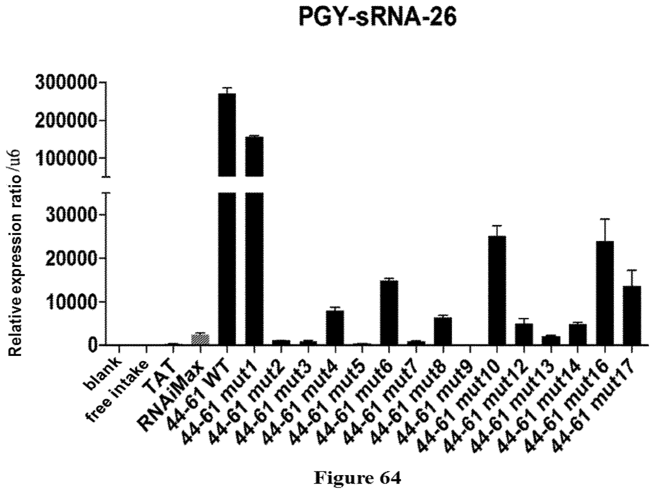

As shown in Figure 64, Jilin-2004(H5N1)-M2-44-61 and its mutant polypeptide sequences all had the ability to carry small RNA into cells, and the ability of wild-type Jilin-2004(H5N1)-M2-44-61, Jilin-2004(H5N1)-M2-44-61 mut1, Jilin-2004(H5N1)-M2-44-61 mut4, Jilin-2004(H5N1)-M2-44-61 mut6, Jilin-2004(H5N1)-M2-44-61 mut8, Jilin-2004(H5N1)-M2-44-61 mut10, Jilin-2004(H5N1)-M2-44-61 mut12, Jilin-2004(H5N1)-M2-44-61 mut14, Jilin-2004(H5N1)-M2-44-61 mut16 and Jilin-2004(H5N1)-M2-44-61 mut17 to carry small RNA into cells were all stronger than the transfection reagent RNAiMax group.

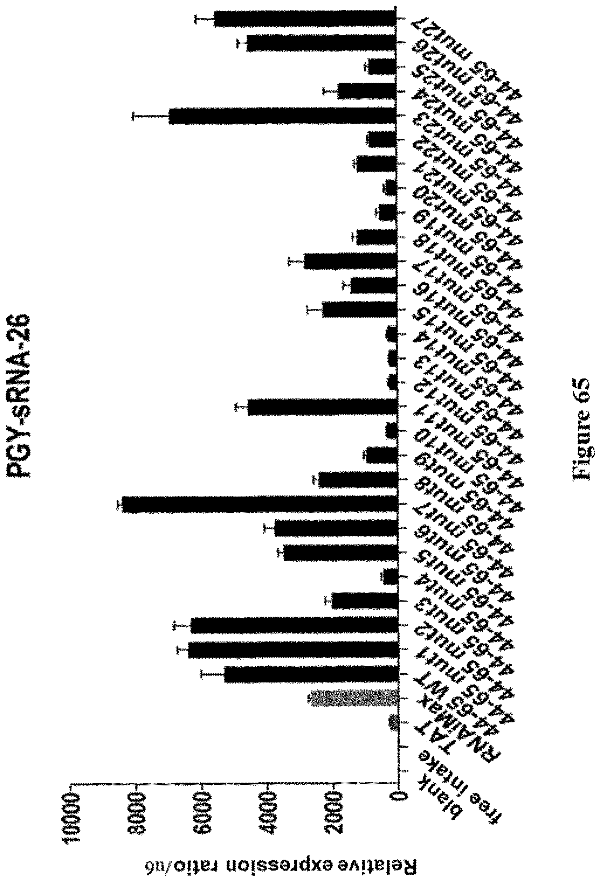

As shown in Figure 65, Jilin-2004(H5N1)-M2-44-65 and its mutant polypeptide sequences all had the ability to carry small RNA into cells, and the ability of wild-type Jilin-2004(H5N1)-44-65, Jilin-2004(H5N1)-M2-44-65 mut1, Jilin-2004(H5N1)-M2-44-65 mut2, Jilin-2004(H5N1)-M2-44-65 mut5, Jilin-2004(H5N1)-M2-44-65 mut6, Jilin-2004(H5N1)-M2-44-65 mut7, Jilin-2004(H5N1)-M2-44-65 mut11, Jilin-2004(H5N1)-M2-44-65 mut15, Jilin-2004(H5N1)-M2-44-65 mut17, Jilin-2004(H5N1)-M2-44-65 mut23, Jilin-2004(H5N1)-M2-44-65 mut26 and Jilin-2004(H5N1)-M2-44-65 mut27 to carry small RNA into cells were all stronger than the transfection reagent RNAiMax group.

As summarized by all the above experimental results, the polypeptides with cell-penetrating peptide function derived from influenza virus could all carry small RNA into cells, and the carrying abilities were all stronger than that of the control cell-penetrating peptide TAT. The ability of most cell-penetrating peptides to carry small RNA into cells were significantly stronger than that of the currently well recognized transfection reagent RNAiMax.

### Example 11-1: Entry of cell-penetrating peptides carrying small RNA and the function

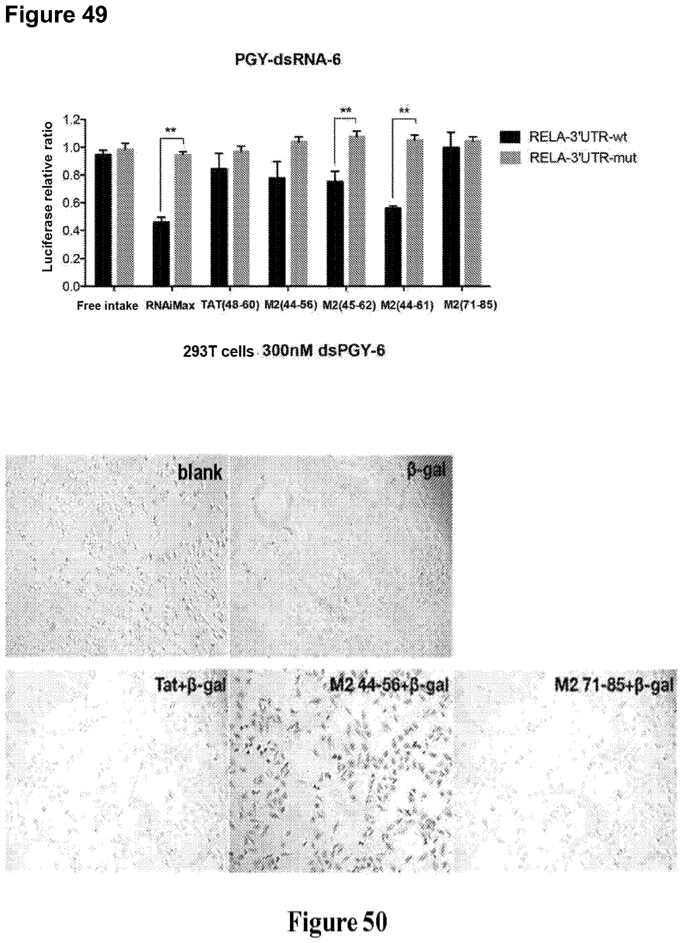

On the basis of Example 9, it was further verified whether the cell-penetrating peptide of the present invention could fulfill the normal function of the small RNA after carrying the small RNA into the cell. Previous experiments have proved that PGY-dsRNA-6 can target and bind the 3'-UTR region of the *RELA* gene and achieve the function of inhibiting its expression. Experiments were performed using Jilin-2004(H5N1)-M2-45-62, Jilin-2004(H5N1)-M2-44-61 and Jilin-2004(HSN1)-M2-44-56 and 293T cells. The cell-penetrating peptide at a concentration of 30 µM was mixed with 300 nM PGY-dsRNA-6 and incubated with the cells, and the luciferase reporter gene in the cells was detected. From the results shown in Figure 49, it can be seen that Jilin-2004(H5N1)-M2-45-62, Jilin-2004(H5N1)-M2-44-61 and Jilin-2004(HSN1)-M2-44-56 could all carry PGY-dsRNA-6 into cells and fulfill its function, and the ability to carry PGY-dsRNA-6 was stronger than that of the positive control TAT. Among them, the cell-penetrating peptide effect of Jilin-2004(H5N1)-M2-44-61 was close to the transfection reagent RNAiMax.

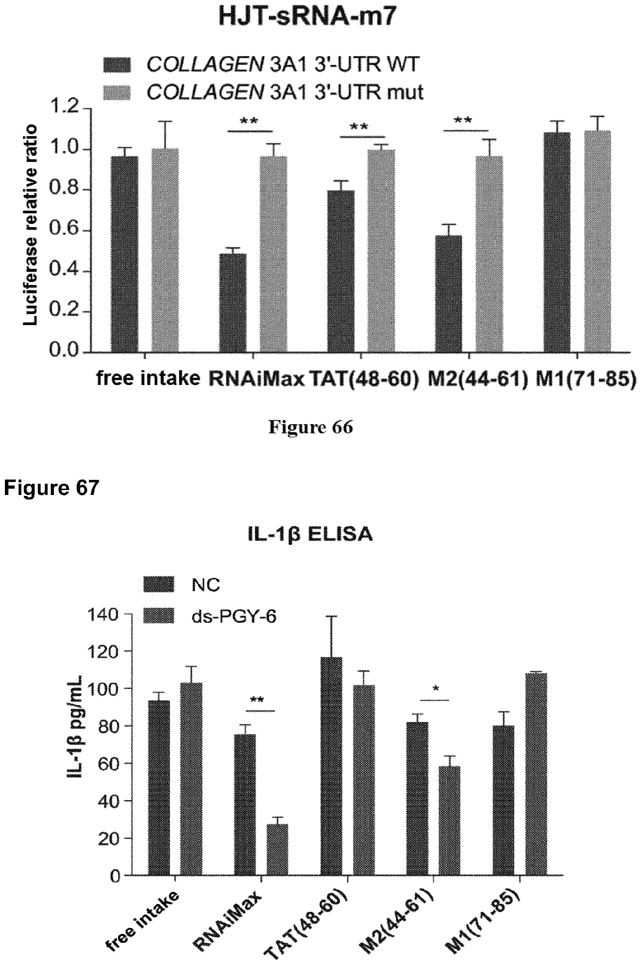

Similarly, HJT-sRNA-m7 can target and bind the 3'-UTR region of the *COLLAGEN3A1* gene and achieve the function of inhibiting its expression. Jilin-2004(H5N1)-M2-44-61 was used in the experiment in HEK293T cells. The cell-penetrating peptide at a concentration of 30 µM was mixed with 300 nM HJT-sRNA-m7 and incubated with the cells, and the luciferase reporter gene in the cells was detected. From the results shown in Figure 66, it can be seen that Jilin-2004(H5N1)-M2-44-61 could carry HJT-sRNA-m7 into cells and fulfill its function, and the ability to carry HJT-sRNA-m7 was stronger than that of the positive control TAT. Among them, the cell-penetrating peptide effect of Jilin-2004(H5N1)-M2-44-61 was close to the transfection reagent RNAiMax.

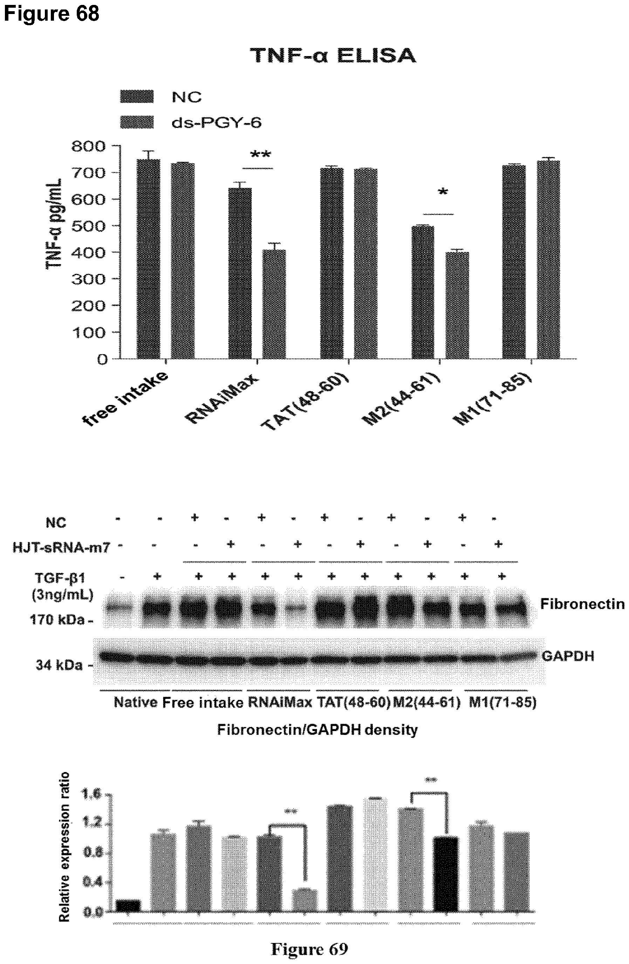

PGY-dsRNA-6 can target and bind the 3'-UTR region of the *RELA* gene and fulfill its anti-inflammatory effect. Figure 67 and Figure 68 showed that in the LPS-induced inflammation model of THP-1, PGY-sRNA-6 carried by Jilin-2004(H5N1)-M2-44-61 effectively reduced IL-1β and TNF-α expression in the supernatant of THP-1 cells. Therefore, Jilin-2004(H5N1)-M2-44-61 could carry small RNA into the cell and fulfill its small RNA function.

HJT-sRNA-m7 can target and bind the 3'-UTR region of the *COLLAGEN3A1* gene and fulfill its fibrosis effect. Figure 69 showed that in the TGF-1β-induced fibrosis model of MRC-5 cells, HJT-sRNA-m7 carried by Jilin-2004(H5N1)-M2-44-61 effectively reduced fibronectin expression in MRC-5 cells. It was again proved that Jilin-2004(H5N1)-M2-44-61 could carry small RNA into the cell and fulfill its small RNA function.

### Example 11-2: Entry of cell-penetrating peptides carrying plasmid and the expression function

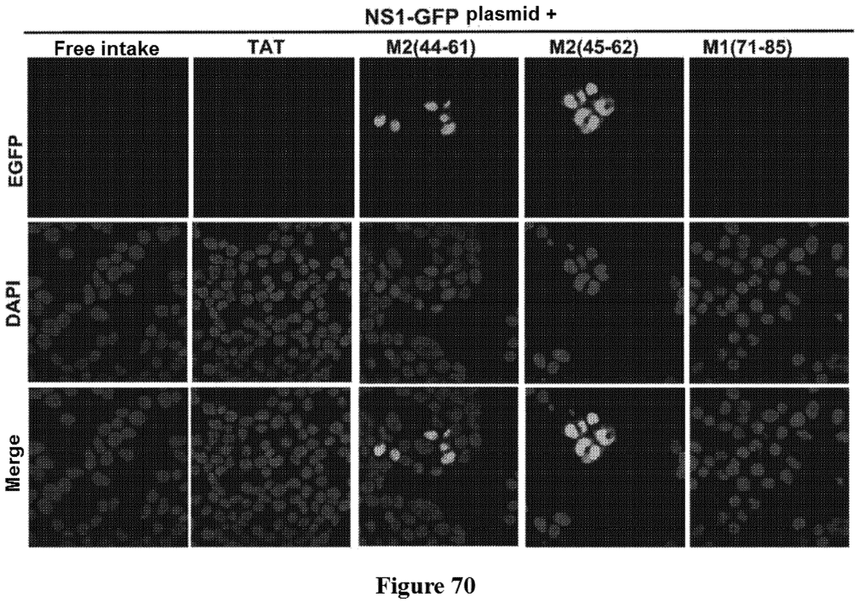

The present invention has proved that the cell-penetrating peptides Jilin-2004(H5N1)-M2-44-61 and Jilin-2004(H5N1)-M2-45-62 derived from influenza virus M2 can carry small RNA into cells and fulfill its own small RNA function. We tried to use a plasmid that could express GFP protein in the nucleus. Figure 70 showed that both Jilin-2004(H5N1)-M2-44-61 and Jilin-2004(H5N1)-M2-45-62 could carry NS1-GFP plasmid into HEK293 cells, and GFP protein was expressed in the nucleus. Meanwhile, the control TAT group had almost no expression of GFP fluorescent protein.

### Example 12: cell-penetrating peptides carry proteins into cells

Previous studies have shown that some cell-penetrating peptides can carry proteins into cells. In order to verify the above properties of the cell-penetrating peptide of the present invention, Jilin-2004(HSN1)-M2-44-56 was used to verify its ability to carry β-galactosidase protein into A549 cells. 50 µM Jilin-2004(HSN1)-M2-44-56 polypeptide and 50 nM β-galactosidase were mixed for 30 min, and then the mixture was incubated with A549 cells for 1 h. Cell entry of β-galactosidase protein was detected by the color test of β-galactosidase, and the results are shown in Figure 50. These results showed that the cell-penetrating peptide of the present invention could carry β-galactosidase protein into the cell, and compared with the positive control TAT at the same concentration, its ability to carry proteins into the cell was significantly stronger.

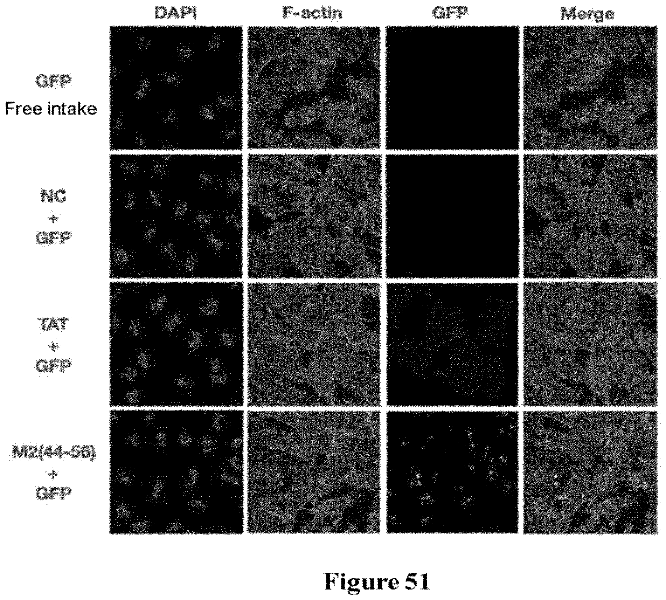

In addition, EGFP protein was used for further verification. As shown in Figure 51, when 3 µM Jilin-2004(HSN1)-M2-44-56 polypeptide and 1µM GFP protein were mixed and incubated with A549 cells for 1 h, cell entry of Jilin-2004(HSN1)-M2-44-56 polypeptide carrying GFP protein was observed, and the effect was stronger than that of the positive control TAT.

### Example 13: Fusion protein of cell-penetrating peptide and EGFP has cell-penetrating peptide function

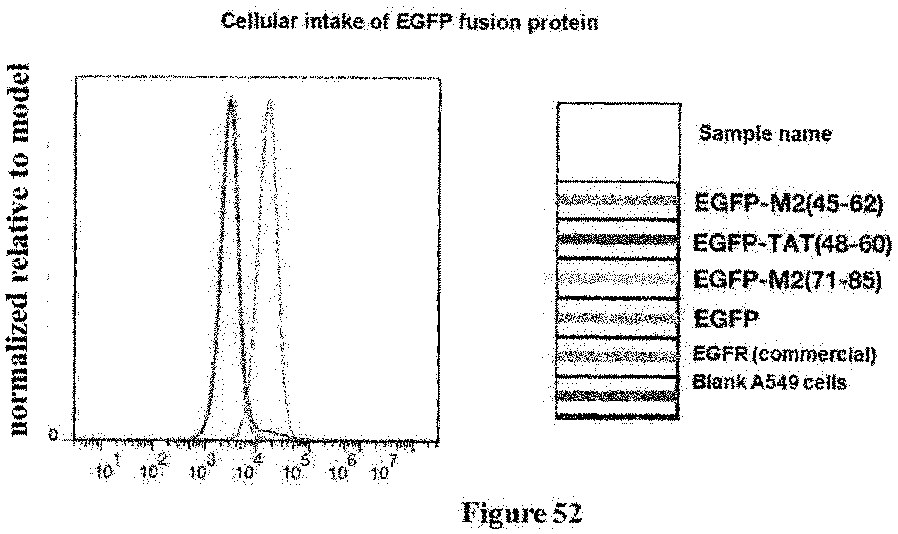

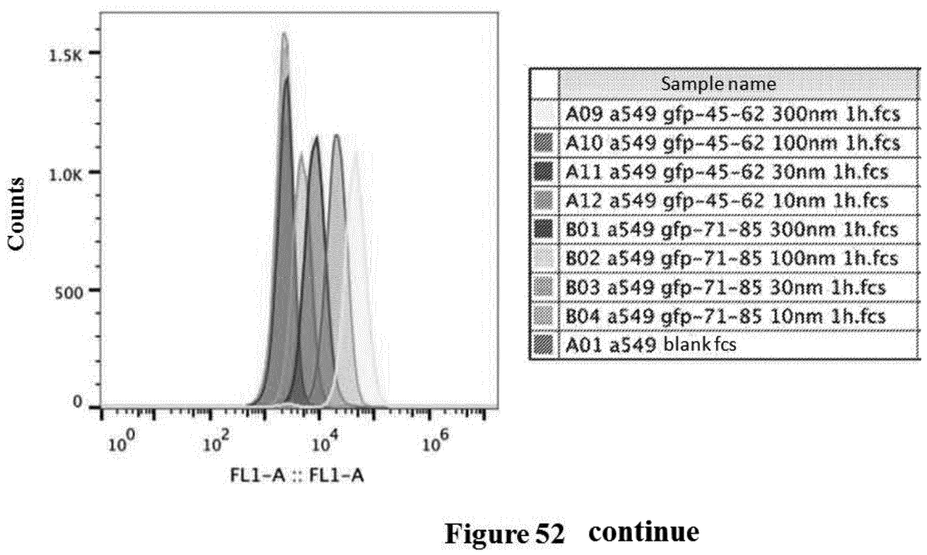

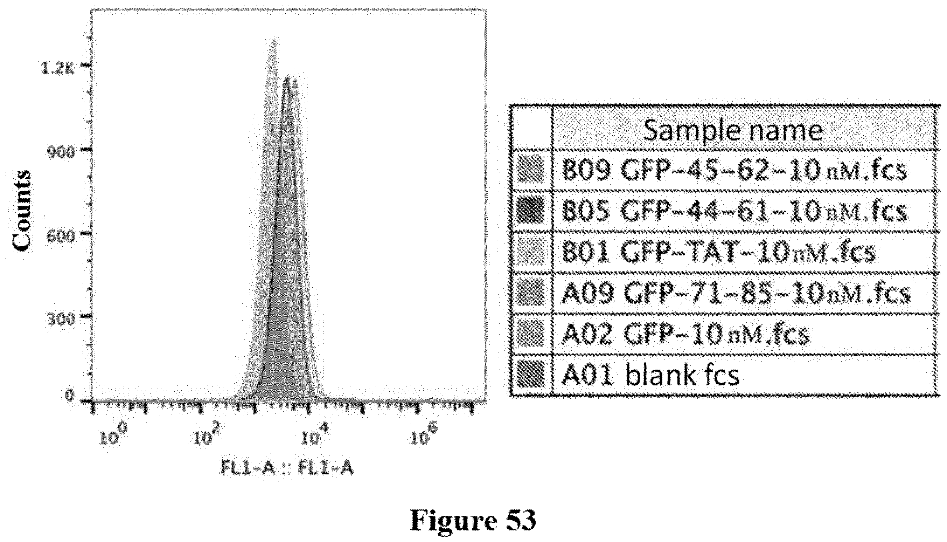

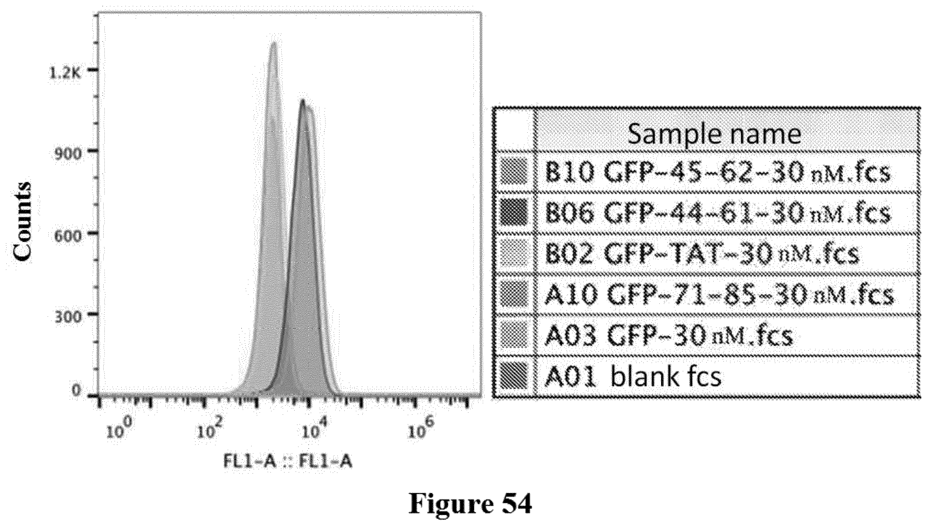

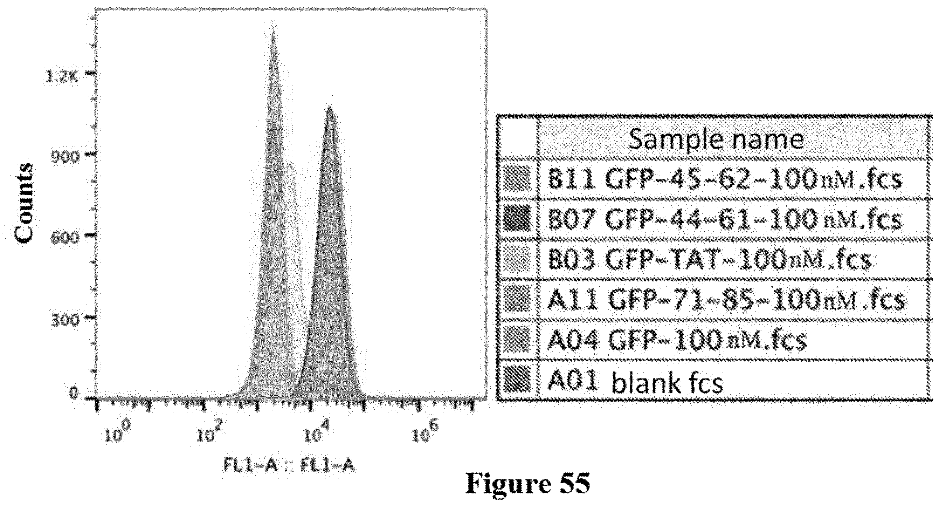

The cell-penetrating peptide of the present invention was further used to study when it was fused with a protein, whether the fusion protein still had the function of cell-penetrating peptides. Figure 52 shows experiments using different concentrations (10 nM, 30 nM, 100 nM and 300 nM) of EGFP-Jilin-2004(H5N1)-M2-45-62 fusion protein and EGFP-TAT and EGFP-Jilin-2004(H5N1)-M2-71-85 as controls, in which the fusion proteins were incubated with A549 cells for 1 h. Figure 53, Figure 54 and Figure 55 respectively show the experimental results using 10 nM, 30 nM and 100 nM of EGFP-Jilin-2004(H5N1)-M2-45-62 and EGFP-Jilin-2004(H5N1)-M2-44-61 fusion proteins, in which the fusion proteins were incubated with A549 cells for 1 h.

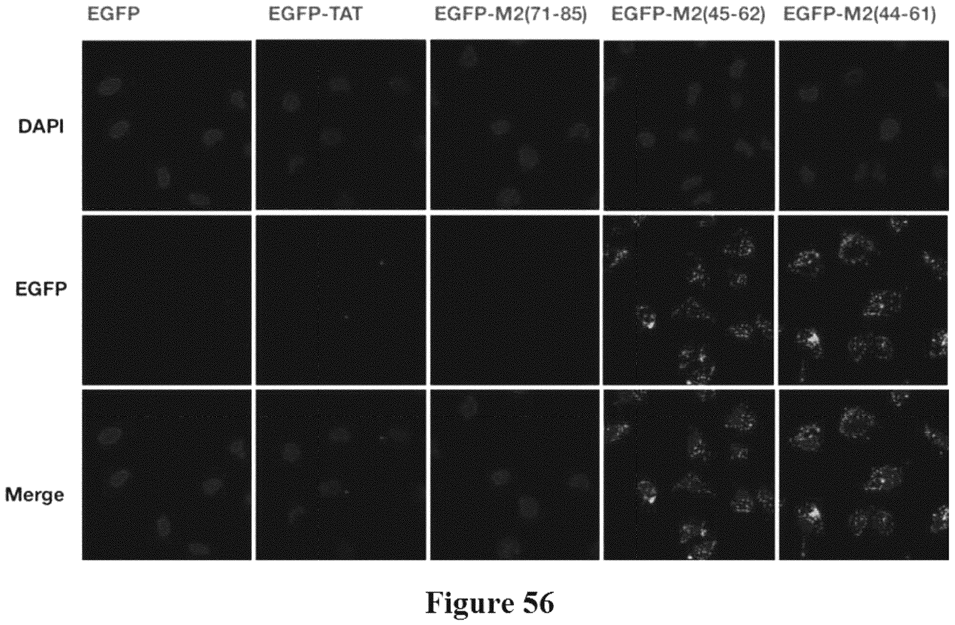

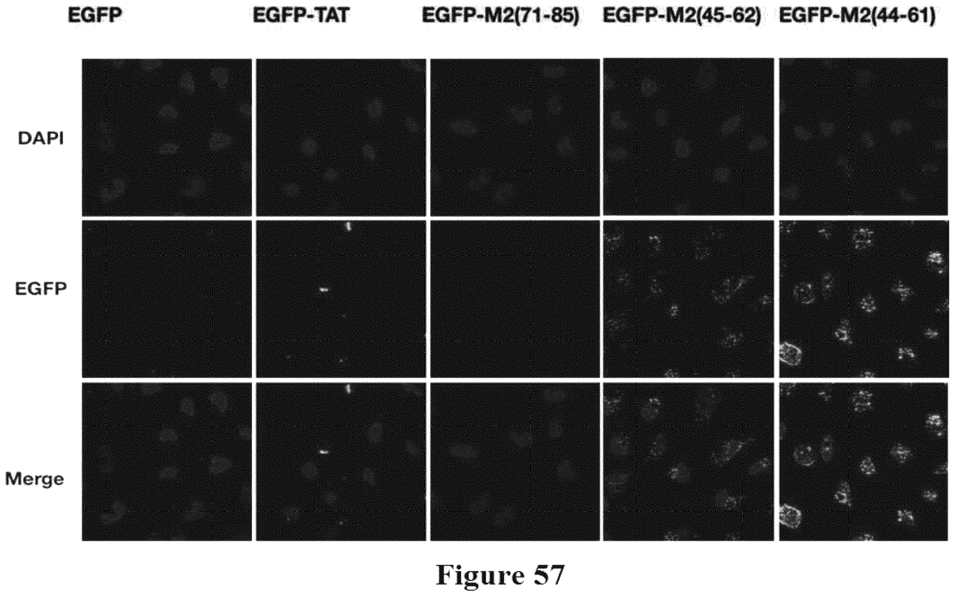

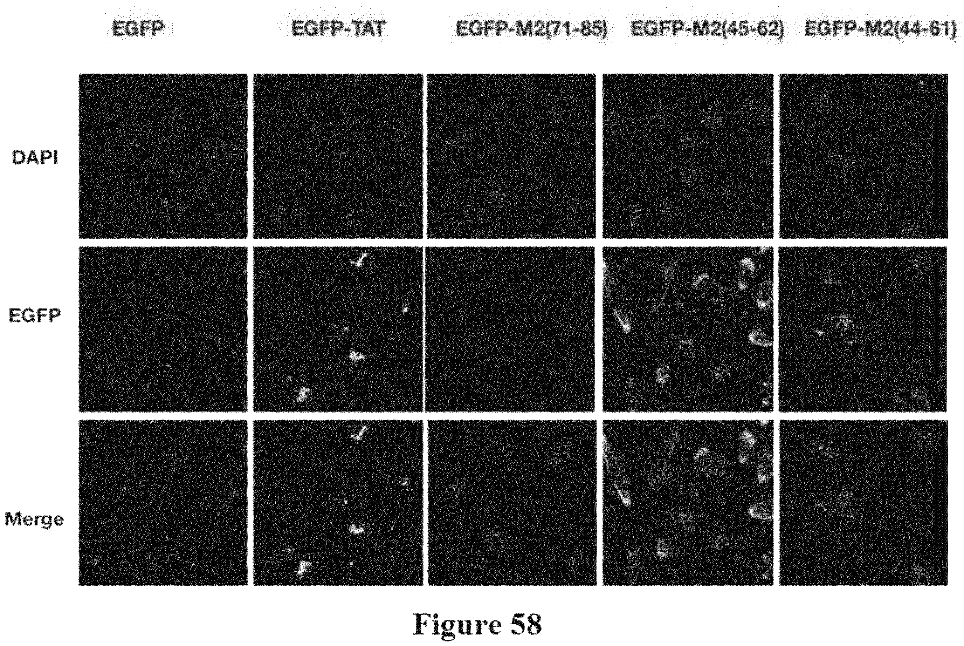

From the results in these figures, it can be seen that EGFP-Jilin-2004(H5N1)-M2-45-62 and EGFP-Jilin-2004(H5N1)-M2-44-61 fusion proteins could enter A549 cells at the above concentrations, and the EGFP-TAT fusion protein was only observed to enter the cells at high concentration (100 nM). Figure 56, Figure 57 and Figure 58 show the results of related confocal laser experiments. Among them, Figure 56 shows the results using 10 nM cell-penetrating peptide, Figure 57 shows the results using 30 nM cell-penetrating peptide, and Figure 57 shows the results using 100 nM, which are consistent with the flow cytometry results of Figure 53 to Figure 55. The above results indicated that the cell-penetrating peptide of the present invention could maintain its cell-penetrating peptide function when fused with other proteins, and could enable the fusion protein to enter cells with high efficiency.

### Example 14: Fusion protein of the cell-penetrating peptide and EGFP could directly enter murine organs

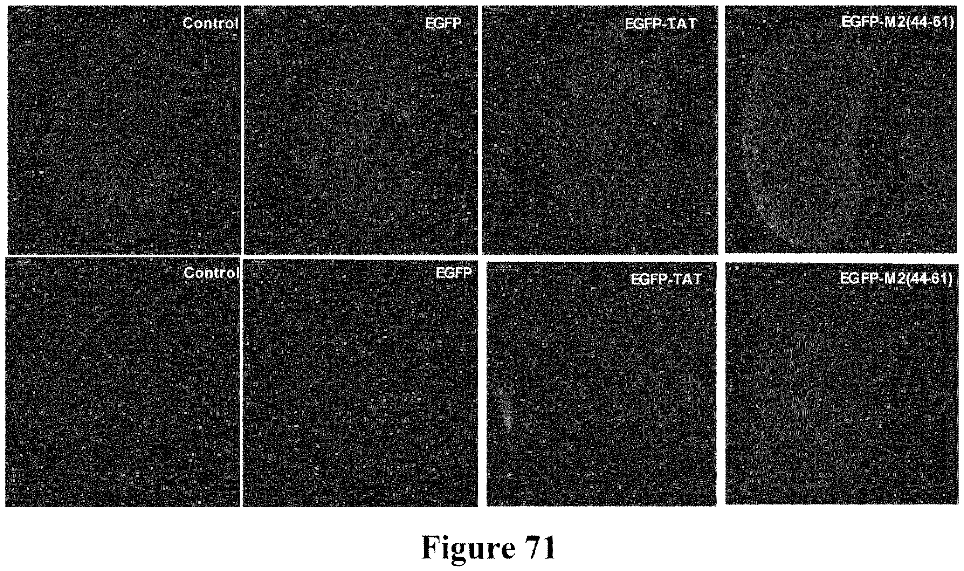

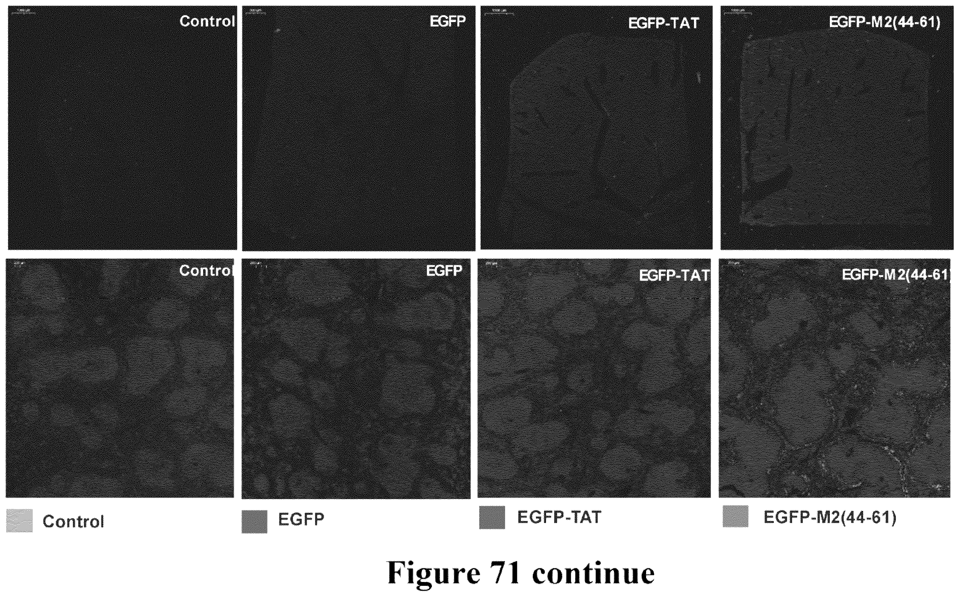

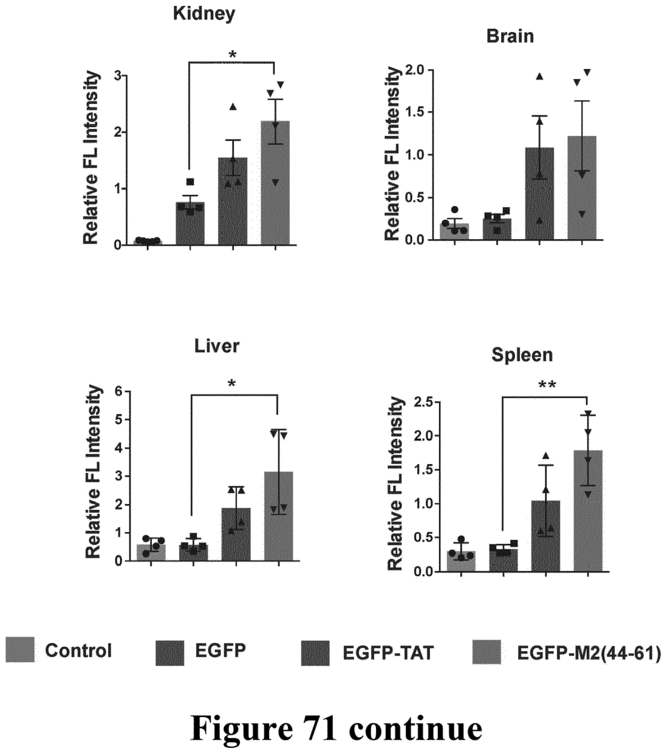

Example 13 proved that EGFP-Jilin-2004(H5N1)-M2-45-62 and EGFP-Jilin-2004(H5N1)-M2-44-61 fusion proteins still had penetrating peptide function and could enter cells efficiently. Figure 71 showed that EGFP-Jilin-2004(H5N1)-M2-44-61 could enter the murine brain, kidney, liver and spleen 1 h after intraperitoneal injection of mice, and its entry effect was stronger than that of cell-penetrating peptide control TAT under the same experimental conditions.

### Example 15: Entry of cell-penetrating peptides carrying small RNA into murine organs

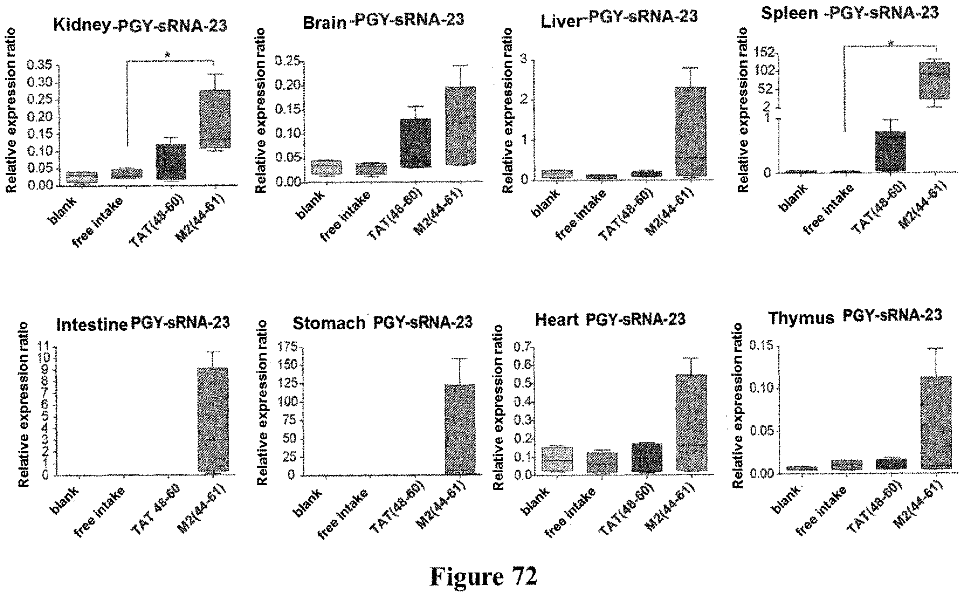

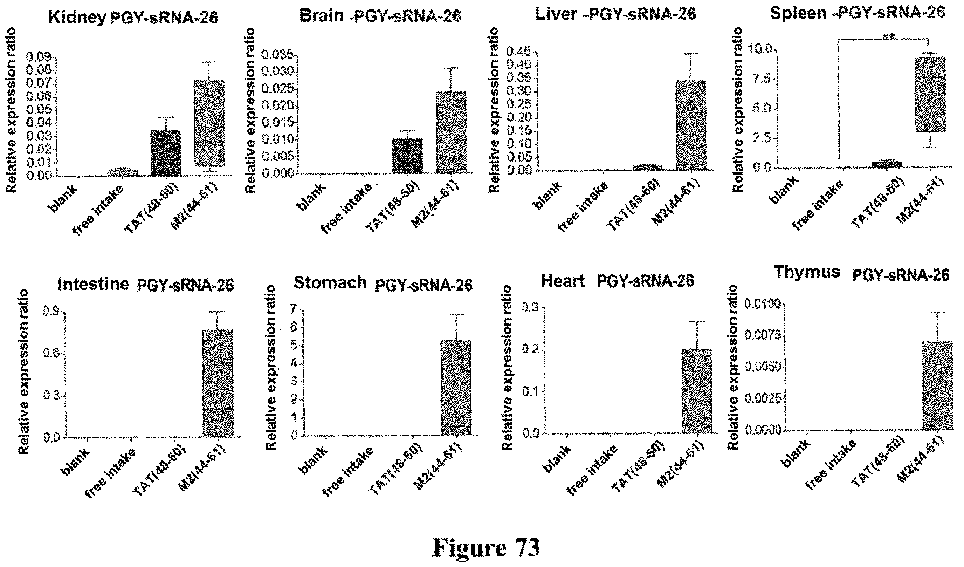

In Example 10, function of the cell-penetrating peptides derived from the influenza virus M2 protein and the control cell-penetrating peptide to carry small RNA into cells was detected by confocal laser detection and real-time quantitative qPCR methods. Jilin-2004(H5N1)-M2-44-61, with the strongest ability to carry small RNA in the cell experiments, was selected to be experimentally verified in mice. Figure 72 and Figure 73 showed that Jilin-2004(H5N1)-M2-44-61 could effectively deliver small RNAs (PGY-sRNA-23 and PGY-sRNA-26) to murine organs. Among them, expressions of small RNA were detected in 8 murine organs or tissues, including heart, liver, spleen, lung, kidney, stomach, small intestine and thymus. The ability of Jilin-2004(H5N1)-M2-44-61 to deliver small RNA was significantly stronger than that of TAT.

### Example 16: Entry of cell-penetrating peptides carrying small RNA into murine organs and the function

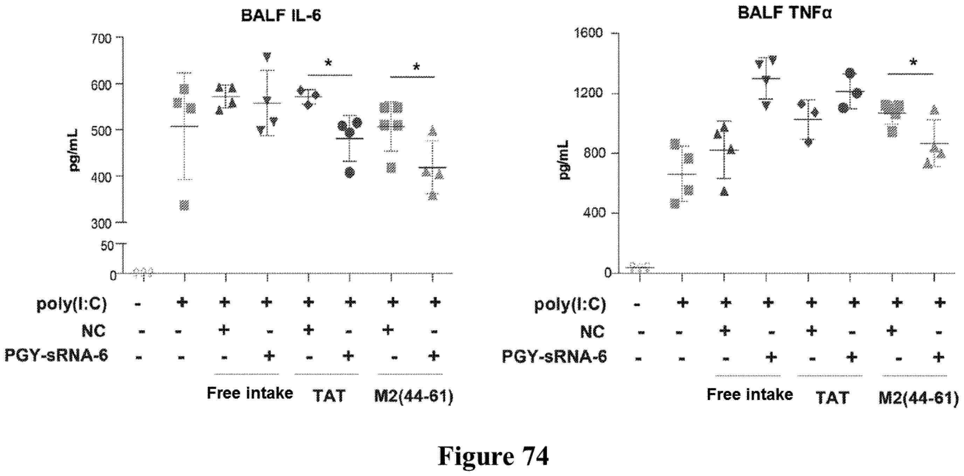

PGY-dsRNA-6 can target and bind the 3'-UTR region of the *RELA* gene and fulfill its anti-inflammatory effect. In the poly(I:C)-induced acute lung injury mouse model, as shown in Figure 74, PGY-sRNA-6 carried by Jilin-2004(H5N1)-M2-44-61effectively reduced IL-6 and TNF-α expression in the alveolar lavage fluid of mice, and reduced acute lung injury in mice. Meanwhile, PGY-sRNA-6 carried by the cell-penetrating peptide control TAT could only slightly reduce the expression of IL-6 in the alveolar lavage fluid of mice, while having no effect on TNF-α expression. The ability of Jilin-2004(H5N1)-M2-44-61to carry small RNA in murine organs was stronger than that of TAT, and the small RNA could perform its function better.

### Example 17: Entry of cell-penetrating peptides carrying small RNA antagomir into murine organs and the function

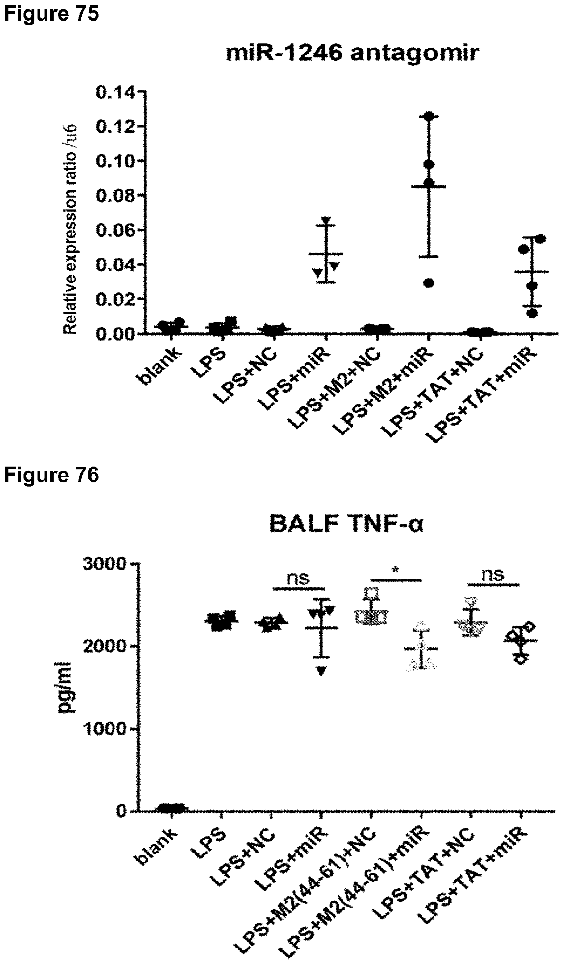

It has been reported in the literature that miR1246 antagomir can effectively reduce the LPS-induced acute lung injury in mice. We verified through experiments whether Jilin-2004(H5N1)-M2-44-61 could carry miR1246 antagomir into murine organs to better fulfill its anti-acute lung injury function. Figure 75 showed that Jilin-2004(H5N1)-M2-44-61 could improve the entry of miR1246 antagomir into lung tissue of mice, while TAT in the control group had almost no effect on the entry of miR1246 antagomir into the lung tissue. As shown in Figure 76, in the LPS-stimulated acute lung injury mouse model, Jilin-2004(H5N1)-M2-44-61 carrying miR1246 antagomir had the best effect on preventing lung injury. Therefore, Jilin-2004(H5N1)-M2-44-61 could facilitate entry of small RNA into mice and fulfill its function.

### Example 18: Entry of cell-penetrating peptides carrying small molecule drug into murine organs and fthe function

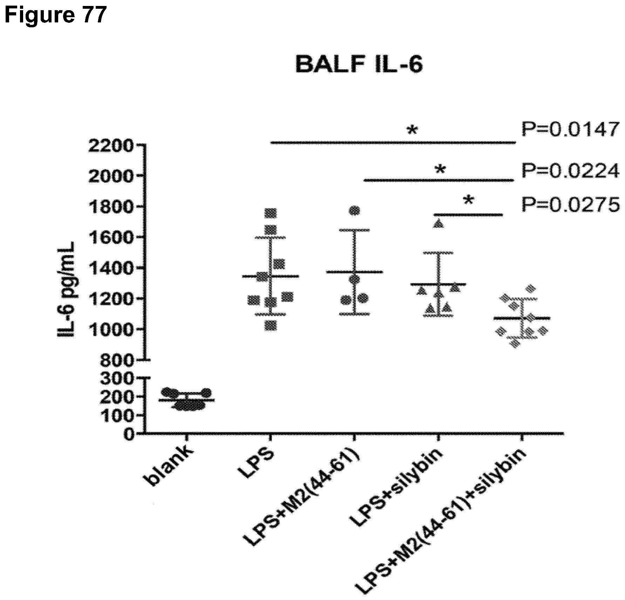

It has been reported in the literature that small molecule drug silybin can effectively reduce the LPS-induced acute lung injury in mice. We verified through experiments whether Jilin-2004(H5N1)-M2-44-61 could carry small molecule drug silybin into murine organs to better fulfill its anti-acute lung injury function. As shown in Figure 77, in the LPS-stimulated acute lung injury mouse model, Jilin-2004(H5N1)-M2-44-61 itself had no effect on preventing lung injury, and the small molecule compound silybin had a tendency to prevent lung injury at this dose. Meanwhile, Jilin-2004(H5N1)-M2-44-61 carrying the small molecule drug silybin had the best effect on preventing lung injury. Therefore, Jilin-2004(H5N1)-M2-44-61 could facilitate entry of small molecule compound into mice and fulfill its function.

## Claims

1. A polypeptide, which has an amino acid sequence selected from the group consisting of:
a. an amino acid sequence comprising amino acid sequence 44-67 of an influenza virus M2 protein or a fragment thereof;
b. an amino acid sequence comprising amino acid sequence 44-67 of the influenza virus M2 protein or the fragment thereof with modification of one or more amino acid residue(s); and
c. an amino acid sequence comprising an amino acid sequence that has at least 60%, at least 70%, at least 80%, at least 85%, at least 90%, at least 95%, or 100% sequence identity with amino acid sequence 44-67 of the influenza virus M2 protein or the fragment thereof,
wherein the fragment is at least 8 amino acids in length, and the polypeptide has the function of cell-penetrating peptides.

2. The polypeptide according to claim 1, wherein the length of the fragment is at least 10 amino acids.

3. The polypeptide according to claim 2, wherein the length of the fragment is at least 13 amino acids.

4. The polypeptide according to any one of claims 1 to 3, wherein the fragment comprises amino acids 44-48 or amino acids 45-48 of the M2 protein.

5. The polypeptide according to claim 4, wherein the fragment comprises amino acids 44-65, amino acids 44-61, amino acids 45-62 or amino acids 44-56 of the influenza virus M2 protein.

6. The polypeptide according to any one of claims 1 to 5, wherein the modification of one or more amino acid residue(s) is substitution, deletion or insertion of one or more amino acid residue(s).

7. The polypeptide according to claim 6, wherein the substitution, deletion or insertion of one or more amino acid residue(s) is substitution, deletion or insertion of 1-10 amino acid residue(s).

8. The polypeptide according to claim 6, wherein the substitution, deletion or insertion of one or more amino acid residue(s) is substitution, deletion or insertion of 1-5, such as 4, 3 or 2 amino acid residue(s).

9. The polypeptide according to any one of claims 6 to 8, wherein at least one of the substitutions is substitution with a hydrophobic amino acid or positively charged amino acid, the insertion is insertion of a hydrophobic amino acid or positively charged amino acid, and the deletion is deletion of a hydrophilic amino acid;
wherein the hydrophobic amino acid is preferably C, A, F, I, L, M, P, V, W, M or Y, more preferably I;
the positively charged amino acid is preferably K, R or H, more preferably K or R;
the hydrophilic amino acid is preferably D, E, Q, S or T.

10. The polypeptide according to claim 9, wherein the hydrophobic amino acid or positively charged amino acid is selected from the group consisting of isoleucine (I), phenylalanine (F) and arginine (R) residues.

11. The polypeptide according to any one of claims 1 to 10, wherein the influenza virus is H1N1, H5N1, H5N6 or H7N9 influenza virus.

12. The polypeptide according to any one of claims 1 to 10, which has an amino acid sequence of the following formula X1-X2-X3-X4-X5-X6-X7-X8-X9-X10-X11-X12-X13-X14-X15-X16-X17-X18-X19-X20-X2 1-X22-X23-X24;
wherein, X1 is D, R, L, I or absent;
X2 is R, I or S;
X3 is L, I, F, A, R or K;
X4 is F, R or I;
X5 is F, R or I;
X6 is K, R or I;
X7 is C, I or R;
X8 is A, I, F, K or R;
X9 is Y, I, F, K or R;
X10 is R or S;
X11 is R, S or absent;
X12 is L, I, F, R or K or absent;
X13 is K, R, T or absent;
X14 is Y, F, R or K or absent;
X15 is G, R, K or absent;
X16 is R, L, F or A or absent;
X17 is I, K, R or T or absent;
X18 is R, I, T or absent;
X19 is G, R, K or absent;
X20 is P or absent;
X21 is S, K or absent;
X22 is T, V or absent;
X23 is E or absent;
X24 is G or absent; or
the polypeptide has an amino acid sequence that has at least 60%, at least 70%, at least 80%, at least 85%, at least 90%, at least 95%, or 100% sequence identity with the amino acid sequence; or
the polypeptide has an amino acid sequence with deletion, insertion or substitution of one or more amino acid residue(s) in the amino acid sequence.

13. A polypeptide, which has an amino acid sequence selected from the group consisting of:
a. an amino acid sequence comprising DRLFFKCIYRRLK;
b. an amino acid sequence comprising amino acid sequence DRLFFKCIYRRLK with substitution, deletion or insertion of one or more amino acid residue(s), and
c. an amino acid sequence comprising an amino acid sequence that has at least 60%, at least 70%, at least 80%, at least 85%, at least 90%, at least 95% or 100% sequence identity with DRLFFKCIYRRLK,
wherein the polypeptide has the function of cell-penetrating peptides.

14. The polypeptide according to claim 13, wherein the substitution, deletion or insertion of one or more amino acid residue(s) is substitution, deletion or insertion of 1-5, such as 4, 3 or 2 amino acid residue(s).

15. The polypeptide according to claim 13 or 14, wherein at least one of the substitutions is substitution with a hydrophobic amino acid or positively charged amino acid, the insertion is insertion of a hydrophobic amino acid or positively charged amino acid, and the deletion is deletion of a hydrophilic amino acid;
wherein the hydrophobic amino acid is preferably C, A, F, I, L, M, P, V, W, M or Y, preferably I;
the positively charged amino acid is preferably K, R or H, more preferably K or R;
the hydrophilic amino acid is preferably D, E, Q, S or T.

16. The polypeptide according to claim 15, wherein the hydrophobic amino acid or positively charged amino acid is selected from the group consisting of I, F and R residues.

17. The polypeptide according to any one of claims 13 to 16, which has an amino acid sequence of the following formula X1-X2-X3-X4-X5-X6-X7-X8-X9-X10-X11-X12-X13;
wherein, X1 is D or R;
X2 is R;
X3 is L, I or F;
X4 is F;
X5 is F;
X6 is K or R;
X7 is C or R;
X8 is I or F;
X9 is Y, I or F;
X10 is R;
X11 is R;
X12 is L, I or F;
and X13 is K or R; or
the polypeptide has an amino acid sequence that has at least 60%, at least 70%, at least 80%, at least 85%, at least 90%, at least 95%, or 100% sequence identity with the amino acid sequence; or an amino acid sequence with substitution, deletion or insertion of one or more amino acid residue(s) in the amino acid sequence.

18. The polypeptide according to claim 13, wherein the polypeptide has an amino acid sequence selected from the group consisting of RRLFFKCIYRRLK, RRLFFRCIYRRLK, RRLFFRRIYRRLK, RRLFFRRIYRRLR, DRIFFKCIYRRLK, DRIFFKCIIRRLK, DRIFFKCIIRRIK, DRFFFKCIYRRLK, DRFFFKCFYRRLK, DRFFFKCFFRRLK, DRFFFKCFFRRFK and RRFFFRRFFRRFR.

19. A polypeptide, which has an amino acid sequence selected from the group consisting of:
a. an amino acid sequence comprising RLFFKCIYRRLKYGLKRG;
b. an amino acid sequence comprising amino acid sequence RLFFKCIYRRLKYGLKRG with substitution, deletion or insertion of one or more amino acid residue(s), and
c. an amino acid sequence comprising an amino acid sequence that has at least 60%, at least 70%, at least 80%, at least 85%, at least 90%, at least 95% or 100% sequence identity with DRLFFKCIYRRLKYGLKR,
wherein the polypeptide has the function of cell-penetrating peptides.

20. The polypeptide according to claim 19, wherein the substitution, deletion or insertion of one or more amino acid residue(s) is substitution, deletion or insertion of 1-6, such as 5, 4, 3 or 2 amino acid residue(s).

21. The polypeptide according to claim 19 or 20, wherein at least one of the substitutions is substitution with a hydrophobic amino acid or positively charged amino acid, the insertion is insertion of a hydrophobic amino acid or positively charged amino acid, and the deletion is deletion of a hydrophilic amino acid;
wherein the hydrophobic amino acid is preferably C, A, F, I, L, M, P, V, W, M or Y, preferably I; the hydrophilic amino acid is preferably D, E, Q, S or T;
the positively charged amino acid is preferably K, R or H, more preferably K or R;
the hydrophilic amino acid is preferably D, E, Q, S or T.

22. The polypeptide according to claim 21, wherein the hydrophobic amino acid or positively charged amino acid is selected from the group consisting of I, F and R residues.

23. The polypeptide according to any one of claims 19 to 22, which has an amino acid sequence of the following formula X1-X2-X3-X4-X5-X6-X7-X8-X9-X10-X11-X12-X13-X14-X15-X16-X17-X18;
wherein, X1 is R or S;
X2 is A, L or F;
X3 is F or I;
X4 is F or I;
X5 is R or K;
X6 is I, C or R;
X7 is A, I or F;
X8 is K, Y or F;
X9 is R or S;
X10 is R or S;
X11 is A, L or F;
X12 is K, R or T;
X13 is Y, ForK;
X14 is G, R or K;
X15 isL, F or A;
X16 is K, R or T;
X17 is R or T; and
X18 is G, R or K; or
the polypeptide has an amino acid sequence that has at least 60%, at least 70%, at least 80%, at least 85%, at least 90%, at least 95%, or 100% sequence identity with the amino acid sequence; or an amino acid sequence with substitution, deletion or insertion of one or more amino acid residue(s) in the amino acid sequence.

24. The polypeptide according to claim 19, wherein the polypeptide has an amino acid sequence selected from the group consisting of RLFFRCIYRRLKYGLKRG, RLFFRRIYRRLKYGLKRG, RLFFRRIYRRLRYGLKRG, RLFFRRIYRRLRYRLKRG, RLFFRRIYRRLRYRLRRG, RLFFRRIYRRLRYRLRRR, RFFFKCIYRRLKYGLKRG, RFFFKCFYRRLKYGLKRG, RFFFKCFFRRLKYGLKRG, RFFFKCFFRRFKYGLKRG, RFFFKCFFRRFKFGLKRG, RFFFKCFFRRFKFGFKRG and RFFFRRFFRRFRFRFRRR.

25. The polypeptide according to claim 19, wherein the polypeptide has an amino acid sequence selected from the group consisting of SLFFKCIYRRLKYGLKRG, RAFFKCIYRRLKYGLKRG, RLIFKCIYRRLKYGLKRG, RLFIKCIYRRLKYGLKRG, RLFFKIIYRRLKYGLKRG, RLFFKCAYRRLKYGLKRG, RLFFKCIKRRLKYGLKRG, RLFFKCIYSRLKYGLKRG, RLFFKCIYRSLKYGLKRG, SLFFKCIYRRLKYGLKRG, RLFFKCIYRRAKYGLKRG, RLFFKCIYRRLTYGLKRG, RLFFKCIYRRLKKGLKRG, RLFFKCIYRRLKYKLKRG, RLFFKCIYRRLKYGAKRG, RLFFKCIYRRLKYGLTRG, RLFFKCIYRRLKYGLKTG and RLFFKCIYRRLKYGLKRK.

26. A polypeptide, which has an amino acid sequence selected from the group consisting of:
a. an amino acid sequence comprising DRLFFKCIYRRLKYGLKRGPST; and
b. an amino acid sequence comprising amino acid sequence DRLFFKCIYRRLKYGLKRGPST with substitution, deletion or insertion of one or more amino acid residue(s),
c. an amino acid sequence comprising an amino acid sequence that has at least 60%, at least 70%, at least 80%, at least 85%, at least 90%, at least 95% or 100% sequence identity with DRLFFKCIYRRLKYGLKRGPST,
wherein the polypeptide has the function of cell-penetrating peptides.

27. The polypeptide according to claim 26, wherein the substitution, deletion or insertion of one or more amino acid residue(s) is substitution, deletion or insertion of 1-10 or 1-6, such as 5, 4, 3 or 2 amino acid residue(s).

28. The polypeptide according to claim 26 or 27, wherein at least one of the substitutions is substitution with a hydrophobic amino acid or positively charged amino acid, the insertion is insertion of a hydrophobic amino acid or positively charged amino acid, and the deletion is deletion of a hydrophilic amino acid;
wherein the hydrophobic amino acid is preferably C, A, F, I, L, M, P, V, W, M or Y, preferably I;
the positively charged amino acid is preferably K, R or H, more preferably K or R;
the hydrophilic amino acid is preferably D, E, Q, S or T.

29. The polypeptide according to claim 28, wherein the hydrophobic amino acid or positively charged amino acid is selected from the group consisting of I, F and R residues.

30. The polypeptide according to any one of claims 26 to 29, which has an amino acid sequence of the following formula X1-X2-X3-X4-X5-X6-X7-X8-X9-X10-X11-X12-X13-X14-X15-X16-X17-X18-X19-X20-X2 1-X22;
wherein, X1 is D or R;
X2 is R;
X3 is L or F;
X4 is F;
X5 is F;
X6 is K or R;
X7 is C or R;
X8 is I or F;
X9 is Y or F;
X10 is R;
X11 is R;
X12 is L or F;
X13 is K or R;
X14 is Y or F;
X15 is G or R;
X16 is L;
X17 is K or R;
X18 is R;
X19 is G or R or K;
X20 is P;
X21 is S or K; and
X22 is T or V; or
the polypeptide has an amino acid sequence that has at least 60%, at least 70%, at least 80%, at least 85%, at least 90%, at least 95%, or 100% sequence identity with the amino acid sequence; or an amino acid sequence with substitution, deletion or insertion of one or more amino acid residue(s) in the amino acid sequence.

31. The polypeptide according to claim 26, wherein the polypeptide has an amino acid sequence selected from the group consisting of DRFFFKCIYRRLKYGLKRGPST, DRFFFKCIYRRLKYGLKRRPST, DRFFFKCIYRRLKFGLKRGPST, DRFFFKCIFRRLKYGLKRGPST, DRFFFKCIFRRLKFGLKRGPST, DRFFFKCFFRRFKFGLKRGPST, RRFFFKCFFRRFKFGLKRGPST, DRFFFKRIYRRLKYRLKRRPST, DRFFFKRIYRRLKYRLKRRPKV, DRFFFKRIFRRLKYRLKRRPST, DRFFFKRIFRRLKFRLKRRPST, DRFFFKRIFRRFKFRLKRRPST, RRFFFKRIFRRFKFRLKRRPST, RRFFFKRIFRRFKFRLKRRPKV, RRLFFKCIYRRLKYGLKRGPST, RRLFFKCIYRRLKYGLKRGPKV, RRFFFKCIYRRLKYGLKRGPST, RRLFFKCIYRRLKYGLKRRPST, RRLFFKCIYRRLKYGLKRKPST, RRLFFRRIYRRLKYGLKRGPST, RRLFFRRIYRRLRYRLRRRPST, DRLFFKRIYRRLKYGLKRGPST, DRLFFRRIYRRLKYGLKRGPST, DRLFFRRIYRRLRYRLRRRPST, DRLFFKCIYRRLKYGLKRRPST, DRLFFKCIYRRLKYRLKRRPST, DRLFFKRIYRRLKYRLKRRPST, RRLFFKRIYRRLKYRLKRRPST and DRLFFKCIYRRLKYGLKRGPKV.

32. A polypeptide, which has an amino acid sequence selected from the group consisting of:
a. an amino acid sequence comprising DRLFFKCIYRRLKYGLKR; and
b. an amino acid sequence comprising amino acid sequence DRLFFKCIYRRLKYGLKR with substitution, deletion or insertion of one or more amino acid residue(s),
c. an amino acid sequence comprising an amino acid sequence that has at least 60%, at least 70%, at least 80%, at least 85%, at least 90%, at least 95% or 100% sequence identity with DRLFFKCIYRRLKYGLKR,
wherein the polypeptide has the function of cell-penetrating peptides.

33. The polypeptide according to claim 32, wherein the substitution, deletion or insertion of one or more amino acid residue(s) is substitution, deletion or insertion of 1-10 or 1-6, such as 5, 4, 3 or 2 amino acid residue(s).

34. The polypeptide according to claim 32 or 33, wherein at least one of the substitutions is substitution with a hydrophobic amino acid or positively charged amino acid, the insertion is insertion of a hydrophobic amino acid or positively charged amino acid, and the deletion is deletion of a hydrophilic amino acid;
wherein the hydrophobic amino acid is preferably C, A, F, I, L, M, P, V, W, M or Y, preferably I;
the positively charged amino acid is preferably K, R or H, more preferably K or R;
the hydrophilic amino acid is preferably D, E, Q, S or T.

35. The polypeptide according to any one of claims 32 to 34, which has an amino acid sequence of the following formula X1-X2-X3-X4-X5-X6-X7-X8-X9-X10-X11-X12-X13-X14-X15-X16-X17-X18;
wherein, X1 is D, R, L or I;
X2 is R or I;
X3 is L, R or K;
X4 is F or R;
X5 is F or R;
X6 is K or I;
X7 is C, R or I;
X8 is I, K or R;
X9 is Y, K or R;
X10 is R;
X11 is R;
X12 is L, R or K;
X13 is K;
X14 is Y or R;
X15 is G;
X16 is L or R;
X17 is K or I; and
X18 is R or I; or
the polypeptide has an amino acid sequence that has at least 60%, at least 70%, at least 80%, at least 85%, at least 90%, at least 95%, or 100% sequence identity with the amino acid sequence; or an amino acid sequence with substitution, deletion or insertion of one or more amino acid residue(s) in the amino acid sequence.

36. The polypeptide according to claim 32, wherein the polypeptide has an amino acid sequence selected from the group consisting of RRLFFKCIYRRLKYGLKR, LRLFFKCIYRRLKYGLKR, DILFFKCIYRRLKYGLKR, DRRFFKCIYRRLKYGLKR, DRLRRKCIYRRLKYGLKR, DRLFFICIYRRLKYGLKR, DRLFFKRIYRRLKYGLKR, DRLFFKIIYRRLKYGLKR, DRLFFKCKKRRLKYGLKR, DRRRRKCRRRRRKYGRKR, DRLFFKCIYRRRKRGLKR, DRLFFKCIYRRLKYGRKR, DRKRRKCKYRRKKYGRKR, DRLFFKCIYRRLKYGLII and IILFFKCIYRRLKYGLKR.

37. A conjugate or composition, wherein the conjugate comprises the polypeptide according to any one of claims 1 to 36 and a moiety conjugated to the polypeptide, the composition comprises the polypeptide according to any one of claims 1 to 36 and a moiety non-covalently linked to the polypeptide; wherein the moiety is a therapeutic moiety, a detectable moiety or a cosmetic moiety, selected from the group consisting of protein, peptide, nucleic acid, antibiotic, anti-inflammatory drug, anti-tumor drug, neuroprotective agent, chemotherapeutic, cytotoxin, radioisotope, fluorescent marker, luminescent substance, chromogenic substance and enzyme;
preferably, the therapeutic moiety is selected from the group consisting of 13-cis-retinoic acid, 2-amino-6-mercaptopurine, 2-CdA, 2-chlorodeoxyadenosine, 5-fluorouracil, 6-thioguanine, 6-mercaptopurine, Accutane, Actinomycin D, adriamycin, Adrucil, Agrylin, Ala-Cort, Aldesleukin, Alemtuzumab, Alitretinoin, Alkaban-AQ, Alkeran, All-trans-retinoicacid, α interferon, Altretamine, Amethopterin, Amifostine, Aminoglutethimide, Anagrelide, Anandron, Anastrozle, Arabinosylcytosine, Aranesp, Aredia, Arimidex, Aromasin, Arsenic trioxide, Asparaginase, ATRA, Avastin, BCG, BCNU, Bevacizumab, Bexarotene, Bicalutamide, BiCNU, Blenoxane, Bleomycin, Bortezomib, Busulfan, Busulfex, C225, Calcium Folinate, Campath, Camptosar, Camptothecin-11, Capecitabine, Carac, Carboplatin, Carmustine, Carmustine Tablet, Casodex, CCNU, CDDP, CeeNU, Daunorubicin, Cetuximab, Chlorambucil, Cisplatin, Citrovorum Factor, Cladribine, Cortisone, Cosmegen, CPT-11, Cyclophosphamide, Cytadren, Cytarabine, Cytarabine Liposome, Cytosar-U, Cytoxan, Dacarbazine, Dactinomycin, Dapomycin α, Daunomycin, Daunorubicin, Daunorubicin Hydrochloride, Daunorubicin Liposome, DaunoXome, Decadron, Delta-Cortef, Deltasone, Denileukin-diftitox, DepoCyt, Dexamethasone, Dexamethasone Acetate, Dexamethasone Sodium Phosphate, Dexasone, Dexrazoxane, DHAD, DIC, Diodex, Docetaxel, Doxil, Doxorubicin, Doxorubicin Liposome, Droxia, DTIC, DTIC-Dome, Duralone, Efudex, Eligard, Ellence, Eloxatin, Elspar, Emcyt, Epirubicin, Epoetin α, Erbitux, Erwinase, Estramusting, Ethyol, Etopophos, Etoposide, Etoposide Phosphate, Eulexin, Evista, Exemestane, Fareston, Faslodex, Femara, Filgrastim, Floxuridine, Fludara, Fludarabine, Fluoroplex, Fluorouracil, Fluoxymesterone, Flutamide, Folinic Acid, FUDR, Fulvestrant, G-CSF, Gefitinib, Gemcitabine, Gemtuzumab, Gemzar, Gleevec, Leuprorelin Acetate, LupronDepot, Matulane, Maxidex, Mechlorethamine, Mechlorethamine Hydrochloride, Medrol, Methylprednisolone, Megace, Megestrol, Megestrol Acetate, Melphalan, Mercaptopurine, Mesna, Mesnex, Methotrexate, Methotrexate Sodium, Methylprednisolone, Mylocel, Letrozole, Neosar, Neulasta, Neumega, Neupogen, Nilandron, Nilutamide, Nitrogen Mustard, Novaldex, Novantrone, Octreotide, Octreotide Acetate, Oncospar, Oncovin, Ontak, Onxal, Oprevelkin, Orapred, Orasone, Oxaliplatin, Paclitaxel, Pamidronate, Panretin, Paraplatin, Pediapred, PEG interferon, Pegaspargase, Pegfilgrastim, PEG-INTRON, PEG-L-Asparaginase, Phenylalanine Mechlorethamine, Platinol, Platinol-AQ, Prednisolone, Prednison, Prelone, Procarbazine, PROCRIT, Proleukin, Polifeprosan 20 with Carmustine Implant, Purinethol, Raloxifene, Rheumatrex, Rituxan, Rituximab, Roveron-A, Rubex, Rubidomycin Hydrochloride, Sandostatin, Sandostatin LAR, Sargramostim, Solu-Cortef, Solu-Medrol, STI-571, Streptozocin, Tamoxifen, Taggretine, Taxol, Taxotere, Temodar, Temozolomide, Teniposide, TESPA, Thalidomide, Thalomid, TheraCys, Thioguanine, Thioguanine Tablet, Thioxophosphamide, Thioplex, Thiotepa, TICE, Toposar, Topotecan, Toremifene, Trastuzumab, Tretinoin, Trexall, Trisenox, TSPA, VCR, Velban, Velcade, Vepesid, Vesanoid, Viadur, Vinblastine, Vinblastine Sulfate, Vincasar Pfs, Vincristine, Vinorelbine, Vinorelbine Tartrate, VLB, VP-16, Vumon, Xeloda, Zanosar, Zevalin, Zinecard, Zoladex, Zoledronic Acid, Zometa, Gliadel, Glivec, GM-CSF, Goserelin, Granulocyte Colony Stimulating Factor, Fluoxymesterone, Herceptin, Hexadrol, Hexalen, Altretamine, HMM, Hycamtin, Hydrea, Hydrocortisone Acetate, Hydrocortisone, Hydrocortisone Sodium Phosphate, Hydrocortisone Sodium Succinate, Hydrocortisone Phosphate, Hydroxyurea, Ibritumomab, Ibritumomab Tiuxetan, Idamycin, Idarubicin, Ifex, Interferon-alpha, Ifosfamide, IL2, IL-11, Imatinib Mesylate, Imidazole Carboxamide, Interferon α, PEG-interferon α, Interleukin 2, Interleukin 11, Leucovorin, Leukeran, Leukine, Leuprorelin, Leurocristine, Leustatin, Liposomal Ara-C, Liquid Pred, Lomustine, L-PAM, L-Sarcolysin, Meticorten, Mitomycin, Mitomycin-C, Mitoxantrone, M-Metacortandracin, MTC, MTX, Mustargen, Mutamycin, Myleran, Iressa, Irinotecan, Isotretinoin, Kidrolase, Lanacort, L-Asparaginasum and LCR, Ganciclovir, Azido Deoxythymidine (AZT), Lamivudine (3TC), Acapolone, acetyl Sodium Sulfone, Alymycin, Alessidine, Mecillinam, Pivmecillinam, Amicycline, Amifloxacin, Amfloxacin Mesylate, Amikacin, Amikacin Sulfate, Aminosalicylic Acid, Sodium Aminosalicylate, Amoxicillin, Amphotericin, Ampicillin, Ampicillin Sodium, Apalcillin Sodium, Apramycin, Aspartate, Aspartate Sulfate, Avilamycin, Avoparcin, Azithromycin, Azlocillin, Azlocillin Sodium, Bacampicillin Hydrochloride, Bacitracin, Bacitracin Methylene Disalicylate, Bacitracin Zinc, Bambermycin, Benzoyl Calcium, Erythromycin Sulfate, Betamycin Sulfate, Biapenem, Beniamycin, Biphenamine Hydrochloride, Biscaptooxypyridine Magnesium Sulfate, Buticacin, Butylosidectin Sulfate, Capreomycin Sulfate, Capadol, Carbenicillin Disodium, Carbenicillin Indene Sodium, Carbenicillin Sodium, Carbenicillin Potassium, Coumarin Sodium, Cefaclor, Cefalexin, Cefamendol, Cefamendole Sodium, Cefamendol Sodium, Cefparo, Ceftriaxone, Cefazoline Sodium, Cefazoline, Cefoperazone, Cefdinir, Cefepime, Cefepime Hydrochloride, Cefanol, Cefoxime, Cefoxime Hydrochloride, Cefmezole, Cefmezole Sodium, Cefniximetam Sodium, Cefonicid Sodium, Cefoperazone Sodium, Cefdinib, Cefotaxime Sodium, Cefotetam, Cefotetam Disodium, Cefotiam Hydrochloride, Cefoxitin, Cefoxitin Sodium, Cefimizole, Cefpiazide Sodium, Cefpiramide, Cefpiramide Sodium, Cefpirome Sulfate, Cefpodoxime Proxetil, Cefprozil, Cefrotidine, Cefsulodine Sodium, Ceftazidime, Ceftibuten, Ceftizoxime Sodium, Ceftriaxone Sodium, Cefuroxime, Cefuroxime Ester, Cefacetonitrile Sodium, Cefexin, Cefexin Hydrochloride, Cefosporin, Cefotaxidine, Cefothiophene Sodium, Cefpirin Sodium, Cefradine, Cetocycline Hydrochloride, Chloramphenicol Acetyl, Chloramphenicol, Chloramphenicol Palmitate, Chloramphenicol Pantothenate Complex, Chloramphenicol Sodium Succinate, Chlorhexidine Aminobenzene Phosphate, Chloroxylphenol, Chlorotetracycline Hydrosulfate, Chlorotetracycline Hydrochloride, Cinoxacin, Ciprofloxacin, Ciprofloxacin Hydrochloride, Cirolemycin, Clarithromycin, Clindfloxacin Hydrochloride, Clindamycin, Clindamycin Hydrochloride, Clindamycin Palmitate Hydrochloride, Clindamycin Phosphate, Clofazimine,Cloxacillin Benzathine, Cloxacillin Sodium, Chlorohydroxyquine, Colistin Mesylate Sodium, Coumarin, Coumarin Sodium, Cyclocillin, Cycloserine, Dafoptine, Dapsone, Datoramycin, Demecycline, Demecycline Hydrochloride, Demecycline, Denofungin, Diaveridine, Dicloxacillin, Dicloxacillin Sodium, Dihydrostreptomycin Sulfate, Dipyrithione, Dierythromycin, Doxycycline, Doxycycline Calcium, Doxycycline Phosphate Complex, Doxycycline Hydrochloride, Droxacin Sodium, Enoxacin, Epicillin, Epitetracycline Hydrochloride, Erythromycin, Erythromycin acistrate, erythromycin estolate, erythromycin ethyl succinate, erythromycin gluceptate, erythromycin lactobionate, erythromycin propionate, erythromycin stearate, ethambutol hydrochloride, ethionamide, fleroxacin, floxacillin, fludalanine, flumequine, fosfomycin, fosfomycin tromethamine, fumoxicillin, furazolium chloride, furazolium tartrate, fusidate sodium, fusidic acid, gentamicin sulfate, gloximonam, gramicidin, haloprogin, hetacillin, hetacillin potassium, hexedine, ibafloxacin, imipenem, isoconazole, isepamicin, isoniazid, josamycin, kanamycin sulfate, kitasamycin, levofuraltadone, levopropylcillin potassium, lexithromycin, lincomycin, lincomycin hydrochloride, lomefloxacin, Lomefloxacin hydrochloride, lomefloxacin mesylate, loracarbef, mafenide, meclocycline, meclocycline sulfosalicylate, megalomicin potassium phosphate, mequidox, meropenem, methacycline, methacycline hydrochloride, methenamine, methenamine hippurate, methenamine mandelate, methicillin sodium, metioprim, metronidazole hydrochloride, metronidazole phosphate, mezlocillin, mezlocillin sodium, minocycline, minocycline hydrochloride, mirincamycin hydrochloride, monensin, monensin sodiumr, nafcillin sodium, nalidixate sodium, nalidixic acid, natainycin, nebramycin, neomycin palmitate, neomycin sulfate, neomycin undecylenate, netilmicin sulfate, neutramycin, nifuiradene, nifuraldezone, nifuratel, nifuratrone, nifurdazil, nifurimide, nifiupirinol, nifurquinazol, nifurthiazole, nitrocycline, nitrofurantoin, nitromide, norfloxacin, novobiocin sodium, ofloxacin, onnetoprim, oxacillin, oxacillin sodium, oximonam, oximonam sodium, oxolinic acid, oxytetracycline, oxytetracycline calcium, oxytetracycline hydrochloride, paldimycin, parachlorophenol, paulomycin, pefloxacin, pefloxacin mesylate, penamecillin, penicillin G benzathine, penicillin G potassium, penicillin G procaine, penicillin G sodium, penicillin V, penicillin V benzathine, penicillin V hydrabamine, penicillin V potassium, pentizidone sodium, phenyl aminosalicylate, piperacillin sodium, pirbenicillin sodium, piridicillin sodium, pirlimycin hydrochloride, pivampicillin hydrochloride, pivampicillin pamoate, pivampicillin probenate, polymyxin B sulfate, porfiromycin, propikacin, pyrazinamide, pyrithione zinc, quindecamine acetate, quinupristin, racephenicol, ramoplanin, ranimycin, relomycin, repromicin, rifabutin, rifametane, rifamexil, rifamide, rifampin, rifapentine, rifaximin, rolitetracycline, rolitetracycline nitrate, rosaramicin, rosaramicin butyrate, rosaramicin propionate, rosaramicin sodium phosphate, rosaramicin stearate, rosoxacin, roxarsone, roxithromycin, sancycline, sanfetrinem sodium, sarmoxicillin, sarpicillin, scopafungin, sisomicin, sisomicin sulfate, sparfloxacin, spectinomycin hydrochloride, spiramycin, stallimycin hydrochloride, steffimycin, streptomycin sulfate, streptonicozid, sulfabenz, sulfabenzamide, sulfacetamide, sulfacetamide sodium, sulfacytine, sulfadiazine, sulfadiazine sodium, sulfadoxine, sulfalene, sulfamerazine, sulfameter, sulfamethazine, sulfamethizole, sulfamethoxazole, sulfamonomethoxine, sulfamoxole, sulfanilate zinc, sulfanitran, sulfasalazine, sulfasomizole, sulfathiazole, sulfazamet, sulfisoxazole, sulfisoxazole acetyl, sulfisboxazole diolamine, sulfomyxin, sulopenem, sultamricillin, suncillin sodium, talampicillin hydrochloride, teicoplanin, temafloxacin hydrochloride, temocillin, tetracycline, tetracycline hydrochloride, tetracycline phosphate complex, tetroxoprim, thiamphenicol, thiphencillin potassium, ticarcillin cresyl sodium, ticarcillin disodium, ticarcillin monosodium, ticlatone, tiodonium chloride, tobramycin, tobramycin sulfate, tosufloxacin, trimethoprim, trimethoprim sulfate, trisulfapyrimidines, troleandomycin, trospectomycin sulfate, tyrothricin, vancomycin, vancomycin hydrochloride, virginiamycin, or zorbamycin, Silybin;
preferably, the detectable moiety is selected from the group consisting of UV-Vis label, near infrared label, luminescent group, phosphorescent group, magnetic spin resonance label, photosensitizer, photocleavable moiety, chelating center, heavy atom, radioisotope, isotope detectable spin resonance label, paramagnetic moiety, chromophore, luminophore, such as metalloporphyrin; benzoporphyrin; azabenzoporphyrin; naphthoporphyrin, phthalocyanine; polycyclic aromatic hydrocarbon, such as perylene, perylene diimide, pyrene; azo dye; xanthene dye; boron dipyrromethene, aza-boron dipyrromethene, cyanine dye, metal-ligand complex such as bipyridine, bipyridyl, phenanthroline, coumarin and acetylacetonate of ruthenium and iridium; acridine and oxazine derivatives such as benzophenoxazine; aza-annulene, squaraine; 8-hydroxyquinoline, polymethine, luminescence-producing nanoparticles such as quantum dot and nanocrystal; quinolone, terbium conjugate; inorganic phosphor; ionophores such as crown ether attachment or derivatization of dyes; Pd(II) octaethylporphyrin; Pt(II)-octaethylporphyrin; Pd(II) tetraphenylporphyrin; Pt(II) tetraphenylporphyrin; Pd(II) meso-tetraphenylporphyrin tetrabenzomorphine; Pt(II) meso-tetraphenylmethyl benzoporphyrin; Pd(II) octaethylporphyrin; Pt(II) octaethylporphyrin; Pd(II) meso-tetra(pentafluorophenyl)porphyrin; Pt(II) meso-tetra(pentafluorophenyl)porphyrin; Ru(II) tris(4,7-diphenyl-1, 10-phenanthroline)(Ru(dpp)₃); Ru(II)tris(1,10-phenanthroline)(Ru(phen)₃), tris(2,2"-bipyridine) ruthenium(II) chloride hexahydrate (Ru(bpy)₃); erythrosine B; fluorescein; fluorescein isothiocyanate (FITC); eosin; iridium (III) ((N-methyl-benzimidazol-2-yl)-7-(diethylamino)-coumarin)); indium (III) ((benzothiazol-2-yl)-7-(diethylamino)-coumarin)-2-(acetylacetonate); Lumogen dye; Macroflex fluorescent red; Macrolex fluorescent yellow; Texas red; rhodamine B; rhodamine 6G; endorhodamine; m-cresol; thymol blue; xylenol blue; cresol red; chlorophenol blue; bromocresol green; bromocresol red; bromothymol blue; Cy2; Cy3; Cy5; Cy5.5; Cy7; 4-nitrophenol; alizarin; phenolphthalein; o-cresolphthalein; chlorophenol red; calcein; bromoxylenol; phenol red; neutral red; nitrazine; 3,4,5,6-tetrabromophenolphthalein; congo red; fluorescein; eosin; 2',7'-dichlorofluorescein; 5(6)-carboxy-fluorescein; carboxynaphthofluorescein; 8-hydroxystyrene-1,3,6-trisulfonic acid; seminaphthodifluoro; seminaphthofluorescein; tris(4,7-diphenyl-1,10-phenanthroline)ruthenium(II) dichloride; (4,7-diphenyl-1,10-phenanthroline)ruthenium(II) tetraphenyl boron; platinum(II) octaethylporphyrin; dialkyl carbocyanine; dioctadecyl cyclodicarbocyanine; fluorenylmethoxycarbonyl chloride; 7-amino-4-methylcoumarin (Amc); green fluorescent protein (such as GFP, GFP-2, tagGFP, turboGFP, eGFP, Emerald, Azami Green, monomer Azami Green, CopGFP, AceGFP, ZsGreen1), yellow fluorescent protein (such as YFP, eYFP, Citrine, Venus, YPet, PhiYFP, ZsYellowl), blue fluorescent protein (such as eBFP, eBFP2, Azurite, mKalamal, GFPuv, Sapphire, T-sapphire), cyan fluorescent protein (such as eCFP, Cerulean, CyPet, AmCyanl, Midoriishi-Cyan), red fluorescent protein (such as mKate, mKate2, mPlum, DsRed monomer, mCherry, mRFP1, DsRed-Express, DsRed2, DsRed-monomer, HcRed-Tandem, HcRedl, AsRed2, eqFP611, mRaspberry, mStrawberry, Jred), orange fluorescent protein (such as mOrange, mKO, Kusabira-Orange, monomer Kusabira-Orange, mTangerine, tdTomato) and any other suitable fluorescent proteins;
preferably, the cosmetic moiety is selected from cosmetic polypeptides, such as palmitoyl pentapeptide 4, palmitoyl tetrapeptide 7, carnosine, acetyl hexapeptide 8, aFGF, bFGF or EGF and botulinus toxin;
preferably, the nucleic acid is a natural or artificial single-stranded or double-stranded DNA or RNA molecule, which comprises one or more selected from the group consisting of: small RNA, DNA, cDNA, decoy DNA, RNA, siRNA, miRNA, shRNA, stRNA, snoRNA, snRNA, PNA, antisense oligomer, plasmid, cosmid, phage, artificial chromosome and other modified nucleic acids.

38. A fusion protein, the fusion protein comprises the polypeptide according to any one of claims 1 to 36 and a moiety fused to the polypeptide, the moiety is selected from the group consisting of antigen, antibody or antigen binding fragment thereof, ligand, receptor, cytokine, transcription regulation factor, fluorescent protein and enzyme;
preferably, the moiety is a therapeutic moiety, such as human growth hormone, bovine growth hormone, porcine growth hormone, growth hormone releasing hormone, growth hormone releasing peptide, granulocyte-colony stimulating factor, granulocyte macrophage-colony stimulating factor, macrophage-colony stimulating factor, erythropoietin, bone morphogenetic protein, interferon, insulin, atrial peptide hormone-III, monoclonal antibody, tumor necrosis factor, macrophage activating factor, interleukin, tumor degradation factor, insulin-like growth factor, epidermal growth factor, tissue plasminogen activator and urokinase; or
preferably, the moiety is a detectable moiety, for example, green fluorescent protein (such as GFP, GFP-2, tagGFP, turboGFP, eGFP, Emerald, Azami Green, monomer Azami Green, CopGFP, AceGFP, ZsGreen1), yellow fluorescent protein (such as YFP, eYFP, Citrine, Venus, YPet, PhiYFP, ZsYellowl), blue fluorescent protein (such as eBFP, eBFP2, Azurite, mKalamal, GFPuv, Sapphire, T-sapphire), cyan fluorescent protein (such as eCFP, Cerulean, CyPet, AmCyanl, Midoriishi-Cyan), red fluorescent protein (such as mKate, mKate2, mPlum, DsRed monomer, mCherry, mRFP1, DsRed-Express, DsRed2, DsRed-monomer, HcRed-Tandem , HcRedl, AsRed2, eqFP611, mRaspberry, mStrawberry, Jred), orange fluorescent protein (such as mOrange, mKO, Kusabira-Orange, monomer Kusabira-Orange, mTangerine, tdTomato) and any other suitable fluorescent proteins;
preferably, the moiety is a cosmetic moiety, selected from cosmetic polypeptides, such as palmitoyl pentapeptide 4, palmitoyl tetrapeptide 7, carnosine, acetyl hexapeptide 8, aFGF, bFGF or EGF, botulinus toxin, elastin and hyaluronic acid.

39. The fusion protein according to claim 38, wherein the polypeptide and the moiety are linked through a linker.

40. A method of allowing a polypeptide to penetrate a cell, the method comprising a step of incubating the polypeptide according to any one of claims 1 to 36, the conjugate or composition according to claim 37, or the fusion protein according to claim 38 or 39, with a cell.

41. The method according to claim 40, wherein the cell is a cell line, for example, an animal or plant cell line, or a microbial cell, such as a bacterial cell or fungal cell, or a primary cell isolated from a subject or a cultured plant cell.

42. The method according to claim 40, wherein the cell is selected from the group consisting of tumor cell, fibroblast, epithelial cell, endothelial cell, immune cell, skin cell and nerve cell; or selected from the group consisting of meristematic cell, stone cell, parenchyma cell, germ cell, root hair cell, duct cell, sieve cell, mesophyll cell and guard cell, epidermal cell and pigment cell.

43. The method according to claim 42, wherein the epithelial cell is selected from digestive tract epithelial cell and respiratory tract epithelial cell.

44. The method according to claim 40, wherein the cell is selected from the group consisting of A549 cell, HCT-116 cell, MKN-45 cell, 293T cell, HUVEC cell, ESF-1 cell, HeLa cell and MRC-5 cell.

45. The method according to any one of claims 40 to 44, wherein the polypeptide, conjugate or composition or fusion protein is incubated with the cell for 5 min to 24 h.

46. The method according to claim 45, wherein the polypeptide, conjugate or composition or fusion protein is incubated with the cell for 30 min to 6 h.

47. The method according to any one of claims 40 to 46, wherein the concentration of the polypeptide, conjugate or composition or fusion protein is 0.01 µM to 100 µM.

48. The method according to claim 47, wherein the concentration of the polypeptide, conjugate or composition or fusion protein is 0.03 µM to 30 µM.

49. A method of introducing a molecule into a cell, the method comprising a step of incubating the molecule and the polypeptide according to any one of claims 1 to 36, the conjugate or composition according to claim 37, or the fusion protein according to claim 38 or 39, with a cell.

50. The method according to claim 49, wherein the molecule is a therapeutic moiety, a detectable moiety or a cosmetic moiety, for example, selected from the group consisting of protein, nucleic acid, peptide, lipid, metabolite, drug and small molecule compound;
preferably, the therapeutic moiety is the therapeutic moiety as defined in claim 37;
preferably, the detectable moiety is the detectable moiety as defined in claim 37;
preferably, the cosmetic moiety is the cosmetic moiety as defined in claim 37;
preferably, the nucleic acid is a natural or artificial single-stranded or double-stranded DNA or RNA molecule, which comprises one or more selected from the group consisting of: small RNA, DNA, cDNA, decoy DNA, RNA, siRNA, miRNA, shRNA, stRNA, snoRNA, snRNA, PNA, antisense oligomer, plasmid, cosmid, phage, artificial chromosome and other modified nucleic acids.

51. The method according to claim 49, wherein the molecule is selected from the group consisting of antibiotic, anti-inflammatory drug, anti-tumor drug, neuroprotective agent, chemotherapeutic, cytotoxin, radioisotope, fluorescent marker, luminescent substance, chromogenic substance, antigen, cytokine, transcription regulation factor and enzyme.

52. The method according to claim 49, wherein the molecule is small RNA.

53. The method according to claim 52, wherein the small RNA is selected from siRNA and microRNA, for example small RNA antagomir; the siRNA is preferably PGY-dsRNA-6, PGY-sRNA-23 and PGY-sRNA-26.

54. The method according to any one of claims 49 to 53, wherein the cell is a cell line, for example, an animal or plant cell line, or a microbial cell, such as a bacterial cell or fungal cell, or a primary cell isolated from a subject or a cultured plant cell.

55. The method according to any one of claims 49 to 53, wherein the cell is selected from the group consisting of tumor cell, fibroblast, epithelial cell, endothelial cell, immune cell, skin cell and nerve cell; or selected from the group consisting of meristematic cell, stone cell, parenchyma cell, germ cell, root hair cell, duct cell, sieve cell, mesophyll cell and guard cell, epidermal cell and pigment cell.

56. The method according to claim 55, wherein the epithelial cell is selected from digestive tract epithelial cell and respiratory tract epithelial cell.

57. The method according to any one of claims 49 to 53, wherein the cell is selected from the group consisting of A549 cell, HCT-116 cell, MKN-45 cell, 293T cell, HUVEC cell, ESF-1 cell, HeLa cell and MRC-5 cell.

58. The polypeptide according to any one of claims 1 to 36, the conjugate or composition according to claim 37, or the fusion protein according to claim 38 or 39, for use in introducing a molecule into a cell.

59. The use according to claim 58, wherein the molecule is a therapeutic moiety, a detectable moiety or a cosmetic moiety, selected from the group consisting of protein, nucleic acid, peptide, lipid, metabolite, drug and small molecule compound;
preferably, the therapeutic moiety is the therapeutic moiety as defined in claim 37;
preferably, the detectable moiety is the detectable moiety as defined in claim 37;
preferably, the cosmetic moiety is the cosmetic moiety as defined in claim 37;
preferably, the nucleic acid is a natural or artificial DNA or RNA molecule, which is single-stranded or double-stranded, which comprises one or more selected from the group consisting of: small RNA, DNA, cDNA, decoy DNA, RNA, siRNA, miRNA, shRNA, stRNA, snoRNA, snRNA, PNA, antisense oligomer, plasmid, cosmid, phage, artificial chromosome and other modified nucleic acids.

60. The use according to claim 58, wherein the molecule is selected from the group consisting of antibiotic, anti-inflammatory drug, anti-tumor drug, neuroprotective agent, chemotherapeutic, cytotoxin, radioisotope, fluorescent marker, luminescent substance, chromogenic substance, antigen, cytokine, transcription regulation factor and enzyme.

61. The use according to claim 58, wherein the molecule is small RNA or plasmid.

62. The use according to claim 61, wherein the small RNA is selected from siRNA and microRNA, for example small RNA antagomir; the siRNA is preferably PGY-dsRNA-6, PGY-sRNA-23 and PGY-sRNA-26.

63. The use according to any one of claims 58 to 62, wherein the cell is a cell line, for example, an animal or plant cell line, or a microbial cell, such as a bacterial cell or fungal cell, or a primary cell isolated from a subject or a cultured plant cell.

64. The use according to any one of claims 58 to 62, wherein the cell is selected from the group consisting of tumor cell, fibroblast, epithelial cell, endothelial cell, immune cell, skin cell and nerve cell; or selected from the group consisting of meristematic cell, stone cell, parenchyma cell, germ cell, root hair cell, duct cell, sieve cell, mesophyll cell and guard cell, epidermal cell and pigment cell.

65. The use according to claim 64, wherein the epithelial cell is selected from digestive tract epithelial cell and respiratory tract epithelial cell.

66. The use according to any one of claims 58 to 62, wherein the cell is selected from the group consisting of A549 cell, HCT-116 cell, MKN-45 cell, 293T cell, HUVEC cell, ESF-1 cell, HeLa cell and MRC-5 cell.

67. A method for the treatment and diagnosis of diseases, for tracing (preferably intracellular tracing) or for cosmetic uses, the method comprising administration of the polypeptide according to any one of claims 1 to 36, the conjugate or composition according to claim 37, or the fusion protein according to claim 38 or 39, to a subject or a cell in need thereof.

68. The method according to claim 67, wherein the method comprises administration of the polypeptide according to any one of claims 1 to 36, the conjugate or composition according to claim 37, or the fusion protein according to claim 38 or 39, as well as a therapeutic moiety, a detectable moiety or a cosmetic moiety selected from the group consisting of antibiotic, anti-inflammatory drug, anti-tumor drug, neuroprotective agent, chemotherapeutic agent, cytotoxin, radioisotope, fluorescent marker, luminescent substance, chromogenic substance, antigen, cytokine, transcription regulation factor, enzyme and nucleic acid, such as plasmid and small RNA, to a subject or a cell in need thereof;
preferably, the therapeutic moiety is the therapeutic moiety as defined in claim 37;
preferably, the detectable moiety is the detectable moiety as defined in claim 37;
preferably, the cosmetic moiety is the cosmetic moiety as defined in claim 37;
wherein preferably, the nucleic acid is a natural or artificial single-stranded or double-stranded DNA or RNA molecule, which comprises one or more selected from the group consisting of: small RNA, DNA, cDNA, decoy DNA, RNA, siRNA, miRNA, shRNA, stRNA, snoRNA, snRNA, PNA, antisense oligomer, plasmid, cosmid, phage, artificial chromosome and other modified nucleic acids.

69. The method according to claim 68, wherein the small RNA is selected from siRNA and microRNA, for example small RNA antagomir; the siRNA is preferably PGY-dsRNA-6, PGY-sRNA-23 and PGY-sRNA-26.

70. The method according to any one of claims 67 to 69, wherein the disease is selected from cancer, viral infection, central nervous system disease, inflammatory disease, autoimmune disease, mitochondrial related disease, and metabolic disease; or the cosmetic use includes eliminating and delaying skin aging, whitening and anti-wrinkle;
preferably, the cancer is selected from the group consisting of acoustic neuroma; adenocarcinoma; adrenal carcinoma; rectal cancer; angiosarcoma such as lymphangiosarcoma, lymphatic endothelial sarcoma and angiosarcoma; cecal cancer; benign monoclonal gammopathy; bile cancer such as cholangiocarcinoma; bladder cancer; breast cancer such as adenocarcinoma of the breast, papillary carcinoma of the breast, breast cancer and medullary carcinoma of the breast; brain cancer such as meningioma, glioblastoma, glioma such as astrocytoma and oligodendroglioma, and medulloblastoma; bronchial carcinoma; carcinoid tumors; cervical cancer such as cervical adenocarcinoma; choriocarcinoma; chordoma; craniopharyngioma; colorectal cancer such as colon cancer, rectal cancer and colorectal adenocarcinoma; connective tissue cancer; epithelial cancer; ependymoma; endothelial sarcoma such as Kaposi's sarcoma and multiple idiopathic hemorrhagic sarcoma; endometrial cancer such as uterine cancer and uterine sarcoma; esophageal cancer such as adenocarcinoma of the esophagus and Barrett's adenocarcinoma; Ewing's sarcoma; eye cancer such as intraocular melanoma and retinoblastoma; familial eosinophilia; gallbladder cancer; gastric cancer such as gastric adenocarcinoma; gastrointestinal stromal tumor; germ cell cancer; head and neck cancer such as head and neck squamous cell carcinoma, oral cancer such as oral squamous cell carcinoma, laryngeal cancer such as laryngeal cancer, pharynx cancer, nasopharyngeal cancer and oropharyngeal cancer; hematopoietic stem cell cancer, for example, leukemia, such as acute lymphocytic leukemia (ALL) such as B-cell ALL and T-cell ALL, acute myeloid leukemia (AML) such as B-cell AML and T-cell AML, chronic myeloid leukemia (CML) such as B-cell CML and T-cell CML, and chronic lymphocytic leukemia (CLL) such as B-cell CLL and T-cell CLL; lymphomas, such as Hodgkin's lymphoma (HL) such as B-cell HL and T-cell HL, and non-Hodgkin's lymphoma (NHL) such as B-cell NHL, such as diffuse large cell lymphoma (DLCL) such as diffuse large B-cell lymphoma, follicular lymphoma, chronic lymphocytic leukemia/small lymphocytic lymphoma (CLL/SLL), mantle cell lymphoma (MCL), marginal zone B-cell lymphoma such as mucous associated lymphoid tissue (MALT) lymphoma, nodular marginal zone B-cell lymphoma and splenic marginal zone B-cell lymphoma, primary mediastinal B-cell lymphoma, Burkitt lymphoma, lymphoplasmacytic lymphoma, hairy cell leukemia (HCL), immunoblastic large cell lymphoma, precursor B-lymphoblastic lymphoma and primary central nervous system (CNS) lymphoma; and T-cell NHL, such as precursor T-lymphoblastic lymphoma/leukemia, peripheral T-cell lymphoma (PTCL) such as cutaneous T-cell lymphoma (CTCL) such as mycosis fungoides and Sezari syndrome, angioimmunoblastic T-cell lymphoma, extranodal natural killer T-cell lymphoma, enteropathic T-cell lymphoma, subcutaneous panniculitis-like T-cell lymphoma and anaplastic large cell lymphoma; a mixture of one or more of the above leukemias/lymphomas; and multiple myeloma (MM), heavy chain diseases such as α chain disease, γ chain disease and µ chain disease; hemangioblastoma; hypopharyngeal carcinoma; inflammatory myofibroblastoma; immune cell amyloidosis; renal cancer such as nephroblastoma; liver cancer such as hepatocellular carcinoma (HCC) and malignant liver cancer; lung cancer such as bronchial carcinoma, small cell lung cancer (SCLC), non-small cell lung cancer (NSCLC) and lung adenocarcinoma; leiomyosarcoma (LMS); mastocytosis, for example, systemic mastocytosis; muscle cancer; myelodysplastic syndrome (MDS); mesothelioma; myelodysplastic disease (MPD) such as polycythemia vera (PV), idiopathic thrombocytosis (ET), unexplained myeloid metaplasia (AMM), also known as myelofibrosis (MF), chronic idiopathic myelofibrosis, chronic myeloid leukemia (CML), chronic neutrophilic leukemia (CNL) and hypereosinophilic syndrome (HES); neuroblastoma; neurofibromatosis such as neurofibromatosis (NF) type 1 or 2 and schwannoma; neuroendocrine cancer such as gastrointestinal pancreatic neuroendocrine tumor (GEP-NET) and carcinoid tumor; osteosarcoma such as bone cancer; ovarian cancer such as cystadenocarcinoma, ovarian embryonic carcinoma and ovarian gland carcinoma; papillary adenocarcinoma; pancreatic cancer such as pancreatic cancer, intraductal papillary mucin-like neoplasm (IPMN) and islet cell tumor; penile cancer such as Paget's disease of the penis and scrotum; pinealoma; primary neuroectodermal tumor (PNT); plasmacytoma; tumor-like syndrome; intraepithelial neoplasia; prostate cancer such as adenocarcinoma of the prostate; rectal cancer; rhabdomyosarcoma; salivary gland cancer; skin cancer such as squamous cell carcinoma (SCC), keratoacanthoma (KA), melanoma and basal cell carcinoma (BCC); small intestine cancer such as cecal cancer; soft tissue sarcoma such as malignant fibrous histiocytoma (MFH), liposarcoma, malignant peripheral nerve sheath tumor (MPNST), chondrosarcoma, fibrosarcoma and myxosarcoma; sebaceous carcinoma; small bowel carcinoma; sweat gland carcinoma; synovial tumor; testicular carcinoma such as seminoma and testicular embryonic cancer; thyroid cancer such as papillary thyroid cancer, papillary thyroid cancer (PTC) and medullary thyroid cancer; urethral cancer; vaginal cancer; and vulvar cancer such as Paget's disease of the vulva;
preferably, the viral infection is selected from poxviridae viral disease, herpesviridae viral disease, adenoviridae viral disease, papillomaviridae and polyomaviridae viral disease, parvoviridae viral disease, hepatotropic DNA viral disease, retroviral viral disease, reoviridae viral disease, bornaviridae viral disease, rhabdoviridae viral disease, filoviridae viral disease, Paramyxoviridae viral disease, orthomyxoviridae viral disease, bunyaviridae viral disease, arenaviridae viral disease, picornaviridae viral disease, caliciviridae viral disease, astroviridae viral disease, coronaviridae viral disease, togaviridae viral disease, flaviviridae viral disease, unspecified viral disease and lentiviral infections; for example, viral infection is infection caused by the following viruses: hepatitis A, B, C virus, influenza virus, varicella virus, herpes simplex virus type I (HSV-I), herpes simplex virus type II (HSV-II), rinderpest virus, respiratory syncytial virus, cytomegalovirus, sea urchin virus, arbovirus, hantavirus, mumps virus, measles virus, rubella virus, human immunodeficiency virus type I (HIV-1), human immunity Defective virus type II (HIV-2), any Togaviridae virus such as dengue virus, alphavirus, flavivirus, coronavirus, rabies virus, green monkey virus, ebola virus, parainfluenza virus, orthomyxovirus, arenavirus, human T-cell leukemia virus type I, human T-cell leukemia virus type II, simian immunodeficiency virus, lentivirus, epstein-barr virus, human herpes virus, cercopithecine herpes virus 1 (B virus) and pox virus;
Preferably, the central nervous system disease is selected from the group consisting of neurodegenerative disease, stroke, epilepsy, traumatic brain injury, shock, HIV dementia, glaucoma, multiple sclerosis, and the like. Stroke is divided into hemorrhagic and ischemic stroke; neurodegenerative disease includes Alzheimer's disease, cerebellar atrophy, multiple sclerosis, primary lateral sclerosis, spinal muscular atrophy, Parkinson's disease, Huntington's disease, Creutzfeldt-Jakob disease, bovine spongiform encephalopathy, ataxia telangiectasia and amyotrophic lateral sclerosis;
preferably, the inflammatory disease is selected from the group consisting of atherosclerosis, arteriosclerosis, autoimmune disorder, multiple sclerosis, systemic lupus erythematosus, polymyalgia rheumatica (PMR), gouty arthritis, degenerative arthritis, tendinitis, bursitis, psoriasis, cystic fibrosis, arthrosteitis, rheumatoid arthritis, inflammatory arthritis, Sjogren's syndrome, giant cell arteritis, progressive systemic sclerosis, ankylosing spondylitis, polymyositis, dermatomyositis, pemphigus, pemphigoid, diabetes such as type I, myasthenia gravis, Hashimoto's thyroiditis, Graves' disease, Goodpasture's disease, mixed connective tissue disease, sclerosing cholangitis, inflammatory bowel disease, Crohn's disease, ulcerative colitis, pernicious anemia, inflammatory skin disease, usual interstitial pneumonia (UIP), asbestosis, silicosis, bronchiectasis, beryllium poisoning, talc disease, pneumoconiosis, sarcomatoid disease, desquamative interstitial pneumonia, lymphoid interstitial pneumonia, giant cell interstitial pneumonia, cellular interstitial pneumonia, exogenous allergies alveolitis, Wegener's granulomatosis and related forms of vasculitis such as temporal arteritis and polyarteritis nodosa, inflammatory skin disease, hepatitis, delayed hypersensitivity reaction such as poison ivy dermatitis, pneumonia, respiratory tract inflammation, adult respiratory distress syndrome (ARDS), encephalitis, immediate hypersensitivity, asthma, hay fever, allergy, acute allergic reaction, rheumatic fever, glomerulonephritis, pyelonephritis, cellulitis, cystitis, chronic cholecystitis, ischemia, regional ischemic injury, reperfusion injury, allograft rejection, host versus graft, appendicitis, arteritis, blepharitis, bronchiolitis, bronchitis, cervicitis, cholangitis, chorioamnionitis, conjunctivitis, lacrimal gland inflammation, dermatomyositis, endocarditis, endometritis, enteritis, enterocolitis, epicondylitis, epididymitis, fasciitis, fibrositis, gastritis, gastroenteritis, gingivitis, ileitis, iritis, laryngitis, myelitis, myocarditis, nephritis, omphalitis, oophoritis, orchitis, ostitis, otitis, pancreatitis, mumps, pericarditis, pharyngitis, pleurisy, phlebitis, pneumonia, proctitis, prostatitis, rhinitis, salpingitis, sinusitis, stomatitis, synovitis, orchitis, tonsillitis, urethritis, cystitis, uveitis, vaginitis, vascular inflammation, vulvitis, vulvovaginitis, vasculitis, chronic bronchitis, osteomyelitis, optic neuritis, temporal arteritis, transverse myelitis, necrotizing fasciitis and necrotizing enterocolitis;
preferably, the autoimmune disease is selected from the group consisting of glomerulonephritis, Goodpasture's syndrome, necrotizing vasculitis, lymphadenitis, nodular periarteritis, systemic lupus erythematosus, rheumatoid disease, arthritis, psoriatic arthritis, systemic lupus erythematosus, psoriasis, ulcerative colitis, systemic sclerosis, dermatomyositis/polymyositis, antiphospholipid antibody syndrome, scleroderma, pemphigus vulgaris, ANCA-related vasculitis such as Wegener's granulomatosis and polyangiitis under the microscope, uveitis, Shouglen's syndrome, Crohn's disease, Wright's syndrome, ankylosing spondylitis, Lyme arthritis, Gulan-Barre syndrome, Hashimoto's thyroiditis and cardiomyopathy;
preferably, the metabolic disease is selected from the group consisting of phenylketonuria, xanthoma, amyloidosis, xanthomas, xanthomas, lipomatous fibroma, lipofibroma, lipofibroma, xanthomatosis, hyperlipidemia, hyperlipemia, hyperlipemia, lipomatosis, hyperliposis, amyloidosis, amyloidosis, galactosemia, obesity, hyperlipidemia, osteomalacia, ricket, osteomalacia and ricket, osteoporosis and diabetes;
preferably, the mitochondrial related disease is selected from the group consisting of Huntington's disease, amyotrophic lateral sclerosis, mitochondrial myopathy, encephalopathy, lactic acidosis and stroke-like episodes (MELAS); myoclonus epilepsy associated with ragged-red fibers (MERRF); neuromuscular relaxation, disorder; neuropathy, ataxia, and retinitis pigmentosa/maternally inherited Leigh syndrome (NARP/MILS); Leber's hereditary optic neuropathy (LHON); Kearns-Sayre syndrome (KSS); Pearson marrow-pancreas syndrome (PMPS); chronic progressive external ophthalmoplegia (CPEO); Wright's syndrome; Alpers syndrome; multiple mitochondrial DNA deficiency syndrome; mitochondrial DNA deficiency syndrome; complex I defect; complex II (succinate dehydrogenase (SDH)) defect; complex III defect; cytochrome c oxidase (COX, complex IV) defect; complex V defect; adenine nucleotide transporter (ANT) defect; pyruvate dehydrogenase (PDH) defect; ethylmalonate aciduria with lactic acidemia; 3-methylglutaconate aciduria with lactic acidemia; refractoriness epilepsy attenuated during infection; Asperger's syndrome attenuated during infection; autism attenuated during infection; attention deficit hyperactivity disorder (ADHD); cerebral palsy attenuated during infection; dyslexia attenuated during infection; maternally inherited thrombocytopenia; leukemia; MNGIE, mitochondrial myopathy, peripheral and autonomic neuropathy, gastrointestinal dysfunction and epilepsy; MARIAHS syndrome (mitochondrial ataxia, recurrent infections, aphasia, hypouricemia/hypomyelination, seizures, and dicarboxylic aciduria); ND6 dystonia; periodic vomiting attenuated during infection; 3-hydroxyisobutyric acid urine with lactic acidemia; diabetes insipidus with lactic acidemia; uridine-responsive neurological symptoms (URNS); family bilateral striatal necrosis (FBSN); aminoglycosides related hearing loss; relaxation cardiomyopathy; splenic lymphoma; tungsten symptoms; multiple mitochondrial DNA deletion symptoms; and renal tubular acidemia/diabetes insipidus/disorder symptoms.

71. The polypeptide according to any one of claims 1 to 36, the conjugate or composition according to claim 37, or the fusion protein according to claim 38 or 39, for use in the treatment and diagnosis of diseases, tracing (preferably intracellular tracing), or cosmetology.

72. The use according to claim 71, wherein the disease is selected from cancer, viral infection, central nervous system disease, inflammatory disease, autoimmune disease, mitochondrial related disease, and metabolic disease,
preferably, the disease is the disease according to claim 70;
preferably, the cosmetology includes eliminating and delaying skin aging, whitening and anti-wrinkle.

73. Use of the polypeptide according to any one of claims 1 to 36, the conjugate or composition according to claim 37, or the fusion protein according to claim 38 or 39, in the preparation of a pharmaceutical composition for the treatment and diagnosis of diseases, tracing (preferably intracellular tracing), or cosmetology.

74. The use according to claim 73, wherein the pharmaceutical composition also comprises a therapeutic moiety, a detectable moiety or a cosmetic moiety, which is selected from the group consisting of antibiotic, anti-inflammatory drug, anti-tumor drug, neuroprotective agent, chemotherapeutic, cytotoxin, radioisotope, fluorescent marker, luminescent substance, chromogenic substance, antigen, cytokine, transcription regulation factor, enzyme and nucleic acid.
preferably, the therapeutic moiety is the therapeutic moiety as defined in claim 37;
preferably, the detectable moiety is the detectable moiety as defined in claim 37;
preferably, the cosmetic moiety is the cosmetic moiety as defined in claim 37;
wherein preferably, the nucleic acid is a natural or artificial DNA or RNA molecule, which is single-stranded or double-stranded, which comprises one or more selected from the group consisting of: small RNA, DNA, cDNA, decoy DNA, RNA, siRNA, miRNA, shRNA, stRNA, snoRNA, snRNA, PNA, antisense oligomer, plasmid, cosmid, phage, artificial chromosome and other modified nucleic acids.

75. The use according to claim 74, wherein the small RNA is selected from siRNA and microRNA, for example small RNA antagomir; the siRNA is preferably PGY-dsRNA-6, PGY-sRNA-23 and PGY-sRNA-26.

76. The use according to any one of claims 73 to 75, wherein the disease is selected from cancer, viral infection, central nervous system disease, inflammatory disease, autoimmune disease, mitochondrial related disease and metabolic disease,
preferably, the disease is the disease according to claim 70;
preferably, the cosmetology includes eliminating and delaying skin aging, whitening and anti-wrinkle.

77. A nucleic acid molecule, the nucleic acid molecule comprising the nucleotide sequence encoding the polypeptide according to any one of claims 1 to 36, or the fusion protein according to claim 38 or 39.

78. A vector, the vector comprising the nucleic acid molecule according to claim 77.

79. A composition, the composition comprising the polypeptide according to any one of claims 1 to 36, the conjugate or composition according to claim 37, or the fusion protein according to claim 38 or 39.

80. The composition according to claim 79, wherein the composition also comprises a therapeutic moiety, a detectable moiety or a cosmetic moiety, which is selected from the group consisting of antibiotic, anti-inflammatory drug, anti-tumor drug, neuroprotective agent, chemotherapeutic, cytotoxin, radioisotope, fluorescent marker, luminescent substance, chromogenic substance, antigen, cytokine, transcription regulation factor, enzyme and nucleic acid;
preferably, the therapeutic moiety is the therapeutic moiety as defined in claim 37;
preferably, the detectable moiety is the detectable moiety as defined in claim 37;
preferably, the cosmetic moiety is the cosmetic moiety as defined in claim 37;
preferably, the nucleic acid is a natural or artificial DNA or RNA molecule, which is single-stranded or double-stranded, which comprises one or more selected from the group consisting of: small RNA, DNA, cDNA, decoy DNA, RNA, siRNA, miRNA, shRNA, stRNA, snoRNA, snRNA, PNA, antisense oligomer, plasmid, cosmid, phage, artificial chromosome and other modified nucleic acids.

81. The composition according to claim 80, wherein the small RNA is selected from siRNA and microRNA, for example small RNA antagomir; the siRNA is preferably PGY-dsRNA-6, PGY-sRNA-23 and PGY-sRNA-26.

82. The composition according to any one of claims 79 to 81, wherein the composition is a pharmaceutical composition and further comprises one or more pharmaceutically acceptable carrier(s).

83. A host cell, which comprises the vector according to claim 78.

84. A method for production the polypeptide according to any one of claims 1 to 36, which comprises culturing the host cell according to claim 83 in a suitable medium, and collecting the polypeptide according to any one of claims 1 to 36 .

85. The polypeptide according to any one of claims 1 to 36, the conjugate or composition according to claim 37, or the fusion protein according to claim 38 or 39, the nucleic acid according to claim 77, the vector according to claim 78, or the composition according to any one of claims 79 to 82, for use in the treatment and diagnosis of diseases, tracing, preferably intracellular tracing, or cosmetology.

86. The polypeptide, conjugate or composition, fusion protein, nucleic acid, vector or composition according to claim 85, wherein the disease is selected from cancer, viral infection, central nervous system disease, inflammatory disease, autoimmune disease, mitochondrial related disease and metabolic disease,
preferably, the disease is the disease as defined in claim 70;
preferably, the cosmetology includes eliminating and delaying skin aging, whitening and anti-wrinkle.


SEQUENCE LISTING

<110>  INSTITUTE OF BASIC MEDICAL SCIENCES CHINESE ACADEMY OF MEDICAL
SCIENCES

<120>  CELL-PENETRATING PEPTIDE BASED ON INFLUENZA VIRUS M2 PROTEIN

<130>  C19W5340

<150>  PCT/CN2018/113755
<151>  2018-11-02

<160>  126

<170>  PatentIn version 3.5

<210>  1
<211>  15
<212>  PRT
<213>  Artificial sequence

<220>
<223>  NC negative control

<400>  1

Glu Arg Gly Leu Gln Arg Arg Arg Phe Val Gln Asn Ala Leu Asn
1               5                   10                  15


<210>  2
<211>  13
<212>  PRT
<213>  Human immunodeficiency virus

<400>  2

Gly Arg Lys Lys Arg Arg Gln Arg Arg Arg Pro Pro Gln
1               5                   10


<210>  3
<211>  54
<212>  PRT
<213>  Influenza virus

<400>  3

Asp Arg Leu Phe Ser Lys Ser Ile Tyr Arg Ile Phe Lys His Gly Leu
1               5                   10                  15


Lys Arg Gly Pro Ser Thr Glu Gly Val Pro Glu Ser Met Arg Glu Glu
            20                  25                  30


Tyr Arg Glu Glu Gln Gln Asn Ala Val Asp Ala Asp Asp Gly His Phe
        35                  40                  45

Val Ser Ile Glu Leu Lys
    50

<210>  4
<211>  18
<212>  PRT
<213>  Influenza virus

<400>  4

Arg Leu Phe Ser Lys Ser Ile Tyr Arg Ile Phe Lys His Gly Leu Lys
1               5                   10                  15

Arg Gly

<210>  5
<211>  23
<212>  PRT
<213>  Influenza virus

<400>  5

Lys Arg Gly Pro Ser Thr Glu Gly Val Pro Glu Ser Met Arg Glu Glu
1               5                   10                  15

Tyr Arg Glu Glu Gln Gln Asn
            20

<210>  6
<211>  54
<212>  PRT
<213>  Influenza A virus

<400>  6

Asp Arg Leu Phe Phe Lys Cys Ile Tyr Arg Arg Leu Lys Tyr Gly Leu
1               5                   10                  15

Lys Arg Gly Pro Ser Thr Ala Gly Val Pro Glu Ser Met Arg Glu Glu
            20                  25                  30

Tyr Arg Gln Glu Gln Gln Asn Ala Val Asp Val Asp Asp Gly His Phe
        35                  40                  45

Val Asn Ile Glu Leu Glu
    50

<210>  7
<211>  18
<212>  PRT
<213>  Influenza A virus

<400>  7

Arg Leu Phe Phe Lys Cys Ile Tyr Arg Arg Leu Lys Tyr Gly Leu Lys
1               5                   10                  15

Arg Gly

<210>  8
<211>  23
<212>  PRT
<213>  Influenza A virus

<400>  8

Lys Arg Gly Pro Ser Thr Ala Gly Val Pro Glu Ser Met Arg Glu Glu
1               5                   10                  15

Tyr Arg Gln Glu Gln Gln Asn
            20

<210>  9
<211>  23
<212>  PRT
<213>  Influenza A virus

<400>  9

Lys Arg Gly Pro Ser Thr Glu Gly Val Pro Glu Ser Met Arg Glu Glu
1               5                   10                  15

Tyr Arg Gln Glu Gln Gln Ser
            20

<210>  10
<211>  24
<212>  PRT
<213>  Influenza A virus

<400>  10

Met Ser Leu Leu Thr Glu Val Glu Thr Pro Thr Arg Asn Gly Trp Glu
1               5                   10                  15

Cys Arg Cys Ser Asp Ser Ser Asp

            20

<210>  11
<211>  19
<212>  PRT
<213>  Influenza A virus

<400>  11

Pro Leu Val Val Ala Ala Ser Ile Ile Gly Ile Leu His Leu Ile Leu
1               5                   10                  15

Trp Ile Leu

<210>  12
<211>  18
<212>  PRT
<213>  Influenza A virus

<400>  12

Asp Arg Leu Phe Phe Lys Cys Ile Tyr Arg Arg Leu Lys Tyr Gly Leu
1               5                   10                  15

Lys Arg

<210>  13
<211>  18
<212>  PRT
<213>  Influenza A virus

<400>  13

Phe Phe Lys Cys Ile Tyr Arg Arg Leu Lys Tyr Gly Leu Lys Arg Gly
1               5                   10                  15

Pro Ser

<210>  14
<211>  18
<212>  PRT
<213>  Influenza A virus

<400>  14

Cys Ile Tyr Arg Arg Leu Lys Tyr Gly Leu Lys Arg Gly Pro Ser Thr
1               5                   10                  15

Glu Gly

<210> 15
<211> 18
<212> PRT
<213> Influenza A virus

<400> 15

Arg Arg Leu Lys Tyr Gly Leu Lys Arg Gly Pro Ser Thr Glu Gly Val
1               5                   10                  15

Pro Glu

<210> 16
<211> 18
<212> PRT
<213> Influenza A virus

<400> 16

Lys Tyr Gly Leu Lys Arg Gly Pro Ser Thr Glu Gly Val Pro Glu Ser
1               5                   10                  15

Met Arg

<210> 17
<211> 18
<212> PRT
<213> Influenza A virus

<400> 17

Leu Lys Arg Gly Pro Ser Thr Glu Gly Val Pro Glu Ser Met Arg Glu
1               5                   10                  15

Glu Tyr

<210> 18
<211> 18
<212> PRT
<213> Influenza A virus

<400> 18

Gly Pro Ser Thr Glu Gly Val Pro Glu Ser Met Arg Glu Glu Tyr Arg

1               5                   10                  15

Gln Glu

<210>  19
<211>  18
<212>  PRT
<213>  Influenza A virus

<400>  19

Thr Glu Gly Val Pro Glu Ser Met Arg Glu Glu Tyr Arg Gln Glu Gln
1               5                   10                  15

Gln Ser

<210>  20
<211>  20
<212>  PRT
<213>  Influenza A virus

<400>  20

Phe Lys Cys Ile Tyr Arg Arg Leu Lys Tyr Gly Leu Lys Arg Gly Pro
1               5                   10                  15

Ser Thr Glu Gly
            20

<210>  21
<211>  21
<212>  PRT
<213>  Influenza A virus

<400>  21

Phe Phe Lys Cys Ile Tyr Arg Arg Leu Lys Tyr Gly Leu Lys Arg Gly
1               5                   10                  15

Pro Ser Thr Glu Gly
            20

<210>  22
<211>  22
<212>  PRT
<213>  Influenza A virus

<400>  22

Leu Phe Phe Lys Cys Ile Tyr Arg Arg Leu Lys Tyr Gly Leu Lys Arg
1               5                   10                  15

Gly Pro Ser Thr Glu Gly
            20

<210>  23
<211>  23
<212>  PRT
<213>  Influenza A virus

<400>  23

Arg Leu Phe Phe Lys Cys Ile Tyr Arg Arg Leu Lys Tyr Gly Leu Lys
1               5                   10                  15

Arg Gly Pro Ser Thr Glu Gly
            20

<210>  24
<211>  24
<212>  PRT
<213>  Influenza A virus

<400>  24

Asp Arg Leu Phe Phe Lys Cys Ile Tyr Arg Arg Leu Lys Tyr Gly Leu
1               5                   10                  15

Lys Arg Gly Pro Ser Thr Glu Gly
            20

<210>  25
<211>  23
<212>  PRT
<213>  Influenza A virus

<400>  25

Asp Arg Leu Phe Phe Lys Cys Ile Tyr Arg Arg Leu Lys Tyr Gly Leu
1               5                   10                  15

Lys Arg Gly Pro Ser Thr Glu
            20

<210>  26
<211>  22
<212>  PRT
<213>  Influenza A virus

<400>  26

Asp Arg Leu Phe Phe Lys Cys Ile Tyr Arg Arg Leu Lys Tyr Gly Leu
1               5                   10                  15

Lys Arg Gly Pro Ser Thr
            20

<210>  27
<211>  21
<212>  PRT
<213>  Influenza A virus

<400>  27

Asp Arg Leu Phe Phe Lys Cys Ile Tyr Arg Arg Leu Lys Tyr Gly Leu
1               5                   10                  15

Lys Arg Gly Pro Ser
            20

<210>  28
<211>  20
<212>  PRT
<213>  Influenza A virus

<400>  28

Asp Arg Leu Phe Phe Lys Cys Ile Tyr Arg Arg Leu Lys Tyr Gly Leu
1               5                   10                  15

Lys Arg Gly Pro
            20

<210>  29
<211>  19
<212>  PRT
<213>  Influenza A virus

<400>  29

Asp Arg Leu Phe Phe Lys Cys Ile Tyr Arg Arg Leu Lys Tyr Gly Leu
1               5                   10                  15

Lys Arg Gly

<210>  30
<211>  18

<212>  PRT
<213>  Influenza A virus

<400>  30

Asp Arg Leu Phe Phe Lys Cys Ile Tyr Arg Arg Leu Lys Tyr Gly Leu
1               5                   10                  15

Lys Arg

<210>  31
<211>  17
<212>  PRT
<213>  Influenza A virus

<400>  31

Asp Arg Leu Phe Phe Lys Cys Ile Tyr Arg Arg Leu Lys Tyr Gly Leu
1               5                   10                  15

Lys

<210>  32
<211>  16
<212>  PRT
<213>  Influenza A virus

<400>  32

Asp Arg Leu Phe Phe Lys Cys Ile Tyr Arg Arg Leu Lys Tyr Gly Leu
1               5                   10                  15

<210>  33
<211>  15
<212>  PRT
<213>  Influenza A virus

<400>  33

Asp Arg Leu Phe Phe Lys Cys Ile Tyr Arg Arg Leu Lys Tyr Gly
1               5                   10                  15

<210>  34
<211>  14
<212>  PRT
<213>  Influenza A virus

<400>  34

Asp Arg Leu Phe Phe Lys Cys Ile Tyr Arg Arg Leu Lys Tyr

1               5                   10


<210>  35
<211>  13
<212>  PRT
<213>  Influenza A virus

<400>  35

Asp Arg Leu Phe Phe Lys Cys Ile Tyr Arg Arg Leu Lys
1               5                   10


<210>  36
<211>  12
<212>  PRT
<213>  Influenza A virus

<400>  36

Asp Arg Leu Phe Phe Lys Cys Ile Tyr Arg Arg Leu
1               5                   10


<210>  37
<211>  11
<212>  PRT
<213>  Influenza A virus

<400>  37

Asp Arg Leu Phe Phe Lys Cys Ile Tyr Arg Arg
1               5                   10


<210>  38
<211>  10
<212>  PRT
<213>  Influenza A virus

<400>  38

Asp Arg Leu Phe Phe Lys Cys Ile Tyr Arg
1               5                   10


<210>  39
<211>  13
<212>  PRT
<213>  Artificial sequence

<220>
<223>  JL-M2-44-56-mut1

<400>  39

Arg Arg Leu Phe Phe Lys Cys Ile Tyr Arg Arg Leu Lys

1                   5                        10


<210>  40
<211>  13
<212>  PRT
<213>  Artificial sequence

<220>
<223>  JL-M2-44-56-mut2

<400>  40

Arg Arg Leu Phe Phe Arg Cys Ile Tyr Arg Arg Leu Lys
1                   5                        10


<210>  41
<211>  13
<212>  PRT
<213>  Artificial sequence

<220>
<223>  JL-M2-44-56-mut3

<400>  41

Arg Arg Leu Phe Phe Arg Arg Ile Tyr Arg Arg Leu Lys
1                   5                        10


<210>  42
<211>  13
<212>  PRT
<213>  Artificial sequence

<220>
<223>  JL-M2-44-56-mut4

<400>  42

Arg Arg Leu Phe Phe Arg Arg Ile Tyr Arg Arg Leu Arg
1                   5                        10


<210>  43
<211>  13
<212>  PRT
<213>  Artificial sequence

<220>
<223>  JL-M2-44-56-mut5

<400>  43

Asp Arg Ile Phe Phe Lys Cys Ile Tyr Arg Arg Leu Lys
1                   5                        10

<210>  44
<211>  13
<212>  PRT
<213>  Artificial sequence

<220>
<223>  JL-M2-44-56-mut6

<400>  44

Asp Arg Ile Phe Phe Lys Cys Ile Ile Arg Arg Leu Lys
1               5                   10


<210>  45
<211>  13
<212>  PRT
<213>  Artificial sequence

<220>
<223>  JL-M2-44-56-mut7

<400>  45

Asp Arg Ile Phe Phe Lys Cys Ile Ile Arg Arg Ile Lys
1               5                   10


<210>  46
<211>  13
<212>  PRT
<213>  Artificial sequence

<220>
<223>  JL-M2-44-56-mut8

<400>  46

Asp Arg Phe Phe Phe Lys Cys Ile Tyr Arg Arg Leu Lys
1               5                   10


<210>  47
<211>  13
<212>  PRT
<213>  Artificial sequence

<220>
<223>  JL-M2-44-56-mut9

<400>  47

Asp Arg Phe Phe Phe Lys Cys Phe Tyr Arg Arg Leu Lys
1               5                   10


<210>  48

<211>  13
<212>  PRT
<213>  Artificial sequence

<220>
<223>  JL-M2-44-56-mut10

<400>  48

Asp Arg Phe Phe Phe Lys Cys Phe Phe Arg Arg Leu Lys
1               5                   10


<210>  49
<211>  13
<212>  PRT
<213>  Artificial sequence

<220>
<223>  JL-M2-44-56-mut11

<400>  49

Asp Arg Phe Phe Phe Lys Cys Phe Phe Arg Arg Phe Lys
1               5                   10


<210>  50
<211>  13
<212>  PRT
<213>  Artificial sequence

<220>
<223>  JL-M2-44-56-mut12

<400>  50

Arg Arg Phe Phe Phe Arg Arg Phe Phe Arg Arg Phe Arg
1               5                   10


<210>  51
<211>  18
<212>  PRT
<213>  Artificial sequence

<220>
<223>  JL-2004(H5N1)-M2-45-62 mut1

<400>  51

Arg Leu Phe Phe Arg Cys Ile Tyr Arg Arg Leu Lys Tyr Gly Leu Lys
1               5                   10                  15


Arg Gly

<210>  52
<211>  18
<212>  PRT
<213>  Artificial sequence

<220>
<223>  JL-2004(H5N1)-M2-45-62 mut2

<400>  52

Arg Leu Phe Phe Arg Arg Ile Tyr Arg Arg Leu Lys Tyr Gly Leu Lys
1               5                   10                  15

Arg Gly

<210>  53
<211>  18
<212>  PRT
<213>  Artificial sequence

<220>
<223>  JL-2004(H5N1)-M2-45-62 mut3

<400>  53

Arg Leu Phe Phe Arg Arg Ile Tyr Arg Arg Leu Arg Tyr Gly Leu Lys
1               5                   10                  15

Arg Gly

<210>  54
<211>  18
<212>  PRT
<213>  Artificial sequence

<220>
<223>  JL-2004(H5N1)-M2-45-62 mut4

<400>  54

Arg Leu Phe Phe Arg Arg Ile Tyr Arg Arg Leu Arg Tyr Arg Leu Lys
1               5                   10                  15

Arg Gly

<210>  55
<211>  18
<212>  PRT

<213>  Artificial sequence

<220>
<223>  JL-2004(H5N1)-M2-45-62 mut5

<400>  55

Arg Leu Phe Phe Arg Arg Ile Tyr Arg Arg Leu Arg Tyr Arg Leu Arg
1               5                   10                  15

Arg Gly

<210>  56
<211>  18
<212>  PRT
<213>  Artificial sequence

<220>
<223>  JL-2004(H5N1)-M2-45-62 mut6

<400>  56

Arg Leu Phe Phe Arg Arg Ile Tyr Arg Arg Leu Arg Tyr Arg Leu Arg
1               5                   10                  15

Arg Arg

<210>  57
<211>  18
<212>  PRT
<213>  Artificial sequence

<220>
<223>  JL-2004(H5N1)-M2-45-62 mut7

<400>  57

Arg Phe Phe Phe Lys Cys Ile Tyr Arg Arg Leu Lys Tyr Gly Leu Lys
1               5                   10                  15

Arg Gly

<210>  58
<211>  18
<212>  PRT
<213>  Artificial sequence

<220>
<223>  JL-2004(H5N1)-M2-45-62 mut8

<400>  58

Arg Phe Phe Phe Lys Cys Phe Tyr Arg Arg Leu Lys Tyr Gly Leu Lys
1               5                   10                  15

Arg Gly

<210>  59
<211>  18
<212>  PRT
<213>  Artificial sequence

<220>
<223>  JL-2004(H5N1)-M2-45-62 mut9

<400>  59

Arg Phe Phe Phe Lys Cys Phe Phe Arg Arg Leu Lys Tyr Gly Leu Lys
1               5                   10                  15

Arg Gly

<210>  60
<211>  18
<212>  PRT
<213>  Artificial sequence

<220>
<223>  JL-2004(H5N1)-M2-45-62 mut10

<400>  60

Arg Phe Phe Phe Lys Cys Phe Phe Arg Arg Phe Lys Tyr Gly Leu Lys
1               5                   10                  15

Arg Gly

<210>  61
<211>  18
<212>  PRT
<213>  Artificial sequence

<220>
<223>  JL-2004(H5N1)-M2-45-62 mut11

<400>  61

Arg Phe Phe Phe Lys Cys Phe Phe Arg Arg Phe Lys Phe Gly Leu Lys

1                   5                   10                  15

Arg Gly

<210>  62
<211>  18
<212>  PRT
<213>  Artificial sequence

<220>
<223>  JL-2004(H5N1)-M2-45-62 mut12

<400>  62

Arg Phe Phe Phe Lys Cys Phe Phe Arg Arg Phe Lys Phe Gly Phe Lys
1                   5                   10                  15

Arg Gly

<210>  63
<211>  18
<212>  PRT
<213>  Artificial sequence

<220>
<223>  JL-2004(H5N1)-M2-45-62 mut13

<400>  63

Arg Phe Phe Phe Arg Arg Phe Phe Arg Arg Phe Arg Phe Arg Phe Arg
1                   5                   10                  15

Arg Arg

<210>  64
<211>  18
<212>  PRT
<213>  Artificial sequence

<220>
<223>  JL-2004(H5N1)-M2-45-62 mut45

<400>  64

Ser Leu Phe Phe Lys Cys Ile Tyr Arg Arg Leu Lys Tyr Gly Leu Lys
1                   5                   10                  15

Arg Gly

<210>  65
<211>  18
<212>  PRT
<213>  Artificial sequence

<220>
<223>  JL-2004(H5N1)-M2-45-62 mut46

<400>  65

Arg Ala Phe Phe Lys Cys Ile Tyr Arg Arg Leu Lys Tyr Gly Leu Lys
1               5                   10                  15

Arg Gly

<210>  66
<211>  18
<212>  PRT
<213>  Artificial sequence

<220>
<223>  JL-2004(H5N1)-M2-45-62 mut47

<400>  66

Arg Leu Ile Phe Lys Cys Ile Tyr Arg Arg Leu Lys Tyr Gly Leu Lys
1               5                   10                  15

Arg Gly

<210>  67
<211>  18
<212>  PRT
<213>  Artificial sequence

<220>
<223>  JL-2004(H5N1)-M2-45-62 mut48

<400>  67

Arg Leu Phe Ile Lys Cys Ile Tyr Arg Arg Leu Lys Tyr Gly Leu Lys
1               5                   10                  15

Arg Gly

<210>  68

<211>  22
<212>  PRT
<213>  Artificial sequence

<220>
<223>  JL-2004(H5N1)-M2-45-62 mut49

<400>  68

Asp Arg Leu Phe Phe Lys Cys Ile Tyr Arg Arg Leu Lys Tyr Gly Leu
1               5                   10                  15

Lys Arg Gly Pro Lys Val
            20

<210>  69
<211>  18
<212>  PRT
<213>  Artificial sequence

<220>
<223>  JL-2004(H5N1)-M2-45-62 mut50

<400>  69

Arg Leu Phe Phe Lys Ile Ile Tyr Arg Arg Leu Lys Tyr Gly Leu Lys
1               5                   10                  15

Arg Gly

<210>  70
<211>  18
<212>  PRT
<213>  Artificial sequence

<220>
<223>  JL-2004(H5N1)-M2-45-62 mut51

<400>  70

Arg Leu Phe Phe Lys Cys Ala Tyr Arg Arg Leu Lys Tyr Gly Leu Lys
1               5                   10                  15

Arg Gly

<210>  71
<211>  18
<212>  PRT
<213>  Artificial sequence

<220>
<223>  JL-2004(H5N1)-M2-45-62 mut52

<400>  71

Arg Leu Phe Phe Lys Cys Ile Lys Arg Arg Leu Lys Tyr Gly Leu Lys
1               5                   10                  15

Arg Gly

<210>  72
<211>  18
<212>  PRT
<213>  Artificial sequence

<220>
<223>  JL-2004(H5N1)-M2-45-62 mut53

<400>  72

Arg Leu Phe Phe Lys Cys Ile Tyr Ser Arg Leu Lys Tyr Gly Leu Lys
1               5                   10                  15

Arg Gly

<210>  73
<211>  18
<212>  PRT
<213>  Artificial sequence

<220>
<223>  JL-2004(H5N1)-M2-45-62 mut54

<400>  73

Arg Leu Phe Phe Lys Cys Ile Tyr Arg Ser Leu Lys Tyr Gly Leu Lys
1               5                   10                  15

Arg Gly

<210>  74
<211>  18
<212>  PRT
<213>  Artificial sequence

<220>
<223>  JL-2004(H5N1)-M2-45-62 mut55

<400>  74

Arg Leu Phe Phe Lys Cys Ile Tyr Arg Arg Ala Lys Tyr Gly Leu Lys
1               5                   10                  15

Arg Gly

<210>  75
<211>  18
<212>  PRT
<213>  Artificial sequence

<220>
<223>  JL-2004(H5N1)-M2-45-62 mut56

<400>  75

Arg Leu Phe Phe Lys Cys Ile Tyr Arg Arg Leu Thr Tyr Gly Leu Lys
1               5                   10                  15

Arg Gly

<210>  76
<211>  18
<212>  PRT
<213>  Artificial sequence

<220>
<223>  JL-2004(H5N1)-M2-45-62 mut57

<400>  76

Arg Leu Phe Phe Lys Cys Ile Tyr Arg Arg Leu Lys Lys Gly Leu Lys
1               5                   10                  15

Arg Gly

<210>  77
<211>  18
<212>  PRT
<213>  Artificial sequence

<220>
<223>  JL-2004(H5N1)-M2-45-62 mut58

<400>  77

Arg Leu Phe Phe Lys Cys Ile Tyr Arg Arg Leu Lys Tyr Lys Leu Lys
1               5                   10                  15

Arg Gly

<210> 78
<211> 18
<212> PRT
<213> Artificial sequence

<220>
<223> JL-2004(H5N1)-M2-45-62 mut59

<400> 78

Arg Leu Phe Phe Lys Cys Ile Tyr Arg Arg Leu Lys Tyr Gly Ala Lys
1               5                   10                  15

Arg Gly

<210> 79
<211> 18
<212> PRT
<213> Artificial sequence

<220>
<223> JL-2004(H5N1)-M2-45-62 mut60

<400> 79

Arg Leu Phe Phe Lys Cys Ile Tyr Arg Arg Leu Lys Tyr Gly Leu Thr
1               5                   10                  15

Arg Gly

<210> 80
<211> 18
<212> PRT
<213> Artificial sequence

<220>
<223> JL-2004(H5N1)-M2-45-62 mut61

<400> 80

Arg Leu Phe Phe Lys Cys Ile Tyr Arg Arg Leu Lys Tyr Gly Leu Lys
1               5                   10                  15

Thr Gly

<210>  81
<211>  18
<212>  PRT
<213>  Artificial sequence

<220>
<223>  JL-2004(H5N1)-M2-45-62 mut62

<400>  81

Arg Leu Phe Phe Lys Cys Ile Tyr Arg Arg Leu Lys Tyr Gly Leu Lys
1               5                   10                  15

Arg Lys

<210>  82
<211>  18
<212>  PRT
<213>  Artificial sequence

<220>
<223>  JL-2004(H5N1)-M2-44-61 mut1

<400>  82

Arg Arg Leu Phe Phe Lys Cys Ile Tyr Arg Arg Leu Lys Tyr Gly Leu
1               5                   10                  15

Lys Arg

<210>  83
<211>  18
<212>  PRT
<213>  Artificial sequence

<220>
<223>  JL-2004(H5N1)-M2-44-61 mut2

<400>  83

Leu Arg Leu Phe Phe Lys Cys Ile Tyr Arg Arg Leu Lys Tyr Gly Leu
1               5                   10                  15

Lys Arg

<210>  84
<211>  18
<212>  PRT

<213>  Artificial sequence

<220>
<223>  JL-2004(H5N1)-M2-44-61 mut3

<400>  84

Asp Ile Leu Phe Phe Lys Cys Ile Tyr Arg Arg Leu Lys Tyr Gly Leu
1               5                   10                  15

Lys Arg

<210>  85
<211>  18
<212>  PRT
<213>  Artificial sequence

<220>
<223>  JL-2004(H5N1)-M2-44-61 mut4

<400>  85

Asp Arg Arg Phe Phe Lys Cys Ile Tyr Arg Arg Leu Lys Tyr Gly Leu
1               5                   10                  15

Lys Arg

<210>  86
<211>  18
<212>  PRT
<213>  Artificial sequence

<220>
<223>  JL-2004(H5N1)-M2-44-61 mut5

<400>  86

Asp Arg Leu Arg Arg Lys Cys Ile Tyr Arg Arg Leu Lys Tyr Gly Leu
1               5                   10                  15

Lys Arg

<210>  87
<211>  18
<212>  PRT
<213>  Artificial sequence

<220>
<223>  JL-2004(H5N1)-M2-44-61 mut6

<400>  87

Asp Arg Leu Phe Phe Ile Cys Ile Tyr Arg Arg Leu Lys Tyr Gly Leu
1               5                   10                  15

Lys Arg

<210>  88
<211>  18
<212>  PRT
<213>  Artificial sequence

<220>
<223>  JL-2004(H5N1)-M2-44-61 mut7

<400>  88

Asp Arg Leu Phe Phe Lys Arg Ile Tyr Arg Arg Leu Lys Tyr Gly Leu
1               5                   10                  15

Lys Arg

<210>  89
<211>  18
<212>  PRT
<213>  Artificial sequence

<220>
<223>  JL-2004(H5N1)-M2-44-61 mut8

<400>  89

Asp Arg Leu Phe Phe Lys Ile Ile Tyr Arg Arg Leu Lys Tyr Gly Leu
1               5                   10                  15

Lys Arg

<210>  90
<211>  18
<212>  PRT
<213>  Artificial sequence

<220>
<223>  JL-2004(H5N1)-M2-44-61 mut9

<400>  90

Asp Arg Leu Phe Phe Lys Cys Lys Lys Arg Arg Leu Lys Tyr Gly Leu

1                   5                   10                  15

Lys Arg

<210>  91
<211>  18
<212>  PRT
<213>  Artificial sequence

<220>
<223>  JL-2004(H5N1)-M2-44-61 mut10

<400>  91

Asp Arg Arg Arg Arg Lys Cys Arg Arg Arg Arg Arg Lys Tyr Gly Arg
1                   5                   10                  15

Lys Arg

<210>  92
<211>  18
<212>  PRT
<213>  Artificial sequence

<220>
<223>  JL-2004(H5N1)-M2-44-61 mut12

<400>  92

Asp Arg Leu Phe Phe Lys Cys Ile Tyr Arg Arg Arg Lys Arg Gly Leu
1                   5                   10                  15

Lys Arg

<210>  93
<211>  18
<212>  PRT
<213>  Artificial sequence

<220>
<223>  JL-2004(H5N1)-M2-44-61 mut13

<400>  93

Asp Arg Leu Phe Phe Lys Cys Ile Tyr Arg Arg Leu Lys Tyr Gly Arg
1                   5                   10                  15

Lys Arg

<210>  94
<211>  18
<212>  PRT
<213>  Artificial sequence

<220>
<223>  JL-2004(H5N1)-M2-44-61 mut14

<400>  94

Asp Arg Lys Arg Arg Lys Cys Lys Tyr Arg Arg Lys Lys Tyr Gly Arg
1               5                   10                  15

Lys Arg

<210>  95
<211>  18
<212>  PRT
<213>  Artificial sequence

<220>
<223>  JL-2004(H5N1)-M2-44-61 mut16

<400>  95

Asp Arg Leu Phe Phe Lys Cys Ile Tyr Arg Arg Leu Lys Tyr Gly Leu
1               5                   10                  15

Ile Ile

<210>  96
<211>  18
<212>  PRT
<213>  Artificial sequence

<220>
<223>  JL-2004(H5N1)-M2-44-61 mut17

<400>  96

Ile Ile Leu Phe Phe Lys Cys Ile Tyr Arg Arg Leu Lys Tyr Gly Leu
1               5                   10                  15

Lys Arg

<210>  97

<211>  22
<212>  PRT
<213>  Artificial sequence

<220>
<223>  JL-2004(H5N1)-M2-44-65 mut1

<400>  97

Asp Arg Phe Phe Phe Lys Cys Ile Tyr Arg Arg Leu Lys Tyr Gly Leu
1               5                   10                  15

Lys Arg Gly Pro Ser Thr
            20

<210>  98
<211>  22
<212>  PRT
<213>  Artificial sequence

<220>
<223>  JL-2004(H5N1)-M2-44-65 mut2

<400>  98

Asp Arg Phe Phe Phe Lys Cys Ile Tyr Arg Arg Leu Lys Tyr Gly Leu
1               5                   10                  15

Lys Arg Arg Pro Ser Thr
            20

<210>  99
<211>  22
<212>  PRT
<213>  Artificial sequence

<220>
<223>  JL-2004(H5N1)-M2-44-65 mut3

<400>  99

Asp Arg Phe Phe Phe Lys Cys Ile Tyr Arg Arg Leu Lys Phe Gly Leu
1               5                   10                  15

Lys Arg Gly Pro Ser Thr
            20

<210>  100
<211>  22
<212>  PRT
<213>  Artificial sequence

<220>
<223>  JL-2004(H5N1)-M2-44-65 mut4

<400>  100

Asp Arg Phe Phe Phe Lys Cys Ile Phe Arg Arg Leu Lys Tyr Gly Leu
1               5                   10                  15

Lys Arg Gly Pro Ser Thr
            20

<210>  101
<211>  22
<212>  PRT
<213>  Artificial sequence

<220>
<223>  JL-2004(H5N1)-M2-44-65 mut5

<400>  101

Asp Arg Phe Phe Phe Lys Cys Ile Phe Arg Arg Leu Lys Phe Gly Leu
1               5                   10                  15

Lys Arg Gly Pro Ser Thr
            20

<210>  102
<211>  22
<212>  PRT
<213>  Artificial sequence

<220>
<223>  JL-2004(H5N1)-M2-44-65 mut6

<400>  102

Asp Arg Phe Phe Phe Lys Cys Phe Phe Arg Arg Phe Lys Phe Gly Leu
1               5                   10                  15

Lys Arg Gly Pro Ser Thr
            20

<210>  103
<211>  22
<212>  PRT
<213>  Artificial sequence

<220>
<223>  JL-2004(H5N1)-M2-44-65 mut7

<400>  103

Arg Arg Phe Phe Phe Lys Cys Phe Phe Arg Arg Phe Lys Phe Gly Leu
1               5                   10                  15

Lys Arg Gly Pro Ser Thr
            20

<210>  104
<211>  22
<212>  PRT
<213>  Artificial sequence

<220>
<223>  JL-2004(H5N1)-M2-44-65 mut8

<400>  104

Asp Arg Phe Phe Phe Lys Arg Ile Tyr Arg Arg Leu Lys Tyr Arg Leu
1               5                   10                  15

Lys Arg Arg Pro Ser Thr
            20

<210>  105
<211>  22
<212>  PRT
<213>  Artificial sequence

<220>
<223>  JL-2004(H5N1)-M2-44-65 mut9

<400>  105

Asp Arg Phe Phe Phe Lys Arg Ile Tyr Arg Arg Leu Lys Tyr Arg Leu
1               5                   10                  15

Lys Arg Arg Pro Lys Val
            20

<210>  106
<211>  22
<212>  PRT
<213>  Artificial sequence

<220>
<223>  JL-2004(H5N1)-M2-44-65 mut10

<400>  106

Asp Arg Phe Phe Phe Lys Arg Ile Phe Arg Arg Leu Lys Tyr Arg Leu
1               5                   10                  15

Lys Arg Arg Pro Ser Thr
            20

<210>  107
<211>  22
<212>  PRT
<213>  Artificial sequence

<220>
<223>  JL-2004(H5N1)-M2-44-65 mut11

<400>  107

Asp Arg Phe Phe Phe Lys Arg Ile Phe Arg Arg Leu Lys Phe Arg Leu
1               5                   10                  15

Lys Arg Arg Pro Ser Thr
            20

<210>  108
<211>  22
<212>  PRT
<213>  Artificial sequence

<220>
<223>  JL-2004(H5N1)-M2-44-65 mut12

<400>  108

Asp Arg Phe Phe Phe Lys Arg Ile Phe Arg Arg Phe Lys Phe Arg Leu
1               5                   10                  15

Lys Arg Arg Pro Ser Thr
            20

<210>  109
<211>  22
<212>  PRT
<213>  Artificial sequence

<220>
<223>  JL-2004(H5N1)-M2-44-65 mut13

<400>  109

Arg Arg Phe Phe Phe Lys Arg Ile Phe Arg Arg Phe Lys Phe Arg Leu
1               5                   10                  15

Lys Arg Arg Pro Ser Thr
            20

<210>  110
<211>  22
<212>  PRT
<213>  Artificial sequence

<220>
<223>  JL-2004(H5N1)-M2-44-65 mut14

<400>  110

Arg Arg Phe Phe Phe Lys Arg Ile Phe Arg Arg Phe Lys Phe Arg Leu
1               5                   10                  15

Lys Arg Arg Pro Lys Val
            20

<210>  111
<211>  22
<212>  PRT
<213>  Artificial sequence

<220>
<223>  JL-2004(H5N1)-M2-44-65 mut15

<400>  111

Arg Arg Leu Phe Phe Lys Cys Ile Tyr Arg Arg Leu Lys Tyr Gly Leu
1               5                   10                  15

Lys Arg Gly Pro Ser Thr
            20

<210>  112
<211>  22
<212>  PRT
<213>  Artificial sequence

<220>
<223>  JL-2004(H5N1)-M2-44-65 mut16

<400>  112

Arg Arg Leu Phe Phe Lys Cys Ile Tyr Arg Arg Leu Lys Tyr Gly Leu
1               5                   10                  15

Lys Arg Gly Pro Lys Val
            20

<210>  113
<211>  22
<212>  PRT

<213>  Artificial sequence

<220>
<223>  JL-2004(H5N1)-M2-44-65 mut17

<400>  113

Arg Arg Phe Phe Phe Lys Cys Ile Tyr Arg Arg Leu Lys Tyr Gly Leu
1               5                   10                  15

Lys Arg Gly Pro Ser Thr
            20

<210>  114
<211>  22
<212>  PRT
<213>  Artificial sequence

<220>
<223>  JL-2004(H5N1)-M2-44-65 mut18

<400>  114

Arg Arg Leu Phe Phe Lys Cys Ile Tyr Arg Arg Leu Lys Tyr Gly Leu
1               5                   10                  15

Lys Arg Arg Pro Ser Thr
            20

<210>  115
<211>  22
<212>  PRT
<213>  Artificial sequence

<220>
<223>  JL-2004(H5N1)-M2-44-65 mut19

<400>  115

Arg Arg Leu Phe Phe Lys Cys Ile Tyr Arg Arg Leu Lys Tyr Gly Leu
1               5                   10                  15

Lys Arg Lys Pro Ser Thr
            20

<210>  116
<211>  22
<212>  PRT
<213>  Artificial sequence

<220>
<223>  JL-2004(H5N1)-M2-44-65 mut20

<400>  116

Arg Arg Leu Phe Phe Arg Arg Ile Tyr Arg Arg Leu Lys Tyr Gly Leu
1               5                   10                  15

Lys Arg Gly Pro Ser Thr
            20

<210>  117
<211>  22
<212>  PRT
<213>  Artificial sequence

<220>
<223>  JL-2004(H5N1)-M2-44-65 mut21

<400>  117

Arg Arg Leu Phe Phe Arg Arg Ile Tyr Arg Arg Leu Arg Tyr Arg Leu
1               5                   10                  15

Arg Arg Arg Pro Ser Thr
            20

<210>  118
<211>  22
<212>  PRT
<213>  Artificial sequence

<220>
<223>  JL-2004(H5N1)-M2-44-65 mut22

<400>  118

Asp Arg Leu Phe Phe Lys Arg Ile Tyr Arg Arg Leu Lys Tyr Gly Leu
1               5                   10                  15

Lys Arg Gly Pro Ser Thr
            20

<210>  119
<211>  22
<212>  PRT
<213>  Artificial sequence

<220>
<223>  JL-2004(H5N1)-M2-44-65 mut23

<400>  119

Asp Arg Leu Phe Phe Arg Arg Ile Tyr Arg Arg Leu Lys Tyr Gly Leu

1               5                   10                  15

Lys Arg Gly Pro Ser Thr
            20

<210>  120
<211>  22
<212>  PRT
<213>  Artificial sequence

<220>
<223>  JL-2004(H5N1)-M2-44-65 mut24

<400>  120

Asp Arg Leu Phe Phe Arg Arg Ile Tyr Arg Arg Leu Arg Tyr Arg Leu
1               5                   10                  15

Arg Arg Arg Pro Ser Thr
            20

<210>  121
<211>  22
<212>  PRT
<213>  Artificial sequence

<220>
<223>  JL-2004(H5N1)-M2-44-65 mut25

<400>  121

Asp Arg Leu Phe Phe Lys Cys Ile Tyr Arg Arg Leu Lys Tyr Gly Leu
1               5                   10                  15

Lys Arg Arg Pro Ser Thr
            20

<210>  122
<211>  22
<212>  PRT
<213>  Artificial sequence

<220>
<223>  JL-2004(H5N1)-M2-44-65 mut26

<400>  122

Asp Arg Leu Phe Phe Lys Cys Ile Tyr Arg Arg Leu Lys Tyr Arg Leu
1               5                   10                  15

Lys Arg Arg Pro Ser Thr

            20

<210>  123
<211>  22
<212>  PRT
<213>  Artificial sequence

<220>
<223>  JL-2004(H5N1)-M2-44-65 mut27

<400>  123

Asp Arg Leu Phe Phe Lys Arg Ile Tyr Arg Arg Leu Lys Tyr Arg Leu
1               5                   10                  15

Lys Arg Arg Pro Ser Thr
            20

<210>  124
<211>  22
<212>  PRT
<213>  Artificial sequence

<220>
<223>  JL-2004(H5N1)-M2-44-65 mut28

<400>  124

Arg Arg Leu Phe Phe Lys Arg Ile Tyr Arg Arg Leu Lys Tyr Arg Leu
1               5                   10                  15

Lys Arg Arg Pro Ser Thr
            20

<210>  125
<211>  720
<212>  DNA
<213>  Artificial sequence

<220>
<223>  Coding sequence of EGFP

<400>  125
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagtaa 720

<210> 126
<211> 225
<212> PRT
<213> Influenza A virus

<400> 126

Met Asp Ser Asn Thr Val Ser Ser Phe Gln Val Asp Cys Phe Leu Trp
1               5                   10                  15

His Val Arg Lys Arg Phe Ala Asp Gln Glu Leu Gly Asp Ala Pro Phe
            20                  25                  30

Leu Asp Arg Leu Arg Arg Asp Gln Lys Ser Leu Arg Gly Arg Gly Asn
        35                  40                  45

Thr Leu Gly Leu Asp Ile Glu Thr Ala Thr Arg Ala Gly Lys Gln Ile
    50                  55                  60

Val Glu Arg Ile Leu Glu Glu Glu Ser Asp Glu Ala Leu Lys Met Pro
65                  70                  75                  80

Ala Ser Arg Tyr Leu Thr Asp Met Thr Leu Glu Glu Met Ser Arg Asp
                85                  90                  95

Trp Phe Met Leu Met Pro Lys Gln Lys Val Ala Gly Ser Leu Cys Ile
            100                 105                 110

Lys Met Asp Gln Ala Ile Met Asp Lys Thr Ile Ile Leu Lys Ala Asn
        115                 120                 125

Phe Ser Val Ile Phe Asp Arg Leu Glu Thr Leu Ile Leu Leu Arg Ala
    130                 135                 140

Phe Thr Glu Glu Gly Ala Ile Val Gly Glu Ile Ser Pro Leu Pro Ser
145                 150                 155                 160

Leu Pro Gly His Thr Gly Glu Asp Val Lys Asn Ala Ile Gly Val Leu
                165                 170                 175

Ile Gly Gly Leu Glu Trp Asn Asp Asn Thr Val Arg Val Ser Glu Thr
            180                 185                 190

Ile Gln Arg Phe Ala Trp Arg Ser Ser Asp Glu Asp Gly Arg Leu Pro
        195                 200                 205

Leu Pro Pro Asn Gln Lys Arg Lys Met Ala Arg Thr Ile Glu Ser Glu
    210                 215                 220

Val
225